# EUROPEAN PATENT APPLICATION

(11) **EP 2 746 266 A1**
(43) Date of publication of application: **25.06.2014**
(21) Application number: 12198168.2
(22) Date of filing: 19.12.2012
(51) Int. Cl.: C07D 303/12, C07D 307/12, C07D 307/16, C07D 405/06, A01N 43/50, A01N 43/653

(54) **New substituted triazoles and imidazoles and their use as fungicides**

(71) Applicant: BASF SE, 67056 Ludwigshafen (DE)
(72) Inventor: Lohmann, Jan Klaas, 67245 Lambsheim (DE)

(57) **Abstract**

The present invention relates to new substituted triazoles and imidazoles compounds of formula I as defined in the description, and the N-oxides, and salts thereof, their preparation and intermediates for preparing them. The invention also relates to the use of these compounds for combating harmful fungi and seed coated with at least one such compound and also to compositions comprising at least one such compound.

## Description

The present invention relates to new substituted triazoles and imidazoles of the formula I

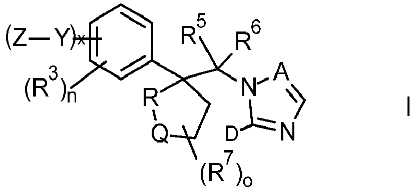

Furthermore the present invention relates to a process for preparing compounds of the formula I.

Furthermore the present invention relates to agrochemical compositions, comprising an auxiliary and at least one compound of formula I an N-oxide or an agriculturally acceptable salt thereof.

Furthermore the present invention relates to the use of a compound of the formula I and/or of an agriculturally acceptable salt thereof or of the compositions for combating phytopathogenic fungi. Furthermore the present invention relates to a method for combating harmful fungi, comprising treating the fungi or the materials, plants, the soil or seeds to be protected against fungal attack with an effective amount of at least one compound of formula I or with a composition. Furthermore the present invention relates to seed, coated with at least one compound of the formula I and/or an agriculturally acceptable salt thereof or with a composition in an amount of from 0.1 to 10 kg per 100 kg of seed.

The preparations of azolylmethyloxirane and their use for controlling phytopathogenic fungi is known from e.g. EP 0443980.

The compounds according to the present invention differ from those described in the above-mentioned publications inter alia by the substitution of the phenyl ring and the epoxide group.

In many cases, in particular at low application rates, the fungicidal activity of the known fungicidal compounds is unsatisfactory. Based on this, it was an object of the present invention to provide compounds having improved activity and/or a broader activity spectrum against phytopathogenic harmful fungi.

Accordingly, it is an object of the present invention to provide compounds having better fungicidal activity and/or better crop plant compatibility.

Surprisingly, these objects are achieved by compounds of the general formula I, as defined below, and by the agriculturally acceptable salts of the compounds of the general formula I. Accordingly, the present invention relates to compounds of formula I 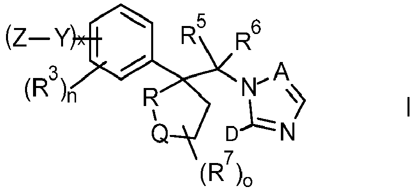 wherein:
R is O or CH₂;
Q is O or CH₂;
wherein either R or Q is O;
A is CH or N;
D is H, halogen or SR^{D}, wherein
   R^{D} is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl or CN;
R³ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R³ is unsubstituted or further substituted by one, two, three or four R^{3a}; wherein R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
   n is 0, 1, 2, 3 or 4;
Y is a direct bond or a divalent group selected from the group consisting of -O-, -S-, SO-, -SO₂-; -NH-, -N(C₁-C₄-alkyl)-, CR¹⁵R⁸-, -CR⁹R¹⁰-CR¹¹R¹²-, -CR¹³=CR¹⁴ and -C≡C-; wherein R⁸,R⁹,R¹⁰,R¹¹,R¹²,R¹³,R¹⁴,R¹⁵ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl, wherein the heteroaryl contains 1, 2, 3 or 4 heteroatoms selected from the group consisting of O, N and S, or phenyl, wherein the heteroaryl or phenyl is unsubstituted (m=0) or substituted by (R⁴)ₘ, wherein
   m is 0, 1, 2, 3, 4 or 5;
   R⁴ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a}; wherein
   R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
   p is 0, 1 or 2;
x is 0, 1, 2, 3 or 4;
R⁵ is H, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl and heteroaryl-C₁-C₄-alkyl, wherein the heteroaryl radical in the last-mentioned groups is 5- or 6-membered and wherein the aliphatic, alicyclic and aromatic moieties of R⁵ are unsubstituted or substituted by one, two, three or four or up to the maximum possible number of R^{5a}; wherein
   R^{5a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R⁶ is H or is selected from the substituents defined for R⁵, wherein the aliphatic, alicyclic and aromatic moieties of R⁶ are unsubstituted or substituted by one, two, three or four or up to the maximum possible number of R^{6a}, wherein R^{6a} is defined as R^{5a};
   or
R⁵ and R⁶ together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S);
   or R⁵ and R⁶ together with the carbon atom to which they are bound (denominated C*) form a group (C*=CR⁵⁵R⁶⁶), wherein
   R⁵⁵ R⁶⁶ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R⁷ is independently selected from H, C₁-C₆-alkyl, C₂-C₆-alkenyl, C₂-C₆-alkynyl, C₃-C₈-cycloalkyl, wherein each of R⁷ is unsubstituted or further substituted by one, two, three or four R^{7a}; wherein
   R^{7a} is independently selected from halogen, OH and C₁-C₆-alkoxy;
   or
   two substituents R⁷: R⁷¹ and R⁷² together with the carbon atom(s) to which they are bound form a saturated three-, four-, five-, six- or seven-membered carbocycle or heterocycle, wherein the heterocycle contains one, two, three or four O atoms;
   o is 0, 1, 2, 3 or 4;
   and the N-oxides and the agricucturally acceptable salts thereof
   with the proviso that
   if R is O, x is 1, R⁵ and R⁶ are H R³ is not halogen.

Furthermore the present invention provides a process for preparing compounds of the formula I. Furthermore the present invention provides an agrochemical composition, comprising an auxiliary and at least one compound of formula I an N-oxide or an agriculturally acceptable salt thereof.

Furthermore compounds of the formula I and/or of an agriculturally acceptable salt thereof or of the compositions can be used for combating phytopathogenic fungi.

Furthermore the present invention provides a method for combating harmful fungi, comprising treating the fungi or the materials, plants, the soil or seeds to be protected against fungal attack with an effective amount of at least one compound of formula I or with a composition. Furthermore the present invention provides seed, coated with at least one compound of the formula I and/or an agriculturally acceptable salt thereof or with a composition in an amount of from 0.1 to 10 kg per 100 kg of seed.

The terms used for organic groups in the definition of the variables are, for example the expression "halogen", collective terms which represent the individual members of these groups of organic units.

The prefix Cₓ-C_{y} denotes the number of possible carbon atoms in the particular case. halogen: fluorine, bromine, chlorine or iodine, especially fluorine, chlorine or bromine; alkyl and the alkyl moieties of composite groups such as, for example, alkoxy, alkylamino, alkoxycarbonyl: saturated straight-chain or branched hydrocarbon radicals having 1 to 10 carbon atoms, for example C₁-C₁₀-akyl, such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl; heptyl, octyl, 2-ethylhexyl and positional isomers thereof; nonyl, decyl and positional isomers thereof;
haloalkyl: straight-chain or branched alkyl groups having 1 to 10 carbon atoms (as mentioned above), where some or all of the hydrogen atoms in these groups are replaced by halogen atoms as mentioned above. In one embodiment, the alkyl groups are substituted at least once or completely by a particular halogen atom, preferably fluorine, chlorine or bromine. In a further embodiment, the alkyl groups are partially or fully halogenated by different halogen atoms; in the case of mixed halogen substitutions, the combination of chlorine and fluorine is preferred. Particular preference is given to (C₁-C₃)-haloalkyl, more preferably (C₁-C₂)-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl or 1,1,1-trifluoroprop-2-yl;
alkenyl and also the alkenyl moieties in composite groups, such as alkenyloxy: unsaturated straight-chain or branched hydrocarbon radicals having 2 to 10 carbon atoms and one double bond in any position. According to the invention, it may be preferred to use small alkenyl groups, such as (C₂-C₄)-alkenyl; on the other hand, it may also be preferred to employ larger alkenyl groups, such as (C₅-C₈)-alkenyl. Examples of alkenyl groups are, for example, C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
alkynyl and the alkynyl moieties in composite groups: straight-chain or branched hydrocarbon groups having 2 to 10 carbon atoms and one or two triple bonds in any position, for example C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
cycloalkyl and also the cycloalkyl moieties in composite groups: mono- or bicyclic saturated hydrocarbon groups having 3 to 10, in particular 3 to 6, carbon ring members, for example C₃-C₆-cycloalkyl, such as cyclopropyl, cyclobutyl, cyclopentyl, cyclohexyl, cycloheptyl or cyclooctyl. Examples of bicyclic radicals comprise bicyclo[2.2.1]heptyl, bicyclo[3.1.1]heptyl, bicyclo[2.2.2]octyl and bicyclo[3.2.1]octyl. In this connection, optionally substituted C₃-C₈-cycloalkyl means a cycloalkyl radical having from 3 to 8 carbon atoms, in which at least one hydrogen atom, for example 1, 2, 3, 4 or 5 hydrogen atoms, is/are replaced by substituents which are inert under the conditions of the reaction. Examples of inert substituents are CN, C₁-C₆-alkyl, C₁-C₄-haloalkyl, C₁-C₆-alkoxy, C₃-C₆-cycloalkyl, and C₁-C₄-alkoxy-C₁-C₆-alkyl;
halocycloalkyl and the halocycloalkyl moieties in halocycloalkoxy, halocycloalkylcarbonyl and the like: monocyclic saturated hydrocarbon groups having 3 to 10 carbon ring members (as mentioned above) in which some or all of the hydrogen atoms may be replaced by halogen atoms as mentioned above, in particular fluorine, chlorine and bromine;
alkoxy: an alkyl group as defined above which is attached via an oxygen, preferably having 1 to 10, more preferably 2 to 6, carbon atoms. Examples are: methoxy, ethoxy, n-propoxy, 1-methyl-ethoxy, butoxy, 1-methylpropoxy, 2-methylpropoxy or 1,1-dimethylethoxy, and also for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy; halogenalkoxy: alkoxy as defined above, where some or all of the hydrogen atoms in these groups are replaced by halogen atoms as described above under haloalkyl, in particular by fluorine, chlorine or bromine. Examples are OCH₂F, OCHF₂, OCF₃, OCH₂Cl, OCHCl₂, OCCl₃, chloro-fluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy; and also 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy or dodecafluorohexoxy.

Depending on the substitution pattern, the compounds according to the invention may have one or more centers of chirality, and are generally obtained in the form of racemates or as diastereomer compositions of erythro and threo forms. The erythro and threo diastereomers of the compounds according to the invention can be separated and isolated in pure form, for example, on the basis of their different solubilities or by column chromatography. Using known methods, such uniform pairs of diastereomers can be used to obtain uniform enantiomers. Suitable for use as antimicrobial agents are both the uniform diastereomers or enantiomers and compositions thereof obtained in the synthesis. This applies correspondingly to the fungicidal compositions. Accordingly, the invention provides both the pure enantiomers or diastereomers and compositions thereof. This applies to the compounds according to the invention and, if appropriate, correspondingly to their precursors. The scope of the present invention includes in particular the (R) and (S) isomers and the racemates of the compounds according to the invention, in particular of the formula I, which have centers of chirality. Suitable compounds of the formula I according to the invention also comprise all possible stereoisomers (cis/trans isomers) and compositions thereof. The compounds according to the invention may be present in various crystal modifications which may differ in their biological activity. They are likewise provided by the present invention.

Owing to the basic character of their heteroatoms, the compounds according to the invention are capable of forming salts or adducts with inorganic or organic acids or with metal ions.

Suitable agriculturally useful salts are especially the salts of those cations or the acid addition salts of those acids whose cations and anions, respectively, have no adverse effect on the fungicidal action of the compounds of the formula I. Thus, suitable cations are in particular the ions of the alkali metals, preferably sodium and potassium, of the alkaline earth metals, preferably calcium, magnesium and barium, and of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and also the anions of C₁-C₄-alkanoic acids, preferably formate, acetate, propionate and butyrate. They can be formed by reacting with an acid of the corresponding anion, preferably hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer compositions. Both, the pure enantiomers or diastereomers and their compositions are subject matter of the present invention.

The compounds of the formula I according to the invention can be prepared by different routes analogously to processes known per se of the prior art (see, for example, the prior art cited at the outset).

The compounds of the formula I in which R is O and Q is CH₂ can be prepared as follows:

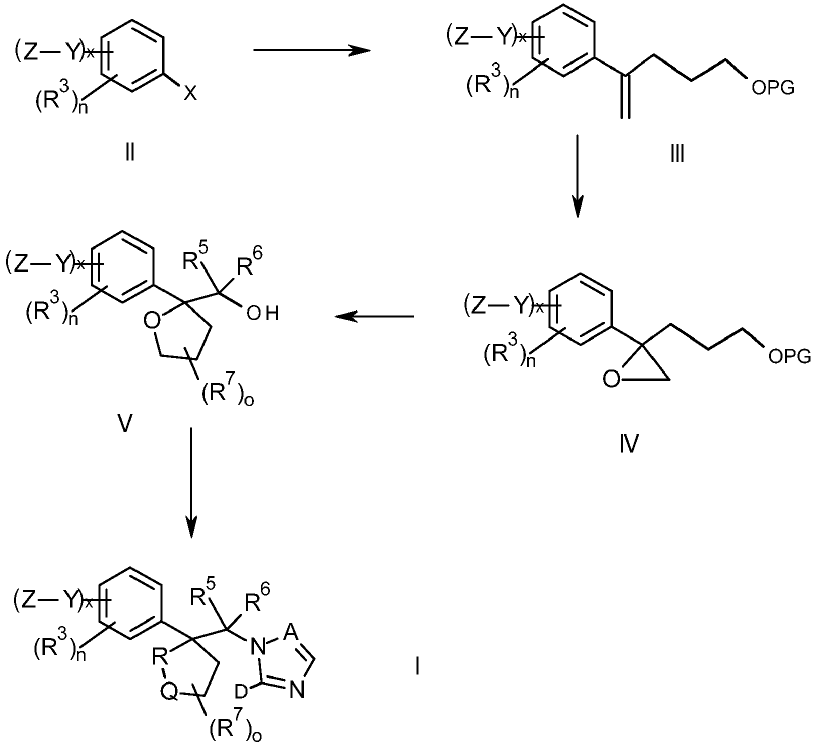

Analogs of compounds IV can be prepared starting from accessible compounds II in cross-coupling / epoxidation / cyclization sequence (Journal of the American Chemical Society, 132(44), 15474-15476; 2010; Journal of the American Chemical Society, 132(23), 7878-7880; 2010; Journal of Organic Chemistry, 69(24), 8387-8393; 2004). Activation by sulfonation or halogenation leads to azole compounds I.

The compounds of the formula I in which R is CH₂ and Q is H can be prepared as follows:

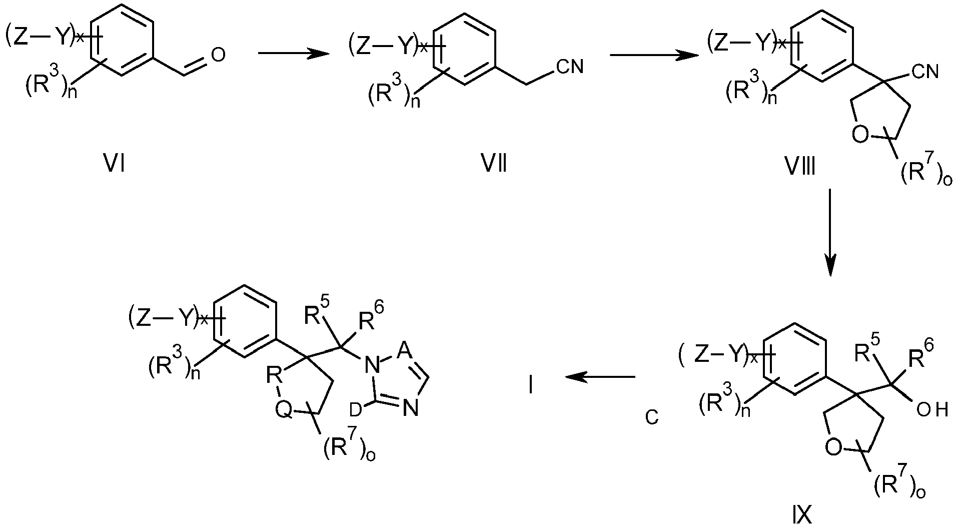

Aldehydes VI are known in literature or can be prepared in analogy to prior art. Reduction followed by activation (sulfonation, halogenation) enables benzyl cyanides VII, which can be transformed into THF-derivative VIII in analogy to W02008076427A2. Reduction of the cyanide either with Hydride equivalents (eg. R5 not H DIBAH, Red-Al, H2, NaBH4) or with metal organic reagents (R5 not H; using R-M such as MeLi, MeMgX, R-Mgx, R-Li) gives hydroxyl compounds IX.

Activation of the hydroxyl group (sulfonation, halogenation) followed by substitution of with an azole compound (triazole, imidazole) leads to compounds I.

If individual inventive compounds cannot be directly obtained by the routes described above, they can be prepared by derivatization of other inventive compounds.

The N-oxides may be prepared from the inventive compounds according to conventional oxidation methods, e. g. by treating compounds I with an organic peracid such as metachloroper-benzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a composition of different N-oxides, which can be separated by conventional methods such as chromatography.

If the synthesis yields compositions of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the following, the intermediate compounds are further described. A skilled person will readily understand that the preferences for the substituents given herein in connection with compounds I apply for the intermediates accordingly. Thereby, the substituents in each case have independently of each other or more preferably in combination the meanings as defined herein.

A further embodiment of the present invention is compounds of formulae III (see above), wherein the variables Z, Y, R³, n are as defined and preferably defined for formula I herein and PG is preferably THP, MOM, TMS, TES, TBDMS, TIPS, TBDPS. In specific embodiments of compounds II according to the present invention, variables Z, Y, R³, n are as defined in tables 1 a-I to 1a-18, 1a-II to 18a-II, 1a-III to 9a-III, 1a-IV to 9a-IV for compounds I, wherein the substituents are specific embodiments independently of each other or in any combination.

A further embodiment of the present invention is compounds of formulae IV (see above), wherein the variables Z, Y, R³, n are as defined and preferably defined for formula I herein and PG is preferably THP, MOM, TMS, TES, TBDMS, TIPS, TBDPS. In specific embodiments of compounds IV according to the present invention, variables Z, Y, R³, n are as defined in tables 1a-I to 1a-18, 1a-II to 18a-II, 1a-III to 9a-III, 1a-IV to 9a-IV for compounds I, wherein the substituents are specific embodiments independently of each other or in any combination.

A further embodiment of the present invention is compounds of formulae V (see above), wherein the variables Z, Y, R³, R⁵, R⁶, R⁷, o, n, x are as defined and preferably defined for formula I herein. In specific embodiments of compounds V according to the present invention, variables , Z, Y, R³, R⁵, R⁶, R⁷, o, n, x are as defined in tables 1a-I to 1a-18, 1a-II to 18a-II 1a-III to 9a-III, 1a-IV to 9a-IV for compounds I, wherein the substituents are specific embodiments independently of each other or in any combination.

A further embodiment of the present invention is compounds of formula VI (see above), wherein variables Z, Y, R³, n are as defined and preferably defined for formula I herein. In specific embodiments of compounds VI according to the present invention, the variables Z, Y, R³ n are as defined in tables 1a-I to 1a-18, 1a-I to 18a-I 1a-III to 9a-III, 1a-IV to 9a-IV for compounds I, wherein the substituents are specific embodiments independently of each other or in any combination.

A further embodiment of the present invention is compounds of formula VII (see above), wherein variables Z, Y, R³, n are as defined and preferably defined for formula I herein. In specific embodiments of compounds VII according to the present invention, the variables Z, Y, R³ n are as defined in tables 1a-I to 1a-18, 1a-II to 18a-II 1a-III to 9a-III 1a-IV to 9a-IV for compounds I, wherein the substituents are specific embodiments independently of each other or in any combination.

A further embodiment of the present invention is compounds of formula VIII (see above), wherein variables Z, Y, R³, R⁷, n, o are as defined and preferably defined for formula I herein. In specific embodiments of compounds VIII according to the present invention, the variables Z, Y, R³, R⁷, n, o are as defined in tables 1a-I to 1a-18, 1a-II to 18a-II 1a-III to 9a-III 1a-IV to 9a-IV for compounds I, wherein the substituents are specific embodiments independently of each other or in any combination.

A further embodiment of the present invention is compounds of formula IX, wherein the variables Z, Y, R³, R⁵, R⁶, R⁷, o, n, x are as defined and preferably defined for formula I herein. In specific embodiments of compounds IX according to the present invention, the variables Z, Y, R³, R⁵, R⁶,R⁷, o, n, x are as defined in tables in tables 1a-I to 1a-18, 1a-II to 18a-II, 1a-II to 9a-III, 1a-IV to 9a-IV for compounds I, wherein the substituents are specific embodiments independently of each other or in any combination.

In the compounds according to the invention I, particular preference is given to the following meanings of the substituents, in each case on their own or in combination.

A in the compounds according to the invention is, according to one embodiment, CH.

A in the compounds according to the invention is, according to one further embodiment, N. According to one embodiment of the invention, Q is O, R is CH₂.

According to one embodiment of the invention, R is O, Q is CH₂.

D in the compounds according to the invention is as defined in claim 1.

According to a further embodiment of the invention, D is selected from H, halogen, SH or S-C₁-C₆-alkyl.

According to one embodiment D is H. According to one further embodiment D is SH. According to one further embodiment D is S-CN.

According to one further embodiment D is halogen. In a special embodiment D is I. In a further special embodiment D is Br. In a further special embodiment D is Cl.

According to one further embodiment D is S-C₁-C₆-alkyl preferably S-methyl, S-ethyl, S-n-propyl, S-i-propyl, S-n-butyl, S-i-butyl or S-t-butyl. In a special embodiment D is S-methyl. In a further special embodiment D is S-ethyl. In a further special embodiment D is S-n-propyl. In a further special embodiment D is S-t-butyl.

According to one further embodiment D is S-C₁-C₆-haloalkyl wherein C₁-C₆-haloalkyl is preferably fully or partially halogenated methyl, ethyl, n-propyl, i-propyl, n-butyl, i-butyl or t-butyl. In a special embodiment D is S-CF₃. In a further special embodiment D is S-CHF₂. In a further special embodiment D is S-CFH₂. In a further special embodiment D is S-CCl₃. In a further special embodiment D is S-CHCl₂. In a further special embodiment D is S-CClH₂. According to one another embodiment D is S-C₂-C₆-alkenyl, wherein C₂-C₆-alkenyl is preferably CH=CH₂, CH₂CH=CH₂, CH=CHCH₃ or C(CH₃)=CH₂. According to one another embodiment D is S-C₂-C₆-haloalkenyl, preferably fully or partially halogenated CH=CH₂, CH₂CH=CH₂, CH=CHCH₃ or C(CH₃)=CH₂. According to one another embodiment D is S-C₂-C₆-alkynyl, wherein C₂-C₆-alkynyl is preferably CCH, CH₂CCH, CH₂CCCH₃. In a special embodiment D is S-CCH. In a further special embodiment D is S-CCCH₃. In a further special embodiment D is S-CCCH(CH₃)₂. In a further special embodiment D is S-CCC(CH₃)₃. According to a specific embodiment D is C₂-C₆-haloalkynyl, more preferably fully or partially halogenated C₂-C₄-alkynyl. In a special embodiment D is fully or partially halogenated C₂-alkynyl.

R³ in the compounds according to the invention is, according to one embodiment, as defined in claim 1.

According to one embodiment n is 0. According to one further embodiment n is 1. According to one further embodiment n is 2. According to one further embodiment n is 3. According to one further embodiment n is 4.

R³ in the compounds according to the invention is, according to a further embodiment, halogen, CN, NO₂, C₁-C₆-alkyl, C₁-C₆-haloalkyl, C₁-C₆-alkoxy, S(O)ₚ(C₁-C₄-alkyl), wherein R³ is unsubstituted or further substituted by one, two, three or four R^{3a}; wherein R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy; p is an integrer and is 0, 1, 2.

According to one embodiment R³ is halogen. According to a specific embodiment R³ is Cl. According to a further specific embodiment R³ is F. According to a further specific embodiment R³ is Br. According to one further embodiment R³ is CN. According to one further embodiment R³ is NO₂. According to one further embodiment R³ is OH. According to one further embodiment R³ is SH.

According to one further embodiment R³ is C₁-C₆-alkyl, preferably methyl, ethyl, n-propyl, i-propyl, n-butyl, i-butyl or t-butyl. In a special embodiment R³ is methyl. In a further special embodiment R³ is ethyl. In a further special embodiment R³ is n-propyl. In a further special embodiment R³ is i-propyl. In a further special embodiment R³ is 1-methylpropyl. In a further special embodiment R³ is n-butyl. In a further special embodiment R³ is i-butyl. In a further special embodiment R³ is t-butyl.

According to a one preferred embodiment R³ is C₁-C₆-alkyl substituted by one, two, three or up to the maximum possible number of identical or different groups R^{a} as defined and preferably herein. According to a specific embodiment R³ is C₁-C₆-haloalkyl, more preferably fully or partially halogenated methyl, ethyl, n-propyl, i-propyl, n-butyl, i-butyl or t-butyl. In a special embodiment R³ is CF₃. In a further special embodiment R³ is CHF₂. In a further special embodiment R³ is CFH₂. In a further special embodiment R³ is CCl₃. In a further special embodiment R³ is CHCl₂. In a further special embodiment R³ is CClH₂. According to a further specific embodiment R³ is C₁-C₆-alkyl, preferably C₁-C₄-alkyl substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R³ is CH₂OH. According to a further specific embodiment R³ is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH₂CH₂CH₂CN. In a special embodiment R³ is CH₂CH₂CN. In a further special embodiment R³ is CH(CH₃)CN. According to a further specific embodiment R³ is C₁-C₄-alkoxy-C₁-C₆-alkyl, more preferably C₁-C₄-alkoxy-C₁-C₄-alkyl. In a special embodiment R³ is CH₂OCH₃. In a further special embodiment R³ is CH₂CH₂OCH₃. In a further special embodiment R³ is CH(CH₃)OCH₃. In a further special embodiment R³ is CH(CH₃)OCH₂CH₃. In a further special embodiment R³ is CH₂CH₂OCH₂CH₃. According to a further specific embodiment R³ is C₁-C₄-haloalkoxy-C₁-C₆-alkyl, more preferably C₁-C₄-alkoxy-C₁-C₄-alkyl. In a special embodiment R³ is CH₂OCF₃. In a further special embodiment R³ is CH₂CH₂OCF₃. In a further special embodiment R³ is CH₂OCCl₃. In a further special embodiment R³ is CH₂CH₂OCCl₃.

According to one another embodiment R³ is C₁-C₆-alkoxy, preferably C₁-C₄-alkoxy. In a special embodiment of the invention R³ is OCH₃. In a further special embodiment of the invention R³ is OCH₂CH₃. In a further special embodiment of the invention R³ is OCH(CH₃)₂. In a further special embodiment of the invention R³ is OCH₂CH₂CH₃. In a further special embodiment of the invention R³ is OC(CH₃)₃.

According to one another embodiment R³ is C₂-C₆-alkenyl, preferably CH=CH₂, CH₂CH=CH₂, CH=CHCH₃ or C(CH₃)=CH₂. In a special embodiment R³ is CH=CH₂. In a further special embodiment R³ is CH₂CH=CH₂. In a further special embodiment R³ is CH₂CH=CHCH₃. In a further special embodiment R³ is CH=CHCH₃ In a further special embodiment R³ is CH₂C(CH₃)=CH₂. In a further special embodiment R³ is C(CH₃)=CH₂. In a further special embodiment R³ is C(CH₃)=C(CH₃)H. In a further special embodiment R³ is C(CH₃)=C(CH₃)₂. In a further special embodiment R³ is CH=C(CH₃)₂.

According to a further preferred embodiment R³ is C₂-C₆-alkenyl, substituted by one, two, three or up to the maximum possible number of identical or different groups R^{a} as defined and preferably herein.

According to a specific embodiment R³ is C₂-C₆-haloalkenyl, more preferably fully or partially halogenated C₂-C₆-alkenyl. In a special embodiment R³ is fully or partially halogenated C₂-alkenyl. In a further special embodiment R³ is CH=CF₂. In a further special embodiment R³ is C=CHF. In a further special embodiment R³ is CF=CF₂. In a further special embodiment R³ is CF=CHF. In a further special embodiment R³ is CF=CH₂. In a further special embodiment R³ is CH=CCl₂. . In a further special embodiment R³ is C=CHCl. In a further special embodiment R³ is CCl=CCl₂. In a further special embodiment R³ is CCl=CHCl. In a further special embodiment R³ is CCl=CH₂. In a further special embodiment R³ is fully or partially halogenated C₃-alkenyl. According to a further specific embodiment R³ is C₂-C₆-alkenyl, preferably C₂-C₄-alkenyl, substituted by OH, more preferably, CH=CHCH₂OH, , CH=C(CH₃)OH. In a further special embodiment R³ is CH=CHCH₂OH. According to a further specific embodiment R³ is C₁-C₄-alkoxy-C₂-C₆-alkenyl, more preferably C₁-C₄-alkoxy-C₂-C₄-alkenyl. In a special embodiment R³ is CH=CHOCH₃. In a further special embodiment R³ is CH=CHCH₂OCH₃. According to a further specific embodiment R³ is C₁-C₄-haloalkoxy-C₂-C₆-alkenyl, more preferably C₁-C₄-haloalkoxy-C₂-C₄-alkenyl. In a special embodiment R³ is CH=CHOCF₃. In a further special embodiment R³ is CH=CHCH₂OCF₃. In a further special embodiment R³ is CH=CHOCCl₃. In a further special embodiment R³ is CH=CHCH₂OCCl₃. According to a further specific embodiment R³ is C₃-C₈-cycloalkyl-C₂-C₆-alkenyl, preferably C₃-C₆-cycloalkyl-C₂-C₄-alkenyl. According to a further specific embodiment R³ is C₃-C₆-halocycloalkyl-C₂-C₄-alkenyl, preferably C₃-C₈-halocycloalkyl-C₂-C₆-alkenyl.

According to one another embodiment R³ is C₂-C₆-alkynyl, preferably CCH, CH₂CCH, CH₂CCCH₃. In a special embodiment R³ is CCH. in a further special embodiment R³ is CCCH₃. In a further special embodiment R³ is CH₂CCH. In a further special embodiment R³ is CH₂CCCH₃. In a further special embodiment R³ is CH₂CCH₂CH₃.

According to a further preferred embodiment R³ is C₂-C₆-alkynyl, substituted by one, two, three or up to the maximum possible number of identical or different groups R^{a} as defined and preferably herein. In a further special embodiment R³ is CC-CH₃.

According to a specific embodiment R³ is C₂-C₆-haloalkynyl, more preferably fully or partially halogenated C₂-C₆-alkynyl. In a special embodiment R³ is CC-Cl. In a special embodiment R³ is CC-Br. In a special embodiment R³ is CC-I.In a special embodiment R³ is fully or partially halogenated C₂-alkynyl. In a further special embodiment R³ is fully or partially halogenated C₃-alkynyl. According to a further specific embodiment R³ is C₂-C₆-alkynyl, preferably C₂-C₄-alkynyl, substituted by OH, more preferably, CCOHln a special embodiment R³ is In a further special embodiment R³. According to a further specific embodiment R³ is C₁-C₄-alkoxy-C₂-C₆-alkynyl, more preferably C₁-C₄-alkoxy-C₂-C₄-alkynyl. In a special embodiment R³ is CCOCH₃. In a further special embodiment R³ is CH₂CCOCH₃. According to a further specific embodiment R³ is C₁-C₄-haloalkoxy-C₂-C₆-alkynyl, more preferably C₁-C₄-haloalkoxy-C₂-C₄-alkynyl. In a special embodiment R³ is CCOCF₃. In a further special embodiment R³ is CH₂CCOCF₃. In a further special embodiment R³ is CCOCCl₃. In a further special embodiment R³ is CH₂CCOCCl₃. According to a further specific embodiment R³ is C₃-C₈-cycloalkyl-C₂-C₆-alkynyl, preferably C₃-C₆-cycloalkyl-C₂-C₄-alkynyl. According to a further specific embodiment R³ is C₃-C₆-halocycloalkyl-C₂-C₄-alkynyl, preferably C₃-Ca-halocycloalkyl-C₂-C₆-alkynyl.

According to one another embodiment R³ is C₃-C₈-cycloalkyl, preferably cyclopropyl, cyclobutyl, cyclopentyl or cyclohexyl, in particular cyclopropyl or cyclobutyl. In a special embodiment R³ is cyclopropyl. In a further special embodiment R³ is cyclobutyl. In a further special embodiment R³ is cyclopentyl. In a further special embodiment R³ is cyclohexyl.

According to one another embodiment R³ is C₃-C₈-cycloalkoxy, preferably C₃-C₆-cycloalkoxy. According to a further preferred embodiment R³ is C₃-C₈-cycloalkyl, substituted by one, two, three or up to the maximum possible number of identical or different groups R^{a} as defined and preferably herein.

According to a specific embodiment R³ is C₃-C₈-halocycloalkyl, more preferably fully or partially halogenated C₃-C₆-cycloalkyl. In a special embodiment R³ is fully or partially halogenated cyclopropyl. In a further special embodiment R³ is 1-Cl-cyclopropyl. In a further special embodiment R³ is 2-Cl-cyclopropyl. In a further special embodiment R³ is 1-F-cyclopropyl. In a further special embodiment R³ is 2-F-cyclopropyl. In a further special embodiment R³ is fully or partially halogenated cyclobutyl. In a further special embodiment R³ is 1-Cl-cyclobutyl. In a further special embodiment R³ is 1-F-cyclobutyl. According to a specific embodiment R³ is C₃-C₈-cycloalkyl substituted by C₁-C₄-alkyl, more preferably is C₃-C₆-cycloalkyl substituted by C₁-C₄-alkyl. In a special embodiment R³ is 1-CH₃-cyclopropyl. According to a specific embodiment R³ is C₃-C₈-cycloalkyl substituted by CN, more preferably is C₃-C₆-cycloalkyl substituted by CN. In a special embodiment R³ is 1-CN-cyclopropyl.According to a further specific embodiment R³ is C₃-C₈-cycloalkyl-C₃-C₈-cycloalkyl, preferably C₃-C₆-cycloalkyl-C₃-C₆-cycloalkyl. In a special embodiment R³ is cyclopropyl-cyclopropyl. According to a further specific embodiment R³ is C₃-C₈-cycloalkyl-C₃-C₈-halocycloalkyl, preferably C₃-C₆-cycloal kyl-C₃-C₆-halocycloalkyl.

According to one another embodiment R³ is C₃-C₈-cydoalkyl-C₁-C₄-alkyl , preferably C₃-C₆-cycloalkyl-C₁-C₄-alkyl. In a special embodiment R³ is CH(CH₃)(cyclopropyl). In a special embodiment R³ is CH₂-(cyclopropyl).

According to a further preferred embodiment R³ is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R³ is C₃-C₈-cycloalkyl-C₁-C₄-haloalkyl, C₃-C₆-cycloalkyl-C₁-C₄-haloalkyl. According to a specific embodiment R³ is C₃-C₈-halocycloalkyl-C₁-C₄-alkyl, C₃-C₆-halocydoalkyl-C₁-C₄-alkyl. In a special embodiment R³ is fully or partially halogenated cyclopropyl-C₁-C₄-alkyl. In a further special embodiment R³ is 1-Cl-cyclopropyl-C₁-C₄-alkyl. In a further special embodiment R³ is 1-F-cyclopropyl-C₁-C₄-alkyl.

According to one another embodiment R³ is NH₂.

According to one another embodiment R³ is NH(C₁-C₄-alkyl). According to a specific embodiment R³ is NH(CH₃). According to a specific embodiment R³ is NH(CH₂CH₃). According to a specific embodiment R³ is NH(CH₂CH₂CH₃). According to a specific embodiment R³ is NH(CH(CH₃)₂). According to a specific embodiment R³ is NH(CH₂CH₂CH₂CHₛ). According to a specific embodiment R³ is NH(C(CH₃)₃).

According to one another embodiment R³ is N(C₁-C₄-alkyl)₂. According to a specific embodiment R³ is N(CH₃)₂. According to a specific embodiment R³ is N(CH₂CH₃)₂. According to a specific embodiment R³ is N(CH₂CH₂CH₃)₂. According to a specific embodiment R³ is N(CH(CH₃)₂)₂. According to a specific embodiment R³ is N(CH₂CH₂CH₂CH₃)₂. According to a specific embodiment R³ is NH(C(CH₃)₃)₂.

According to one another embodiment R³ is NH(C₃-C₈-cycloalkyl) preferably NH(C₃-C₆-cycloalkyl). According to a specific embodiment R³ is NH(cyclopropyl). According to a specific embodiment R³ is NH(cyclobutyl). According to a specific embodiment R³ is NH(cyclopentyl). According to a specific embodiment R³ is NH(cyclohexyl).

According to one another embodiment R³ is N(C₃-C₈-cycloalkyl)₂ preferably N(C₃-C₆-cycloalkyl)₂. According to a specific embodiment R³ is N(cyclopropyl)₂. According to a specific embodiment R³ is N(cyclobutyl)₂. According to a specific embodiment R³ is N(cyclopentyl)₂. According to a specific embodiment R³ is N(cyclohexyl)₂.

According to one another embodiment R³ is S(O)ₚ(C₁-C₄-alkyl) wherein p is 0, 1, 2, preferably S(O)ₚ(C₁-C₄-alkyl) wherein p is 2. According to a specific embodiment R³ is SO₂CH₃. According to a specific embodiment R³ is SO₂CF₃.

According to one another embodiment R³ is C(=O)(-C₁-C₄-alkyl). According to a specific embodiment R³ is C(=O)CH_{3.}. According to a further specific embodiment R³ is C(=O)CH₂CH_{3.} According to a further specific embodiment R³ is C(=O)CH₂CH₂CH_{3.} According to a further specific embodiment R³ is C(=O)CH(CH₃)_{2.} According to a further specific embodiment R³ is C(=O)C(CH₃)_{3.} According to one another embodiment R³ is C(=O)OH.

According to one another embodiment R³ is C(=O)(-O-C₁-C₄-alkyl). According to a specific embodiment R³ is C(=O)OCH_{3.}. According to a further specific embodiment R³ is C(=O)OCH₂CH_{3.} According to a further specific embodiment R³ is C(=O)OCH₂CH₂CH_{3.} According to a further specific embodiment R³ is C(=O)OCH(CH₃)_{2.} According to a further specific embodiment R³ is C(=O)OC(CH₃)_{3.}

According to one another embodiment R³ is C(=O)-NH(C₁-C₄-alkyl). According to a specific embodiment R³ is C(=O)NHCH_{3.}. According to a further specific embodiment R³ is C(=O)NHCH₂CH₃. According to a further specific embodiment R³ is C(=O)NHCH₂CH₂CH_{3.} According to a further specific embodiment R³ is C(=O)NHCH(CH₃)_{2.} According to a further specific embodiment R³ is C(=O)NHC(CH₃)_{3.}

According to one another embodiment R³ is C(=O)-N(C₁-C₄-alkyl)₂. According to a specific embodiment R³ is C(=O)N(CH₃)₂. According to a further specific embodiment R³ is C(=O)N(CH₂CH₃)₂. According to a further specific embodiment R³ is C(=O)N(CH₂CH₂CH₃)_{2.} According to a further specific embodiment R³ is C(=O)N(CH(CH₃)₂)₂. According to a further specific embodiment R³ is C(=O)N(C(CH₃)₃)₂.

According to one another embodiment R³ is C(=O)-NH(C₃-C₆-cycloalkyl). According to a specific embodiment R³ is C(=O)NH(cyclopropyl).. According to a further specific embodiment R³ is C(=O)NH(cydobutyl). According to a further specific embodiment R³ is C(=O)NH(cyclopentyl)_{.} According to a further specific embodiment R³ is C(=O)NH(cyclohexyl).

According to one another embodiment R³ is C(=O)-N(C₃-C₆-cycloalkyl)₂. According to a specific embodiment R³ is C(=O)N(cyclopropyl)₂. According to a further specific embodiment R³ is C(=O)N(cyclobutyl)₂. According to a further specific embodiment R³ is C(=O)N(cyclopentyl)₂. According to a further specific embodiment R³ is C(=O)N(cyclohexyl)₂.

According to one specific embodiment thereof, said R² is in the 2-positon of the phenyl ring. According to one specific embodiment thereof, said R³ is in the 3-positon of the phenyl ring. According to one further specific embodiment thereof, said R³ is in the 4-positon of the phenyl ring. According to one specific embodiment thereof, said R³ is in the 2,3-positon of the phenyl ring. According to one specific embodiment thereof, said R³ is in the 2,4-positon of the phenyl ring. According to one specific embodiment thereof, said R³ is in the 2,5-positon of the phenyl ring. According to one specific embodiment thereof, said R³ is in the 2,6-positon of the phenyl ring. According to one specific embodiment thereof, said R³ is in the 3,4-positon of the phenyl ring. According to one specific embodiment thereof, said R³ is in the 3,5-positon of the phenyl ring. According to one specific embodiment thereof, said R³ is in the 3,6-positon of the phenyl ring. According to one specific embodiment thereof, said R³ is in the 2,4,6-positon of the phenyl ring.

Particularly preferred embodiments of (R³)ₙ according to the invention are in Table P below, wherein each line of lines P1 to P155 corresponds to one particular embodiment of the invention, wherein P1 to P155 are also in any combination a preferred embodiment of the present invention.

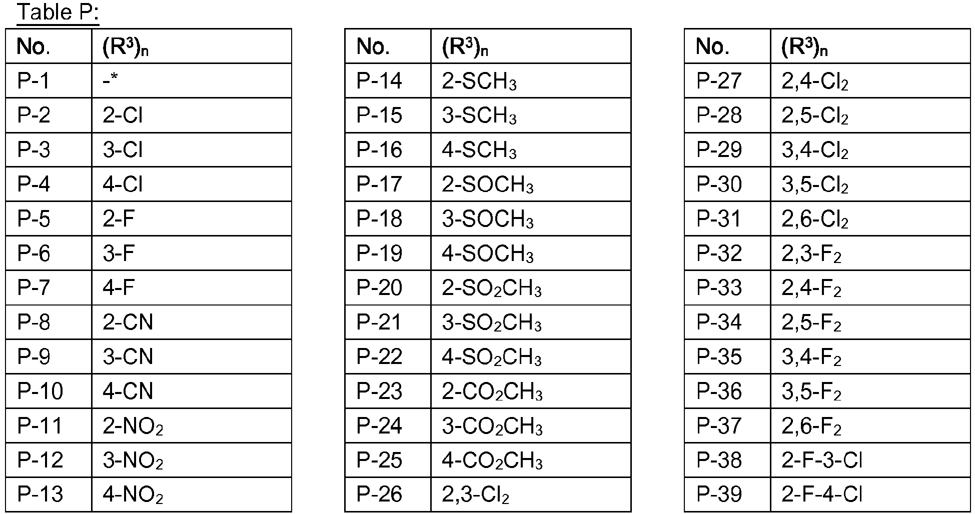

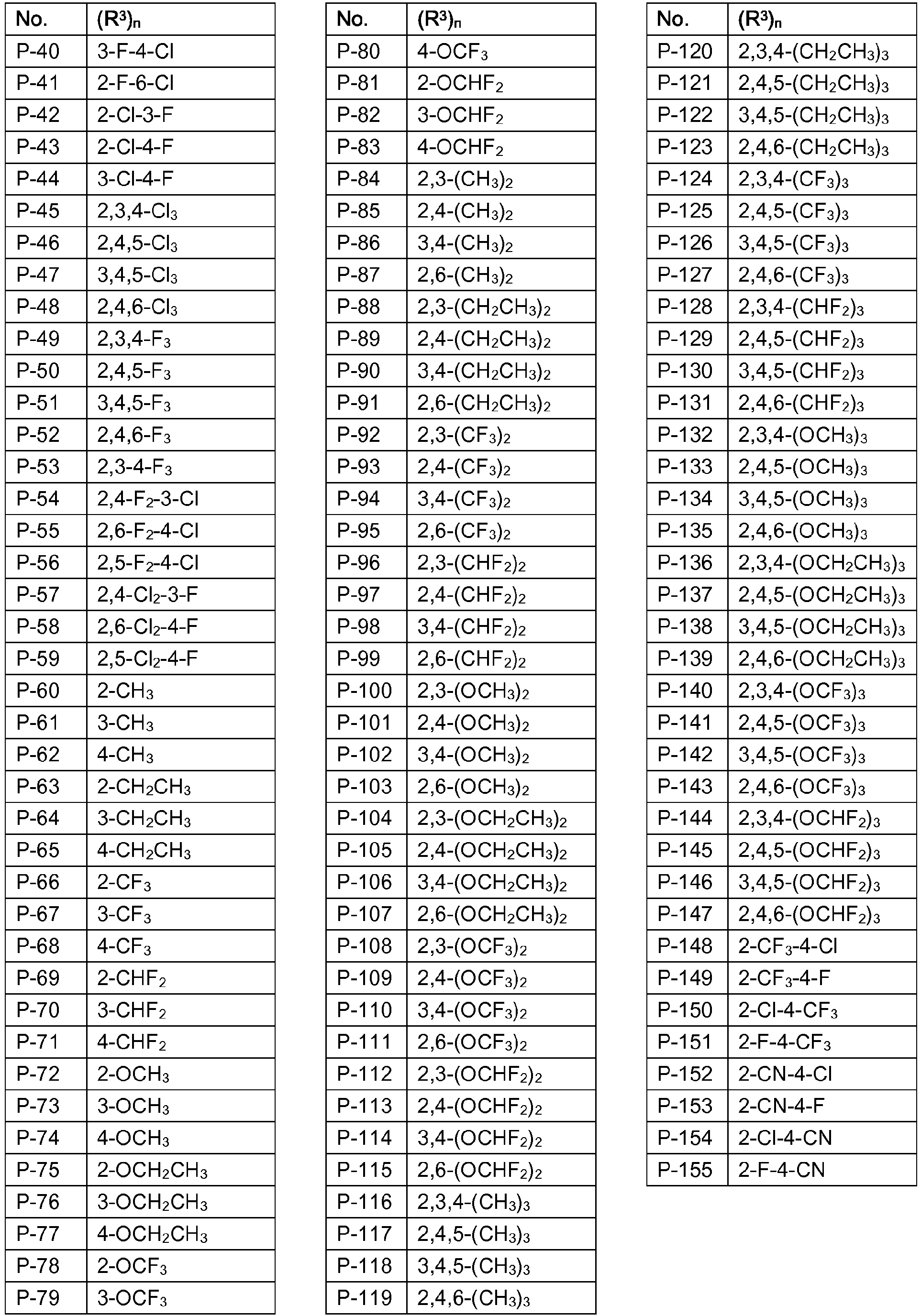

* means that n=0

Y in the compounds according to the invention is, according to one embodiment, as defined in claim 1.

Y in the compounds according to the invention is, according to a further embodiment, a direct bond or a divalent group selected from the group consisting of -O-, -S-, SO-, -SO₂-.

According to a specific embodiment Y is a direct bond. According to a further specific embodiment Y is -O-. According to a further specific embodiment Y is -S-. According to a further specific embodiment Y is -SO-. According to a further specific embodiment Y is -SO₂-. According to a further specific embodiment Y is -NH-. According to a further specific embodiment Y is -N(C₁-C₄-alkyl)-, wherein C₁-C₄-alkyl is preferably methyl, ethyl, n-propyl, i-propyl, n-butyl, i-butyl or t-butyl.

According to a further specific embodiment Y is -CH₂-. According to a further specific embodiment Y is -CH₂CH₂-. According to a further specific embodiment Y is -CH=CH-.

According to a further specific embodiment Y is -C≡C-.

Z in the compounds according to the invention is, according to one embodiment, as defined in claim 1.

Z in the compounds according to the invention is, according to a further embodiment, is phenyl or a five- or six-membered heteroaryl selected from the group consisting of pyrimidin-2-yl, pyrimidin-3-yl, pyrimidin-4-yl, pyridin-2-yl, pyridin-3-yl, pyridin-4-yl, thiazol-2-yl, thiazol-4-yl, thiazol-5-yl, isothiazol-3-yl, isothiazol-4-yl, isothiazol-5-yl, pyrazin-2-yl, pyridazin-3-yl, 1,3,5-triazin-2-yl and 1,2,4-triazin-3-yl; and wherein the phenyl is unsubstituted carries one, two, three or four independently selected radicals R⁴ , and wherein the heteroaryl is unsubstitutedcarries one, two or three radicals R⁴, wherein R⁴ is as defined or preferably defined below.

According to one specific embodiment Z is phenyl which carries one, two, three or four independently selected radicals R⁴ as defined or preferably defined below. According to one specific embodiment Z is pyrimidin-2-yl which carries one, two, three or four independently selected radicals R⁴ as defined or preferably defined below. According to one specific embodiment Z is pyridin-3-yl, which carries one, two, three or four independently selected radicals R⁴ as defined or preferably defined below. According to one specific embodiment Z is pyridin-4-yl, which carries one, two, three or four independently selected radicals R⁴ as defined or preferably defined below. According to one specific embodiment Z is thiazol-2-yl, which carries one, two, three or four independently selected radicals R⁴ as defined or preferably defined below.

According to one embodiment x is 0. According to one further embodiment x is 1. According to one further embodiment x is 2. According to one further embodiment x is 3. According to one further embodiment x is 4.

According to one embodiment if R is O and Q is CH₂ x is not 0.

R⁴ in the compounds according to the invention is, according to one embodiment, as defined in claim 1.

R⁴ in the compounds according to the invention is, according to a further embodiment, halogen, C₁-C₆-alkyl, C₁-C₆-haloalkyl, C₁-C₆-alkoxy, C₁-C₆-haloalkoxy, wherein R⁴ is substituted by one, two, three or four R^{4a}; wherein R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy; wherein m is 1, 2 or 3.

According to one embodiment m is 0. According to one embodiment m is 1. According to one further embodiment m is 2. According to one further embodiment m is 3. According to one further embodiment m is 4.

According to one specific embodiment thereof, said R⁴ is in the 2-positon of the phenyl ring. According to one specific embodiment thereof, said R⁴ is in the 3-positon of the phenyl ring. According to one further specific embodiment thereof, said R⁴ is in the 4-positon of the phenyl ring. According to one specific embodiment thereof, said R⁴ is in the 2,3-positon of the phenyl ring. According to one specific embodiment thereof, said R⁴ is in the 2,4-positon of the phenyl ring. According to one specific embodiment thereof, said R⁴ is in the 2,5-positon of the phenyl ring. According to one specific embodiment thereof, said R⁴ is in the 2,6-positon of the phenyl ring. According to one specific embodiment thereof, said R⁴ is in the 3,4-positon of the phenyl ring. According to one specific embodiment thereof, said R⁴ is in the 3,5-positon of the phenyl ring. According to one specific embodiment thereof, said R⁴ is in the 3,6-positon of the phenyl ring. According to one specific embodiment thereof, said R⁴ is in the 2,4,6-positon of the phenyl ring.

According to one embodiment R⁴ is halogen. According to a specific embodiment R⁴ is Cl. According to a further specific embodiment R⁴ is F. According to a further specific embodiment R⁴ is Br. According to a further specific embodiment R⁴ is CN. According to a further specific embodiment R⁴ is NO₂. According to a further specific embodiment R⁴ is OH. According to a further specific embodiment R⁴ is SH.

According to one further embodiment R⁴ is C₁-C₆-alkyl, preferably methyl, ethyl, n-propyl, i-propyl, n-butyl, i-butyl or t-butyl. In a special embodiment R⁴ is methyl. In a further special embodiment R⁴ is ethyl. In a further special embodiment R⁴ is n-propyl. In a further special embodiment R⁴ is i-propyl. In a further special embodiment R⁴ is 1-methylpropyl. In a further special embodiment R⁴ is n-butyl. In a further special embodiment R⁴ is i-butyl. In a further special embodiment R⁴ is t-butyl.

According to a one preferred embodiment R⁴ is C₁-C₆-alkyl substituted by one, two, three or up to the maximum possible number of identical or different groups R^{a} as defined and preferably herein. According to a specific embodiment R⁴ is C₁-C₆-haloalkyl, more preferably fully or partially halogenated methyl, ethyl, n-propyl, i-propyl, n-butyl, i-butyl or t-butyl. In a special embodiment R⁴ is CF₃. In a further special embodiment R⁴ is CHF₂. In a further special embodiment R⁴ is CFH₂. In a further special embodiment R⁴ is CCl₃ In a further special embodiment R⁴ is CHCl₂. In a further special embodiment R⁴ is CClH₂. According to a further specific embodiment R⁴ is C₁-C₆-alkyl, preferably C₁-C₄-alkyl substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R⁴ is CH₂OH. According to a further specific embodiment R⁴ is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH₂CH₂CH₂CN. In a special embodiment R⁴ is CH₂CH₂CN. In a further special embodiment R⁴ is CH(CH₃)CN. According to a further specific embodiment R⁴ is C₁-C₄-alkoxy-C₁-C₆-alkyl, more preferably C₁-C₄-alkoxy-C₁-C₄-alkyl. In a special embodiment R⁴ is CH₂OCH₃. In a further special embodiment R⁴ is CH₂CH₂OCH₃. In a further special embodiment R⁴ is CH(CH₃)OCH₃. In a further special embodiment R⁴ is CH(CH₃)OCH₂CH₃. In a further special embodiment R⁴ is CH₂CH₂OCH₂CH₃. According to a further specific embodiment R⁴ is C₁-C₄-haloalkoxy-C₁-C₆-alkyl, more preferably C₁-C₄-alkoxy-C₁-C₄-alkyl. In a special embodiment R⁴ is CH₂OCF₃. In a further special embodiment R⁴ is CH₂CH₂OCF₃. In a further special embodiment R⁴ is CH₂OCCl₃. In a further special embodiment R⁴ is CH₂CH₂OCCl₃.

According to one another embodiment R⁴ is C₁-C₆-alkoxy, preferably C₁-C₄-alkoxy. In a special embodiment of the invention R⁴ is OCH₃. In a further special embodiment of the invention R⁴ is OCH₂CH₃.In a further special embodiment of the invention R⁴ is OCH(CH₃)₂. In a further special embodiment of the invention R⁴ is OCH₂CH₂CH₃. In a further special embodiment of the invention R⁴ is OC(CH₃)₃.

According to one another embodiment R⁴ is C₁-C₆-haloalkoxy, preferably C₁-C₄-haloalkoxy. In a special embodiment of the invention R⁴ is OCF₃. In a further special embodiment of the invention R⁴ is OCHF₂.

According to one another embodiment R⁴ is C₂-C₆-alkenyl, preferably CH=CH₂, CH₂CH=CH₂, CH=CHCH₃ or C(CH₃)=CH₂. In a special embodiment R⁴ is CH=CH₂. In a further special embodiment R⁴ is CH₂CH=CH₂. In a further special embodiment R⁴ is CH₂CH=CHCH₃. In a further special embodiment R⁴ is CH=CHCH₃ In a further special embodiment R⁴ is CH₂C(CH₃)=CH₂. In a further special embodiment R⁴ is C(CH₃)=CH₂. In a further special embodiment R⁴ is C(CH₃)=C(CH₃)H. In a further special embodiment R⁴ is C(CH₃)=C(CH₃)₂. In a further special embodiment R⁴ is CH=C(CH₃)₂.

According to a further preferred embodiment R⁴ is C₂-C₆-alkenyl, substituted by one, two, three or up to the maximum possible number of identical or different groups R^{a} as defined and preferably herein.

According to a specific embodiment R⁴ is C₂-C₆-haloalkenyl, more preferably fully or partially halogenated C₂-C₆-alkenyl. In a special embodiment R⁴ is fully or partially halogenated C₂-alkenyl. In a further special embodiment R⁴ is fully or partially halogenated C₃-alkenyl. According to a further specific embodiment R⁴ is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R⁴ is CH=CHOH. In a further special embodiment R⁴ is CH=CHCH₂OH. According to a further specific embodiment R⁴ is C₁-C₄-alkoxy-C₂-C₆-alkenyl, more preferably C₁-C₄-alkoxy-C₂-C₄-alkenyl. In a special embodiment R⁴ is CH=CHOCH₃. In a further special embodiment R⁴ is CH=CHCH₂OCH₃. According to a further specific embodiment R⁴ is C₁-C₄-haloalkoxy-C₂-C₆-alkenyl, more preferably C₁-C₄-haloalkoxy-C₂-C₄-alkenyl. In a special embodiment R⁴ is CH=CHOCF₃. In a further special embodiment R⁴ is CH=CHCH₂OCF₃. In a further special embodiment R⁴ is CH=CHOCCl₃. In a further special embodiment R⁴ is CH=CHCH₂OCCl₃. According to a further specific embodiment R⁴ is C₃-C₈-cycloalkyl-C₂-C₆-alkenyl, preferably C₃-C₆-cycloalkyl-C₂-C₄-alkenyl. According to a further specific embodiment R⁴ is C₃-C₆-halocycloalkyl-C₂-C₄-alkenyl, preferably C₃-C₈-halocycloalkyl-C₂-C₆-alkenyl.

According to one another embodiment R⁴ is C₂-C₆-alkynyl, preferably CCH, CH₂CCH, CH₂CCCH₃. In a special embodiment R⁴ is CCH. in a further special embodiment R⁴ is CCCH₃. In a further special embodiment R⁴ is CH₂CCH. In a further special embodiment R⁴ is CH₂CCCH₃. In a further special embodiment R⁴ is CH₂CCH₂CH₃.

According to a further preferred embodiment R⁴ is C₂-C₆-alkynyl, substituted by one, two, three or up to the maximum possible number of identical or different groups R^{a} as defined and preferably herein.

According to a specific embodiment R⁴ is C₂-C₆-haloalkynyl, more preferably fully or partially halogenated C₂-C₆-alkynyl. In a special embodiment R⁴ is fully or partially halogenated C₂-alkynyl. In a further special embodiment R⁴ is fully or partially halogenated C₃-alkynyl. According to a further specific embodiment R⁴ is C₂-C₆-alkynyl, preferably C₂-C₄-alkynyl, substituted by OH, more preferably, CCOH, CH₂CCOH. In a special embodiment R⁴ is CCOH. In a further special embodiment R⁴ is CH₂CCOH. According to a further specific embodiment R⁴ is C₁-C₄-alkoxy-C₂-C₆-alkynyl, more preferably C₁-C₄-alkoxy-C₂-C₄-alkynyl. In a special embodiment R⁴ is CCOCH₃. In a further special embodiment R⁴ is CH₂CCOCH₃. According to a further specific embodiment R⁴ is C₁-C₄-haloalkoxy-C₂-C₆-alkynyl, more preferably C₁-C₄-haloalkoxy-C₂-C₄-alkynyl. In a special embodiment R⁴ is CCOCF₃. In a further special embodiment R⁴ is CH₂CCOCF₃. In a further special embodiment R⁴ is CCOCCl₃. In a further special embodiment R⁴ is CH₂CCOCCl₃. According to a further specific embodiment R⁴ is C₃-C₈-cycloalkyl-C₂-C₆-alkynyl, preferably C₃-C₆-cycloalkyl-C₂-C₄-alkynyl. According to a further specific embodiment R⁴ is C₃-C₆-halocycloalkyl-C₂-C₄-alkynyl, preferably C₃-C₈-halocycloalkyl-C₂-C₆-alkynyl.

According to one another embodiment R⁴ is C₃-C₈-cycloalkyl, preferably cyclopropyl, cyclobutyl, cyclopentyl or cyclohexyl, in particular cyclopropyl or cyclobutyl. In a special embodiment R⁴ is cyclopropyl. In a further special embodiment R⁴ is cyclobutyl. In a further special embodiment R⁴ is cyclopentyl. In a further special embodiment R⁴ is cyclohexyl.

According to one another embodiment R⁴ is C₃-C₈-cycloalkoxy, preferably C₃-C₆-cycloalkoxy. In a special embodiment R⁴ is O-cyclopropyl.

According to a further preferred embodiment R⁴ is C₃-C₈-cycloalkyl, substituted by one, two, three or up to the maximum possible number of identical or different groups R^{a} as defined and preferably herein.

According to a specific embodiment R⁴ is C₃-C₈-halocycloalkyl, more preferably fully or partially halogenated C₃-C₆-cycloalkyl. In a special embodiment R⁴ is fully or partially halogenated cyclopropyl. In a further special embodiment R⁴ is 1-Cl-cyclopropyl. In a further special embodiment R⁴ is 2-Cl-cyclopropyl. In a further special embodiment R⁴ is 1-F-cyclopropyl. In a further special embodiment R⁴ is 2-F-cyclopropyl. In a further special embodiment R⁴ is fully or partially halogenated cyclobutyl. In a further special embodiment R⁴ is 1-Cl-cyclobutyl. In a further special embodiment R⁴ is 1-F-cyclobutyl. In a further special embodiment R⁴ is 3,3-(Cl)₂-cyclobutyl. In a further special embodiment R⁴ is 3,3-(F)₂-cyclobutyl.According to a specific embodiment R⁴ is C₃-C₈-cycloalkyl substituted by C₁-C₄-alkyl, more preferably is C₃-C₆-cycloalkyl substituted by C₁-C₄-alkyl. In a special embodiment R⁴ is 1-CH₃-cyclopropyl. According to a specific embodiment R⁴ is C₃-C₈-cycloalkyl substituted by CN, more preferably is C₃-C₆-cycloalkyl substituted by CN. In a special embodiment R⁴ is 1-CN-cyclopropyl.According to a further specific embodiment R⁴ is C₃-C₈-cycloalkyl-C₃-C₈-cycloalkyl, preferably C₃-C₆-cycloalkyl-C₃-C₆-cycloalkyl. In a special embodiment R⁴ is cyclopropyl-cyclopropyl. In a special embodiment R⁴ is 2-cyclopropyl-cyclopropyl. According to a further specific embodiment R⁴ is C₃-C₈-cycloalkyl-C₃-C₈-halocycloalkyl, preferably C₃-C₆-cycloalkyl-C₃-C₆-halocycloalkyl.

According to one another embodiment R⁴ is C₃-C₈-cycloalkyl-C₁-C₄-alkyl, preferably C₃-C₆-cycloalkyl-C₁-C₄-alkyl. In a special embodiment R⁴ is CH(CH₃)(cyclopropyl). In a further special embodiment R⁴ is In a special embodiment R⁴ is CH₂-(cyclopropyl).

According to a further preferred embodiment R⁴ is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R⁴ is C₃-C₈-cycloalkyl-C₁-C₄-haloalkyl, C₃-C₆-cycloalkyl-C₁-C₄-haloalkyl. According to a specific embodiment R⁴ is C₃-C₈-halocycloalkyl-C₁-C₄-alkyl, C₃-C₆-halocycloalkyl-C₁-C₄-alkyl. In a special embodiment R⁴ is fully or partially halogenated cyclopropyl-C₁-C₄-alkyl. In a further special embodiment R⁴ is 1-Cl-cyclopropyl-C₁-C₄-alkyl. In a further special embodiment R⁴ is 1-F-cyclopropyl-C₁-C₄-alkyl.

According to one another embodiment R⁴ is NH₂.

According to one another embodiment R⁴ is NH(C₁-C₄-alkyl). According to a specific embodiment R⁴ is NH(CH₃). According to a specific embodiment R⁴ is NH(CH₂CH₃). According to a specific embodiment R⁴ is NH(CH₂CH₂CH₃). According to a specific embodiment R⁴ is NH(CH(CH₃)₂). According to a specific embodiment R⁴ is NH(CH₂CH₂CH₂CH₃). According to a specific embodiment R⁴ is NH(C(CH₃)₃).

According to one another embodiment R⁴ is N(C₁-C₄-alkyl)₂. According to a specific embodiment

R⁴ is N(CH₃)₂. According to a specific embodiment R⁴ is N(CH₂CH₃)₂. According to a specific embodiment R⁴ is N(CH₂CH₂CH₃)₂. According to a specific embodiment R⁴ is N(CH(CH₃)₂)₂. According to a specific embodiment R⁴ is N(CH₂CH₂CH₂CH₃)₂. According to a specific embodiment R⁴ is NH(C(CH₃)₃)_{2.}

According to one another embodiment R⁴ is NH(C₃-C₈-cycloalkyl) preferably NH(C₃-C₆-cycloalkyl). According to a specific embodiment R⁴ is NH(cyclopropyl). According to a specific embodiment R⁴ is NH(cyclobutyl). According to a specific embodiment R⁴ is NH(cyclopentyl). According to a specific embodiment R⁴ is NH(cyclohexyl).

According to one another embodiment R⁴ is N(C₃-C₈-cycloalkyl)₂ preferably N(C₃-C₆-cycloalkyl)₂. According to a specific embodiment R⁴ is N(cyclopropyl)₂. According to a specific embodiment R⁴ is N(cyclobutyl)₂. According to a specific embodiment R⁴ is N(cyclopentyl)₂. According to a specific embodiment R⁴ is N(cyclohexyl)₂.

According to one another embodiment R⁴ is S(O)ₚ(C₁-C₄-alkyl) wherein p is 0, 1, 2, preferably S(O)ₚ(C₁-C₄-alkyl) wherein p is 2. According to a specific embodiment R⁴ is SO₂CH₃. According to a specific embodiment R⁴ is SO₂CF₃.

According to one another embodiment R⁴ is C(=O)(-C₁-C₄-alkyl). According to a specific embodiment R⁴ is C(=O)CH₃. According to a further specific embodiment R⁴ is C(=O)CH₂CH₃. According to a further specific embodiment R⁴ is C(=O)CH₂CH₂CH₃. According to a further specific embodiment R⁴ is C(=O)CH(CH₃)₂. According to a further specific embodiment R⁴ is C(=O)C(CH₃)₃.

According to one another embodiment R⁴ is C(=O)OH.

According to one another embodiment R⁴ is C(=O)(-O-C₁-C₄-alkyl). According to a specific embodiment R⁴ is C(=O)OCH₃. According to a further specific embodiment R⁴ is C(=O)OCH₂CH₃. According to a further specific embodiment R⁴ is C(=O)OCH₂CH₂CH₃. According to a further specific embodiment R⁴ is C(=O)OCH(CH₃)₂. According to a further specific embodiment R⁴ is C(=O)OC(CH₃)₃.

According to one another embodiment R⁴ is C(=O)-NH(C₁-C₄-alkyl). According to a specific embodiment R⁴ is C(=O)NHCH₃. According to a further specific embodiment R⁴ is C(=O)NHCH₂CH₃. According to a further specific embodiment R⁴ is C(=O)NHCH₂CH₂CH₃. According to a further specific embodiment R⁴ is C(=O)NHCH(CH₃)₂. According to a further specific embodiment R⁴ is C(=O)NHC(CH₃)₃.

According to one another embodiment R⁴ is C(=O)-N(C₁-C₄-alkyl)₂. According to a specific embodiment R⁴ is C(=O)N(CH₃)₂. According to a further specific embodiment R⁴ is C(=O)N(CH₂CH₃)₂. According to a further specific embodiment R⁴ is C(=O)N(CH₂CH₂CH₃)₂. According to a further specific embodiment R⁴ is C(=O)N(CH(CH₃)₂)₂. According to a further specific embodiment R⁴ is C(=O)N(C(CH₃)₃)₂.

According to one another embodiment R⁴ is C(=O)-NH(C₃-C₆-cycloalkyl). According to a specific embodiment R⁴ is C(=O)NH(cyclopropyl). According to a further specific embodiment R⁴ is C(=O)NH(cyclobutyl). According to a further specific embodiment R⁴ is C(=O)NH(cyclopentyl). According to a further specific embodiment R⁴ is C(=O)NH(cyclohexyl).

According to one another embodiment R⁴ is C(=O)-N(C₃-C₆-cycloalkyl)₂. According to a specific embodiment R⁴ is C(=O)N(cyclopropyl)₂. According to a further specific embodiment R⁴ is C(=O)N(cyclobutyl)₂. According to a further specific embodiment R⁴ is C(=O)N(cyclopentyl)₂. According to a further specific embodiment R⁴ is C(=O)N(cyclohexyl)₂.

The above mentioned list of particularly preferred embodiments of R⁴ is independent for each m = 1, m = 2, m = 3, m = 4 and m= 5 and is independent within m = 2, m = 3, m = 4 and m= 5. Particularly preferred embodiments of R⁴ₘ according to the invention are in Table X below, wherein each line of lines X1-1 to X1-145 corresponds to one particular embodiment of the invention, wherein X1-1 to X1-145 are also in any combination a preferred embodiment of the present invention

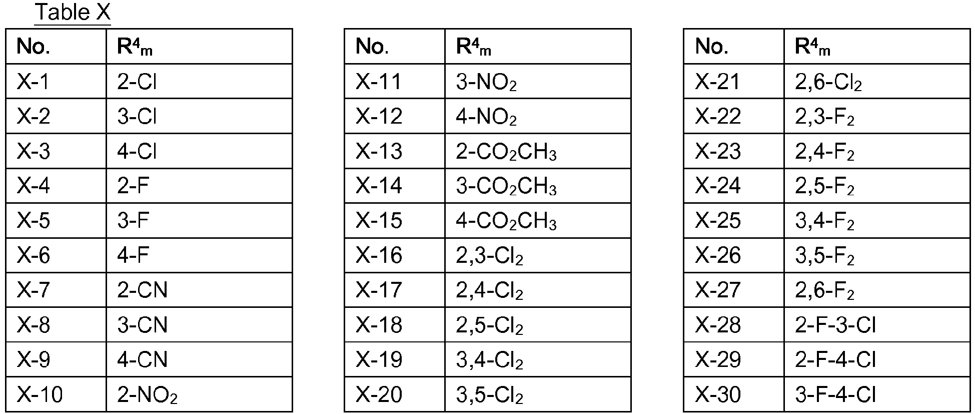

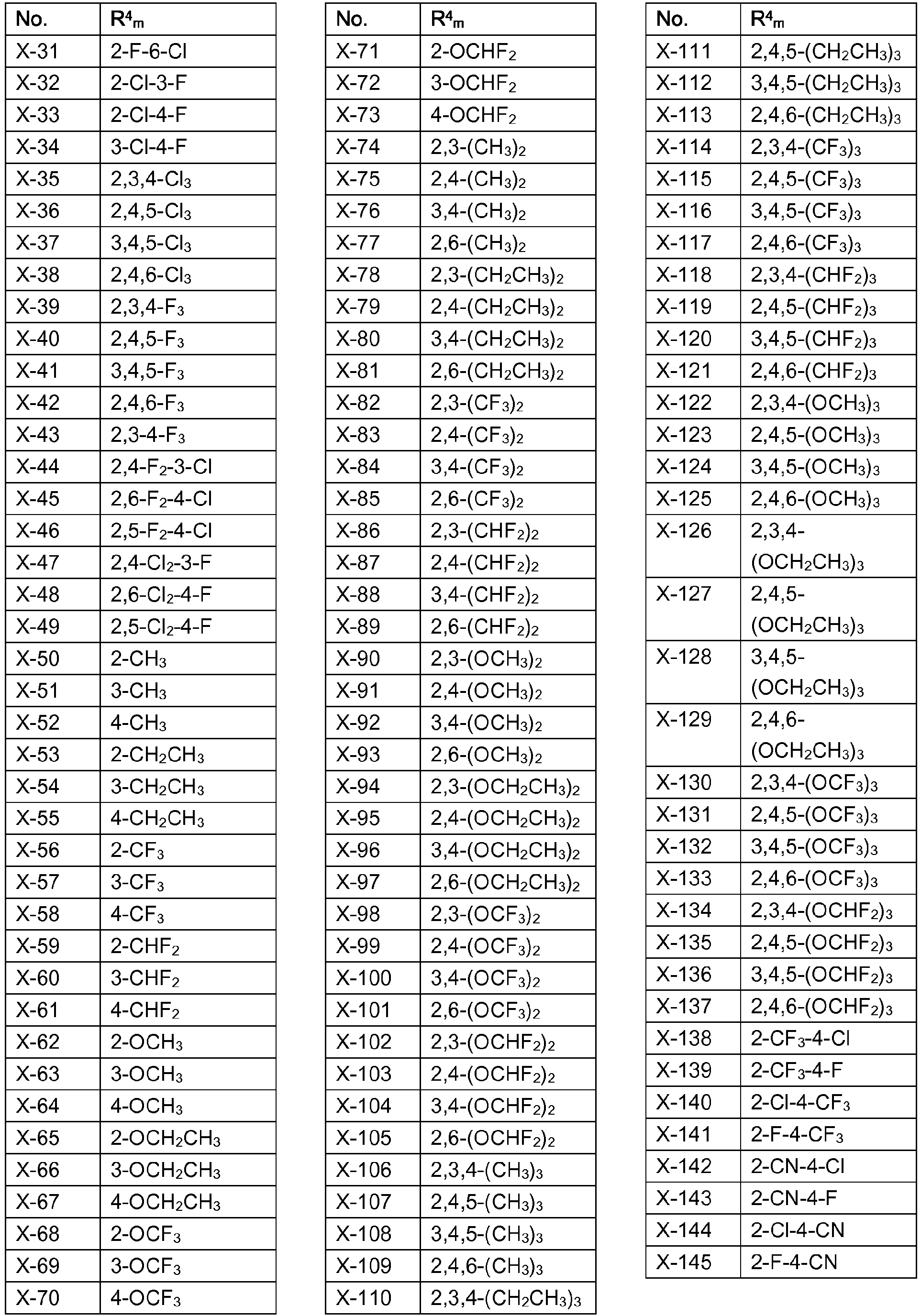

Particularly preferred embodiments of Z-R⁴ₘ according to the invention are in Table Y below, wherein each line of lines Y-1 to Y-145 corresponds to one particular embodiment of the invention, wherein Y-1 to Y-145 are also in any combination a preferred embodiment of the present invention

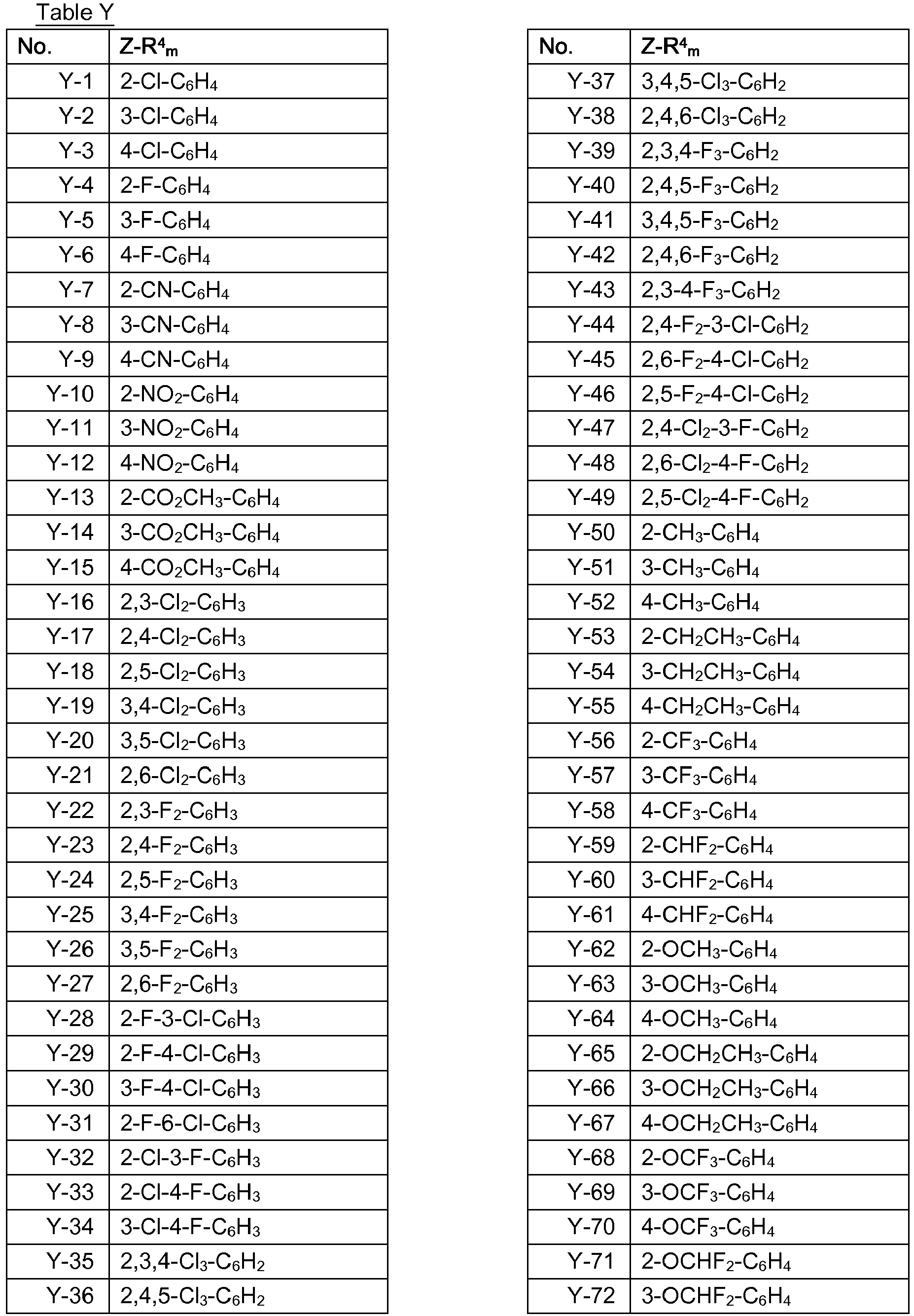

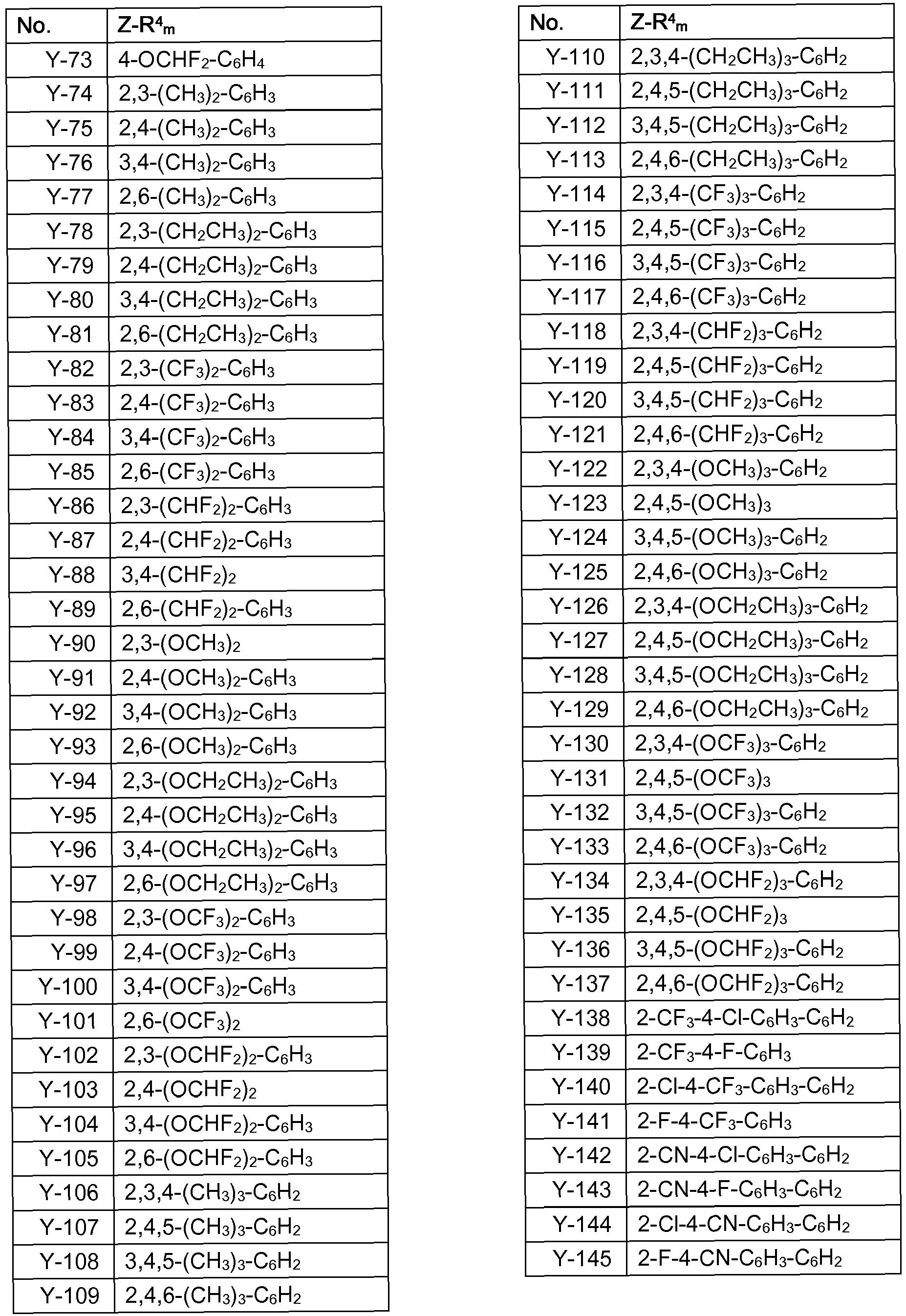

Particularly preferred embodiments of Z-R⁴ₘ according to the invention are in Table Z below, wherein each line of lines Z-1 to Z-103 corresponds to one particular embodiment of the invention, wherein Z-1 to Z-1 03 are also in any combination a preferred embodiment of the present invention

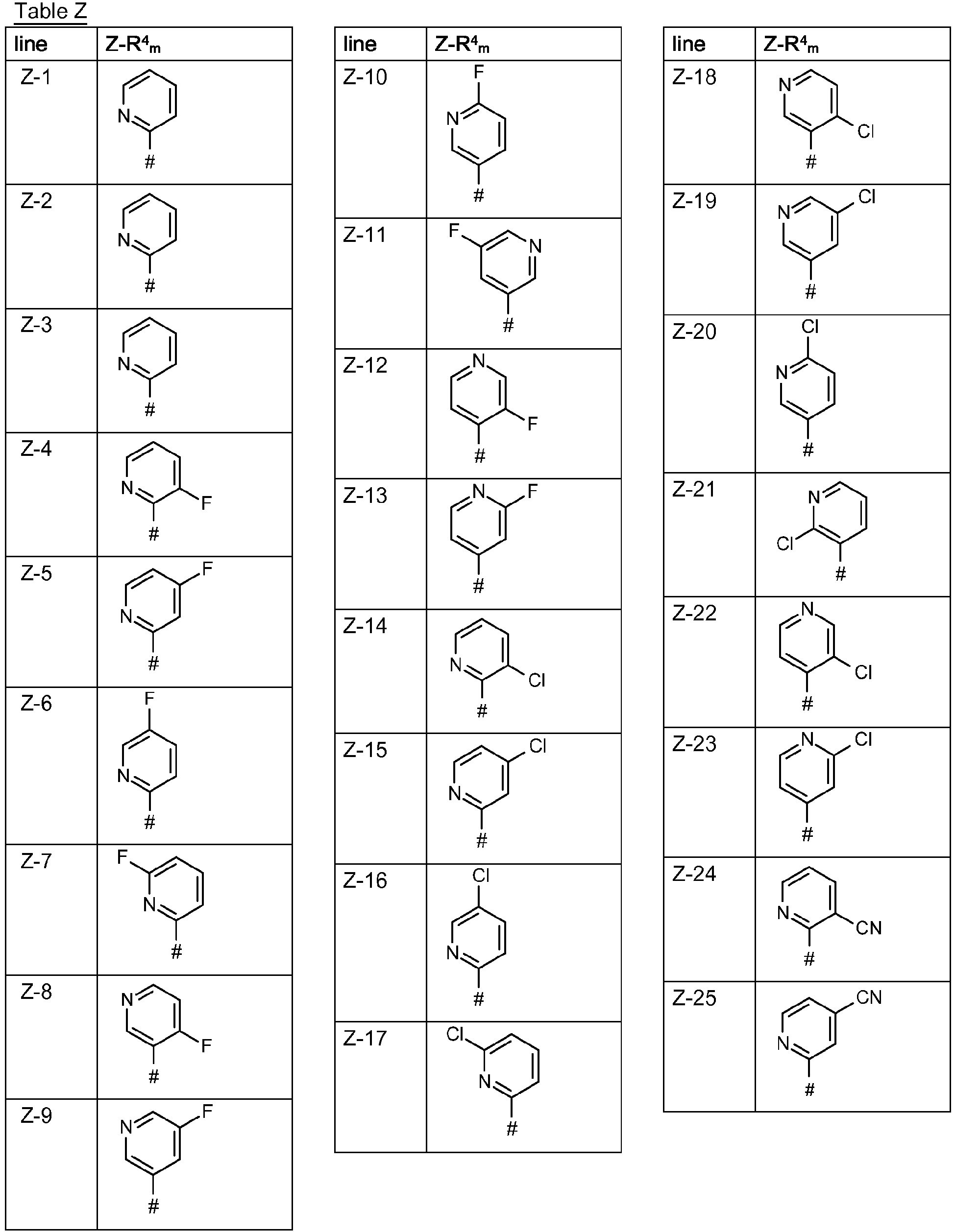

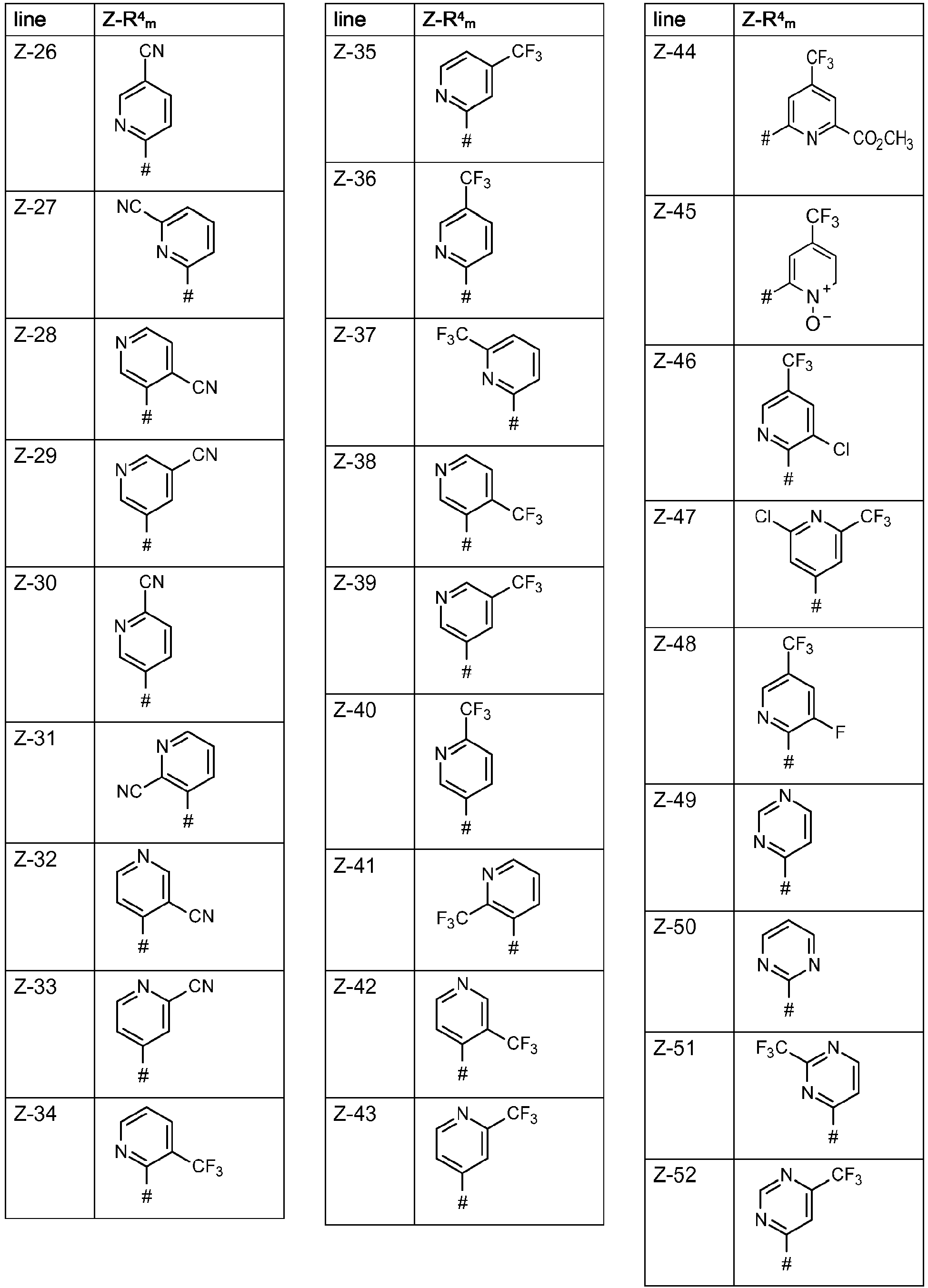

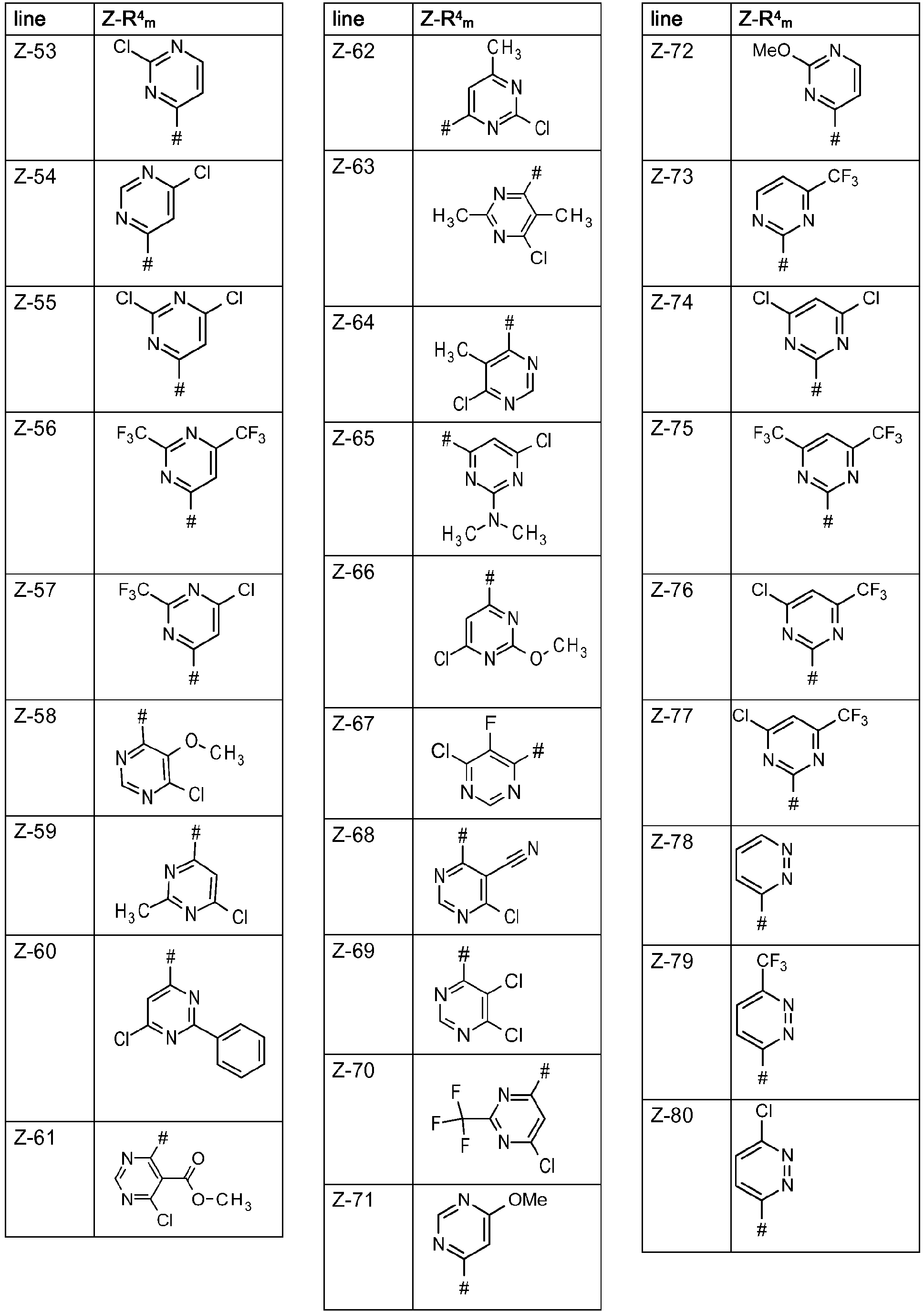

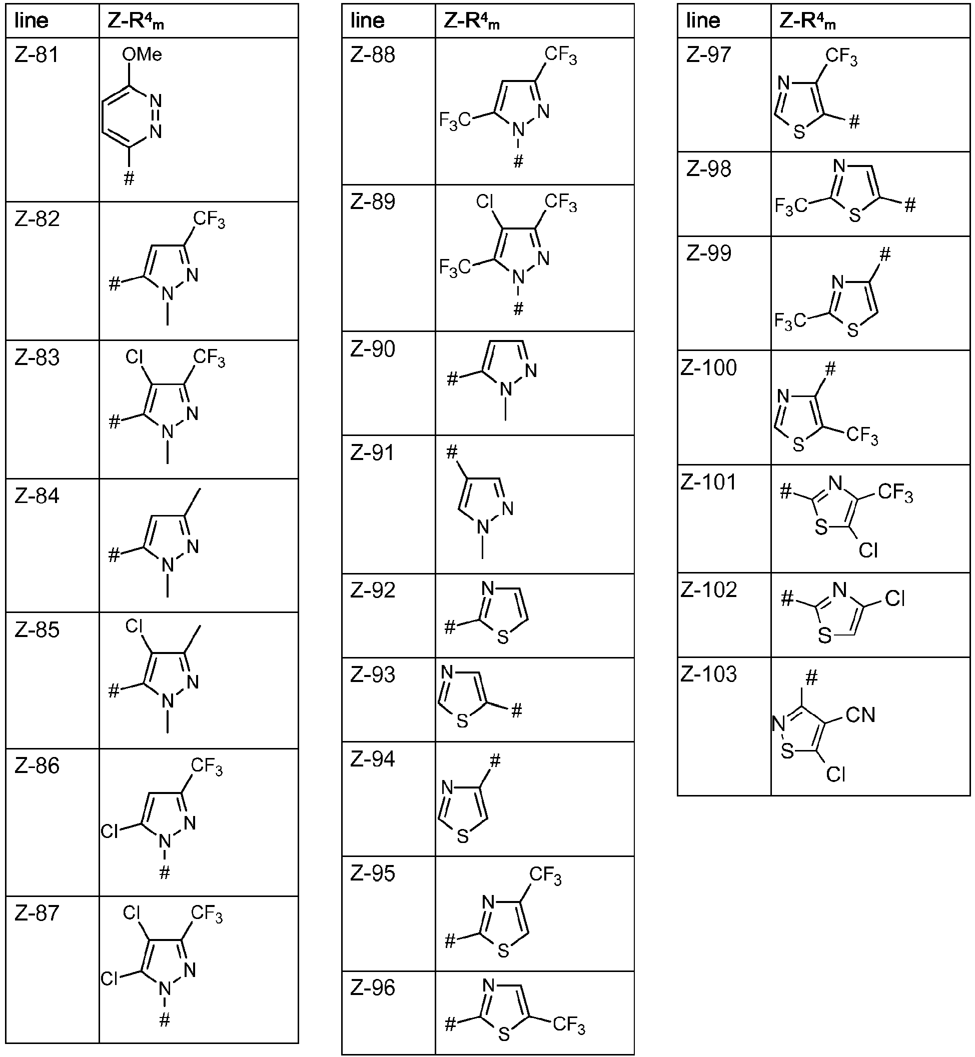

R⁵ and R⁶ in the compounds according to the invention are independently of another, according to one embodiment, as defined in claim 1.

R⁵ and R⁶ in the compounds according to the invention are independently of another, according to a further embodiment, H.

According to one embodiment R⁵ and R⁶ in the compounds according to the invention are independently of another halogen. According to a specific embodiment R⁵ and R⁶ are independently Cl. According to a further specific embodiment R⁵ and R⁶ are independently F. According to a further specific embodiment R⁵ and R⁶ are independently Br. According to a further specific embodiment R⁵ and R⁶ are independently CN. According to a further specific embodiment R⁵ and R⁶ are independently NO₂**.** According to a further specific embodiment R⁵ and R⁶ are independently OH. According to a further specific embodiment R⁵ and R⁶ are independently SH. In a further special embodiment R⁵ is H and R⁶ is Cl. In a further special embodiment R⁵ is H and R⁶ is Br. In a further special embodiment R⁵ is H and R⁶ is F.

According to a further embodiment of the invention, R⁵ and R⁶ are independently selected from C₁-C₆-alkyl, C₂-C₆-alkenyl, C₂-C₆-alkynyl, C₃-C₈-cycloalkyl, wherein the R¹ are in each case unsubstituted or are substituted by R^{a} and/or R^{b} as defined and preferably herein.

According to a further embodiment of the invention, R⁵ and R⁶ are independently selected from C₁-C₃-alkyl, C₂-C₆-alkenyl, C₂-C₆-alkynyl, C₃-C₈-cycloalkyl, wherein the R⁵ and R⁶ are independently in each case unsubstituted or are substituted by R^{a} and/or R^{b} as defined and preferably herein.

According to one embodiment R⁵ and R⁶ are independently C₁-C₆-alkyl, preferably methyl, ethyl, n-propyl, i-propyl, n-butyl, i-butyl or t-butyl. In a special embodiment R⁵ and R⁶ are independently methyl. In a further special embodiment R⁵ and R⁶ are independently ethyl. In a further special embodiment R⁵ and R⁶ are independently n-propyl. In a further special embodiment R⁵ and R⁶ are independently i-propyl. In a further special embodiment R⁵ and R⁶ are independently 1-methylpropyl. In a further special embodiment R⁵ and R⁶ are independently n-butyl. In a further special embodiment R⁵ and R⁶ are independently i-butyl. In a further special embodiment R⁵ and R⁶ are independently t-butyl.

In a further special embodiment R⁵ is H and R⁶ is CH₃. In a further special embodiment R⁵ is H and R⁶ is CH₂CH₃. In a further special embodiment R⁵ is H and R⁶ is CH₂CH₂CH₃. In a further special embodiment R⁵ is H and R⁶ is CH(CH₃)₂. In a further special embodiment R⁵ is CH₃ and R⁶ is CH₃. In a further special embodiment R⁵ is CH₂CH₃ and R⁶ is CH₂CH₃.

According to a one preferred embodiment R⁵ and R⁶ are independently C₁-C⁶-alkyl substituted by one, two, three or up to the maximum possible number of identical or different groups R^{a} as defined and preferably herein.

According to a specific embodiment R⁵ and R⁶ are independently C₁-C₆-haloalkyl, more preferably fully or partially halogenated methyl, ethyl, n-propyl, i-propyl, n-butyl, i-butyl or t-butyl. In a special embodiment R⁵ and R⁶ are independently CF₃. In a further special embodiment R⁵ and R⁶ are independently CHF₂. In a further special embodiment R⁵ and R⁶ are independently CFH₂. In a further special embodiment R⁵ and R⁶ are independently CCl₃. In a further special embodiment R⁵ and R⁶ are independently CHCl₂. In a further special embodiment R⁵ and R⁶ are independently CClH₂. According to a further specific embodiment R⁵ and R⁶ are independently C₁-C⁶-alkyl, preferably C₁-C₄-alkyl substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R⁵ and R⁶ are independently CH₂OH. In a further special embodiment R⁵ and R⁶ are independently CH₂CH₂OH. According to a further specific embodiment R⁵ and R⁶ are independently C₁-C⁶-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH₂CH₂CH₂CN. In a special embodiment R⁵ and R⁶ are independently CH₂CH₂CN. In a further special embodiment R⁵ and R⁶ are independently CH(CH₃)CN. According to a further specific embodiment R⁵ and R⁶ are independently C₁-C₄-alkoxy-C₁-C₆-alkyl, more preferably C₁-C₄-alkoxy-C₁-C₄-alkyl. In a special embodiment R⁵ and R⁶ are independently CH₂OCH₃. In a further special embodiment R⁵ and R⁶ are independently CH₂CH₂OCH₃. In a further special embodiment R⁵ and R⁶ are independently CH(CH₃)OCH₃. In a further special embodiment R⁵ and R⁶ are independently CH(CH₃)OCH₂CH₃. In a further special embodiment R⁵ and R⁶ are independently CH₂CH₂OCH₂CH₃. According to a further specific embodiment R⁵ and R⁶ are independently C₁-C₄-haloalkoxy-C₁-C₆-alkyl, more preferably C₁-C₄-alkoxy-C₁-C₄-alkyl. In a special embodiment R⁵ and R⁶ are independently CH₂OCF₃. In a further special embodiment R⁵ and R⁶ are independently CH₂CH₂OCF₃. In a further special embodiment R⁵ and R⁶ are independently CH₂OCCl₃. In a further special embodiment R⁵ and R⁶ are independently CH₂CH₂OCCl₃.

According to one another embodiment R⁵ and R⁶ are independently C₁-C₆-alkoxy, preferably C₁-C₄-alkoxy. In a special embodiment of the invention R⁵ and R⁶ are independently OCH₃. In a further special embodiment of the invention R⁵ and R⁶ are independently OCH₂CH₃. In a further special embodiment of the invention R⁵ and R⁶ are independently OCH(CH₃)₂. In a further special embodiment of the invention R⁵ and R⁶ are independentlyOCH₂CH₂CH₃. In a further special embodiment of the invention R⁵ and R⁶ are independently OC(CH₃)₃.

According to one another embodiment R⁵ and R⁶ are independently C₂-C₆-alkenyl, preferably CH=CH₂, CH₂CH=CH₂, CH=CHCH₃ or C(CH₃)=CH₂. In a special embodiment R⁵ and R⁶ are independently CH=CH₂. In a further special embodiment R⁵ and R⁶ are independently CH₂CH=CH₂. In a further special embodiment R⁵ and R⁶ are independently CH₂C(CH3)=CH₂. In a further special embodiment R⁵ and R⁶ are independently CH₂C(CH₃)=CHCH₃. In a further special embodiment R⁵ and R⁶ are independently CH₂C(CH₃)=C(CH₃)₂. In a further special embodiment R⁵ and R⁶ are independently CH₂CH=CHCH₃. In a further special embodiment R⁵ and R⁶ are independently CH=CHCH₃ In a further special embodiment R⁵ and R⁶ are independently CH₂C(CH₃)=CH₂. In a further special embodiment R⁵ and R⁶ are independently C(CH₃)=CH₂. In a further special embodiment R⁵ and R⁶ are independently C(CH₃)=C(CH₃)H. In a further special embodiment R⁵ and R⁶ are independently C(CH₃)=C(CH₃)₂. In a further special embodiment R⁵ and R⁶ are independently CH=C(CH₃)₂.

According to a further preferred embodiment R⁵ and R⁶ are independently C₂-C₆-alkenyl, substituted by one, two, three or up to the maximum possible number of identical or different groups R^{a} as defined and preferably herein.

According to a specific embodiment R⁵ and R⁶ are independently C₂-C₆-haloalkenyl, more preferably fully or partially halogenated C₂-C₆-alkenyl. In a special embodiment R⁵ and R⁶ are independently fully or partially halogenated C₂-alkenyl. In a further special embodiment R⁵ and R⁶ are independently fully or partially halogenated C₃-alkenyl. In a special embodiment R⁵ and R⁶ are independently C(Cl)=CH₂. In a special embodiment R⁵ and R⁶ are independently C(Cl)=CClH. In a further special embodiment R⁵ and R⁶ are independently C(H)=CH(Cl). In a further special embodiment R⁵ and R⁶ are independently C(H)=CCl₂. In a further special embodiment R⁵ and R⁶ are independently C(Cl)=CCl₂. In a special embodiment R⁵ and R⁶ are independently C(Cl)=CH₂. In a further special embodiment R⁵ and R⁶ are independently C(H)=CH(F). In a further special embodiment R⁵ and R⁶ are independently C(H)=CF₂. In a further special embodiment R⁵ and R⁶ are independently C(F)=CF₂. In a special embodiment R⁵ and R⁶ are independently C(F)=CFH. According to a further specific embodiment R⁵ and R⁶ are independently C₂-C₆-alkenyl, preferably C₂-C₄-alkenyl, substituted by OH, more preferably, CH=CHCH₂OH. In a further special embodiment R⁵ and R⁶ are independently CH=CHCH₂OH. According to a further specific embodiment R⁵ and R⁶ are independently C₁-C₄-alkoxy-C₂-C⁶-alkenyl, more preferably C₁-C₄-alkoxy-C₂-C₄-alkenyl. In a special embodiment R⁵ and R⁶ are independently CH=CHOCH₃. In a further special embodiment R⁵ and R⁶ are independently CH=CHCH₂OCH₃. According to a further specific embodiment R⁵ and R⁶ are independently C₁-C₄-haloalkoxy-C₂-C₆-alkenyl, more preferably C₁-C₄-haloalkoxy-C₂-C₄-alkenyl. In a further special embodiment R⁵ and R⁶ are independently CH=CHCH₂OCF₃. In a further special embodiment R⁵ and R⁶ are independently CH=CHCH₂OCCl₃. According to a further specific embodiment R⁵ and R⁶ are independently C₃-C₈-cycloalkyl-C₂-C₆-alkenyl, preferably C₃-C⁶-cycloalkyl-C₂-C₄-alkenyl. According to a further specific embodiment R⁵ and R⁶ are independently C₃-C₆-halocycloalkyl-C₂-C₄-alkenyl, preferably C₃-C₈-halocycloalkyl-C₂-C⁶-alkenyl. In a further special embodiment R⁵ and R⁶ are independently CH=CH(C₃H₅). In a further special embodiment R⁵ and R⁶ are independently CH=CH(C₄H₇). In a further special embodiment R⁵ and R⁶ are independently CH=C(H)(ClC₃H₄). In a further special embodiment R⁵ and R⁶ are independently CH=C(H)(FC₃H₄). In a further special embodiment R⁵ and R⁶ are independently CH=C(H)(ClC₄H₆). In a further special embodiment R⁵ and R⁶ are independently CH=C(H)(FC₄H₆).

According to one another embodiment R⁵ and R⁶ are independently C₂-C⁶-alkynyl, preferably CCH, CH₂CCH, CH₂CCCH₃. In a special embodiment R⁵ and R⁶ are independently CCH. In a further special embodiment R⁵ and R⁶ are independently CCCH₃. In a further special embodiment R⁵ and R⁶ are independently CCCH(CH₃)₂. In a further special embodiment R⁵ and R⁶ are independently CCC(CH₃)₃. In a further special embodiment R⁵ and R⁶ are independently CH₂CCH. In a further special embodiment R⁵ and R⁶ are independently CH₂CCCH₃. In a further special embodiment R⁵ and R⁶ are independently CC(C₂H₅) In a further special embodiment R⁵ and R⁶ are independently CH₂CCH₂CH₃.

According to a further preferred embodiment R⁵ and R⁶ are independently C₂-C₆-alkynyl, substituted by one, two, three or up to the maximum possible number of identical or different groups R^{a} as defined and preferably herein.

According to a specific embodiment R⁵ and R⁶ are independently C₂-C₆-haloalkynyl, more preferably fully or partially halogenated C₂-C₆-alkynyl. In a special embodiment R⁵ and R⁶ are independently fully or partially halogenated C₂-alkynyl. In a further special embodiment R⁵ and R⁶ are independently fully or partially halogenated C₃-alkynyl. In a further special embodiment R⁵ and R⁶ are independently CCCI. In a further special embodiment R⁵ and R⁶ are independently CCBr. In a further special embodiment R⁵ and R⁶ are independently CC-I. In a further special embodiment R⁵ and R⁶ are independently CH₂-CCCl. In a further special embodiment R⁵ and R⁶ are independently CH₂-CCBr. In a further special embodiment R⁵ and R⁶ are independently CH₂-CC-I. According to a further specific embodiment R⁵ and R⁶ are independently C₂-C⁶-alkynyl, preferably C₂-C₄-alkynyl, substituted by OH. In a special embodiment R⁵ and R⁶ are independently CC-C(OH)(CH₃)₂. According to a further specific embodiment R⁵ and R⁶ are independently C₁-C₄-alkoxy-C₂-C₆-alkynyl, more preferably C₁-C₄-alkoxy-C₂-C₄-alkynyl. In a special embodiment R⁵ and R⁶ are independently CCOCH₃. In a special embodiment R⁵ and R⁶ are independently CC-CH₂-OCH₃. In a special embodiment R⁵ and R⁶ are independently CC-C(OCH₃)(CH₃)₂. In a further special embodiment R⁵ and R⁶ are independently CH₂CCOCH₃. According to a further specific embodiment R⁵ and R⁶ are independently C₁-C₄-haloalkoxy-C₂-C⁶-alkynyl, more preferably C₁-C₄-haloalkoxy-C₂-C₄-alkynyl. In a further special embodiment R⁵ and R⁶ are independently CC-CH₂OCCl₃. In a further special embodiment R⁵ and R⁶ are independently CC-CH₂OCF₃ According to a further specific embodiment R⁵ and R⁶ are independently C₃-C₈-cycloalkyl-C₂-C₆-alkynyl, preferably C₃-C⁶-cycloalkyl-C₂-C₄-alkynyl. In a special embodiment R⁵ and R⁶ are independently CC(C₃H₅). In a special embodiment R⁵ and R⁶ are independently CC(C₄H₇). In a special embodiment R⁵ and R⁶ are independently CCCH₂(C₃H₅). In a special embodiment R⁵ and R⁶ are independently CC-CH₂-C₄H₇). According to a further specific embodiment R⁵ and R⁶ are independently C₃-C₆-halocycloalkyl-C₂-C₄-alkynyl, preferably C₃-C₈-halocycloalkyl-C₂-C₆-alkynyl. In a special embodiment R⁵ and R⁶ are independently CC(C₃H₄Cl). In a special embodiment R⁵ and R⁶ are independently CC(C₃H₄F). In a special embodiment R⁵ and R⁶ are independently CC(C₄H₆Cl). In a special embodiment R⁵ and R⁶ are independently CC(C₄H₆F).

According to one another embodiment R⁵ and R⁶ are independently C₃-C₈-cycloalkyl, preferably cyclopropyl, cyclobutyl, cyclopentyl or cyclohexyl, in particular cyclopropyl or cyclobutyl. In a special embodiment R⁵ and R⁶ are independently cyclopropyl. In a further special embodiment R⁵ and R⁶ are independently cyclobutyl. In a further special embodiment R⁵ and R⁶ are independently cyclopentyl. In a further special embodiment R⁵ and R⁶ are independently cyclohexyl.

According to a further preferred embodiment R⁵ and R⁶ are independently C₃-C₈-cycloalkyl, substituted by one, two, three or up to the maximum possible number of identical or different groups R^{a} as defined and preferably herein.

According to a specific embodiment R⁵ and R⁶ are independently C₃-C₈-halocycloalkyl, more preferably fully or partially halogenated C₃-C⁶-cycloalkyl. In a special embodiment R⁵ and R⁶ are independently fully or partially halogenated cyclopropyl. In a further special embodiment R⁵ and R⁶ are independently 1-Cl-cyclopropyl. In a further special embodiment R⁵ and R⁶ are independently 2-Cl-cyclopropyl. In a further special embodiment R⁵ and R⁶ are independently 1-F-cyclopropyl. In a further special embodiment R⁵ and R⁶ are independently 2-F-cyclopropyl. In a further special embodiment R⁵ and R⁶ are independently fully or partially halogenated cyclobutyl. In a further special embodiment R⁵ and R⁶ are independently 1-Cl-cyclobutyl. In a further special embodiment R⁵ and R⁶ are independently 1-F-cyclobutyl. In a further special embodiment R⁵ and R⁶ are independently 2-Cl-cyclobutyl. In a further special embodiment R⁵ and R⁶ are independently 3-Cl-cyclobutyl. In a further special embodiment R⁵ and R⁶ are independently 2-F-cyclobutyl. In a further special embodiment R⁵ and R⁶ are independently 3-F-cyclobutyl. In a further special embodiment R⁵ and R⁶ are independently 3,3-(Cl)₂-cyclobutyl. In a further special embodiment R⁵ and R⁶ are independently 3,3-(F)₂-cyclobutyl. According to a specific embodiment R⁵ and R⁶ are independently C₃-C₈-cycloalkyl substituted by C₁-C₄-alkyl, more preferably is C₃-C⁶-cycloalkyl substituted by C₁-C₄-alkyl. In a special embodiment R⁵ and R⁶ are independently 1-CH₃-cyclopropyl. In a further special embodiment R⁵ and R⁶ are independently 2-CH₃-cyclopropyl. In a further special embodiment R⁵ and R⁶ are independently 1-CH₃-cyclobutyl. In a further special embodiment R⁵ and R⁶ are independently 2-CH₃-cyclobutyl. In a further special embodiment R⁵ and R⁶ are independently 3-CH₃-cyclobutyl. In a further special embodiment R⁵ and R⁶ are independently 3,3-(CH₃)₂-cyclobutyl. According to a specific embodiment R⁵ and R⁶ are independently C₃-C₈-cycloalkyl substituted by CN, more preferably is C₃-C⁶-cycloalkyl substituted by CN. In a special embodiment R⁵ and R⁶ are independently 1-CN-cyclopropyl. In a special embodiment R⁵ and R⁶ are independently 2-CN-cyclopropyl. According to a further specific embodiment R⁵ and R⁶ are independently C₃-C₈-cycloalkyl-C₃-C₈-cycloalkyl, preferably C₃-C₆-cycloalkyl-C₃-C₆-cycloalkyl. In a special embodiment R⁵ and R⁶ are independently 1-cyclopropyl-cyclopropyl. In a very special embodiment R⁵ and R⁶ are independently 2-cyclopropyl-cyclopropyl. According to a further specific embodiment R⁵ and R⁶ are independently C₃-C₈-cycloalkyl-C₃-C₈-halocycloalkyl, preferably C₃-C₆-cycloalkyl-C₃-C⁶-halocycloalkyl.

According to one another embodiment R⁵ and R⁶ are independently C₃-C₈-cycloalkyl-C₁-C₄-alkyl, preferably C₃-C⁶-cycloalkyl-C₁-C₄-alkyl. In a special embodiment R⁵ and R⁶ are independently CH(CH₃)(cyclopropyl). In a special embodiment R⁵ and R⁶ are independently CH₂-(cyclopropyl). In a special embodiment R⁵ and R⁶ are independently CH(CH₃)(cyclobutyl). In a special embodiment R⁵ and R⁶ are independently CH₂-(cyclobutyl). In a special embodiment R⁵ and R⁶ are independently CH₂CH₂-(cyclopropyl). In a special embodiment R⁵ and R⁶ are independently CH₂CH₂-(cyclobutyl).

According to a further preferred embodiment R⁵ and R⁶ are independently C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R⁵ and R⁶ are independently C₃-C₈-cycloalkyl-C₁-C₄-haloalkyl, C₃-C⁶-cycloalkyl-C₁-C₄-haloalkyl. According to a specific embodiment R⁵ and R⁶ are independently C₃-C₈-halocycloalkyl-C₁-C₄-alkyl, C₃-C⁶-halocycloalkyl-C₁-C₄-alkyl. In a special embodiment R⁵ and R⁶ are independently fully or partially halogenated cyclopropyl-C₁-C₄-alkyl. In a further special embodiment R⁵ and R⁶ are independently 1-Cl-cyclopropyl-C₁-C₄-alkyl. In a further special embodiment R⁵ and R⁶ are independently 1-F-cyclopropyl-C₁-C₄-alkyl. In a further very special embodiment R⁵ and R⁶ are independently CH₂-1-Cl-cyclopropyl. In a further very special embodiment R⁵ and R⁶ are independently CH₂-1-F-cyclopropyl. In a further very special embodiment R⁵ and R⁶ are independently CH(CH₃)-1-Cl-cyclopropyl. In a further very special embodiment R⁵ and R⁶ are independently C(CH₃)₂-1-F-cyclopropyl. In a further very special embodiment R⁵ and R⁶ are independently CH₂-1-F-cyclobutyl. In a further very special embodiment R⁵ and R⁶ are independently CH₂-1-Cl-cyclobutyl.

According to one embodiment R⁵ and R⁶ are independently phenyl.

According to a one preferred embodiment R⁵ and R⁶ are independently phenyl substituted by one, two, three or up to the maximum possible number of identical or different groups R^{b} as defined and preferably herein.

According to a specific embodiment R⁵ and R⁶ are independently phenyl substituted by one, two or three halogen atoms, preferably by one, two or three Cl or F. In a special embodiment R⁵ and R⁶ are independently 2-Cl-phenyl. In a further special embodiment R⁵ and R⁶ are independently 2-F-phenyl. In a further special embodiment R⁵ and R⁶ are independently 4-Cl-phenyl. In a further special embodiment R⁵ and R⁶ are independently 4-Cl-phenyl. In a further special embodiment R⁵ and R⁶ are independently 4-F-phenyl. In a further special embodiment R⁵ and R⁶ are independently 4-F-phenyl. In a further special embodiment R⁵ and R⁶ are independently 2,4-Cl₂-phenyl. In a further special embodiment R⁵ and R⁶ are independently 2,4-F₂-phenyl. In a further special embodiment R⁵ and R⁶ are independently 2-Cl-4-F-phenyl. In a further special embodiment R⁵ and R⁶ are independently 2-F-4-Cl-phenyl. In a further special embodiment R⁵ and R⁶ are independently 2,4,6-Cl₃-phenyl. In a further special embodiment R⁵ and R⁶ are independently 2,4,6-F₃-phenyl.

According to a specific embodiment R⁵ and R⁶ are independently phenyl substituted by one, two or three CN or OH groups. In a special embodiment R⁵ and R⁶ are independently 2-OH-phenyl. In a further special embodiment R⁵ and R⁶ are independently 4-OH-phenyl. In a further special embodiment R⁵ and R⁶ are independently 2,4-OH₂-phenyl. In a further special embodiment R⁵ and R⁶ are independently 2,4,6-OH₃-phenyl.

According to a specific embodiment R⁵ and R⁶ are independently phenyl substituted by one, two or three C₁-C₄-alkyl or C₁-C₄-haloalkyl groups, preferably methyl, ethyl, n-propyl, i-propyl, n-butyl, i-butyl, t-butyl or CFs, CHF₂, CFH₂, CCl₃, CHCl₂, CClH₂. In a special embodiment R⁵ and R⁶ are independently 2-CH₃-phenyl. In a further special embodiment R⁵ and R⁶ are independently 2-CF₃-phenyl. In a further special embodiment R⁵ and R⁶ are independently 4-CH₃-phenyl. In a further special embodiment R⁵ and R⁶ are independently 4-CF₃-phenyl.

According to a specific embodiment R⁵ and R⁶ are independently phenyl substituted by one, two or three C₁-C₄-alkoxy or C₁-C₄-haloalkoxy groups, preferably preferably C₁-C₄-alkoxy, more preferably CH₃O, CH₃CH₂O, CH₃CH₂CH₂O, CH₂(CH₃)CH₂O, CH₃CH(CH₃)O, CH₃CH₂CH₂CH₂O, CF₃O, CCl₃O. In a special embodiment R⁵ and R⁶ are independently 2-CH₃O-phenyl. In a further special embodiment R⁵ and R⁶ are independently 2-CF₃O-phenyl. In a further special embodiment R⁵ and R⁶ are independently 4-CH₃O-phenyl. In a further special embodiment R⁵ and R⁶ are independently 4-CF₃O-phenyl.

According to one embodiment R⁵ and R⁶ are independently phenyl-C₁-C₄-alkyl, preferably phenyl-C₁-C₂-alkyl. In a special embodiment R⁵ and R⁶ are independently benzyl.

According to a one preferred embodiment R⁵ and R⁶ are independently phenyl-C₁-C₄-alkyl therein the alkyl moiety can be substituted by one, two, three or up to the maximum possible number of identical or different groups R^{a} as defined and preferably herein, in particular selected from halogen, in particular Cl and F, C₁-C₄-alkoxy, in particular OCH₃, C₁-C₄-alkyl, in particular CH₃ or C₂H⁵, and CN, and phenyl can be substituted by one, two, three or up to the maximum possible number of identical or different groups R^{b} as defined and preferably herein in particular selected from halogen, in particular Cl and F, C₁-C₄-alkoxy, in particular OCH₃, C₁-C₄-alkyl, in particular CH₃ or C₂H₅, and CN. In a special embodiment R⁵ and R⁶ are independently CH₂-(4-Cl)-phenyl. In a further special embodiment R⁵ and R⁶ are independently CH₂-(4-CH₃)-phenyl. In a further special embodiment R⁵ and R⁶ are independently CH₂-(4-OCH₃)-phenyl. In a further special embodiment R⁵ and R⁶ are independently CH₂-(4-F)-phenyl. In a further special embodiment R⁵ and R⁶ are independently CH₂-(2,4-Cl₂)-phenyl. In a further special embodiment R⁵ and R⁶ are independently CH₂-(2,4-F₂)-phenyl.

According to a one preferred embodiment R⁵ and R⁶ together with the carbon atom to which they are bound form a saturated or partially unsaturated three-, four-, five-, six- or seven-membered carbocycle. In a special embodiment R⁵ and R⁶ form cyclopropyl. In a further special embodiment R⁵ and R⁶ form cyclobutyl. In a further special embodiment R⁵ and R⁶ form cyclopenyl. In a further special embodiment R⁵ and R⁶ form cyclohexyl. Further preffered are cyclopentenyl, cyclopentadienyl and cyclohexenyl.

According to a one preferred embodiment carbocycle as defined above carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S). Preferred are halogen such as Cl, Br, F, C₁-C₆-haloalkyl and C₁-C₆-alkoxy.

According to a one preferred embodiment R⁵ and R⁶ together with the carbon atom to which they are bound form a a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S). Preffered R⁵ and R⁶ form cyclopropanone, cyclopentanone, cyclopropanethione, cyclopentanethione, 5-oxazolone, cyclohexane-1,4-dione, cyclohexane-1,4-dithione, cyclohex-2-ene-1,4-dione or cyclohex-2-ene-1,4-dithione. Further preffered are oxirane, aziridine, thiirane, oxetane, azetidine, thiethane, 2-tetrahydrofuranyl, 3-tetrahydrofuranyl, 2-tetrahydrothienyl and 3-tetrahydrothienyl.

According to a one preferred embodiment R⁵ and R⁶ together with the carbon atom to which they are bound (denominated C*) form a group (C*=CR⁵⁵R⁶⁶), wherein R⁵⁵, R⁶⁶ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl.

According to a one preferred embodiment R⁵ and R⁶ together with the carbon atom to which they are bound (denominated C*) form a group (C*=CR⁵⁵R⁶⁶), wherein R⁵⁵, R⁶⁶ are independently selected from hydrogen, halogen and C₁-C₆-alkyl.

In a special embodiment R⁵ and R⁶ form C*=CH₂. In a special embodiment R⁵ and R⁶ form C*=CCl₂. In a special embodiment R⁵ and R⁶ form C*=CHCl. In a special embodiment R⁵ and R⁶ form C*=CHBr. In a special embodiment R⁵ and R⁶ form C*=CHF. In a special embodiment R⁵ and R⁶ form C*=CHCH₃. In a special embodiment R⁵ and R⁶ form C*=C(CH₃)₂. In a special embodiment R⁵ and R⁶ form C*=CH(CH₂CH₃). In a special embodiment R⁵ and R⁶ form C*=C(CH₂CH₃)₂.

R⁷ in the compounds according to the invention is, according to one embodiment, as defined in claim 1.

R⁷ in the compounds according to the invention is, according to a further embodiment, C₁-C₆-alkyl or C₃-C₆-cycloalkyl, wherein R⁷ is substituted by one, two, three or four R^{7a}; wherein R^{7a} is independently selected from halogen.

According to one embodiment o is 0. According to one embodiment o is 1. According to one further embodiment o is 2. According to one further embodiment o is 3. According to one further embodiment o is 4.

According to one further embodiment R⁷ is C₁-C₆-alkyl, preferably methyl, ethyl, n-propyl, i-propyl, n-butyl, i-butyl or t-butyl. In a special embodiment R⁷ is methyl. In a further special embodiment R⁷ is ethyl. In a further special embodiment R⁷ is n-propyl. In a further special embodiment R⁷ is i-propyl. In a further special embodiment R⁷ is 1-methylpropyl. In a further special embodiment R⁷ is n-butyl. In a further special embodiment R⁷ is i-butyl. In a further special embodiment R⁷ is t-butyl.

According to a one preferred embodiment R⁴ is C₁-C₆-alkyl substituted by one, two, three or up to the maximum possible number of identical or different groups R^{a} as defined and preferably herein. According to a specific embodiment R⁷ is C₁-C₆-haloalkyl, more preferably fully or partially halogenated methyl, ethyl, n-propyl, i-propyl, n-butyl, i-butyl or t-butyl. In a special embodiment R⁷ is CF₃. In a further special embodiment R⁷ is CHF₂. In a further special embodiment R⁷ is CFH₂. In a further special embodiment R⁷ is CCl₃. In a further special embodiment R⁷ is CHCl₂. In a further special embodiment R⁷ is CClH₂. According to a further specific embodiment R⁷ is C₁-C₆-alkyl, preferably C₁-C₇-alkyl substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R⁷ is CH₂OH. According to a further specific embodiment R⁷ is C₁-C₇-alkoxy-C₁-C₆-alkyl, more preferably C₁-C ₇-alkoxy-C₁-C₇-alkyl. In a special embodiment R⁷ is CH₂OCH₃. In a further special embodiment R ⁷ is CH₂CH₂OCH₃. In a further special embodiment R⁷ is CH(CH₃)OCH₃. In a further special embodiment R⁷ is CH(CH₃)OCH₂CH_{3.} In a further special embodiment R⁷ is CH₂CH₂OCH₂CH₃. According to a further preferred embodiment R⁷ is C₂-C₆-alkenyl, substituted by one, two, three or up to the maximum possible number of identical or different groups R^{a} as defined and preferably herein.

According to a specific embodiment R⁷ is C₂-C₆-haloalkenyl, more preferably fully or partially halogenated C₂-C₆-alkenyl. In a special embodiment R⁷ is fully or partially halogenated C₂-alkenyl. In a further special embodiment R⁷ is fully or partially halogenated C₃-alkenyl. According to a further specific embodiment R⁷ is C₂-C₆-alkenyl, preferably C₂-C₇-alkenyl, substituted by OH, more preferably, CH=CHOH, CH=CHCH₂OH, C(CH₃)=CHOH, CH=C(CH₃)OH. In a special embodiment R⁷ is CH=CHOH. In a further special embodiment R⁷ is CH=CHCH₂OH. According to a further specific embodiment R⁷ is C₁-C₇-alkoxy-C₂-C₆-alkenyl, more preferably C₁-C₇-alkoxy-C₂-C₇-alkenyl. In a special embodiment R⁷ is CH=CHOCH₃. In a further special embodiment R⁷ is CH=CHCH₂OCH₃.

According to one another embodiment R⁷ is C₂-C₆-alkynyl, preferably CCH, CH₂CCH, CH₂CCCH₃. In a special embodiment R⁷ is CCH. in a further special embodiment R⁷ is CCCH₃. In a further special embodiment R⁷ is CH₂CCH. In a further special embodiment R⁷ is CH₂CCCH₃. In a further special embodiment R⁷ is CH₂CCH₂CH₃.

According to a further preferred embodiment R⁷ is C₂-C₆-alkynyl, substituted by one, two, three or up to the maximum possible number of identical or different groups R^{a} as defined and preferably herein.

According to a specific embodiment R⁷ is C₂-C₆-haloalkynyl, more preferably fully or partially halogenated C₂-C₆-alkynyl. In a special embodiment R⁷ is fully or partially halogenated C₂-alkynyl. In a further special embodiment R⁷ is fully or partially halogenated C₃-alkynyl. According to a further specific embodiment R⁷ is C₂-C₆-alkynyl, preferably C₂-C₇-alkynyl, substituted by OH, more preferably, CCOH, CH₂CCOH. In a special embodiment R⁷ is CCOH. In a further special embodiment R⁷ is CH₂CCOH. According to a further specific embodiment R⁷ is C₁-C₇-alkoxy-C₂-C₆-alkynyl, more preferably C₁-C₇-alkoxy-C₂-C₇-alkynyl. In a special embodiment R⁷ is CCOCH₃. In a further special embodiment R⁷ is CH₂CCOCH₃.

According to one another embodiment R⁷ is C₃-C₈-cycloalkyl, preferably cyclopropyl, cyclobutyl, cyclopentyl or cyclohexyl, in particular cyclopropyl or cyclobutyl. In a special embodiment R⁷ is cyclopropyl. In a further special embodiment R⁷ is cyclobutyl. In a further special embodiment R⁷ is cyclopentyl. In a further special embodiment R⁷ is cyclohexyl.

According to one another embodiment R⁷ is C₃-C₈-cycloalkoxy, preferably C₃-C₆-cycloalkoxy. In a special embodiment R⁷ is O-cyclopropyl.

According to a further preferred embodiment R⁷ is C₃-C₈-cycloalkyl, substituted by one, two, three or up to the maximum possible number of identical or different groups R^{a} as defined and preferably herein.

According to a specific embodiment R⁷ is C₃-C₈-halocycloalkyl, more preferably fully or partially halogenated C₃-C₆-cycloalkyl. In a special embodiment R⁷ is fully or partially halogenated cyclopropyl. In a further special embodiment R⁷ is 1-Cl-cyclopropyl. In a further special embodiment R⁷ is 2-Cl-cyclopropyl. In a further special embodiment R⁷ is 1-F-cyclopropyl. In a further special embodiment R⁷ is 2-F-cyclopropyl. In a further special embodiment R⁷ is fully or partially halogenated cyclobutyl. In a further special embodiment R⁷ is 1-Cl-cyclobutyl. In a further special embodiment R⁷ is 1-F-cyclobutyl. In a further special embodiment R⁷ is 3,3-(Cl)₂-cyclobutyl. In a further special embodiment R⁷ is 3,3-(F)₂-cyclobutyl.

According to one another embodiment two substituents R⁷: R⁷¹ and R⁷² together with the carbon atom(s) to which they are bound form a saturated three-, four-, five-, six- or seven-membered carbocycle or heterocycle, wherein the heterocycle contains one, two, three or four O atoms. According to a one preferred embodiment R⁷¹ and R⁷² together with the carbon atom to which they are bound form a saturated or partially unsaturated three-, four-, five-, six- or seven-membered carbocycle. In a special embodiment R⁵ and R⁶ form cyclopropyl. In a further special embodiment R⁵ and R⁶ form cyclobutyl. In a further special embodiment R⁵ and R⁶ form cyclopenyl. In a further special embodiment R⁵ and R⁶ form cyclohexyl. Further preffered are cyclopentenyl, cyclopentadienyl and cyclohexenyl.

According to a one preferred embodiment R⁷¹ and R⁷² together with the carbon atom to which they are bound form a a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S). Preffered R⁷¹ and R⁷² form cyclopropanone, cyclopentanone, cyclopropanethione, cyclopentanethione, 5-oxazolone, cyclohexane-1,4-dione, cyclohexane-1,4-dithione, cyclohex-2-ene-1,4-dione or cyclohex-2-ene-1,4-dithione. Further preffered are oxirane, aziridine, thiirane, oxetane, azetidine, thiethane, 2-tetrahydrofuranyl, 3-tetrahydrofuranyl, 2-tetrahydrothienyl and 3-tetrahydrothienyl.

According to one embodiment, the present invention relates to compounds of the formulae I.A to I.H

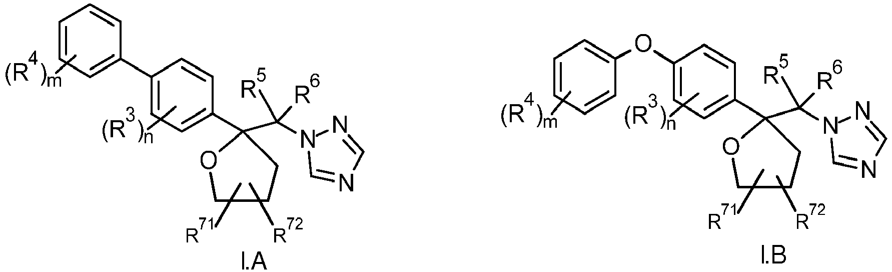

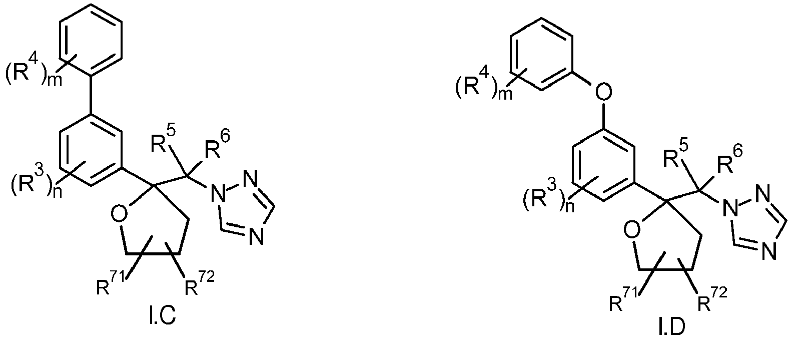

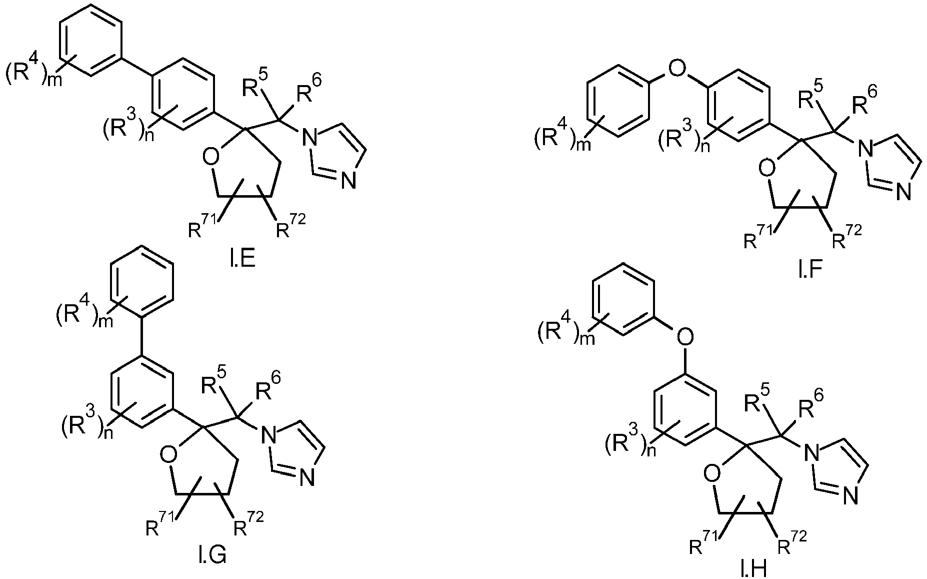

Here, the variables are as defined elsewhere herein for formula I, or as defined as being preferred for formula I.

According to one embodiment, the present invention relates to compounds of the formulae II.A to II.H

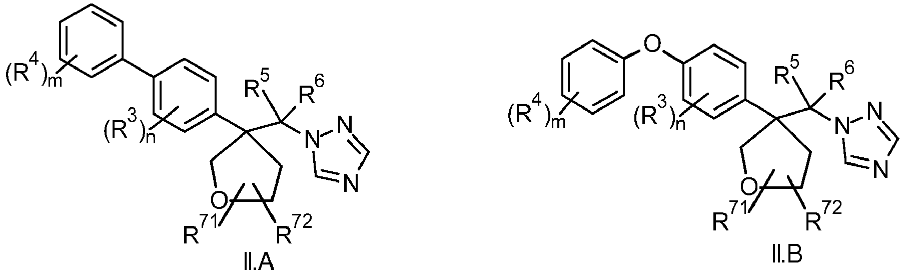

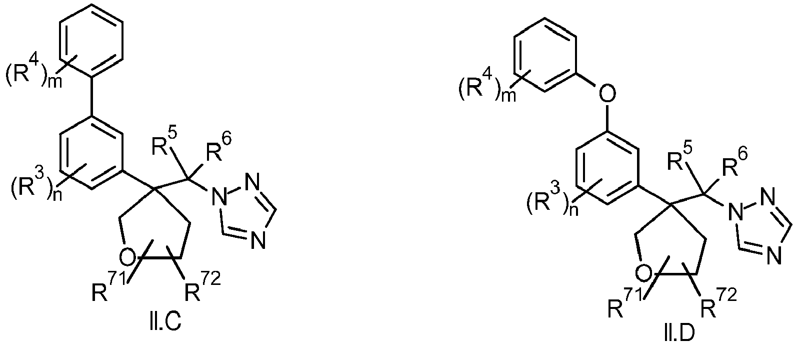

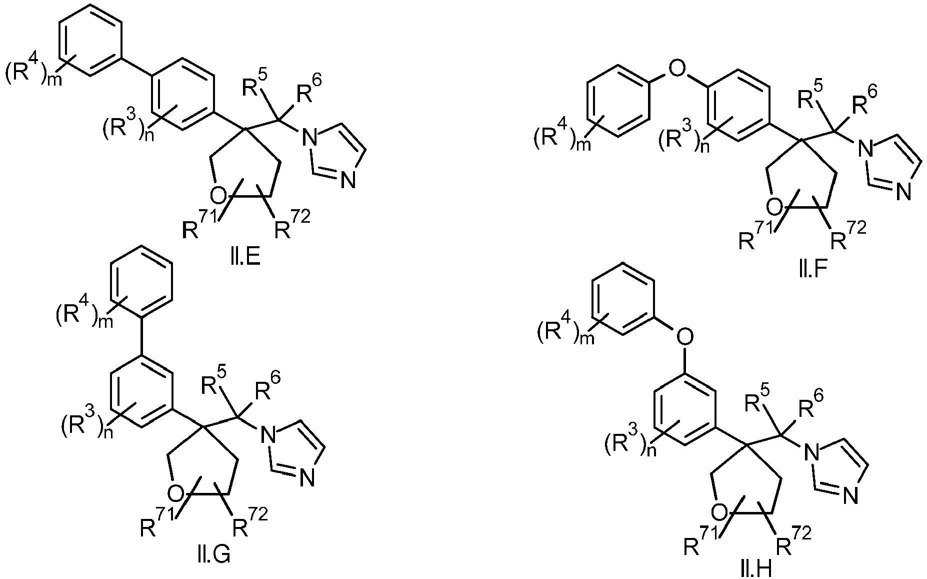

Here, the variables are as defined elsewhere herein for formula I, or as defined as being preferred for formula I.

Preference is given to the compounds I according to the invention compiled in Tables 1a-I to 18a-I, 1a-II to 18a-II below with the proviso as defined above. The groups mentioned for a substituent in the tables are furthermore per se, independently of the combination in which they are mentioned, a particularly preferred aspect of the substituent in question.

### Table 1a-I

Compounds of the formula I.A, I.B. I.C, I.D, I.E, I.F, I.G, I.H in which the combination of R⁵ and R⁶ corresponds to line A-1 of Table A and the meaning for the combination of (R³)ₙ, (R⁴)ₘ, R⁷¹ and R⁷² for each individual compound corresponds in each case to one line of Table B (compounds I.A.A1.B1 to I.A.A1.B2340, compounds I.B.A1.B1 to I.B.A1.B2340; compounds I.C.A1.B1 to I.C.A1.B2340; compounds I.D.A1.B1 to I.D.A1.B2340; compounds I.E.A1.B1 to I.E.A1.B2340; compounds I.F.A1.B1 to I.F.A1.B2340; compounds I.G.A1.B1 to I.G.A1.B2340; compounds I.H.A1.B1 to I.H.A1.B2340).

### Table 2a-I

Compounds of the formula I.A, I.B. I.C, I.D, I.E, I.F, I.G, I.H in which the combination of R⁵ and R⁶ corresponds to line A-2 of Table A and the meaning for the combination of (R³)ₙ, (R⁴)ₘ, R⁷¹ and R⁷² for each individual compound corresponds in each case to one line of Table B (compounds I.A.A2.B1 to I.A.A2.B2340, compounds I.B.A2.B1 to I.B.A2.B2340; compounds I.C.A2.B1 to I.C.A2.B2340; compounds I.D.A2.B1 to I.D.A2.B2340; compounds I.E.A2.B1 to I.E.A2.B2340; compounds I.F.A2.B1 to I.F.A2.B2340; compounds I.G.A2.B1 to I.G.A2.B2340; compounds I.H.A2.B1 to I.H.A2.B2340).

### Table 3a-I

Compounds of the formula I.A, I.B. I.C, I.D, I.E, I.F, I.G, I.H in which the combination of R⁵ and R⁶ corresponds to line A-3 of Table A and the meaning for the combination of (R³)ₙ, (R⁴)ₘ, R⁷¹ and R⁷² for each individual compound corresponds in each case to one line of Table B (compounds I.A.A3.B1 to I.A.A3.B2340, compounds I.B.A3.B1 to I.B.A3.B2340; compounds I.C.A3.B1 to I.C.A3.B2340; compounds I.D.A3.B1 to I.D.A3.B2340; compounds I.E.A3.B1 to I.E.A3.B2340; compounds I.F.A3.B1 to I.F.A3.B2340; compounds I.G.A3.B1 to I.G.A3.B2340; compounds I.H.A3.B1 to I.H.A3.B2340).

### Table 4a-I

Compounds of the formula I.A, I.B. I.C, I.D, I.E, I.F, I.G, I.H in which the combination of R⁵ and R⁶ corresponds to line A-4 of Table A and the meaning for the combination of (R³)ₙ, (R⁴)ₘ, R⁷¹ and R⁷² for each individual compound corresponds in each case to one line of Table B (compounds I.A.A4.B1 to I.A.A4.B2340, compounds I.B.A4.B1 to I.B.A4.B2340; compounds I.C.A4.B1 to I.C.A4.B2340; compounds I.D.A4.B1 to I.D.A4.B2340; compounds I.E.A4.B1 to I.E.A4.B2340; compounds I.F.A4.B1 to I.F.A4.B2340; compounds I.G.A4.B1 to I.G.A4.B2340; compounds I.H.A4.B1 to I.H.A4.B2340).

### Table 5a-I

Compounds of the formula I.A, I.B. I.C, I.D, I.E, I.F, I.G, I.H in which the combination of R⁵ and R⁶ corresponds to line A-5 of Table A and the meaning for the combination of (R³)ₙ, (R⁴)ₘ, R⁷¹ and R⁷² for each individual compound corresponds in each case to one line of Table B (compounds I.A.A5.B1 to I.A.A5.B2340, compounds I.B.A5.B1 to I.B.A5.B2340; compounds I.C.A5.B1 to I.C.A5.B2340; compounds I.D.A5.B1 to I.D.A5.B2340; compounds I.E.A5.B1 to I.E.A5.B2340; compounds I.F.A5.B1 to I.F.A5.B2340; compounds I.G.A5.B1 to I.G.A5.B2340; compounds I.H.A5.B1 to I.H.A5.B2340).

### Table 6a-I

Compounds of the formula I.A, I.B. I.C, I.D, I.E, I.F, I.G, I.H in which the combination of R⁵ and R⁶ corresponds to line A-6 of Table A and the meaning for the combination of (R³)ₙ, (R⁴)ₘ, R⁷¹ and R⁷² for each individual compound corresponds in each case to one line of Table B (compounds I.A.A6.B1 to I.A.A6.B2340, compounds I.B.A6.B1 to I.B.A6.B2340; compounds I.C.A6.B1 to I.C.A6.B2340; compounds I.D.A6.B1 to I.D.A6.B2340; compounds I.E.A6.B1 to I.E.A6.B2340; compounds I.F.A6.B1 to I.F.A6.B2340; compounds I.G.A6.B1 to I.G.A6.B2340; compounds I.H.A6.B1 to I.H.A6.B2340).

### Table 7a-I

Compounds of the formula I.A, I.B. I.C, I.D, I.E, I.F, I.G, I.H in which the combination of R⁵ and R⁶ corresponds to line A-7 of Table A and the meaning for the combination of (R³)ₙ, (R⁴)ₘ, R⁷¹ and R⁷² for each individual compound corresponds in each case to one line of Table B (compounds I.A.A7.B1 to I.A.A7.B2340, compounds I.B.A7.B1 to I.B.A7.B2340; compounds I.C.A7.B1 to I.C.A7.B2340; compounds I.D.A7.B1 to I.D.A7.B2340; compounds I.E.A7.B1 to I.E.A7.B2340; compounds I.F.A7.B1 to I.F.A7.B2340; compounds I.G.A7.B1 to I.G.A7.B2340; compounds I.H.A7.B1 to I.H.A7.B2340).

### Table 8a-I

Compounds of the formula I.A, I.B. I.C, I.D, I.E, I.F, I.G, I.H in which the combination of R⁵ and R⁶ corresponds to line A-8 of Table A and the meaning for the combination of (R³)ₙ, (R⁴)ₘ, R⁷¹ and R⁷² for each individual compound corresponds in each case to one line of Table B (compounds I.A.A8.B1 to I.A.A8.B2340, compounds I.B.A8.B1 to I.B.A8.B2340; compounds I.C.A8.B1 to I.C.A8.B2340; compounds I.D.A8.B1 to I.D.A8.B2340; compounds I.E.A8.B1 to I.E.A8.B2340; compounds I.F.A8.B1 to I.F.A8.B2340; compounds I.G.A8.B1 to I.G.A8.B2340; compounds I.H.A8.B1 to I.H.A8.B2340).

### Table 9a-I

Compounds of the formula I.A, I.B. I.C, I.D, I.E, I.F, I.G, I.H in which the combination of R⁵ and R⁶ corresponds to line A-9 of Table A and the meaning for the combination of (R³)ₙ, (R⁴)ₘ, R⁷¹ and R⁷² for each individual compound corresponds in each case to one line of Table B (compounds I.A.A9.B1 to I.A.A9.B2340, compounds I.B.A9.B1 to I.B.A9.B2340; compounds I.C.A9.B1 to I.C.A9.B2340; compounds I.D.A9.B1 to I.D.A9.B2340; compounds I.E.A9.B1 to I.E.A9.B2340; compounds I.F.A9.B1 to I.F.A9.B2340; compounds I.G.A9.B1 to I.G.A9.B2340; compounds I.H.A9.B1 to I.H.A9.B2340).

### Table 10a-I

Compounds of the formula I.A, I.B. I.C, I.D, I.E, I.F, I.G, I.H in which the combination of R⁵ and R⁶ corresponds to line A-10 of Table A and the meaning for the combination of (R³)ₙ, (R⁴)ₘ, R⁷¹ and R⁷² for each individual compound corresponds in each case to one line of Table B (compounds I.A.A10.B1 to I.A.A10.B2340, compounds I.B.A10.B1 to I.B.A10.B2340; compounds I.C.A10.B1 to I.C.A10.B2340; compounds I.D.A10.B1 to I.D.A10.B2340; compounds I.E.A10.B1 to I.E.A10.B2340; compounds I.F.A10.B1 to I.F.A10.B2340; compounds I.G.A10.B1 to I.G.A10.B2340; compounds I.H.A10.B1 to I.H.A10.B2340).

### Table 11a-I

Compounds of the formula I.A, I.B. I.C, I.D, I.E, I.F, I.G, I.H in which the combination of R⁵ and R⁶ corresponds to line A-11 of Table A and the meaning for the combination of (R³)ₙ, (R⁴)ₘ, R⁷¹ and R⁷² for each individual compound corresponds in each case to one line of Table B (compounds I.A.A11.B1 to I.A.A11.B2340, compounds I.B.A11.B1 to I.B.A11.B2340; compounds I.C.A11.B1 to I.C.A11.B2340; compounds I.D.A11.B1 to I.D.A11.B2340; compounds I.E.A11.B1 to I.E.A11.B2340; compounds I.F.A11.B1 to I.F.A11.B2340; compounds I.G.A11.B1 to I.G.A11.B2340; compounds I.H.A11.B1 to I.H.A11.B2340).

### Table 12a-I

Compounds of the formula I.A, I.B. I.C, I.D, I.E, I.F, I.G, I.H in which the combination of R⁵ and R⁶ corresponds to line A-12 of Table A and the meaning for the combination of (R³)ₙ, (R⁴)ₘ, R⁷¹ and R⁷² for each individual compound corresponds in each case to one line of Table B (compounds I.A.A12.B1 to I.A.A12.B2340, compounds I.B.A12.B1 to I.B.A12.B2340; compounds I.C.A12.B1 to I.C.A12.B2340; compounds I.D.A12.B1 to I.D.A12.B2340; compounds I.E.A12.B1 to I.E.A12.B2340; compounds I.F.A12.B1 to I.F.A12.B2340; compounds I.G.A12.B1 to I.G.A12.B2340; compounds I.H.A12.B1 to I.H.A12.B2340).

### Table 13a-I

Compounds of the formula I.A, I.B. I.C, I.D, I.E, I.F, I.G, I.H in which the combination of R⁵ and R⁶ corresponds to line A-13 of Table A and the meaning for the combination of (R³)ₙ, (R⁴)ₘ, R⁷¹ and R⁷² for each individual compound corresponds in each case to one line of Table B (compounds I.A.A13.B1 to I.A.A13.B2340, compounds I.B.A13.B1 to I.B.A13.B2340; compounds I.C.A13.B1 to I.C.A13.B2340; compounds I.D.A13.B1 to I.D.A13.B2340; compounds I.E.A13.B1 to I.E.A13.B2340; compounds I.F.A13.B1 to I.F.A13.B2340; compounds I.G.A13.B1 to I.G.A13.B2340; compounds I.H.A13.B1 to I.H.A13.B2340).

### Table 14a-I

Compounds of the formula I.A, I.B. I.C, I.D, I.E, I.F, I.G, I.H in which the combination of R⁵ and R⁶ corresponds to line A-114 of Table A and the meaning for the combination of (R³)ₙ, (R⁴)ₘ, R⁷¹ and R⁷² for each individual compound corresponds in each case to one line of Table B (compounds I.A.A14.B1 to I.A.A14.B2340, compounds I.B.A14.B1 to I.B.A14.B2340; compounds I.C.A14.B1 to I.C.A14.B2340; compounds I.D.A14.B1 to I.D.A14.B2340; compounds I.E.A14.B1 to I.E.A14.B2340; compounds I.F.A14.B1 to I.F.A14.B2340; compounds I.G.A14.B1 to I.G.A14.B2340; compounds I.H.A14.B1 to I.H.A14.B2340).

### Table 15a-I

Compounds of the formula I.A, I.B. I.C, I.D, I.E, I.F, I.G, I.H in which the combination of R⁵ and R⁶ corresponds to line A-15 of Table A and the meaning for the combination of (R³)ₙ, (R⁴)ₘ, R⁷¹ and R⁷² for each individual compound corresponds in each case to one line of Table B (compounds I.A.A15.B1 to I.A.A15.B2340, compounds I.B.A15.B1 to I.B.A15.B2340; compounds I.C.A15.B1 to I.C.A15.B2340; compounds I.D.A15.B1 to I.D.A15.B2340; compounds I.E.A15.B1 to I.E.A15.B2340; compounds I.F.A15.B1 to I.F.A15.B2340; compounds I.G.A15.B1 to I.G.A15.B2340; compounds I.H.A15.B1 to I.H.A15.B2340).

### Table 16a-I

Compounds of the formula I.A, I.B. I.C, I.D, I.E, I.F, I.G, I.H in which the combination of R⁵ and R⁶ corresponds to line A-16 of Table A and the meaning for the combination of (R³)ₙ, (R⁴)ₘ, R⁷¹ and R⁷² for each individual compound corresponds in each case to one line of Table B (compounds I.A.A16.B1 to I.A.A16.B2340, compounds I.B.A16.B1 to I.B.A16.B2340; compounds I.C.A16.B1 to I.C.A16.B2340; compounds I.D.A16.B1 to I.D.A16.B2340; compounds I.E.A16.B1 to I.E.A16.B2340; compounds I.F.A16.B1 to I.F.A16.B2340; compounds I.G.A16.B1 to I.G.A16.B2340; compounds I.H.A16.B1 to I.H.A16.B2340).

### Table 17a-I

Compounds of the formula I.A, I.B. I.C, I.D, I.E, I.F, I.G, I.H in which the combination of R⁵ and R⁶ corresponds to line A-17 of Table A and the meaning for the combination of (R³)ₙ, (R⁴)ₘ, R⁷¹ and R⁷² for each individual compound corresponds in each case to one line of Table B (compounds I.A.A17.B1 to I.A.A17.B2340, compounds I.B.A17.B1 to I.B.A17.B2340; compounds I.C.A17.B1 to I.C.A17.B2340; compounds I.D.A17.B1 to I.D.A17.B2340; compounds I.E.A17.B1 to I.E.A17.B2340; compounds I.F.A17.B1 to I.F.A17.B2340; compounds I.G.A17.B1 to I.G.A17.B2340; compounds I.H.A17.B1 to I.H.A17.B2340).

### Table 18a-I

Compounds of the formula I.A, I.B. I.C, I.D, I.E, I.F, I.G, I.H in which the combination of R⁵ and R⁶ corresponds to line A-18 of Table A and the meaning for the combination of (R³)ₙ, (R⁴)ₘ, R⁷¹ and R⁷² for each individual compound corresponds in each case to one line of Table B (compounds I.A.A18.B1 to I.A.A18.B2340, compounds I.B.A18.B1 to I.B.A18.B2340; compounds I.C.A18.B1 to I.C.A18.B2340; compounds I.D.A18.B1 to I.D.A18.B2340; compounds I.E.A18.B1 to I.E.A18.B2340; compounds I.F.A18.B1 to I.F.A18.B2340; compounds I.G.A18.B1 to I.G.A18.B2340; compounds I.H.A18.B1 to I.H.A18.B2340).

### Table 1a-II

Compounds of the formula II.A, II.B. II.C, II.D, II.E, II.F, II.G, II.H in which the combination of R⁵ and R⁶ corresponds to line A-1 of Table A and the meaning for the combination of (R³)ₙ, (R⁴)ₘ, R⁷¹ and R⁷² for each individual compound corresponds in each case to one line of Table B (compounds II.A.A1.B1 to II.A.A1.B2340, compounds II.B.A1.B1 to II.B.A1.B2340; compounds II.C.A1.B1 to II.C.A1.B2340; compounds II.D.A1.B1 to II.D.A1.B2340; compounds II.E.A1.B1 to II.E.A1.B2340; compounds II.F.A1.B1 to II.F.A1.B2340; compounds II.G.A1.B1 to II.G.A1.B2340; compounds II.H.A1.B1 to II.H.A1.B2340).

### Table 2a-II

Compounds of the formula II.A, II.B. II.C, II.D, II.E, II.F, II.G, II.H in which the combination of R⁵ and R⁶ corresponds to line A-2 of Table A and the meaning for the combination of (R³)ₙ, (R⁴)ₘ, R⁷¹ and R⁷² for each individual compound corresponds in each case to one line of Table B (compounds II.A.A2.B1 to II.A.A2.B2340, compounds II.B.A2.B1 to II.B.A2.B2340; compounds II.C.A2.Bl to II.C.A2.B2340; compounds II.D.A2.B1 to II.D.A2.B2340; compounds II.E.A2.Bl to II.E.A2.B2340; compounds II.F.A2.B1 to II.F.A2.B2340; compounds II.G.A2.B1 to II.G.A2.B2340; compounds II.H.A2.Bl to II.H.A2.B2340).

### Table 3a-II

Compounds of the formula II.A, II.B. II.C, II.D, II.E, II.F, II.G, II.H in which the combination of R⁵ and R⁶ corresponds to line A-3 of Table A and the meaning for the combination of (R³)ₙ, (R⁴)ₘ, R⁷¹ and R⁷² for each individual compound corresponds in each case to one line of Table B (compounds II.A.A3.B1 to II.A.A3.B2340, compounds II.B.A3.B1 to II.B.A3.B2340; compounds II.C.A3.B1 to II.C.A3.B2340; compounds II.D.A3.B1 to II.D.A3.B2340; compounds II.E.A3.B1 to II.E.A3.B2340; compounds II.F.A3.B1 to II.F.A3.B2340; compounds II.G.A3.B1 to II.G.A3.B2340; compounds II.H.A3.B1 to II.H.A3.B2340).

### Table 4a-II

Compounds of the formula II.A, II.B. II.C, II.D, II.E, II.F, II.G, II.H in which the combination of R⁵ and R⁶ corresponds to line A-4 of Table A and the meaning for the combination of (R³)ₙ, (R⁴)ₘ, R⁷¹ and R⁷² for each individual compound corresponds in each case to one line of Table B (compounds II.A.A4.B1 to II.A.A4.B2340, compounds II.B.A4.B1 to II.B.A4.B2340; compounds II.C.A4.B1 to II.C.A4.B2340; compounds II.D.A4.B1 to II.D.A4.B2340; compounds II.E.A4.B1 to II.E.A4.B2340; compounds II.F.A4.B1 to II.F.A4.B2340; compounds II.G.A4.B1 to II.G.A4.B2340; compounds II.H.A4.B1 to II.H.A4.B2340).

### Table 5a-II

Compounds of the formula II.A, II.B. II.C, II.D, II.E, II.F, II.G, II.H in which the combination of R⁵ and R⁶ corresponds to line A-5 of Table A and the meaning for the combination of (R³)ₙ, (R⁴)ₘ, R⁷¹ and R⁷² for each individual compound corresponds in each case to one line of Table B (compounds II.A.A5.B1 to II.A.A5.B2340, compounds II.B.A5.B1 to II.B.A5.B2340; compounds II.C.A5.B1 to II.C.A5.B2340; compounds II.D.A5.B1 to II.D.A5.B2340; compounds II.E.A5.B1 to II.E.A5.B2340; compounds II.F.A5.B1 to II.F.A5.B2340; compounds II.G.A5.B1 to II.G.A5.B2340; compounds II.H.A5.B1 to II.H.A5.B2340).

### Table 6a-II

Compounds of the formula II.A, II.B. II.C, II.D, II.E, II.F, II.G, II.H in which the combination of R⁵ and R⁶ corresponds to line A-6 of Table A and the meaning for the combination of (R³)ₙ, (R⁴)ₘ, R⁷¹ and R⁷² for each individual compound corresponds in each case to one line of Table B (compounds II.A.A6.B1 to II.A.A6.B2340, compounds II.B.A6.B1 to II.B.A6.B2340; compounds II.C.A6.B1 to II.C.A6.B2340; compounds II.D.A6.B1 to II.D.A6.B2340; compounds II.E.A6.B1 to II.E.A6.B2340; compounds II.F.A6.B1 to II.F.A6.B2340; compounds II.G.A6.B1 to II.G.A6.B2340; compounds II.H.A6.B1 to II.H.A6.B2340).

### Table 7a-II

Compounds of the formula II.A, II.B. II.C, II.D, II.E, II.F, II.G, II.H in which the combination of R⁵ and R⁶ corresponds to line A-7 of Table A and the meaning for the combination of (R³)ₙ, (R⁴)ₘ, R⁷¹ and R⁷² for each individual compound corresponds in each case to one line of Table B (compounds II.A.A7.B1 to II.A.A7.B2340, compounds II.B.A7.B1 to II.B.A7.B2340; compounds II.C.A7.B1 to II.C.A7.B2340; compounds II.D.A7.B1 to II.D.A7.B2340; compounds II.E.A7.B1 to II.E.A7.B2340; compounds II.F.A7.B1 to II.F.A7.B2340; compounds II.G.A7.B1 to II.G.A7.B2340; compounds II.H.A7.B1 to II.H.A7.B2340).

### Table 8a-II

Compounds of the formula II.A, II.B. II.C, II.D, II.E, II.F, II.G, II.H in which the combination of R⁵ and R⁶ corresponds to line A-8 of Table A and the meaning for the combination of (R³)ₙ, (R⁴)ₘ, R⁷¹ and R⁷² for each individual compound corresponds in each case to one line of Table B (compounds II.A.A8.B1 to II.A.A8.B2340, compounds II.B.A8.B1 to II.B.A8.B2340; compounds II.C.A8.B1 to II.C.A8.B2340; compounds II.D.A8.B1 to II.D.A8.B2340; compounds II.E.A8.B1 to II.E.A8.B2340; compounds II.F.A8.B1 to II.F.A8.B2340; compounds II.G.A8.B1 to II.G.A8.B2340; compounds II.H.A8.B1 to II.H.A8.B2340).

### Table 9a-II

Compounds of the formula II.A, II.B. II.C, II.D, II.E, II.F, II.G, II.H in which the combination of R⁵ and R⁶ corresponds to line A-9 of Table A and the meaning for the combination of (R³)ₙ, (R⁴)ₘ, R⁷¹ and R⁷² for each individual compound corresponds in each case to one line of Table B (compounds II.A.A9.B1 to II.A.A9.B2340, compounds II.B.A9.B1 to II.B.A9.B2340; compounds II.C.A9.B1 to II.C.A9.B2340; compounds II.D.A9.B1 to II.D.A9.B2340; compounds II.E.A9.B1 to II.E.A9.B2340; compounds II.F.A9.B1 to II.F.A9.B2340; compounds II.G.A9.B1 to II.G.A9.B2340; compounds II.H.A9.B1 to II.H.A9.B2340).

### Table 10a-II

Compounds of the formula II.A, II.B. II.C, II.D, II.E, II.F, II.G, II.H in which the combination of R⁵ and R⁶ corresponds to line A-10 of Table A and the meaning for the combination of (R³)ₙ, (R⁴)ₘ, R⁷¹ and R⁷² for each individual compound corresponds in each case to one line of Table B (compounds II.A.A10.B1 to II.A.A10.B2340, compounds II.B.A10.B1 to II.B.A10.B2340; compounds II.C.A10.B1 to II.C.A10.B2340; compounds II.D.A10.B1 to II.D.A10.B2340; compounds II.E.A10.B1 to II.E.A10.B2340; compounds II.F.A10.B1 to II.F.A10.B2340; compounds II.G.A10.B1 to II.G.A10.B2340; compounds II.H.A10.B1 to II.H.A10.B2340).

### Table 11a-II

Compounds of the formula II.A, II.B. II.C, II.D, II.E, II.F, II.G, II.H in which the combination of R⁵ and R⁶ corresponds to line A-11 of Table A and the meaning for the combination of (R³)ₙ, (R⁴)ₘ, R⁷¹ and R⁷² for each individual compound corresponds in each case to one line of Table B (compounds II.A.A11.B1 to II.A.A11.B2340, compounds II.B.A11.B1 to II.B.A11.B2340; compounds II.C.A11.B1 to II.C.A11.B2340; compounds II.D.A11.B1 to II.D.A11.B2340; compounds II.E.A11.B1 to II.E.A11.B2340; compounds II.F.A11.B1 to II.F.A11.B2340; compounds II.G.A11.B1 to II.G.A11.B2340; compounds II.H.A11.B1 to II.H.A11.B2340).

### Table 12a-II

Compounds of the formula II.A, II.B. II.C, II.D, II.E, II.F, II.G, II.H in which the combination of R⁵ and R⁶ corresponds to line A-12 of Table A and the meaning for the combination of (R³)ₙ, (R⁴)ₘ, R⁷¹ and R⁷² for each individual compound corresponds in each case to one line of Table B (compounds II.A.A12.B1 to II.A.A12.B2340, compounds II.B.A12.B1 to II.B.A12.B2340; compounds II.C.A12.B1 to II.C.A12.B2340; compounds II.D.A12.B1 to II.D.A12.B2340; compounds II.E.A12.B1 to II.E.A12.B2340; compounds II.F.A12.B1 to II.F.A12.B2340; compounds II.G.A12.B1 to II.G.A12.B2340; compounds II.H.A12.B1 to II.H.A12.B2340).

### Table 13a-II

Compounds of the formula II.A, II.B. II.C, II.D, II.E, II.F, II.G, II.H in which the combination of R⁵ and R⁶ corresponds to line A-13 of Table A and the meaning for the combination of (R³)ₙ, (R⁴)ₘ, R⁷¹ and R⁷² for each individual compound corresponds in each case to one line of Table B (compounds II.A.A13.B1 to II.A.A13.B2340, compounds II.B.A13.B1 to II.B.A13.B2340; compounds II.C.A13.B1 to II.C.A13.B2340; compounds II.D.A13.B1 to II.D.A13.B2340; compounds II.E.A13.B1 to II.E.A13.B2340; compounds II.F.A13.B1 to II.F.A13.B2340; compounds II.G.A13.B1 to II.G.A13.B2340; compounds II.H.A13.B1 to II.H.A13.B2340).

### Table 14a-II

Compounds of the formula II.A, II.B. II.C, II.D, II.E, II.F, II.G, II.H in which the combination of R⁵ and R⁶ corresponds to line A-114 of Table A and the meaning for the combination of (R³)ₙ, (R⁴)ₘ, R⁷¹ and R⁷² for each individual compound corresponds in each case to one line of Table B (compounds II.A.A14.B1 to II.A.A14.B2340, compounds II.B.A14.B1 to II.B.A14.B2340; compounds II.C.A14.B1 to II.C.A14.B2340; compounds II.D.A14.B1 to II.D.A14.B2340; compounds II.E.A14.B1 to II.E.A14.B2340; compounds II.F.A14.B1 to II.F.A14.B2340; compounds II.G.A14.B1 to II.G.A14.B2340; compounds II.H.A14.B1 to II.H.A14.B2340).

### Table 15a-II

Compounds of the formula II.A, II.B. II.C, II.D, II.E, II.F, II.G, II.H in which the combination of R⁵ and R⁶ corresponds to line A-15 of Table A and the meaning for the combination of (R³)ₙ, (R⁴)ₘ, R⁷¹ and R⁷² for each individual compound corresponds in each case to one line of Table B (compounds II.A.A15.B1 to II.A.A15.B2340, compounds II.B.A15.B1 to II.B.A15.B2340; compounds II.C.A15.B1 to II.C.A15.B2340; compounds II.D.A15.B1 to II.D.A15.B2340; compounds II.E.A15.B1 to II.E.A15.B2340; compounds II.F.A15.B1 to II.F.A15.B2340; compounds II.G.A15.B1 to II.G.A15.B2340; compounds II.H.A15.B1 to II.H.A15.B2340).

### Table 16a-II

Compounds of the formula II.A, II.B. II.C, II.D, II.E, II.F, II.G, II.H in which the combination of R⁵ and R⁶ corresponds to line A-16 of Table A and the meaning for the combination of (R³)ₙ, (R⁴)ₘ, R⁷¹ and R⁷² for each individual compound corresponds in each case to one line of Table B (compounds II.A.A16.B1 to II.A.A16.B2340, compounds II.B.A16.B1 to II.B.A16.B2340; compounds II.C.A16.B1 to II.C.A16.B2340; compounds II.D.A16.B1 to II.D.A16.B2340; compounds II.E.A16.B1 to II.E.A16.B2340; compounds II.F.A16.B1 to II.F.A16.B2340; compounds II.G.A16.B1 to II.G.A16.B2340; compounds II.H.A16.B1 to II.H.A16.B2340).

### Table 17a-II

Compounds of the formula II.A, II.B. II.C, II.D, II.E, II.F, II.G, II.H in which the combination of R⁵ and R⁶ corresponds to line A-17 of Table A and the meaning for the combination of (R³)ₙ, (R⁴)ₘ, R⁷¹ and R⁷² for each individual compound corresponds in each case to one line of Table B (compounds II.A.A17.B1 to II.A.A17.B2340, compounds II.B.A17.B1 to II.B.A17.B2340; compounds II.C.A17.B1 to II.C.A17.B2340; compounds II.D.A17.B1 to II.D.A17.B2340; compounds II.E.A17.B1 to II.E.A17.B2340; compounds II.F.A17.B1 to II.F.A17.B2340; compounds II.G.A17.B1 to II.G.A17.B2340; compounds II.H.A17.B1 to II.H.A17.B2340).

### Table 18a-II

Compounds of the formula II.A, II.B. II.C, II.D, II.E, II.F, II.G, II.H in which the combination of R⁵ and R⁶ corresponds to line A-18 of Table A and the meaning for the combination of (R³)ₙ, (R⁴)ₘ, R⁷¹ and R⁷² for each individual compound corresponds in each case to one line of Table B (compounds II.A.A18.B1 to II.A.A18.B2340, compounds II.B.A18.B1 to II.B.A18.B2340; compounds II.C.A18.B1 to II.C.A18.B2340; compounds II.D.A18.B1 to II.D.A18.B2340; compounds II.E.A18.B1 to II.E.A18.B2340; compounds II.F.A18.B1 to II.F.A18.B2340; compounds II.G.A18.B1 to II.G.A18.B2340; compounds II.H.A18.B1 to II.H.A18.B2340).

**Table A:**

| **line** | **R⁵** | **R⁶** |
|---|---|---|
| A-1 | H | H |
| A-2 | Cl | H |
| A-3 | Br | H |
| A-4 | F | H |
| A-5 | CH₃ | H |
| A-6 | CH₂CH₃ | H |
| A-7 | CH₂CH₂CH₃ | H |
| A-8 | CH(CH₃)₂ | H |
| A-9 | OCH₃ | H |
| A-10 | OCH₂CH₃ | H |
| A-11 | OCH(CH₃)₂ | H |
| A-12 | CH₃ | CH₃ |
| A-13 | CH₂CH₃ | CH₂CH₃ |
| A-14 | =CH₂ | |
| A-15 | cyclopropyl | |
| A-16 | cyclobutyl | |
| A-17 | cyclopentyl | |
| A-18 | cyclohexyl | |

**Table B**

| **line** | **(R⁴)ₘ** | **(R³)ₙ** | **R⁷¹** | **R⁷²** |
|---|---|---|---|---|
| B-1 | H | H | CH₃ | H |
| B-2 | 2-F | H | CH₃ | H |
| B-3 | 3-F | H | CH₃ | H |
| B-4 | 4-F | H | CH₃ | H |
| B-5 | 2-Cl | H | CH₃ | H |
| B-6 | 3-Cl | H | CH₃ | H |
| B-7 | 4-Cl | H | CH₃ | H |
| B-8 | 2-CF₃ | H | CH₃ | H |
| B-9 | 3-CF₃ | H | CH₃ | H |
| B-10 | 4-CF₃ | H | CH₃ | H |
| B-11 | 2,4-F₂ | H | CH₃ | H |
| B-12 | 2,4-Cl₂ | H | CH₃ | H |
| B-13 | 2,6-Cl₂ | H | CH₃ | H |
| B-14 | 2-CN | H | CH₃ | H |
| B-15 | 3-CN | H | CH₃ | H |
| B-16 | 4-CN | H | CH₃ | H |
| B-17 | 2-F-4-CN | H | CH₃ | H |
| B-18 | 2-Cl-4-CN | H | CH₃ | H |
| B-19 | 2-Cl-4-CF3 | H | CH₃ | H |
| B-20 | 2-F-4-CF3 | H | CH₃ | H |
| B-21 | H | 2-Cl | CH₃ | H |
| B-22 | 2-F | 2-Cl | CH₃ | H |
| B-23 | 3-F | 2-Cl | CH₃ | H |
| B-24 | 4-F | 2-Cl | CH₃ | H |
| B-25 | 2-Cl | 2-Cl | CH₃ | H |
| B-26 | 3-Cl | 2-Cl | CH₃ | H |
| B-27 | 4-Cl | 2-Cl | CH₃ | H |
| B-28 | 2-CF₃ | 2-Cl | CH₃ | H |
| B-29 | 3-CF₃ | 2-Cl | CH₃ | H |
| B-30 | 4-CF₃ | 2-Cl | CH₃ | H |
| B-31 | 2,4-F₂ | 2-Cl | CH₃ | H |
| B-32 | 2,4-Cl₂ | 2-Cl | CH₃ | H |
| B-33 | 2,6-Cl₂ | 2-Cl | CH₃ | H |
| B-34 | 2-CN | 2-Cl | CH₃ | H |
| B-35 | 3-CN | 2-Cl | CH₃ | H |
| B-36 | 4-CN | 2-Cl | CH₃ | H |
| B-37 | 2-F-4-CN | 2-Cl | CH₃ | H |
| B-38 | 2-Cl-4-CN | 2-Cl | CH₃ | H |
| B-39 | 2-Cl-4-CF3 | 2-Cl | CH₃ | H |
| B-40 | 2-F-4-CF3 | 2-Cl | CH₃ | H |
| B-41 | H | 6-Cl | CH₃ | H |
| B-42 | 2-F | 6-Cl | CH₃ | H |
| B-43 | 3-F | 6-Cl | CH₃ | H |
| B-44 | 4-F | 6-Cl | CH₃ | H |
| B-45 | 2-Cl | 6-Cl | CH₃ | H |
| B-46 | 3-Cl | 6-Cl | CH₃ | H |
| B-47 | 4-Cl | 6-Cl | CH₃ | H |
| B-48 | 2-CF₃ | 6-Cl | CH₃ | H |
| B-49 | 3-CF₃ | 6-Cl | CH₃ | H |
| B-50 | 4-CF₃ | 6-Cl | CH₃ | H |
| B-51 | 2,4-F₂ | 6-Cl | CH₃ | H |
| B-52 | 2,4-Cl₂ | 6-Cl | CH₃ | H |
| B-53 | 2,6-Cl₂ | 6-Cl | CH₃ | H |
| B-54 | 2-CN | 6-Cl | CH₃ | H |
| B-55 | 3-CN | 6-Cl | CH₃ | H |
| B-56 | 4-CN | 6-Cl | CH₃ | H |
| B-57 | 2-F-4-CN | 6-Cl | CH₃ | H |
| B-58 | 2-Cl-4-CN | 6-Cl | CH₃ | H |
| B-59 | 2-Cl-4-CF3 | 6-Cl | CH₃ | H |
| B-60 | 2-F-4-CF3 | 6-Cl | CH₃ | H |
| B-61 | H | 2-Br | CH₃ | H |
| B-62 | 2-F | 2-Br | CH₃ | H |
| B-63 | 3-F | 2-Br | CH₃ | H |
| B-64 | 4-F | 2-Br | CH₃ | H |
| B-65 | 2-Cl | 2-Br | CH₃ | H |
| B-66 | 3-Cl | 2-Br | CH₃ | H |
| B-67 | 4-Cl | 2-Br | CH₃ | H |
| B-68 | 2-CF₃ | 2-Br | CH₃ | H |
| B-69 | 3-CF₃ | 2-Br | CH₃ | H |
| B-70 | 4-CF₃ | 2-Br | CH₃ | H |
| B-71 | 2,4-F₂ | 2-Br | CH₃ | H |
| B-72 | 2,4-Cl₂ | 2-Br | CH₃ | H |
| B-73 | 2,6-Cl₂ | 2-Br | CH₃ | H |
| B-74 | 2-CN | 2-Br | CH₃ | H |
| B-75 | 3-CN | 2-Br | CH₃ | H |
| B-76 | 4-CN | 2-Br | CH₃ | H |
| B-77 | 2-F-4-CN | 2-Br | CH₃ | H |
| B-78 | 2-Cl-4-CN | 2-Br | CH₃ | H |
| B-79 | 2-Cl-4-CF3 | 2-Br | CH₃ | H |
| B-80 | 2-F-4-CF3 | 2-Br | CH₃ | H |
| B-81 | H | 6-Br | CH₃ | H |
| B-82 | 2-F | 6-Br | CH₃ | H |
| B-83 | 3-F | 6-Br | CH₃ | H |
| B-84 | 4-F | 6-Br | CH₃ | H |
| B-85 | 2-Cl | 6-Br | CH₃ | H |
| B-86 | 3-Cl | 6-Br | CH₃ | H |
| B-87 | 4-Cl | 6-Br | CH₃ | H |
| B-88 | 2-CF₃ | 6-Br | CH₃ | H |
| B-89 | 3-CF₃ | 6-Br | CH₃ | H |
| B-90 | 4-CF₃ | 6-Br | CH₃ | H |
| B-91 | 2,4-F₂ | 6-Br | CH₃ | H |
| B-92 | 2,4-Cl₂ | 6-Br | CH₃ | H |
| B-93 | 2,6-Cl₂ | 6-Br | CH₃ | H |
| B-94 | 2-CN | 6-Br | CH₃ | H |
| B-95 | 3-CN | 6-Br | CH₃ | H |
| B-96 | 4-CN | 6-Br | CH₃ | H |
| B-97 | 2-F-4-CN | 6-Br | CH₃ | H |
| B-98 | 2-Cl-4-CN | 6-Br | CH₃ | H |
| B-99 | 2-Cl-4-CF3 | 6-Br | CH₃ | H |
| B-100 | 2-F-4-CF3 | 6-Br | CH₃ | H |
| B-101 | H | 2-F | CH₃ | H |
| B-102 | 2-F | 2-F | CH₃ | H |
| B-103 | 3-F | 2-F | CH₃ | H |
| B-104 | 4-F | 2-F | CH₃ | H |
| B-105 | 2-Cl | 2-F | CH₃ | H |
| B-106 | 3-Cl | 2-F | CH₃ | H |
| B-107 | 4-Cl | 2-F | CH₃ | H |
| B-108 | 2-CF₃ | 2-F | CH₃ | H |
| B-109 | 3-CF₃ | 2-F | CH₃ | H |
| B-110 | 4-CF₃ | 2-F | CH₃ | H |
| B-111 | 2,4-F₂ | 2-F | CH₃ | H |
| B-112 | 2,4-Cl₂ | 2-F | CH₃ | H |
| B-113 | 2,6-Cl₂ | 2-F | CH₃ | H |
| B-114 | 2-CN | 2-F | CH₃ | H |
| B-115 | 3-CN | 2-F | CH₃ | H |
| B-116 | 4-CN | 2-F | CH₃ | H |
| B-117 | 2-F-4-CN | 2-F | CH₃ | H |
| B-118 | 2-Cl-4-CN | 2-F | CH₃ | H |
| B-119 | 2-Cl-4-CF3 | 2-F | CH₃ | H |
| B-120 | 2-F-4-CF3 | 2-F | CH₃ | H |
| B-121 | H | 6-F | CH₃ | H |
| B-122 | 2-F | 6-F | CH₃ | H |
| B-123 | 3-F | 6-F | CH₃ | H |
| B-124 | 4-F | 6-F | CH₃ | H |
| B-125 | 2-Cl | 6-F | CH₃ | H |
| B-126 | 3-Cl | 6-F | CH₃ | H |
| B-127 | 4-Cl | 6-F | CH₃ | H |
| B-128 | 2-CF₃ | 6-F | CH₃ | H |
| B-129 | 3-CF₃ | 6-F | CH₃ | H |
| B-130 | 4-CF₃ | 6-F | CH₃ | H |
| B-131 | 2,4-F₂ | 6-F | CH₃ | H |
| B-132 | 2,4-Cl₂ | 6-F | CH₃ | H |
| B-133 | 2,6-Cl₂ | 6-F | CH₃ | H |
| B-134 | 2-CN | 6-F | CH₃ | H |
| B-135 | 3-CN | 6-F | CH₃ | H |
| B-136 | 4-CN | 6-F | CH₃ | H |
| B-137 | 2-F-4-CN | 6-F | CH₃ | H |
| B-138 | 2-Cl-4-CN | 6-F | CH₃ | H |
| B-139 | 2-Cl-4-CF3 | 6-F | CH₃ | H |
| B-140 | 2-F-4-CF3 | 6-F | CH₃ | H |
| B-141 | H | 2-CN | CH₃ | H |
| B-142 | 2-F | 2-CN | CH₃ | H |
| B-143 | 3-F | 2-CN | CH₃ | H |
| B-144 | 4-F | 2-CN | CH₃ | H |
| B-145 | 2-Cl | 2-CN | CH₃ | H |
| B-146 | 3-Cl | 2-CN | CH₃ | H |
| B-147 | 4-Cl | 2-CN | CH₃ | H |
| B-148 | 2-CF₃ | 2-CN | CH₃ | H |
| B-149 | 3-CF₃ | 2-CN | CH₃ | H |
| B-150 | 4-CF₃ | 2-CN | CH₃ | H |
| B-151 | 2,4-F₂ | 2-CN | CH₃ | H |
| B-152 | 2,4-Cl₂ | 2-CN | CH₃ | H |
| B-153 | 2,6-Cl₂ | 2-CN | CH₃ | H |
| B-154 | 2-CN | 2-CN | CH₃ | H |
| B-155 | 3-CN | 2-CN | CH₃ | H |
| B-156 | 4-CN | 2-CN | CH₃ | H |
| B-157 | 2-F-4-CN | 2-CN | CH₃ | H |
| B-158 | 2-Cl-4-CN | 2-CN | CH₃ | H |
| B-159 | 2-Cl-4-CF3 | 2-CN | CH₃ | H |
| B-160 | 2-F-4-CF3 | 2-CN | CH₃ | H |
| B-161 | H | 6-CN | CH₃ | H |
| B-162 | 2-F | 6-CN | CH₃ | H |
| B-163 | 3-F | 6-CN | CH₃ | H |
| B-164 | 4-F | 6-CN | CH₃ | H |
| B-165 | 2-Cl | 6-CN | CH₃ | H |
| B-166 | 3-Cl | 6-CN | CH₃ | H |
| B-167 | 4-Cl | 6-CN | CH₃ | H |
| B-168 | 2-CF₃ | 6-CN | CH₃ | H |
| B-169 | 3-CF₃ | 6-CN | CH₃ | H |
| B-170 | 4-CF₃ | 6-CN | CH₃ | H |
| B-171 | 2,4-F₂ | 6-CN | CH₃ | H |
| B-172 | 2,4-Cl₂ | 6-CN | CH₃ | H |
| B-173 | 2,6-Cl₂ | 6-CN | CH₃ | H |
| B-174 | 2-CN | 6-CN | CH₃ | H |
| B-175 | 3-CN | 6-CN | CH₃ | H |
| B-176 | 4-CN | 6-CN | CH₃ | H |
| B-177 | 2-F-4-CN | 6-CN | CH₃ | H |
| B-178 | 2-Cl-4-CN | 6-CN | CH₃ | H |
| B-179 | 2-Cl-4-CF3 | 6-CN | CH₃ | H |
| B-180 | 2-F-4-CF3 | 6-CN | CH₃ | H |
| B-181 | H | 2-CF₃ | CH₃ | H |
| B-182 | 2-F | 2-CF₃ | CH₃ | H |
| B-183 | 3-F | 2-CF₃ | CH₃ | H |
| B-184 | 4-F | 2-CF₃ | CH₃ | H |
| B-185 | 2-Cl | 2-CF₃ | CH₃ | H |
| B-186 | 3-Cl | 2-CF₃ | CH₃ | H |
| B-187 | 4-Cl | 2-CF₃ | CH₃ | H |
| B-188 | 2-CF₃ | 2-CF₃ | CH₃ | H |
| B-189 | 3-CF₃ | 2-CF₃ | CH₃ | H |
| B-190 | 4-CF₃ | 2-CF₃ | CH₃ | H |
| B-191 | 2,4-F₂ | 2-CF₃ | CH₃ | H |
| B-192 | 2,4-Cl₂ | 2-CF₃ | CH₃ | H |
| B-193 | 2,6-Cl₂ | 2-CF₃ | CH₃ | H |
| B-194 | 2-CN | 2-CF₃ | CH₃ | H |
| B-195 | 3-CN | 2-CF₃ | CH₃ | H |
| B-196 | 4-CN | 2-CF₃ | CH₃ | H |
| B-197 | 2-F-4-CN | 2-CF₃ | CH₃ | H |
| B-198 | 2-Cl-4-CN | 2-CF₃ | CH₃ | H |
| B-199 | 2-Cl-4-CF3 | 2-CF₃ | CH₃ | H |
| B-200 | 2-F-4-CF3 | 2-CF₃ | CH₃ | H |
| B-201 | H | 6-CF₃ | CH₃ | H |
| B-202 | 2-F | 6-CF₃ | CH₃ | H |
| B-203 | 3-F | 6-CF₃ | CH₃ | H |
| B-204 | 4-F | 6-CF₃ | CH₃ | H |
| B-205 | 2-Cl | 6-CF₃ | CH₃ | H |
| B-206 | 3-Cl | 6-CF₃ | CH₃ | H |
| B-207 | 4-Cl | 6-CF₃ | CH₃ | H |
| B-208 | 2-CF₃ | 6-CF₃ | CH₃ | H |
| B-209 | 3-CF₃ | 6-CF₃ | CH₃ | H |
| B-210 | 4-CF₃ | 6-CF₃ | CH₃ | H |
| B-211 | 2,4-F₂ | 6-CF₃ | CH₃ | H |
| B-212 | 2,4-Cl₂ | 6-CF₃ | CH₃ | H |
| B-213 | 2,6-Cl₂ | 6-CF₃ | CH₃ | H |
| B-214 | 2-CN | 6-CF₃ | CH₃ | H |
| B-215 | 3-CN | 6-CF₃ | CH₃ | H |
| B-216 | 4-CN | 6-CF₃ | CH₃ | H |
| B-217 | 2-F-4-CN | 6-CF₃ | CH₃ | H |
| B-218 | 2-Cl-4-CN | 6-CF₃ | CH₃ | H |
| B-219 | 2-Cl-4-CF3 | 6-CF₃ | CH₃ | H |
| B-220 | 2-F-4-CF3 | 6-CF₃ | CH₃ | H |
| B-221 | H | 2-OCH₃ | CH₃ | H |
| B-222 | 2-F | 2-OCH₃ | CH₃ | H |
| B-223 | 3-F | 2-OCH₃ | CH₃ | H |
| B-224 | 4-F | 2-OCH₃ | CH₃ | H |
| B-225 | 2-Cl | 2-OCH₃ | CH₃ | H |
| B-226 | 3-Cl | 2-OCH₃ | CH₃ | H |
| B-227 | 4-Cl | 2-OCH₃ | CH₃ | H |
| B-228 | 2-CF₃ | 2-OCH₃ | CH₃ | H |
| B-229 | 3-CF₃ | 2-OCH₃ | CH₃ | H |
| B-230 | 4-CF₃ | 2-OCH₃ | CH₃ | H |
| B-231 | 2,4-F₂ | 2-OCH₃ | CH₃ | H |
| B-232 | 2,4-Cl₂ | 2-OCH₃ | CH₃ | H |
| B-233 | 2,6-Cl₂ | 2-OCH₃ | CH₃ | H |
| B-234 | 2-CN | 2-OCH₃ | CH₃ | H |
| B-235 | 3-CN | 2-OCH₃ | CH₃ | H |
| B-236 | 4-CN | 2-OCH₃ | CH₃ | H |
| B-237 | 2-F-4-CN | 2-OCH₃ | CH₃ | H |
| B-238 | 2-Cl-4-CN | 2-OCH₃ | CH₃ | H |
| B-239 | 2-Cl-4-CF3 | 2-OCH₃ | CH₃ | H |
| B-240 | 2-F-4-CF3 | 2-OCH₃ | CH₃ | H |
| B-241 | H | 6-OCH₃ | CH₃ | H |
| B-242 | 2-F | 6-OCH₃ | CH₃ | H |
| B-243 | 3-F | 6-OCH₃ | CH₃ | H |
| B-244 | 4-F | 6-OCH₃ | CH₃ | H |
| B-245 | 2-Cl | 6-OCH₃ | CH₃ | H |
| B-246 | 3-Cl | 6-OCH₃ | CH₃ | H |
| B-247 | 4-Cl | 6-OCH₃ | CH₃ | H |
| B-248 | 2-CF₃ | 6-OCH₃ | CH₃ | H |
| B-249 | 3-CF₃ | 6-OCH₃ | CH₃ | H |
| B-250 | 4-CF₃ | 6-OCH₃ | CH₃ | H |
| B-251 | 2,4-F₂ | 6-OCH₃ | CH₃ | H |
| B-252 | 2,4-Cl₂ | 6-OCH₃ | CH₃ | H |
| B-253 | 2,6-Cl₂ | 6-OCH₃ | CH₃ | H |
| B-254 | 2-CN | 6-OCH₃ | CH₃ | H |
| B-255 | 3-CN | 6-OCH₃ | CH₃ | H |
| B-256 | 4-CN | 6-OCH₃ | CH₃ | H |
| B-257 | 2-F-4-CN | 6-OCH₃ | CH₃ | H |
| B-258 | 2-Cl-4-CN | 6-OCH₃ | CH₃ | H |
| B-259 | 2-Cl-4-CF3 | 6-OCH₃ | CH₃ | H |
| B-260 | 2-F-4-CF3 | 6-OCH₃ | CH₃ | H |
| B-261 | H | H | CH₂CH₃ | H |
| B-262 | 2-F | H | CH₂CH₃ | H |
| B-263 | 3-F | H | CH₂CH₃ | H |
| B-264 | 4-F | H | CH₂CH₃ | H |
| B-265 | 2-Cl | H | CH₂CH₃ | H |
| B-266 | 3-Cl | H | CH₂CH₃ | H |
| B-267 | 4-Cl | H | CH₂CH₃ | H |
| B-268 | 2-CF₃ | H | CH₂CH₃ | H |
| B-269 | 3-CF₃ | H | CH₂CH₃ | H |
| B-270 | 4-CF₃ | H | CH₂CH₃ | H |
| B-271 | 2,4-F₂ | H | CH₂CH₃ | H |
| B-272 | 2,4-Cl₂ | H | CH₂CH₃ | H |
| B-273 | 2,6-Cl₂ | H | CH₂CH₃ | H |
| B-274 | 2-CN | H | CH₂CH₃ | H |
| B-275 | 3-CN | H | CH₂CH₃ | H |
| B-276 | 4-CN | H | CH₂CH₃ | H |
| B-277 | 2-F-4-CN | H | CH₂CH₃ | H |
| B-278 | 2-Cl-4-CN | H | CH₂CH₃ | H |
| B-279 | 2-Cl-4-CF3 | H | CH₂CH₃ | H |
| B-280 | 2-F-4-CF3 | H | CH₂CH₃ | H |
| B-281 | H | 2-Cl | CH₂CH₃ | H |
| B-282 | 2-F | 2-Cl | CH₂CH₃ | H |
| B-283 | 3-F | 2-Cl | CH₂CH₃ | H |
| B-284 | 4-F | 2-Cl | CH₂CH₃ | H |
| B-285 | 2-Cl | 2-Cl | CH₂CH₃ | H |
| B-286 | 3-Cl | 2-Cl | CH₂CH₃ | H |
| B-287 | 4-Cl | 2-Cl | CH₂CH₃ | H |
| B-288 | 2-CF₃ | 2-Cl | CH₂CH₃ | H |
| B-289 | 3-CF₃ | 2-Cl | CH₂CH₃ | H |
| B-290 | 4-CF₃ | 2-Cl | CH₂CH₃ | H |
| B-291 | 2,4-F₂ | 2-Cl | CH₂CH₃ | H |
| B-292 | 2,4-Cl₂ | 2-Cl | CH₂CH₃ | H |
| B-293 | 2,6-Cl₂ | 2-Cl | CH₂CH₃ | H |
| B-294 | 2-CN | 2-Cl | CH₂CH₃ | H |
| B-295 | 3-CN | 2-Cl | CH₂CH₃ | H |
| B-296 | 4-CN | 2-Cl | CH₂CH₃ | H |
| B-297 | 2-F-4-CN | 2-Cl | CH₂CH₃ | H |
| B-298 | 2-Cl-4-CN | 2-Cl | CH₂CH₃ | H |
| B-299 | 2-Cl-4-CF3 | 2-Cl | CH₂CH₃ | H |
| B-300 | 2-F-4-CF3 | 2-Cl | CH₂CH₃ | H |
| B-301 | H | 6-Cl | CH₂CH₃ | H |
| B-302 | 2-F | 6-Cl | CH₂CH₃ | H |
| B-303 | 3-F | 6-Cl | CH₂CH₃ | H |
| B-304 | 4-F | 6-Cl | CH₂CH₃ | H |
| B-305 | 2-Cl | 6-Cl | CH₂CH₃ | H |
| B-306 | 3-Cl | 6-Cl | CH₂CH₃ | H |
| B-307 | 4-Cl | 6-Cl | CH₂CH₃ | H |
| B-308 | 2-CF₃ | 6-Cl | CH₂CH₃ | H |
| B-309 | 3-CF₃ | 6-Cl | CH₂CH₃ | H |
| B-310 | 4-CF₃ | 6-Cl | CH₂CH₃ | H |
| B-311 | 2,4-F₂ | 6-Cl | CH₂CH₃ | H |
| B-312 | 2,4-Cl₂ | 6-Cl | CH₂CH₃ | H |
| B-313 | 2,6-Cl₂ | 6-Cl | CH₂CH₃ | H |
| B-314 | 2-CN | 6-Cl | CH₂CH₃ | H |
| B-315 | 3-CN | 6-Cl | CH₂CH₃ | H |
| B-316 | 4-CN | 6-Cl | CH₂CH₃ | H |
| B-317 | 2-F-4-CN | 6-Cl | CH₂CH₃ | H |
| B-318 | 2-Cl-4-CN | 6-Cl | CH₂CH₃ | H |
| B-319 | 2-Cl-4-CF3 | 6-Cl | CH₂CH₃ | H |
| B-320 | 2-F-4-CF3 | 6-Cl | CH₂CH₃ | H |
| B-321 | H | 2-Br | CH₂CH₃ | H |
| B-322 | 2-F | 2-Br | CH₂CH₃ | H |
| B-323 | 3-F | 2-Br | CH₂CH₃ | H |
| B-324 | 4-F | 2-Br | CH₂CH₃ | H |
| B-325 | 2-Cl | 2-Br | CH₂CH₃ | H |
| B-326 | 3-Cl | 2-Br | CH₂CH₃ | H |
| B-327 | 4-Cl | 2-Br | CH₂CH₃ | H |
| B-328 | 2-CF₃ | 2-Br | CH₂CH₃ | H |
| B-329 | 3-CF₃ | 2-Br | CH₂CH₃ | H |
| B-330 | 4-CF₃ | 2-Br | CH₂CH₃ | H |
| B-331 | 2,4-F₂ | 2-Br | CH₂CH₃ | H |
| B-332 | 2,4-Cl₂ | 2-Br | CH₂CH₃ | H |
| B-333 | 2,6-Cl₂ | 2-Br | CH₂CH₃ | H |
| B-334 | 2-CN | 2-Br | CH₂CH₃ | H |
| B-335 | 3-CN | 2-Br | CH₂CH₃ | H |
| B-336 | 4-CN | 2-Br | CH₂CH₃ | H |
| B-337 | 2-F-4-CN | 2-Br | CH₂CH₃ | H |
| B-338 | 2-Cl-4-CN | 2-Br | CH₂CH₃ | H |
| B-339 | 2-Cl-4-CF3 | 2-Br | CH₂CH₃ | H |
| B-340 | 2-F-4-CF3 | 2-Br | CH₂CH₃ | H |
| B-341 | H | 6-Br | CH₂CH₃ | H |
| B-342 | 2-F | 6-Br | CH₂CH₃ | H |
| B-343 | 3-F | 6-Br | CH₂CH₃ | H |
| B-344 | 4-F | 6-Br | CH₂CH₃ | H |
| B-345 | 2-Cl | 6-Br | CH₂CH₃ | H |
| B-346 | 3-Cl | 6-Br | CH₂CH₃ | H |
| B-347 | 4-Cl | 6-Br | CH₂CH₃ | H |
| B-348 | 2-CF₃ | 6-Br | CH₂CH₃ | H |
| B-349 | 3-CF₃ | 6-Br | CH₂CH₃ | H |
| B-350 | 4-CF₃ | 6-Br | CH₂CH₃ | H |
| B-351 | 2,4-F₂ | 6-Br | CH₂CH₃ | H |
| B-352 | 2,4-Cl₂ | 6-Br | CH₂CH₃ | H |
| B-353 | 2,6-Cl₂ | 6-Br | CH₂CH₃ | H |
| B-354 | 2-CN | 6-Br | CH₂CH₃ | H |
| B-355 | 3-CN | 6-Br | CH₂CH₃ | H |
| B-356 | 4-CN | 6-Br | CH₂CH₃ | H |
| B-357 | 2-F-4-CN | 6-Br | CH₂CH₃ | H |
| B-358 | 2-Cl-4-CN | 6-Br | CH₂CH₃ | H |
| B-359 | 2-Cl-4-CF3 | 6-Br | CH₂CH₃ | H |
| B-360 | 2-F-4-CF3 | 6-Br | CH₂CH₃ | H |
| B-361 | H | 2-F | CH₂CH₃ | H |
| B-362 | 2-F | 2-F | CH₂CH₃ | H |
| B-363 | 3-F | 2-F | CH₂CH₃ | H |
| B-364 | 4-F | 2-F | CH₂CH₃ | H |
| B-365 | 2-Cl | 2-F | CH₂CH₃ | H |
| B-366 | 3-Cl | 2-F | CH₂CH₃ | H |
| B-367 | 4-Cl | 2-F | CH₂CH₃ | H |
| B-368 | 2-CF₃ | 2-F | CH₂CH₃ | H |
| B-369 | 3-CF₃ | 2-F | CH₂CH₃ | H |
| B-370 | 4-CF₃ | 2-F | CH₂CH₃ | H |
| B-371 | 2,4-F₂ | 2-F | CH₂CH₃ | H |
| B-372 | 2,4-Cl₂ | 2-F | CH₂CH₃ | H |
| B-373 | 2,6-Cl₂ | 2-F | CH₂CH₃ | H |
| B-374 | 2-CN | 2-F | CH₂CH₃ | H |
| B-375 | 3-CN | 2-F | CH₂CH₃ | H |
| B-376 | 4-CN | 2-F | CH₂CH₃ | H |
| B-377 | 2-F-4-CN | 2-F | CH₂CH₃ | H |
| B-378 | 2-Cl-4-CN | 2-F | CH₂CH₃ | H |
| B-379 | 2-Cl-4-CF3 | 2-F | CH₂CH₃ | H |
| B-380 | 2-F-4-CF3 | 2-F | CH₂CH₃ | H |
| B-381 | H | 6-F | CH₂CH₃ | H |
| B-382 | 2-F | 6-F | CH₂CH₃ | H |
| B-383 | 3-F | 6-F | CH₂CH₃ | H |
| B-384 | 4-F | 6-F | CH₂CH₃ | H |
| B-385 | 2-Cl | 6-F | CH₂CH₃ | H |
| B-386 | 3-Cl | 6-F | CH₂CH₃ | H |
| B-387 | 4-Cl | 6-F | CH₂CH₃ | H |
| B-388 | 2-CF₃ | 6-F | CH₂CH₃ | H |
| B-389 | 3-CF₃ | 6-F | CH₂CH₃ | H |
| B-390 | 4-CF₃ | 6-F | CH₂CH₃ | H |
| B-391 | 2,4-F₂ | 6-F | CH₂CH₃ | H |
| B-392 | 2,4-Cl₂ | 6-F | CH₂CH₃ | H |
| B-393 | 2,6-Cl₂ | 6-F | CH₂CH₃ | H |
| B-394 | 2-CN | 6-F | CH₂CH₃ | H |
| B-395 | 3-CN | 6-F | CH₂CH₃ | H |
| B-396 | 4-CN | 6-F | CH₂CH₃ | H |
| B-397 | 2-F-4-CN | 6-F | CH₂CH₃ | H |
| B-398 | 2-Cl-4-CN | 6-F | CH₂CH₃ | H |
| B-399 | 2-Cl-4-CF3 | 6-F | CH₂CH₃ | H |
| B-400 | 2-F-4-CF3 | 6-F | CH₂CH₃ | H |
| B-401 | H | 2-CN | CH₂CH₃ | H |
| B-402 | 2-F | 2-CN | CH₂CH₃ | H |
| B-403 | 3-F | 2-CN | CH₂CH₃ | H |
| B-404 | 4-F | 2-CN | CH₂CH₃ | H |
| B-405 | 2-Cl | 2-CN | CH₂CH₃ | H |
| B-406 | 3-Cl | 2-CN | CH₂CH₃ | H |
| B-407 | 4-Cl | 2-CN | CH₂CH₃ | H |
| B-408 | 2-CF₃ | 2-CN | CH₂CH₃ | H |
| B-409 | 3-CF₃ | 2-CN | CH₂CH₃ | H |
| B-410 | 4-CF₃ | 2-CN | CH₂CH₃ | H |
| B-411 | 2,4-F₂ | 2-CN | CH₂CH₃ | H |
| B-412 | 2,4-Cl₂ | 2-CN | CH₂CH₃ | H |
| B-413 | 2,6-Cl₂ | 2-CN | CH₂CH₃ | H |
| B-414 | 2-CN | 2-CN | CH₂CH₃ | H |
| B-415 | 3-CN | 2-CN | CH₂CH₃ | H |
| B-416 | 4-CN | 2-CN | CH₂CH₃ | H |
| B-417 | 2-F-4-CN | 2-CN | CH₂CH₃ | H |
| B-418 | 2-Cl-4-CN | 2-CN | CH₂CH₃ | H |
| B-419 | 2-Cl-4-CF3 | 2-CN | CH₂CH₃ | H |
| B-420 | 2-F-4-CF3 | 2-CN | CH₂CH₃ | H |
| B-421 | H | 6-CN | CH₂CH₃ | H |
| B-422 | 2-F | 6-CN | CH₂CH₃ | H |
| B-423 | 3-F | 6-CN | CH₂CH₃ | H |
| B-424 | 4-F | 6-CN | CH₂CH₃ | H |
| B-425 | 2-Cl | 6-CN | CH₂CH₃ | H |
| B-426 | 3-Cl | 6-CN | CH₂CH₃ | H |
| B-427 | 4-Cl | 6-CN | CH₂CH₃ | H |
| B-428 | 2-CF₃ | 6-CN | CH₂CH₃ | H |
| B-429 | 3-CF₃ | 6-CN | CH₂CH₃ | H |
| B-430 | 4-CF₃ | 6-CN | CH₂CH₃ | H |
| B-431 | 2,4-F₂ | 6-CN | CH₂CH₃ | H |
| B-432 | 2,4-Cl₂ | 6-CN | CH₂CH₃ | H |
| B-433 | 2,6-Cl₂ | 6-CN | CH₂CH₃ | H |
| B-434 | 2-CN | 6-CN | CH₂CH₃ | H |
| B-435 | 3-CN | 6-CN | CH₂CH₃ | H |
| B-436 | 4-CN | 6-CN | CH₂CH₃ | H |
| B-437 | 2-F-4-CN | 6-CN | CH₂CH₃ | H |
| B-438 | 2-Cl-4-CN | 6-CN | CH₂CH₃ | H |
| B-439 | 2-Cl-4-CF3 | 6-CN | CH₂CH₃ | H |
| B-440 | 2-F-4-CF3 | 6-CN | CH₂CH₃ | H |
| B-441 | H | 2-CF₃ | CH₂CH₃ | H |
| B-442 | 2-F | 2-CF₃ | CH₂CH₃ | H |
| B-443 | 3-F | 2-CF₃ | CH₂CH₃ | H |
| B-444 | 4-F | 2-CF₃ | CH₂CH₃ | H |
| B-445 | 2-Cl | 2-CF₃ | CH₂CH₃ | H |
| B-446 | 3-Cl | 2-CF₃ | CH₂CH₃ | H |
| B-447 | 4-Cl | 2-CF₃ | CH₂CH₃ | H |
| B-448 | 2-CF₃ | 2-CF₃ | CH₂CH₃ | H |
| B-449 | 3-CF₃ | 2-CF₃ | CH₂CH₃ | H |
| B-450 | 4-CF₃ | 2-CF₃ | CH₂CH₃ | H |
| B-451 | 2,4-F₂ | 2-CF₃ | CH₂CH₃ | H |
| B-452 | 2,4-Cl₂ | 2-CF₃ | CH₂CH₃ | H |
| B-453 | 2,6-Cl₂ | 2-CF₃ | CH₂CH₃ | H |
| B-454 | 2-CN | 2-CF₃ | CH₂CH₃ | H |
| B-455 | 3-CN | 2-CF₃ | CH₂CH₃ | H |
| B-456 | 4-CN | 2-CF₃ | CH₂CH₃ | H |
| B-457 | 2-F-4-CN | 2-CF₃ | CH₂CH₃ | H |
| B-458 | 2-Cl-4-CN | 2-CF₃ | CH₂CH₃ | H |
| B-459 | 2-Cl-4-CF3 | 2-CF₃ | CH₂CH₃ | H |
| B-460 | 2-F-4-CF3 | 2-CF₃ | CH₂CH₃ | H |
| B-461 | H | 6-CF₃ | CH₂CH₃ | H |
| B-462 | 2-F | 6-CF₃ | CH₂CH₃ | H |
| B-463 | 3-F | 6-CF₃ | CH₂CH₃ | H |
| B-464 | 4-F | 6-CF₃ | CH₂CH₃ | H |
| B-465 | 2-Cl | 6-CF₃ | CH₂CH₃ | H |
| B-466 | 3-Cl | 6-CF₃ | CH₂CH₃ | H |
| B-467 | 4-Cl | 6-CF₃ | CH₂CH₃ | H |
| B-468 | 2-CF₃ | 6-CF₃ | CH₂CH₃ | H |
| B-469 | 3-CF₃ | 6-CF₃ | CH₂CH₃ | H |
| B-470 | 4-CF₃ | 6-CF₃ | CH₂CH₃ | H |
| B-471 | 2,4-F₂ | 6-CF₃ | CH₂CH₃ | H |
| B-472 | 2,4-Cl₂ | 6-CF₃ | CH₂CH₃ | H |
| B-473 | 2,6-Cl₂ | 6-CF₃ | CH₂CH₃ | H |
| B-474 | 2-CN | 6-CF₃ | CH₂CH₃ | H |
| B-475 | 3-CN | 6-CF₃ | CH₂CH₃ | H |
| B-476 | 4-CN | 6-CF₃ | CH₂CH₃ | H |
| B-477 | 2-F-4-CN | 6-CF₃ | CH₂CH₃ | H |
| B-478 | 2-Cl-4-CN | 6-CF₃ | CH₂CH₃ | H |
| B-479 | 2-Cl-4-CF3 | 6-CF₃ | CH₂CH₃ | H |
| B-480 | 2-F-4-CF3 | 6-CF₃ | CH₂CH₃ | H |
| B-481 | H | 2-OCH₃ | CH₂CH₃ | H |
| B-482 | 2-F | 2-OCH₃ | CH₂CH₃ | H |
| B-483 | 3-F | 2-OCH₃ | CH₂CH₃ | H |
| B-484 | 4-F | 2-OCH₃ | CH₂CH₃ | H |
| B-485 | 2-Cl | 2-OCH₃ | CH₂CH₃ | H |
| B-486 | 3-Cl | 2-OCH₃ | CH₂CH₃ | H |
| B-487 | 4-Cl | 2-OCH₃ | CH₂CH₃ | H |
| B-488 | 2-CF₃ | 2-OCH₃ | CH₂CH₃ | H |
| B-489 | 3-CF₃ | 2-OCH₃ | CH₂CH₃ | H |
| B-490 | 4-CF₃ | 2-OCH₃ | CH₂CH₃ | H |
| B-491 | 2,4-F₂ | 2-OCH₃ | CH₂CH₃ | H |
| B-492 | 2,4-Cl₂ | 2-OCH₃ | CH₂CH₃ | H |
| B-493 | 2,6-Cl₂ | 2-OCH₃ | CH₂CH₃ | H |
| B-494 | 2-CN | 2-OCH₃ | CH₂CH₃ | H |
| B-495 | 3-CN | 2-OCH₃ | CH₂CH₃ | H |
| B-496 | 4-CN | 2-OCH₃ | CH₂CH₃ | H |
| B-497 | 2-F-4-CN | 2-OCH₃ | CH₂CH₃ | H |
| B-498 | 2-Cl-4-CN | 2-OCH₃ | CH₂CH₃ | H |
| B-499 | 2-Cl-4-CF3 | 2-OCH₃ | CH₂CH₃ | H |
| B-500 | 2-F-4-CF3 | 2-OCH₃ | CH₂CH₃ | H |
| B-501 | H | 6-OCH₃ | CH₂CH₃ | H |
| B-502 | 2-F | 6-OCH₃ | CH₂CH₃ | H |
| B-503 | 3-F | 6-OCH₃ | CH₂CH₃ | H |
| B-504 | 4-F | 6-OCH₃ | CH₂CH₃ | H |
| B-505 | 2-Cl | 6-OCH₃ | CH₂CH₃ | H |
| B-506 | 3-Cl | 6-OCH₃ | CH₂CH₃ | H |
| B-507 | 4-Cl | 6-OCH₃ | CH₂CH₃ | H |
| B-508 | 2-CF₃ | 6-OCH₃ | CH₂CH₃ | H |
| B-509 | 3-CF₃ | 6-OCH₃ | CH₂CH₃ | H |
| B-510 | 4-CF₃ | 6-OCH₃ | CH₂CH₃ | H |
| B-511 | 2,4-F₂ | 6-OCH₃ | CH₂CH₃ | H |
| B-512 | 2,4-Cl₂ | 6-OCH₃ | CH₂CH₃ | H |
| B-513 | 2,6-Cl₂ | 6-OCH₃ | CH₂CH₃ | H |
| B-514 | 2-CN | 6-OCH₃ | CH₂CH₃ | H |
| B-515 | 3-CN | 6-OCH₃ | CH₂CH₃ | H |
| B-516 | 4-CN | 6-OCH₃ | CH₂CH₃ | H |
| B-517 | 2-F-4-CN | 6-OCH₃ | CH₂CH₃ | H |
| B-518 | 2-Cl-4-CN | 6-OCH₃ | CH₂CH₃ | H |
| B-519 | 2-Cl-4-CF3 | 6-OCH₃ | CH₂CH₃ | H |
| B-520 | 2-F-4-CF3 | 6-OCH₃ | CH₂CH₃ | H |
| B-521 | H | H | CH₂CH₂CH₃ | H |
| B-522 | 2-F | H | CH₂CH₂CH₃ | H |
| B-523 | 3-F | H | CH₂CH₂CH₃ | H |
| B-524 | 4-F | H | CH₂CH₂CH₃ | H |
| B-525 | 2-Cl | H | CH₂CH₂CH₃ | H |
| B-526 | 3-Cl | H | CH₂CH₂CH₃ | H |
| B-527 | 4-Cl | H | CH₂CH₂CH₃ | H |
| B-528 | 2-CF₃ | H | CH₂CH₂CH₃ | H |
| B-529 | 3-CF₃ | H | CH₂CH₂CH₃ | H |
| B-530 | 4-CF₃ | H | CH₂CH₂CH₃ | H |
| B-531 | 2,4-F₂ | H | CH₂CH₂CH₃ | H |
| B-532 | 2,4-Cl₂ | H | CH₂CH₂CH₃ | H |
| B-533 | 2,6-Cl₂ | H | CH₂CH₂CH₃ | H |
| B-534 | 2-CN | H | CH₂CH₂CH₃ | H |
| B-535 | 3-CN | H | CH₂CH₂CH₃ | H |
| B-536 | 4-CN | H | CH₂CH₂CH₃ | H |
| B-537 | 2-F-4-CN | H | CH₂CH₂CH₃ | H |
| B-538 | 2-Cl-4-CN | H | CH₂CH₂CH₃ | H |
| B-539 | 2-Cl-4-CF3 | H | CH₂CH₂CH₃ | H |
| B-540 | 2-F-4-CF3 | H | CH₂CH₂CH₃ | H |
| B-541 | H | 2-Cl | CH₂CH₂CH₃ | H |
| B-542 | 2-F | 2-Cl | CH₂CH₂CH₃ | H |
| B-543 | 3-F | 2-Cl | CH₂CH₂CH₃ | H |
| B-544 | 4-F | 2-Cl | CH₂CH₂CH₃ | H |
| B-545 | 2-Cl | 2-Cl | CH₂CH₂CH₃ | H |
| B-546 | 3-Cl | 2-Cl | CH₂CH₂CH₃ | H |
| B-547 | 4-Cl | 2-Cl | CH₂CH₂CH₃ | H |
| B-548 | 2-CF₃ | 2-Cl | CH₂CH₂CH₃ | H |
| B-549 | 3-CF₃ | 2-Cl | CH₂CH₂CH₃ | H |
| B-550 | 4-CF₃ | 2-Cl | CH₂CH₂CH₃ | H |
| B-551 | 2,4-F₂ | 2-Cl | CH₂CH₂CH₃ | H |
| B-552 | 2,4-Cl₂ | 2-Cl | CH₂CH₂CH₃ | H |
| B-553 | 2,6-Cl₂ | 2-Cl | CH₂CH₂CH₃ | H |
| B-554 | 2-CN | 2-Cl | CH₂CH₂CH₃ | H |
| B-555 | 3-CN | 2-Cl | CH₂CH₂CH₃ | H |
| B-556 | 4-CN | 2-Cl | CH₂CH₂CH₃ | H |
| B-557 | 2-F-4-CN | 2-Cl | CH₂CH₂CH₃ | H |
| B-558 | 2-Cl-4-CN | 2-Cl | CH₂CH₂CH₃ | H |
| B-559 | 2-Cl-4-CF3 | 2-Cl | CH₂CH₂CH₃ | H |
| B-560 | 2-F-4-CF3 | 2-Cl | CH₂CH₂CH₃ | H |
| B-561 | H | 6-Cl | CH₂CH₂CH₃ | H |
| B-562 | 2-F | 6-Cl | CH₂CH₂CH₃ | H |
| B-563 | 3-F | 6-Cl | CH₂CH₂CH₃ | H |
| B-564 | 4-F | 6-Cl | CH₂CH₂CH₃ | H |
| B-565 | 2-Cl | 6-Cl | CH₂CH₂CH₃ | H |
| B-566 | 3-Cl | 6-Cl | CH₂CH₂CH₃ | H |
| B-567 | 4-Cl | 6-Cl | CH₂CH₂CH₃ | H |
| B-568 | 2-CF₃ | 6-Cl | CH₂CH₂CH₃ | H |
| B-569 | 3-CF₃ | 6-Cl | CH₂CH₂CH₃ | H |
| B-570 | 4-CF₃ | 6-Cl | CH₂CH₂CH₃ | H |
| B-571 | 2,4-F₂ | 6-Cl | CH₂CH₂CH₃ | H |
| B-572 | 2,4-Cl₂ | 6-Cl | CH₂CH₂CH₃ | H |
| B-573 | 2,6-Cl₂ | 6-Cl | CH₂CH₂CH₃ | H |
| B-574 | 2-CN | 6-Cl | CH₂CH₂CH₃ | H |
| B-575 | 3-CN | 6-Cl | CH₂CH₂CH₃ | H |
| B-576 | 4-CN | 6-Cl | CH₂CH₂CH₃ | H |
| B-577 | 2-F-4-CN | 6-Cl | CH₂CH₂CH₃ | H |
| B-578 | 2-Cl-4-CN | 6-Cl | CH₂CH₂CH₃ | H |
| B-579 | 2-Cl-4-CF3 | 6-Cl | CH₂CH₂CH₃ | H |
| B-580 | 2-F-4-CF3 | 6-Cl | CH₂CH₂CH₃ | H |
| B-581 | H | 2-Br | CH₂CH₂CH₃ | H |
| B-582 | 2-F | 2-Br | CH₂CH₂CH₃ | H |
| B-583 | 3-F | 2-Br | CH₂CH₂CH₃ | H |
| B-584 | 4-F | 2-Br | CH₂CH₂CH₃ | H |
| B-585 | 2-Cl | 2-Br | CH₂CH₂CH₃ | H |
| B-586 | 3-Cl | 2-Br | CH₂CH₂CH₃ | H |
| B-587 | 4-Cl | 2-Br | CH₂CH₂CH₃ | H |
| B-588 | 2-CF₃ | 2-Br | CH₂CH₂CH₃ | H |
| B-589 | 3-CF₃ | 2-Br | CH₂CH₂CH₃ | H |
| B-590 | 4-CF₃ | 2-Br | CH₂CH₂CH₃ | H |
| B-591 | 2,4-F₂ | 2-Br | CH₂CH₂CH₃ | H |
| B-592 | 2,4-Cl₂ | 2-Br | CH₂CH₂CH₃ | H |
| B-593 | 2,6-Cl₂ | 2-Br | CH₂CH₂CH₃ | H |
| B-594 | 2-CN | 2-Br | CH₂CH₂CH₃ | H |
| B-595 | 3-CN | 2-Br | CH₂CH₂CH₃ | H |
| B-596 | 4-CN | 2-Br | CH₂CH₂CH₃ | H |
| B-597 | 2-F-4-CN | 2-Br | CH₂CH₂CH₃ | H |
| B-598 | 2-Cl-4-CN | 2-Br | CH₂CH₂CH₃ | H |
| B-599 | 2-Cl-4-CF3 | 2-Br | CH₂CH₂CH₃ | H |
| B-600 | 2-F-4-CF3 | 2-Br | CH₂CH₂CH₃ | H |
| B-601 | H | 6-Br | CH₂CH₂CH₃ | H |
| B-602 | 2-F | 6-Br | CH₂CH₂CH₃ | H |
| B-603 | 3-F | 6-Br | CH₂CH₂CH₃ | H |
| B-604 | 4-F | 6-Br | CH₂CH₂CH₃ | H |
| B-605 | 2-Cl | 6-Br | CH₂CH₂CH₃ | H |
| B-606 | 3-Cl | 6-Br | CH₂CH₂CH₃ | H |
| B-607 | 4-Cl | 6-Br | CH₂CH₂CH₃ | H |
| B-608 | 2-CF₃ | 6-Br | CH₂CH₂CH₃ | H |
| B-609 | 3-CF₃ | 6-Br | CH₂CH₂CH₃ | H |
| B-610 | 4-CF₃ | 6-Br | CH₂CH₂CH₃ | H |
| B-611 | 2,4-F₂ | 6-Br | CH₂CH₂CH₃ | H |
| B-612 | 2,4-Cl₂ | 6-Br | CH₂CH₂CH₃ | H |
| B-613 | 2,6-Cl₂ | 6-Br | CH₂CH₂CH₃ | H |
| B-614 | 2-CN | 6-Br | CH₂CH₂CH₃ | H |
| B-615 | 3-CN | 6-Br | CH₂CH₂CH₃ | H |
| B-616 | 4-CN | 6-Br | CH₂CH₂CH₃ | H |
| B-617 | 2-F-4-CN | 6-Br | CH₂CH₂CH₃ | H |
| B-618 | 2-Cl-4-CN | 6-Br | CH₂CH₂CH₃ | H |
| B-619 | 2-Cl-4-CF3 | 6-Br | CH₂CH₂CH₃ | H |
| B-620 | 2-F-4-CF3 | 6-Br | CH₂CH₂CH₃ | H |
| B-621 | H | 2-F | CH₂CH₂CH₃ | H |
| B-622 | 2-F | 2-F | CH₂CH₂CH₃ | H |
| B-623 | 3-F | 2-F | CH₂CH₂CH₃ | H |
| B-624 | 4-F | 2-F | CH₂CH₂CH₃ | H |
| B-625 | 2-Cl | 2-F | CH₂CH₂CH₃ | H |
| B-626 | 3-Cl | 2-F | CH₂CH₂CH₃ | H |
| B-627 | 4-Cl | 2-F | CH₂CH₂CH₃ | H |
| B-628 | 2-CF₃ | 2-F | CH₂CH₂CH₃ | H |
| B-629 | 3-CF₃ | 2-F | CH₂CH₂CH₃ | H |
| B-630 | 4-CF₃ | 2-F | CH₂CH₂CH₃ | H |
| B-631 | 2,4-F₂ | 2-F | CH₂CH₂CH₃ | H |
| B-632 | 2,4-Cl₂ | 2-F | CH₂CH₂CH₃ | H |
| B-633 | 2,6-Cl₂ | 2-F | CH₂CH₂CH₃ | H |
| B-634 | 2-CN | 2-F | CH₂CH₂CH₃ | H |
| B-635 | 3-CN | 2-F | CH₂CH₂CH₃ | H |
| B-636 | 4-CN | 2-F | CH₂CH₂CH₃ | H |
| B-637 | 2-F-4-CN | 2-F | CH₂CH₂CH₃ | H |
| B-638 | 2-Cl-4-CN | 2-F | CH₂CH₂CH₃ | H |
| B-639 | 2-Cl-4-CF3 | 2-F | CH₂CH₂CH₃ | H |
| B-640 | 2-F-4-CF3 | 2-F | CH₂CH₂CH₃ | H |
| B-641 | H | 6-F | CH₂CH₂CH₃ | H |
| B-642 | 2-F | 6-F | CH₂CH₂CH₃ | H |
| B-643 | 3-F | 6-F | CH₂CH₂CH₃ | H |
| B-644 | 4-F | 6-F | CH₂CH₂CH₃ | H |
| B-645 | 2-Cl | 6-F | CH₂CH₂CH₃ | H |
| B-646 | 3-Cl | 6-F | CH₂CH₂CH₃ | H |
| B-647 | 4-Cl | 6-F | CH₂CH₂CH₃ | H |
| B-648 | 2-CF₃ | 6-F | CH₂CH₂CH₃ | H |
| B-649 | 3-CF₃ | 6-F | CH₂CH₂CH₃ | H |
| B-650 | 4-CF₃ | 6-F | CH₂CH₂CH₃ | H |
| B-651 | 2,4-F₂ | 6-F | CH₂CH₂CH₃ | H |
| B-652 | 2,4-Cl₂ | 6-F | CH₂CH₂CH₃ | H |
| B-653 | 2,6-Cl₂ | 6-F | CH₂CH₂CH₃ | H |
| B-654 | 2-CN | 6-F | CH₂CH₂CH₃ | H |
| B-655 | 3-CN | 6-F | CH₂CH₂CH₃ | H |
| B-656 | 4-CN | 6-F | CH₂CH₂CH₃ | H |
| B-657 | 2-F-4-CN | 6-F | CH₂CH₂CH₃ | H |
| B-658 | 2-Cl-4-CN | 6-F | CH₂CH₂CH₃ | H |
| B-659 | 2-Cl-4-CF3 | 6-F | CH₂CH₂CH₃ | H |
| B-660 | 2-F-4-CF3 | 6-F | CH₂CH₂CH₃ | H |
| B-661 | H | 2-CN | CH₂CH₂CH₃ | H |
| B-662 | 2-F | 2-CN | CH₂CH₂CH₃ | H |
| B-663 | 3-F | 2-CN | CH₂CH₂CH₃ | H |
| B-664 | 4-F | 2-CN | CH₂CH₂CH₃ | H |
| B-665 | 2-Cl | 2-CN | CH₂CH₂CH₃ | H |
| B-666 | 3-Cl | 2-CN | CH₂CH₂CH₃ | H |
| B-667 | 4-Cl | 2-CN | CH₂CH₂CH₃ | H |
| B-668 | 2-CF₃ | 2-CN | CH₂CH₂CH₃ | H |
| B-669 | 3-CF₃ | 2-CN | CH₂CH₂CH₃ | H |
| B-670 | 4-CF₃ | 2-CN | CH₂CH₂CH₃ | H |
| B-671 | 2,4-F₂ | 2-CN | CH₂CH₂CH₃ | H |
| B-672 | 2,4-Cl₂ | 2-CN | CH₂CH₂CH₃ | H |
| B-673 | 2,6-Cl₂ | 2-CN | CH₂CH₂CH₃ | H |
| B-674 | 2-CN | 2-CN | CH₂CH₂CH₃ | H |
| B-675 | 3-CN | 2-CN | CH₂CH₂CH₃ | H |
| B-676 | 4-CN | 2-CN | CH₂CH₂CH₃ | H |
| B-677 | 2-F-4-CN | 2-CN | CH₂CH₂CH₃ | H |
| B-678 | 2-Cl-4-CN | 2-CN | CH₂CH₂CH₃ | H |
| B-679 | 2-Cl-4-CF3 | 2-CN | CH₂CH₂CH₃ | H |
| B-680 | 2-F-4-CF3 | 2-CN | CH₂CH₂CH₃ | H |
| B-681 | H | 6-CN | CH₂CH₂CH₃ | H |
| B-682 | 2-F | 6-CN | CH₂CH₂CH₃ | H |
| B-683 | 3-F | 6-CN | CH₂CH₂CH₃ | H |
| B-684 | 4-F | 6-CN | CH₂CH₂CH₃ | H |
| B-685 | 2-Cl | 6-CN | CH₂CH₂CH₃ | H |
| B-686 | 3-Cl | 6-CN | CH₂CH₂CH₃ | H |
| B-687 | 4-Cl | 6-CN | CH₂CH₂CH₃ | H |
| B-688 | 2-CF₃ | 6-CN | CH₂CH₂CH₃ | H |
| B-689 | 3-CF₃ | 6-CN | CH₂CH₂CH₃ | H |
| B-690 | 4-CF₃ | 6-CN | CH₂CH₂CH₃ | H |
| B-691 | 2,4-F₂ | 6-CN | CH₂CH₂CH₃ | H |
| B-692 | 2,4-Cl₂ | 6-CN | CH₂CH₂CH₃ | H |
| B-693 | 2,6-Cl₂ | 6-CN | CH₂CH₂CH₃ | H |
| B-694 | 2-CN | 6-CN | CH₂CH₂CH₃ | H |
| B-695 | 3-CN | 6-CN | CH₂CH₂CH₃ | H |
| B-696 | 4-CN | 6-CN | CH₂CH₂CH₃ | H |
| B-697 | 2-F-4-CN | 6-CN | CH₂CH₂CH₃ | H |
| B-698 | 2-Cl-4-CN | 6-CN | CH₂CH₂CH₃ | H |
| B-699 | 2-Cl-4-CF3 | 6-CN | CH₂CH₂CH₃ | H |
| B-700 | 2-F-4-CF3 | 6-CN | CH₂CH₂CH₃ | H |
| B-701 | H | 2-CF₃ | CH₂CH₂CH₃ | H |
| B-702 | 2-F | 2-CF₃ | CH₂CH₂CH₃ | H |
| B-703 | 3-F | 2-CF₃ | CH₂CH₂CH₃ | H |
| B-704 | 4-F | 2-CF₃ | CH₂CH₂CH₃ | H |
| B-705 | 2-Cl | 2-CF₃ | CH₂CH₂CH₃ | H |
| B-706 | 3-Cl | 2-CF₃ | CH₂CH₂CH₃ | H |
| B-707 | 4-Cl | 2-CF₃ | CH₂CH₂CH₃ | H |
| B-708 | 2-CF₃ | 2-CF₃ | CH₂CH₂CH₃ | H |
| B-709 | 3-CF₃ | 2-CF₃ | CH₂CH₂CH₃ | H |
| B-710 | 4-CF₃ | 2-CF₃ | CH₂CH₂CH₃ | H |
| B-711 | 2,4-F₂ | 2-CF₃ | CH₂CH₂CH₃ | H |
| B-712 | 2,4-Cl₂ | 2-CF₃ | CH₂CH₂CH₃ | H |
| B-713 | 2,6-Cl₂ | 2-CF₃ | CH₂CH₂CH₃ | H |
| B-714 | 2-CN | 2-CF₃ | CH₂CH₂CH₃ | H |
| B-715 | 3-CN | 2-CF₃ | CH₂CH₂CH₃ | H |
| B-716 | 4-CN | 2-CF₃ | CH₂CH₂CH₃ | H |
| B-717 | 2-F-4-CN | 2-CF₃ | CH₂CH₂CH₃ | H |
| B-718 | 2-Cl-4-CN | 2-CF₃ | CH₂CH₂CH₃ | H |
| B-719 | 2-Cl-4-CF3 | 2-CF₃ | CH₂CH₂CH₃ | H |
| B-720 | 2-F-4-CF3 | 2-CF₃ | CH₂CH₂CH₃ | H |
| B-721 | H | 6-CF₃ | CH₂CH₂CH₃ | H |
| B-722 | 2-F | 6-CF₃ | CH₂CH₂CH₃ | H |
| B-723 | 3-F | 6-CF₃ | CH₂CH₂CH₃ | H |
| B-724 | 4-F | 6-CF₃ | CH₂CH₂CH₃ | H |
| B-725 | 2-Cl | 6-CF₃ | CH₂CH₂CH₃ | H |
| B-726 | 3-Cl | 6-CF₃ | CH₂CH₂CH₃ | H |
| B-727 | 4-Cl | 6-CF₃ | CH₂CH₂CH₃ | H |
| B-728 | 2-CF₃ | 6-CF₃ | CH₂CH₂CH₃ | H |
| B-729 | 3-CF₃ | 6-CF₃ | CH₂CH₂CH₃ | H |
| B-730 | 4-CF₃ | 6-CF₃ | CH₂CH₂CH₃ | H |
| B-731 | 2,4-F₂ | 6-CF₃ | CH₂CH₂CH₃ | H |
| B-732 | 2,4-Cl₂ | 6-CF₃ | CH₂CH₂CH₃ | H |
| B-733 | 2,6-Cl₂ | 6-CF₃ | CH₂CH₂CH₃ | H |
| B-734 | 2-CN | 6-CF₃ | CH₂CH₂CH₃ | H |
| B-735 | 3-CN | 6-CF₃ | CH₂CH₂CH₃ | H |
| B-736 | 4-CN | 6-CF₃ | CH₂CH₂CH₃ | H |
| B-737 | 2-F-4-CN | 6-CF₃ | CH₂CH₂CH₃ | H |
| B-738 | 2-Cl-4-CN | 6-CF₃ | CH₂CH₂CH₃ | H |
| B-739 | 2-Cl-4-CF3 | 6-CF₃ | CH₂CH₂CH₃ | H |
| B-740 | 2-F-4-CF3 | 6-CF₃ | CH₂CH₂CH₃ | H |
| B-741 | H | 2-OCH₃ | CH₂CH₂CH₃ | H |
| B-742 | 2-F | 2-OCH₃ | CH₂CH₂CH₃ | H |
| B-743 | 3-F | 2-OCH₃ | CH₂CH₂CH₃ | H |
| B-744 | 4-F | 2-OCH₃ | CH₂CH₂CH₃ | H |
| B-745 | 2-Cl | 2-OCH₃ | CH₂CH₂CH₃ | H |
| B-746 | 3-Cl | 2-OCH₃ | CH₂CH₂CH₃ | H |
| B-747 | 4-Cl | 2-OCH₃ | CH₂CH₂CH₃ | H |
| B-748 | 2-CF₃ | 2-OCH₃ | CH₂CH₂CH₃ | H |
| B-749 | 3-CF₃ | 2-OCH₃ | CH₂CH₂CH₃ | H |
| B-750 | 4-CF₃ | 2-OCH₃ | CH₂CH₂CH₃ | H |
| B-751 | 2,4-F₂ | 2-OCH₃ | CH₂CH₂CH₃ | H |
| B-752 | 2,4-Cl₂ | 2-OCH₃ | CH₂CH₂CH₃ | H |
| B-753 | 2,6-Cl₂ | 2-OCH₃ | CH₂CH₂CH₃ | H |
| B-754 | 2-CN | 2-OCH₃ | CH₂CH₂CH₃ | H |
| B-755 | 3-CN | 2-OCH₃ | CH₂CH₂CH₃ | H |
| B-756 | 4-CN | 2-OCH₃ | CH₂CH₂CH₃ | H |
| B-757 | 2-F-4-CN | 2-OCH₃ | CH₂CH₂CH₃ | H |
| B-758 | 2-Cl-4-CN | 2-OCH₃ | CH₂CH₂CH₃ | H |
| B-759 | 2-Cl-4-CF3 | 2-OCH₃ | CH₂CH₂CH₃ | H |
| B-760 | 2-F-4-CF3 | 2-OCH₃ | CH₂CH₂CH₃ | H |
| B-761 | H | 6-OCH₃ | CH₂CH₂CH₃ | H |
| B-762 | 2-F | 6-OCH₃ | CH₂CH₂CH₃ | H |
| B-763 | 3-F | 6-OCH₃ | CH₂CH₂CH₃ | H |
| B-764 | 4-F | 6-OCH₃ | CH₂CH₂CH₃ | H |
| B-765 | 2-Cl | 6-OCH₃ | CH₂CH₂CH₃ | H |
| B-766 | 3-Cl | 6-OCH₃ | CH₂CH₂CH₃ | H |
| B-767 | 4-Cl | 6-OCH₃ | CH₂CH₂CH₃ | H |
| B-768 | 2-CF₃ | 6-OCH₃ | CH₂CH₂CH₃ | H |
| B-769 | 3-CF₃ | 6-OCH₃ | CH₂CH₂CH₃ | H |
| B-770 | 4-CF₃ | 6-OCH₃ | CH₂CH₂CH₃ | H |
| B-771 | 2,4-F₂ | 6-OCH₃ | CH₂CH₂CH₃ | H |
| B-772 | 2,4-Cl₂ | 6-OCH₃ | CH₂CH₂CH₃ | H |
| B-773 | 2,6-Cl₂ | 6-OCH₃ | CH₂CH₂CH₃ | H |
| B-774 | 2-CN | 6-OCH₃ | CH₂CH₂CH₃ | H |
| B-775 | 3-CN | 6-OCH₃ | CH₂CH₂CH₃ | H |
| B-776 | 4-CN | 6-OCH₃ | CH₂CH₂CH₃ | H |
| B-777 | 2-F-4-CN | 6-OCH₃ | CH₂CH₂CH₃ | H |
| B-778 | 2-Cl-4-CN | 6-OCH₃ | CH₂CH₂CH₃ | H |
| B-779 | 2-Cl-4-CF3 | 6-OCH₃ | CH₂CH₂CH₃ | H |
| B-780 | 2-F-4-CF3 | 6-OCH₃ | CH₂CH₂CH₃ | H |
| B-781 | H | H | CH₃ | CH₃ |
| B-782 | 2-F | H | CH₃ | CH₃ |
| B-783 | 3-F | H | CH₃ | CH₃ |
| B-784 | 4-F | H | CH₃ | CH₃ |
| B-785 | 2-Cl | H | CH₃ | CH₃ |
| B-786 | 3-Cl | H | CH₃ | CH₃ |
| B-787 | 4-Cl | H | CH₃ | CH₃ |
| B-788 | 2-CF₃ | H | CH₃ | CH₃ |
| B-789 | 3-CF₃ | H | CH₃ | CH₃ |
| B-790 | 4-CF₃ | H | CH₃ | CH₃ |
| B-791 | 2,4-F₂ | H | CH₃ | CH₃ |
| B-792 | 2,4-Cl₂ | H | CH₃ | CH₃ |
| B-793 | 2,6-Cl₂ | H | CH₃ | CH₃ |
| B-794 | 2-CN | H | CH₃ | CH₃ |
| B-795 | 3-CN | H | CH₃ | CH₃ |
| B-796 | 4-CN | H | CH₃ | CH₃ |
| B-797 | 2-F-4-CN | H | CH₃ | CH₃ |
| B-798 | 2-Cl-4-CN | H | CH₃ | CH₃ |
| B-799 | 2-Cl-4-CF3 | H | CH₃ | CH₃ |
| B-800 | 2-F-4-CF3 | H | CH₃ | CH₃ |
| B-801 | H | 2-Cl | CH₃ | CH₃ |
| B-802 | 2-F | 2-Cl | CH₃ | CH₃ |
| B-803 | 3-F | 2-Cl | CH₃ | CH₃ |
| B-804 | 4-F | 2-Cl | CH₃ | CH₃ |
| B-805 | 2-Cl | 2-Cl | CH₃ | CH₃ |
| B-806 | 3-Cl | 2-Cl | CH₃ | CH₃ |
| B-807 | 4-Cl | 2-Cl | CH₃ | CH₃ |
| B-808 | 2-CF₃ | 2-Cl | CH₃ | CH₃ |
| B-809 | 3-CF₃ | 2-Cl | CH₃ | CH₃ |
| B-810 | 4-CF₃ | 2-Cl | CH₃ | CH₃ |
| B-811 | 2,4-F₂ | 2-Cl | CH₃ | CH₃ |
| B-812 | 2,4-Cl₂ | 2-Cl | CH₃ | CH₃ |
| B-813 | 2,6-Cl₂ | 2-Cl | CH₃ | CH₃ |
| B-814 | 2-CN | 2-Cl | CH₃ | CH₃ |
| B-815 | 3-CN | 2-Cl | CH₃ | CH₃ |
| B-816 | 4-CN | 2-Cl | CH₃ | CH₃ |
| B-817 | 2-F-4-CN | 2-Cl | CH₃ | CH₃ |
| B-818 | 2-Cl-4-CN | 2-Cl | CH₃ | CH₃ |
| B-819 | 2-Cl-4-CF3 | 2-Cl | CH₃ | CH₃ |
| B-820 | 2-F-4-CF3 | 2-Cl | CH₃ | CH₃ |
| B-821 | H | 6-Cl | CH₃ | CH₃ |
| B-822 | 2-F | 6-Cl | CH₃ | CH₃ |
| B-823 | 3-F | 6-Cl | CH₃ | CH₃ |
| B-824 | 4-F | 6-Cl | CH₃ | CH₃ |
| B-825 | 2-Cl | 6-Cl | CH₃ | CH₃ |
| B-826 | 3-Cl | 6-Cl | CH₃ | CH₃ |
| B-827 | 4-Cl | 6-Cl | CH₃ | CH₃ |
| B-828 | 2-CF₃ | 6-Cl | CH₃ | CH₃ |
| B-829 | 3-CF₃ | 6-Cl | CH₃ | CH₃ |
| B-830 | 4-CF₃ | 6-Cl | CH₃ | CH₃ |
| B-831 | 2,4-F₂ | 6-Cl | CH₃ | CH₃ |
| B-832 | 2,4-Cl₂ | 6-Cl | CH₃ | CH₃ |
| B-833 | 2,6-Cl₂ | 6-Cl | CH₃ | CH₃ |
| B-834 | 2-CN | 6-Cl | CH₃ | CH₃ |
| B-835 | 3-CN | 6-Cl | CH₃ | CH₃ |
| B-836 | 4-CN | 6-Cl | CH₃ | CH₃ |
| B-837 | 2-F-4-CN | 6-Cl | CH₃ | CH₃ |
| B-838 | 2-Cl-4-CN | 6-Cl | CH₃ | CH₃ |
| B-839 | 2-Cl-4-CF3 | 6-Cl | CH₃ | CH₃ |
| B-840 | 2-F-4-CF3 | 6-Cl | CH₃ | CH₃ |
| B-841 | H | 2-Br | CH₃ | CH₃ |
| B-842 | 2-F | 2-Br | CH₃ | CH₃ |
| B-843 | 3-F | 2-Br | CH₃ | CH₃ |
| B-844 | 4-F | 2-Br | CH₃ | CH₃ |
| B-845 | 2-Cl | 2-Br | CH₃ | CH₃ |
| B-846 | 3-Cl | 2-Br | CH₃ | CH₃ |
| B-847 | 4-Cl | 2-Br | CH₃ | CH₃ |
| B-848 | 2-CF₃ | 2-Br | CH₃ | CH₃ |
| B-849 | 3-CF₃ | 2-Br | CH₃ | CH₃ |
| B-850 | 4-CF₃ | 2-Br | CH₃ | CH₃ |
| B-851 | 2,4-F₂ | 2-Br | CH₃ | CH₃ |
| B-852 | 2,4-Cl₂ | 2-Br | CH₃ | CH₃ |
| B-853 | 2,6-Cl₂ | 2-Br | CH₃ | CH₃ |
| B-854 | 2-CN | 2-Br | CH₃ | CH₃ |
| B-855 | 3-CN | 2-Br | CH₃ | CH₃ |
| B-856 | 4-CN | 2-Br | CH₃ | CH₃ |
| B-857 | 2-F-4-CN | 2-Br | CH₃ | CH₃ |
| B-858 | 2-Cl-4-CN | 2-Br | CH₃ | CH₃ |
| B-859 | 2-Cl-4-CF3 | 2-Br | CH₃ | CH₃ |
| B-860 | 2-F-4-CF3 | 2-Br | CH₃ | CH₃ |
| B-861 | H | 6-Br | CH₃ | CH₃ |
| B-862 | 2-F | 6-Br | CH₃ | CH₃ |
| B-863 | 3-F | 6-Br | CH₃ | CH₃ |
| B-864 | 4-F | 6-Br | CH₃ | CH₃ |
| B-865 | 2-Cl | 6-Br | CH₃ | CH₃ |
| B-866 | 3-Cl | 6-Br | CH₃ | CH₃ |
| B-867 | 4-Cl | 6-Br | CH₃ | CH₃ |
| B-868 | 2-CF₃ | 6-Br | CH₃ | CH₃ |
| B-869 | 3-CF₃ | 6-Br | CH₃ | CH₃ |
| B-870 | 4-CF₃ | 6-Br | CH₃ | CH₃ |
| B-871 | 2,4-F₂ | 6-Br | CH₃ | CH₃ |
| B-872 | 2,4-Cl₂ | 6-Br | CH₃ | CH₃ |
| B-873 | 2,6-Cl₂ | 6-Br | CH₃ | CH₃ |
| B-874 | 2-CN | 6-Br | CH₃ | CH₃ |
| B-875 | 3-CN | 6-Br | CH₃ | CH₃ |
| B-876 | 4-CN | 6-Br | CH₃ | CH₃ |
| B-877 | 2-F-4-CN | 6-Br | CH₃ | CH₃ |
| B-878 | 2-Cl-4-CN | 6-Br | CH₃ | CH₃ |
| B-879 | 2-Cl-4-CF3 | 6-Br | CH₃ | CH₃ |
| B-880 | 2-F-4-CF3 | 6-Br | CH₃ | CH₃ |
| B-881 | H | 2-F | CH₃ | CH₃ |
| B-882 | 2-F | 2-F | CH₃ | CH₃ |
| B-883 | 3-F | 2-F | CH₃ | CH₃ |
| B-884 | 4-F | 2-F | CH₃ | CH₃ |
| B-885 | 2-Cl | 2-F | CH₃ | CH₃ |
| B-886 | 3-Cl | 2-F | CH₃ | CH₃ |
| B-887 | 4-Cl | 2-F | CH₃ | CH₃ |
| B-888 | 2-CF₃ | 2-F | CH₃ | CH₃ |
| B-889 | 3-CF₃ | 2-F | CH₃ | CH₃ |
| B-890 | 4-CF₃ | 2-F | CH₃ | CH₃ |
| B-891 | 2,4-F₂ | 2-F | CH₃ | CH₃ |
| B-892 | 2,4-Cl₂ | 2-F | CH₃ | CH₃ |
| B-893 | 2,6-Cl₂ | 2-F | CH₃ | CH₃ |
| B-894 | 2-CN | 2-F | CH₃ | CH₃ |
| B-895 | 3-CN | 2-F | CH₃ | CH₃ |
| B-896 | 4-CN | 2-F | CH₃ | CH₃ |
| B-897 | 2-F-4-CN | 2-F | CH₃ | CH₃ |
| B-898 | 2-Cl-4-CN | 2-F | CH₃ | CH₃ |
| B-899 | 2-Cl-4-CF3 | 2-F | CH₃ | CH₃ |
| B-900 | 2-F-4-CF3 | 2-F | CH₃ | CH₃ |
| B-901 | H | 6-F | CH₃ | CH₃ |
| B-902 | 2-F | 6-F | CH₃ | CH₃ |
| B-903 | 3-F | 6-F | CH₃ | CH₃ |
| B-904 | 4-F | 6-F | CH₃ | CH₃ |
| B-905 | 2-Cl | 6-F | CH₃ | CH₃ |
| B-906 | 3-Cl | 6-F | CH₃ | CH₃ |
| B-907 | 4-Cl | 6-F | CH₃ | CH₃ |
| B-908 | 2-CF₃ | 6-F | CH₃ | CH₃ |
| B-909 | 3-CF₃ | 6-F | CH₃ | CH₃ |
| B-910 | 4-CF₃ | 6-F | CH₃ | CH₃ |
| B-911 | 2,4-F₂ | 6-F | CH₃ | CH₃ |
| B-912 | 2,4-Cl₂ | 6-F | CH₃ | CH₃ |
| B-913 | 2,6-Cl₂ | 6-F | CH₃ | CH₃ |
| B-914 | 2-CN | 6-F | CH₃ | CH₃ |
| B-915 | 3-CN | 6-F | CH₃ | CH₃ |
| B-916 | 4-CN | 6-F | CH₃ | CH₃ |
| B-917 | 2-F-4-CN | 6-F | CH₃ | CH₃ |
| B-918 | 2-Cl-4-CN | 6-F | CH₃ | CH₃ |
| B-919 | 2-Cl-4-CF3 | 6-F | CH₃ | CH₃ |
| B-920 | 2-F-4-CF3 | 6-F | CH₃ | CH₃ |
| B-921 | H | 2-CN | CH₃ | CH₃ |
| B-922 | 2-F | 2-CN | CH₃ | CH₃ |
| B-923 | 3-F | 2-CN | CH₃ | CH₃ |
| B-924 | 4-F | 2-CN | CH₃ | CH₃ |
| B-925 | 2-Cl | 2-CN | CH₃ | CH₃ |
| B-926 | 3-Cl | 2-CN | CH₃ | CH₃ |
| B-927 | 4-Cl | 2-CN | CH₃ | CH₃ |
| B-928 | 2-CF₃ | 2-CN | CH₃ | CH₃ |
| B-929 | 3-CF₃ | 2-CN | CH₃ | CH₃ |
| B-930 | 4-CF₃ | 2-CN | CH₃ | CH₃ |
| B-931 | 2,4-F₂ | 2-CN | CH₃ | CH₃ |
| B-932 | 2,4-Cl₂ | 2-CN | CH₃ | CH₃ |
| B-933 | 2,6-Cl₂ | 2-CN | CH₃ | CH₃ |
| B-934 | 2-CN | 2-CN | CH₃ | CH₃ |
| B-935 | 3-CN | 2-CN | CH₃ | CH₃ |
| B-936 | 4-CN | 2-CN | CH₃ | CH₃ |
| B-937 | 2-F-4-CN | 2-CN | CH₃ | CH₃ |
| B-938 | 2-Cl-4-CN | 2-CN | CH₃ | CH₃ |
| B-939 | 2-Cl-4-CF3 | 2-CN | CH₃ | CH₃ |
| B-940 | 2-F-4-CF3 | 2-CN | CH₃ | CH₃ |
| B-941 | H | 6-CN | CH₃ | CH₃ |
| B-942 | 2-F | 6-CN | CH₃ | CH₃ |
| B-943 | 3-F | 6-CN | CH₃ | CH₃ |
| B-944 | 4-F | 6-CN | CH₃ | CH₃ |
| B-945 | 2-Cl | 6-CN | CH₃ | CH₃ |
| B-946 | 3-Cl | 6-CN | CH₃ | CH₃ |
| B-947 | 4-Cl | 6-CN | CH₃ | CH₃ |
| B-948 | 2-CF₃ | 6-CN | CH₃ | CH₃ |
| B-949 | 3-CF₃ | 6-CN | CH₃ | CH₃ |
| B-950 | 4-CF₃ | 6-CN | CH₃ | CH₃ |
| B-951 | 2,4-F₂ | 6-CN | CH₃ | CH₃ |
| B-952 | 2,4-Cl₂ | 6-CN | CH₃ | CH₃ |
| B-953 | 2,6-Cl₂ | 6-CN | CH₃ | CH₃ |
| B-954 | 2-CN | 6-CN | CH₃ | CH₃ |
| B-955 | 3-CN | 6-CN | CH₃ | CH₃ |
| B-956 | 4-CN | 6-CN | CH₃ | CH₃ |
| B-957 | 2-F-4-CN | 6-CN | CH₃ | CH₃ |
| B-958 | 2-Cl-4-CN | 6-CN | CH₃ | CH₃ |
| B-959 | 2-Cl-4-CF3 | 6-CN | CH₃ | CH₃ |
| B-960 | 2-F-4-CF3 | 6-CN | CH₃ | CH₃ |
| B-961 | H | 2-CF₃ | CH₃ | CH₃ |
| B-962 | 2-F | 2-CF₃ | CH₃ | CH₃ |
| B-963 | 3-F | 2-CF₃ | CH₃ | CH₃ |
| B-964 | 4-F | 2-CF₃ | CH₃ | CH₃ |
| B-965 | 2-Cl | 2-CF₃ | CH₃ | CH₃ |
| B-966 | 3-Cl | 2-CF₃ | CH₃ | CH₃ |
| B-967 | 4-Cl | 2-CF₃ | CH₃ | CH₃ |
| B-968 | 2-CF₃ | 2-CF₃ | CH₃ | CH₃ |
| B-969 | 3-CF₃ | 2-CF₃ | CH₃ | CH₃ |
| B-970 | 4-CF₃ | 2-CF₃ | CH₃ | CH₃ |
| B-971 | 2,4-F₂ | 2-CF₃ | CH₃ | CH₃ |
| B-972 | 2,4-Cl₂ | 2-CF₃ | CH₃ | CH₃ |
| B-973 | 2,6-Cl₂ | 2-CF₃ | CH₃ | CH₃ |
| B-974 | 2-CN | 2-CF₃ | CH₃ | CH₃ |
| B-975 | 3-CN | 2-CF₃ | CH₃ | CH₃ |
| B-976 | 4-CN | 2-CF₃ | CH₃ | CH₃ |
| B-977 | 2-F-4-CN | 2-CF₃ | CH₃ | CH₃ |
| B-978 | 2-Cl-4-CN | 2-CF₃ | CH₃ | CH₃ |
| B-979 | 2-Cl-4-CF3 | 2-CF₃ | CH₃ | CH₃ |
| B-980 | 2-F-4-CF3 | 2-CF₃ | CH₃ | CH₃ |
| B-981 | H | 6-CF₃ | CH₃ | CH₃ |
| B-982 | 2-F | 6-CF₃ | CH₃ | CH₃ |
| B-983 | 3-F | 6-CF₃ | CH₃ | CH₃ |
| B-984 | 4-F | 6-CF₃ | CH₃ | CH₃ |
| B-985 | 2-Cl | 6-CF₃ | CH₃ | CH₃ |
| B-986 | 3-Cl | 6-CF₃ | CH₃ | CH₃ |
| B-987 | 4-Cl | 6-CF₃ | CH₃ | CH₃ |
| B-988 | 2-CF₃ | 6-CF₃ | CH₃ | CH₃ |
| B-989 | 3-CF₃ | 6-CF₃ | CH₃ | CH₃ |
| B-990 | 4-CF₃ | 6-CF₃ | CH₃ | CH₃ |
| B-991 | 2,4-F₂ | 6-CF₃ | CH₃ | CH₃ |
| B-992 | 2,4-Cl₂ | 6-CF₃ | CH₃ | CH₃ |
| B-993 | 2,6-Cl₂ | 6-CF₃ | CH₃ | CH₃ |
| B-994 | 2-CN | 6-CF₃ | CH₃ | CH₃ |
| B-995 | 3-CN | 6-CF₃ | CH₃ | CH₃ |
| B-996 | 4-CN | 6-CF₃ | CH₃ | CH₃ |
| B-997 | 2-F-4-CN | 6-CF₃ | CH₃ | CH₃ |
| B-998 | 2-Cl-4-CN | 6-CF₃ | CH₃ | CH₃ |
| B-999 | 2-Cl-4-CF3 | 6-CF₃ | CH₃ | CH₃ |
| -1000 | 2-F-4-CF3 | 6-CF₃ | CH₃ | CH₃ |
| -1001 | H | 2-OCH₃ | CH₃ | CH₃ |
| -1002 | 2-F | 2-OCH₃ | CH₃ | CH₃ |
| -1003 | 3-F | 2-OCH₃ | CH₃ | CH₃ |
| -1004 | 4-F | 2-OCH₃ | CH₃ | CH₃ |
| -1005 | 2-Cl | 2-OCH₃ | CH₃ | CH₃ |
| -1006 | 3-Cl | 2-OCH₃ | CH₃ | CH₃ |
| -1007 | 4-Cl | 2-OCH₃ | CH₃ | CH₃ |
| -1008 | 2-CF₃ | 2-OCH₃ | CH₃ | CH₃ |
| -1009 | 3-CF₃ | 2-OCH₃ | CH₃ | CH₃ |
| -1010 | 4-CF₃ | 2-OCH₃ | CH₃ | CH₃ |
| -1011 | 2,4-F₂ | 2-OCH₃ | CH₃ | CH₃ |
| -1012 | 2,4-Cl₂ | 2-OCH₃ | CH₃ | CH₃ |
| -1013 | 2,6-Cl₂ | 2-OCH₃ | CH₃ | CH₃ |
| -1014 | 2-CN | 2-OCH₃ | CH₃ | CH₃ |
| -1015 | 3-CN | 2-OCH₃ | CH₃ | CH₃ |
| -1016 | 4-CN | 2-OCH₃ | CH₃ | CH₃ |
| -1017 | 2-F-4-CN | 2-OCH₃ | CH₃ | CH₃ |
| -1018 | 2-Cl-4-CN | 2-OCH₃ | CH₃ | CH₃ |
| -1019 | 2-Cl-4-CF3 | 2-OCH₃ | CH₃ | CH₃ |
| -1020 | 2-F-4-CF3 | 2-OCH₃ | CH₃ | CH₃ |
| -1021 | H | 6-OCH₃ | CH₃ | CH₃ |
| -1022 | 2-F | 6-OCH₃ | CH₃ | CH₃ |
| -1023 | 3-F | 6-OCH₃ | CH₃ | CH₃ |
| -1024 | 4-F | 6-OCH₃ | CH₃ | CH₃ |
| -1025 | 2-Cl | 6-OCH₃ | CH₃ | CH₃ |
| -1026 | 3-Cl | 6-OCH₃ | CH₃ | CH₃ |
| -1027 | 4-Cl | 6-OCH₃ | CH₃ | CH₃ |
| -1028 | 2-CF₃ | 6-OCH₃ | CH₃ | CH₃ |
| -1029 | 3-CF₃ | 6-OCH₃ | CH₃ | CH₃ |
| -1030 | 4-CF₃ | 6-OCH₃ | CH₃ | CH₃ |
| -1031 | 2,4-F₂ | 6-OCH₃ | CH₃ | CH₃ |
| -1032 | 2,4-Cl₂ | 6-OCH₃ | CH₃ | CH₃ |
| -1033 | 2,6-Cl₂ | 6-OCH₃ | CH₃ | CH₃ |
| -1034 | 2-CN | 6-OCH₃ | CH₃ | CH₃ |
| -1035 | 3-CN | 6-OCH₃ | CH₃ | CH₃ |
| -1036 | 4-CN | 6-OCH₃ | CH₃ | CH₃ |
| -1037 | 2-F-4-CN | 6-OCH₃ | CH₃ | CH₃ |
| -1038 | 2-Cl-4-CN | 6-OCH₃ | CH₃ | CH₃ |
| -1039 | 2-Cl-4-CF3 | 6-OCH₃ | CH₃ | CH₃ |
| -1040 | 2-F-4-CF3 | 6-OCH₃ | CH₃ | CH₃ |
| -1041 | H | H | CH₂CH₃ | CH₂CH₃ |
| -1042 | 2-F | H | CH₂CH₃ | CH₂CH₃ |
| -1043 | 3-F | H | CH₂CH₃ | CH₂CH₃ |
| -1044 | 4-F | H | CH₂CH₃ | CH₂CH₃ |
| -1045 | 2-Cl | H | CH₂CH₃ | CH₂CH₃ |
| -1046 | 3-Cl | H | CH₂CH₃ | CH₂CH₃ |
| -1047 | 4-Cl | H | CH₂CH₃ | CH₂CH₃ |
| -1048 | 2-CF₃ | H | CH₂CH₃ | CH₂CH₃ |
| -1049 | 3-CF₃ | H | CH₂CH₃ | CH₂CH₃ |
| -1050 | 4-CF₃ | H | CH₂CH₃ | CH₂CH₃ |
| -1051 | 2,4-F₂ | H | CH₂CH₃ | CH₂CH₃ |
| -1052 | 2,4-Cl₂ | H | CH₂CH₃ | CH₂CH₃ |
| -1053 | 2,6-Cl₂ | H | CH₂CH₃ | CH₂CH₃ |
| -1054 | 2-CN | H | CH₂CH₃ | CH₂CH₃ |
| -1055 | 3-CN | H | CH₂CH₃ | CH₂CH₃ |
| -1056 | 4-CN | H | CH₂CH₃ | CH₂CH₃ |
| -1057 | 2-F-4-CN | H | CH₂CH₃ | CH₂CH₃ |
| -1058 | 2-Cl-4-CN | H | CH₂CH₃ | CH₂CH₃ |
| -1059 | 2-Cl-4-CF3 | H | CH₂CH₃ | CH₂CH₃ |
| -1060 | 2-F-4-CF3 | H | CH₂CH₃ | CH₂CH₃ |
| -1061 | H | 2-Cl | CH₂CH₃ | CH₂CH₃ |
| -1062 | 2-F | 2-Cl | CH₂CH₃ | CH₂CH₃ |
| -1063 | 3-F | 2-Cl | CH₂CH₃ | CH₂CH₃ |
| -1064 | 4-F | 2-Cl | CH₂CH₃ | CH₂CH₃ |
| -1065 | 2-Cl | 2-Cl | CH₂CH₃ | CH₂CH₃ |
| -1066 | 3-Cl | 2-Cl | CH₂CH₃ | CH₂CH₃ |
| -1067 | 4-Cl | 2-Cl | CH₂CH₃ | CH₂CH₃ |
| -1068 | 2-CF₃ | 2-Cl | CH₂CH₃ | CH₂CH₃ |
| -1069 | 3-CF₃ | 2-Cl | CH₂CH₃ | CH₂CH₃ |
| -1070 | 4-CF₃ | 2-Cl | CH₂CH₃ | CH₂CH₃ |
| -1071 | 2,4-F₂ | 2-Cl | CH₂CH₃ | CH₂CH₃ |
| -1072 | 2,4-Cl₂ | 2-Cl | CH₂CH₃ | CH₂CH₃ |
| -1073 | 2,6-Cl₂ | 2-Cl | CH₂CH₃ | CH₂CH₃ |
| -1074 | 2-CN | 2-Cl | CH₂CH₃ | CH₂CH₃ |
| -1075 | 3-CN | 2-Cl | CH₂CH₃ | CH₂CH₃ |
| -1076 | 4-CN | 2-Cl | CH₂CH₃ | CH₂CH₃ |
| -1077 | 2-F-4-CN | 2-Cl | CH₂CH₃ | CH₂CH₃ |
| -1078 | 2-Cl-4-CN | 2-Cl | CH₂CH₃ | CH₂CH₃ |
| -1079 | 2-Cl-4-CF3 | 2-Cl | CH₂CH₃ | CH₂CH₃ |
| -1080 | 2-F-4-CF3 | 2-Cl | CH₂CH₃ | CH₂CH₃ |
| -1081 | H | 6-Cl | CH₂CH₃ | CH₂CH₃ |
| -1082 | 2-F | 6-Cl | CH₂CH₃ | CH₂CH₃ |
| -1083 | 3-F | 6-Cl | CH₂CH₃ | CH₂CH₃ |
| -1084 | 4-F | 6-Cl | CH₂CH₃ | CH₂CH₃ |
| -1085 | 2-Cl | 6-Cl | CH₂CH₃ | CH₂CH₃ |
| -1086 | 3-Cl | 6-Cl | CH₂CH₃ | CH₂CH₃ |
| -1087 | 4-Cl | 6-Cl | CH₂CH₃ | CH₂CH₃ |
| -1088 | 2-CF₃ | 6-Cl | CH₂CH₃ | CH₂CH₃ |
| -1089 | 3-CF₃ | 6-Cl | CH₂CH₃ | CH₂CH₃ |
| -1090 | 4-CF₃ | 6-Cl | CH₂CH₃ | CH₂CH₃ |
| -1091 | 2,4-F₂ | 6-Cl | CH₂CH₃ | CH₂CH₃ |
| -1092 | 2,4-Cl₂ | 6-Cl | CH₂CH₃ | CH₂CH₃ |
| -1093 | 2,6-Cl₂ | 6-Cl | CH₂CH₃ | CH₂CH₃ |
| -1094 | 2-CN | 6-Cl | CH₂CH₃ | CH₂CH₃ |
| -1095 | 3-CN | 6-Cl | CH₂CH₃ | CH₂CH₃ |
| -1096 | 4-CN | 6-Cl | CH₂CH₃ | CH₂CH₃ |
| -1097 | 2-F-4-CN | 6-Cl | CH₂CH₃ | CH₂CH₃ |
| -1098 | 2-Cl-4-CN | 6-Cl | CH₂CH₃ | CH₂CH₃ |
| -1099 | 2-Cl-4-CF3 | 6-Cl | CH₂CH₃ | CH₂CH₃ |
| -1100 | 2-F-4-CF3 | 6-Cl | CH₂CH₃ | CH₂CH₃ |
| -1101 | H | 2-Br | CH₂CH₃ | CH₂CH₃ |
| -1102 | 2-F | 2-Br | CH₂CH₃ | CH₂CH₃ |
| -1103 | 3-F | 2-Br | CH₂CH₃ | CH₂CH₃ |
| -1104 | 4-F | 2-Br | CH₂CH₃ | CH₂CH₃ |
| -1105 | 2-Cl | 2-Br | CH₂CH₃ | CH₂CH₃ |
| -1106 | 3-Cl | 2-Br | CH₂CH₃ | CH₂CH₃ |
| -1107 | 4-Cl | 2-Br | CH₂CH₃ | CH₂CH₃ |
| -1108 | 2-CF₃ | 2-Br | CH₂CH₃ | CH₂CH₃ |
| -1109 | 3-CF₃ | 2-Br | CH₂CH₃ | CH₂CH₃ |
| -1110 | 4-CF₃ | 2-Br | CH₂CH₃ | CH₂CH₃ |
| -1111 | 2,4-F₂ | 2-Br | CH₂CH₃ | CH₂CH₃ |
| -1112 | 2,4-Cl₂ | 2-Br | CH₂CH₃ | CH₂CH₃ |
| -1113 | 2,6-Cl₂ | 2-Br | CH₂CH₃ | CH₂CH₃ |
| -1114 | 2-CN | 2-Br | CH₂CH₃ | CH₂CH₃ |
| -1115 | 3-CN | 2-Br | CH₂CH₃ | CH₂CH₃ |
| -1116 | 4-CN | 2-Br | CH₂CH₃ | CH₂CH₃ |
| -1117 | 2-F-4-CN | 2-Br | CH₂CH₃ | CH₂CH₃ |
| -1118 | 2-Cl-4-CN | 2-Br | CH₂CH₃ | CH₂CH₃ |
| -1119 | 2-Cl-4-CF3 | 2-Br | CH₂CH₃ | CH₂CH₃ |
| -1120 | 2-F-4-CF3 | 2-Br | CH₂CH₃ | CH₂CH₃ |
| -1121 | H | 6-Br | CH₂CH₃ | CH₂CH₃ |
| -1122 | 2-F | 6-Br | CH₂CH₃ | CH₂CH₃ |
| -1123 | 3-F | 6-Br | CH₂CH₃ | CH₂CH₃ |
| -1124 | 4-F | 6-Br | CH₂CH₃ | CH₂CH₃ |
| -1125 | 2-Cl | 6-Br | CH₂CH₃ | CH₂CH₃ |
| -1126 | 3-Cl | 6-Br | CH₂CH₃ | CH₂CH₃ |
| -1127 | 4-Cl | 6-Br | CH₂CH₃ | CH₂CH₃ |
| -1128 | 2-CF₃ | 6-Br | CH₂CH₃ | CH₂CH₃ |
| -1129 | 3-CF₃ | 6-Br | CH₂CH₃ | CH₂CH₃ |
| -1130 | 4-CF₃ | 6-Br | CH₂CH₃ | CH₂CH₃ |
| -1131 | 2,4-F₂ | 6-Br | CH₂CH₃ | CH₂CH₃ |
| -1132 | 2,4-Cl₂ | 6-Br | CH₂CH₃ | CH₂CH₃ |
| -1133 | 2,6-Cl₂ | 6-Br | CH₂CH₃ | CH₂CH₃ |
| -1134 | 2-CN | 6-Br | CH₂CH₃ | CH₂CH₃ |
| -1135 | 3-CN | 6-Br | CH₂CH₃ | CH₂CH₃ |
| -1136 | 4-CN | 6-Br | CH₂CH₃ | CH₂CH₃ |
| -1137 | 2-F-4-CN | 6-Br | CH₂CH₃ | CH₂CH₃ |
| -1138 | 2-CI-4-CN | 6-Br | CH₂CH₃ | CH₂CH₃ |
| -1139 | 2-CI-4-CF3 | 6-Br | CH₂CH₃ | CH₂CH₃ |
| -1140 | 2-F-4-CF3 | 6-Br | CH₂CH₃ | CH₂CH₃ |
| -1141 | H | 2-F | CH₂CH₃ | CH₂CH₃ |
| -1142 | 2-F | 2-F | CH₂CH₃ | CH₂CH₃ |
| -1143 | 3-F | 2-F | CH₂CH₃ | CH₂CH₃ |
| -1144 | 4-F | 2-F | CH₂CH₃ | CH₂CH₃ |
| -1145 | 2-Cl | 2-F | CH₂CH₃ | CH₂CH₃ |
| -1146 | 3-Cl | 2-F | CH₂CH₃ | CH₂CH₃ |
| -1147 | 4-Cl | 2-F | CH₂CH₃ | CH₂CH₃ |
| -1148 | 2-CF₃ | 2-F | CH₂CH₃ | CH₂CH₃ |
| -1149 | 3-CF₃ | 2-F | CH₂CH₃ | CH₂CH₃ |
| -1150 | 4-CF₃ | 2-F | CH₂CH₃ | CH₂CH₃ |
| -1151 | 2,4-F₂ | 2-F | CH₂CH₃ | CH₂CH₃ |
| -1152 | 2,4-C1₂ | 2-F | CH₂CH₃ | CH₂CH₃ |
| -1153 | 2,6-C1₂ | 2-F | CH₂CH₃ | CH₂CH₃ |
| -1154 | 2-CN | 2-F | CH₂CH₃ | CH₂CH₃ |
| -1155 | 3-CN | 2-F | CH₂CH₃ | CH₂CH₃ |
| -1156 | 4-CN | 2-F | CH₂CH₃ | CH₂CH₃ |
| -1157 | 2-F-4-CN | 2-F | CH₂CH₃ | CH₂CH₃ |
| -1158 | 2-Cl-4-CN | 2-F | CH₂CH₃ | CH₂CH₃ |
| -1159 | 2-CI-4-CF3 | 2-F | CH₂CH₃ | CH₂CH₃ |
| -1160 | 2-F-4-CF3 | 2-F | CH₂CH₃ | CH₂CH₃ |
| -1161 | H | 6-F | CH₂CH₃ | CH₂CH₃ |
| -1162 | 2-F | 6-F | CH₂CH₃ | CH₂CH₃ |
| -1163 | 3-F | 6-F | CH₂CH₃ | CH₂CH₃ |
| -1164 | 4-F | 6-F | CH₂CH₃ | CH₂CH₃ |
| -1165 | 2-Cl | 6-F | CH₂CH₃ | CH₂CH₃ |
| -1166 | 3-Cl | 6-F | CH₂CH₃ | CH₂CH₃ |
| -1167 | 4-Cl | 6-F | CH₂CH₃ | CH₂CH₃ |
| -1168 | 2-CF₃ | 6-F | CH₂CH₃ | CH₂CH₃ |
| -1169 | 3-CF₃ | 6-F | CH₂CH₃ | CH₂CH₃ |
| -1170 | 4-CF₃ | 6-F | CH₂CH₃ | CH₂CH₃ |
| -1171 | 2,4-F₂ | 6-F | CH₂CH₃ | CH₂CH₃ |
| -1172 | 2,4-Cl₂ | 6-F | CH₂CH₃ | CH₂CH₃ |
| -1173 | 2,6-Cl₂ | 6-F | CH₂CH₃ | CH₂CH₃ |
| -1174 | 2-CN | 6-F | CH₂CH₃ | CH₂CH₃ |
| -1175 | 3-CN | 6-F | CH₂CH₃ | CH₂CH₃ |
| -1176 | 4-CN | 6-F | CH₂CH₃ | CH₂CH₃ |
| -1177 | 2-F-4-CN | 6-F | CH₂CH₃ | CH₂CH₃ |
| -1178 | 2-Cl-4-CN | 6-F | CH₂CH₃ | CH₂CH₃ |
| -1179 | 2-Cl-4-CF3 | 6-F | CH₂CH₃ | CH₂CH₃ |
| -1180 | 2-F-4-CF3 | 6-F | CH₂CH₃ | CH₂CH₃ |
| -1181 | H | 2-CN | CH₂CH₃ | CH₂CH₃ |
| -1182 | 2-F | 2-CN | CH₂CH₃ | CH₂CH₃ |
| -1183 | 3-F | 2-CN | CH₂CH₃ | CH₂CH₃ |
| -1184 | 4-F | 2-CN | CH₂CH₃ | CH₂CH₃ |
| -1185 | 2-Cl | 2-CN | CH₂CH₃ | CH₂CH₃ |
| -1186 | 3-Cl | 2-CN | CH₂CH₃ | CH₂CH₃ |
| -1187 | 4-Cl | 2-CN | CH₂CH₃ | CH₂CH₃ |
| -1188 | 2-CF₃ | 2-CN | CH₂CH₃ | CH₂CH₃ |
| -1189 | 3-CF₃ | 2-CN | CH₂CH₃ | CH₂CH₃ |
| -1190 | 4-CF₃ | 2-CN | CH₂CH₃ | CH₂CH₃ |
| -1191 | 2,4-F₂ | 2-CN | CH₂CH₃ | CH₂CH₃ |
| -1192 | 2,4-Cl₂ | 2-CN | CH₂CH₃ | CH₂CH₃ |
| -1193 | 2,6-Cl₂ | 2-CN | CH₂CH₃ | CH₂CH₃ |
| -1194 | 2-CN | 2-CN | CH₂CH₃ | CH₂CH₃ |
| -1195 | 3-CN | 2-CN | CH₂CH₃ | CH₂CH₃ |
| -1196 | 4-CN | 2-CN | CH₂CH₃ | CH₂CH₃ |
| -1197 | 2-F-4-CN | 2-CN | CH₂CH₃ | CH₂CH₃ |
| -1198 | 2-Cl-4-CN | 2-CN | CH₂CH₃ | CH₂CH₃ |
| -1199 | 2-Cl-4-CF3 | 2-CN | CH₂CH₃ | CH₂CH₃ |
| -1200 | 2-F-4-CF3 | 2-CN | CH₂CH₃ | CH₂CH₃ |
| -1201 | H | 6-CN | CH₂CH₃ | CH₂CH₃ |
| -1202 | 2-F | 6-CN | CH₂CH₃ | CH₂CH₃ |
| -1203 | 3-F | 6-CN | CH₂CH₃ | CH₂CH₃ |
| -1204 | 4-F | 6-CN | CH₂CH₃ | CH₂CH₃ |
| -1205 | 2-Cl | 6-CN | CH₂CH₃ | CH₂CH₃ |
| -1206 | 3-Cl | 6-CN | CH₂CH₃ | CH₂CH₃ |
| -1207 | 4-Cl | 6-CN | CH₂CH₃ | CH₂CH₃ |
| -1208 | 2-CF₃ | 6-CN | CH₂CH₃ | CH₂CH₃ |
| -1209 | 3-CF₃ | 6-CN | CH₂CH₃ | CH₂CH₃ |
| -1210 | 4-CF₃ | 6-CN | CH₂CH₃ | CH₂CH₃ |
| -1211 | 2,4-F₂ | 6-CN | CH₂CH₃ | CH₂CH₃ |
| -1212 | 2,4-Cl₂ | 6-CN | CH₂CH₃ | CH₂CH₃ |
| -1213 | 2,6-Cl₂ | 6-CN | CH₂CH₃ | CH₂CH₃ |
| -1214 | 2-CN | 6-CN | CH₂CH₃ | CH₂CH₃ |
| -1215 | 3-CN | 6-CN | CH₂CH₃ | CH₂CH₃ |
| -1216 | 4-CN | 6-CN | CH₂CH₃ | CH₂CH₃ |
| -1217 | 2-F-4-CN | 6-CN | CH₂CH₃ | CH₂CH₃ |
| -1218 | 2-Cl-4-CN | 6-CN | CH₂CH₃ | CH₂CH₃ |
| -1219 | 2-Cl-4-CF3 | 6-CN | CH₂CH₃ | CH₂CH₃ |
| -1220 | 2-F-4-CF3 | 6-CN | CH₂CH₃ | CH₂CH₃ |
| -1221 | H | 2-CF₃ | CH₂CH₃ | CH₂CH₃ |
| -1222 | 2-F | 2-CF₃ | CH₂CH₃ | CH₂CH₃ |
| -1223 | 3-F | 2-CF₃ | CH₂CH₃ | CH₂CH₃ |
| -1224 | 4-F | 2-CF₃ | CH₂CH₃ | CH₂CH₃ |
| -1225 | 2-Cl | 2-CF₃ | CH₂CH₃ | CH₂CH₃ |
| -1226 | 3-Cl | 2-CF₃ | CH₂CH₃ | CH₂CH₃ |
| -1227 | 4-Cl | 2-CF₃ | CH₂CH₃ | CH₂CH₃ |
| -1228 | 2-CF₃ | 2-CF₃ | CH₂CH₃ | CH₂CH₃ |
| -1229 | 3-CF₃ | 2-CF₃ | CH₂CH₃ | CH₂CH₃ |
| -1230 | 4-CF₃ | 2-CF₃ | CH₂CH₃ | CH₂CH₃ |
| -1231 | 2,4-F₂ | 2-CF₃ | CH₂CH₃ | CH₂CH₃ |
| -1232 | 2,4-Cl₂ | 2-CF₃ | CH₂CH₃ | CH₂CH₃ |
| -1233 | 2,6-Cl₂ | 2-CF₃ | CH₂CH₃ | CH₂CH₃ |
| -1234 | 2-CN | 2-CF₃ | CH₂CH₃ | CH₂CH₃ |
| -1235 | 3-CN | 2-CF₃ | CH₂CH₃ | CH₂CH₃ |
| -1236 | 4-CN | 2-CF₃ | CH₂CH₃ | CH₂CH₃ |
| -1237 | 2-F-4-CN | 2-CF₃ | CH₂CH₃ | CH₂CH₃ |
| -1238 | 2-Cl-4-CN | 2-CF₃ | CH₂CH₃ | CH₂CH₃ |
| -1239 | 2-Cl-4-CF3 | 2-CF₃ | CH₂CH₃ | CH₂CH₃ |
| -1240 | 2-F-4-CF3 | 2-CF₃ | CH₂CH₃ | CH₂CH₃ |
| -1241 | H | 6-CF₃ | CH₂CH₃ | CH₂CH₃ |
| -1242 | 2-F | 6-CF₃ | CH₂CH₃ | CH₂CH₃ |
| -1243 | 3-F | 6-CF₃ | CH₂CH₃ | CH₂CH₃ |
| -1244 | 4-F | 6-CF₃ | CH₂CH₃ | CH₂CH₃ |
| -1245 | 2-Cl | 6-CF₃ | CH₂CH₃ | CH₂CH₃ |
| -1246 | 3-Cl | 6-CF₃ | CH₂CH₃ | CH₂CH₃ |
| -1247 | 4-Cl | 6-CF₃ | CH₂CH₃ | CH₂CH₃ |
| -1248 | 2-CF₃ | 6-CF₃ | CH₂CH₃ | CH₂CH₃ |
| -1249 | 3-CF₃ | 6-CF₃ | CH₂CH₃ | CH₂CH₃ |
| -1250 | 4-CF₃ | 6-CF₃ | CH₂CH₃ | CH₂CH₃ |
| -1251 | 2,4-F₂ | 6-CF₃ | CH₂CH₃ | CH₂CH₃ |
| -1252 | 2,4-Cl₂ | 6-CF₃ | CH₂CH₃ | CH₂CH₃ |
| -1253 | 2,6-Cl₂ | 6-CF₃ | CH₂CH₃ | CH₂CH₃ |
| -1254 | 2-CN | 6-CF₃ | CH₂CH₃ | CH₂CH₃ |
| -1255 | 3-CN | 6-CF₃ | CH₂CH₃ | CH₂CH₃ |
| -1256 | 4-CN | 6-CF₃ | CH₂CH₃ | CH₂CH₃ |
| -1257 | 2-F-4-CN | 6-CF₃ | CH₂CH₃ | CH₂CH₃ |
| -1258 | 2-Cl-4-CN | 6-CF₃ | CH₂CH₃ | CH₂CH₃ |
| -1259 | 2-Cl-4-CF3 | 6-CF₃ | CH₂CH₃ | CH₂CH₃ |
| -1260 | 2-F-4-CF3 | 6-CF₃ | CH₂CH₃ | CH₂CH₃ |
| -1261 | H | 2-OCH₃ | CH₂CH₃ | CH₂CH₃ |
| -1262 | 2-F | 2-OCH₃ | CH₂CH₃ | CH₂CH₃ |
| -1263 | 3-F | 2-OCH₃ | CH₂CH₃ | CH₂CH₃ |
| -1264 | 4-F | 2-OCH₃ | CH₂CH₃ | CH₂CH₃ |
| -1265 | 2-Cl | 2-OCH₃ | CH₂CH₃ | CH₂CH₃ |
| -1266 | 3-Cl | 2-OCH₃ | CH₂CH₃ | CH₂CH₃ |
| -1267 | 4-Cl | 2-OCH₃ | CH₂CH₃ | CH₂CH₃ |
| -1268 | 2-CF₃ | 2-OCH₃ | CH₂CH₃ | CH₂CH₃ |
| -1269 | 3-CF₃ | 2-OCH₃ | CH₂CH₃ | CH₂CH₃ |
| -1270 | 4-CF₃ | 2-OCH₃ | CH₂CH₃ | CH₂CH₃ |
| -1271 | 2,4-F₂ | 2-OCH₃ | CH₂CH₃ | CH₂CH₃ |
| -1272 | 2,4-Cl₂ | 2-OCH₃ | CH₂CH₃ | CH₂CH₃ |
| -1273 | 2,6-Cl₂ | 2-OCH₃ | CH₂CH₃ | CH₂CH₃ |
| -1274 | 2-CN | 2-OCH₃ | CH₂CH₃ | CH₂CH₃ |
| -1275 | 3-CN | 2-OCH₃ | CH₂CH₃ | CH₂CH₃ |
| -1276 | 4-CN | 2-OCH₃ | CH₂CH₃ | CH₂CH₃ |
| -1277 | 2-F-4-CN | 2-OCH₃ | CH₂CH₃ | CH₂CH₃ |
| -1278 | 2-Cl-4-CN | 2-OCH₃ | CH₂CH₃ | CH₂CH₃ |
| -1279 | 2-Cl-4-CF3 | 2-OCH₃ | CH₂CH₃ | CH₂CH₃ |
| -1280 | 2-F-4-CF3 | 2-OCH₃ | CH₂CH₃ | CH₂CH₃ |
| -1281 | H | 6-OCH₃ | CH₂CH₃ | CH₂CH₃ |
| -1282 | 2-F | 6-OCH₃ | CH₂CH₃ | CH₂CH₃ |
| -1283 | 3-F | 6-OCH₃ | CH₂CH₃ | CH₂CH₃ |
| -1284 | 4-F | 6-OCH₃ | CH₂CH₃ | CH₂CH₃ |
| -1285 | 2-Cl | 6-OCH₃ | CH₂CH₃ | CH₂CH₃ |
| -1286 | 3-Cl | 6-OCH₃ | CH₂CH₃ | CH₂CH₃ |
| -1287 | 4-Cl | 6-OCH₃ | CH₂CH₃ | CH₂CH₃ |
| -1288 | 2-CF₃ | 6-OCH₃ | CH₂CH₃ | CH₂CH₃ |
| -1289 | 3-CF₃ | 6-OCH₃ | CH₂CH₃ | CH₂CH₃ |
| -1290 | 4-CF₃ | 6-OCH₃ | CH₂CH₃ | CH₂CH₃ |
| -1291 | 2,4-F₂ | 6-OCH₃ | CH₂CH₃ | CH₂CH₃ |
| -1292 | 2,4-Cl₂ | 6-OCH₃ | CH₂CH₃ | CH₂CH₃ |
| -1293 | 2,6-Cl₂ | 6-OCH₃ | CH₂CH₃ | CH₂CH₃ |
| -1294 | 2-CN | 6-OCH₃ | CH₂CH₃ | CH₂CH₃ |
| -1295 | 3-CN | 6-OCH₃ | CH₂CH₃ | CH₂CH₃ |
| -1296 | 4-CN | 6-OCH₃ | CH₂CH₃ | CH₂CH₃ |
| -1297 | 2-F-4-CN | 6-OCH₃ | CH₂CH₃ | CH₂CH₃ |
| -1298 | 2-Cl-4-CN | 6-OCH₃ | CH₂CH₃ | CH₂CH₃ |
| -1299 | 2-Cl-4-CF3 | 6-OCH₃ | CH₂CH₃ | CH₂CH₃ |
| -1300 | 2-F-4-CF3 | 6-OCH₃ | CH₂CH₃ | CH₂CH₃ |
| -1301 | H | H | -CH₂-CH₂- | |
| -1302 | 2-F | H | -CH₂-CH₂- | |
| -1303 | 3-F | H | -CH₂-CH₂- | |
| -1304 | 4-F | H | -CH₂-CH₂- | |
| -1305 | 2-Cl | H | -CH₂-CH₂- | |
| -1306 | 3-Cl | H | -CH₂-CH₂- | |
| -1307 | 4-Cl | H | -CH₂-CH₂- | |
| -1308 | 2-CF₃ | H | -CH₂-CH₂- | |
| -1309 | 3-CF₃ | H | -CH₂-CH₂- | |
| -1310 | 4-CF₃ | H | -CH₂-CH₂- | |
| -1311 | 2,4-F₂ | H | -CH₂-CH₂- | |
| -1312 | 2,4-Cl₂ | H | -CH₂-CH₂- | |
| -1313 | 2,6-Cl₂ | H | -CH₂-CH₂- | |
| -1314 | 2-CN | H | -CH₂-CH₂- | |
| -1315 | 3-CN | H | -CH₂-CH₂- | |
| -1316 | 4-CN | H | -CH₂-CH₂- | |
| -1317 | 2-F-4-CN | H | -CH₂-CH₂- | |
| -1318 | 2-Cl-4-CN | H | -CH₂-CH₂- | |
| -1319 | 2-Cl-4-CF3 | H | -CH₂-CH₂- | |
| -1320 | 2-F-4-CF3 | H | -CH₂-CH₂- | |
| -1321 | H | 2-Cl | -CH₂-CH₂- | |
| -1322 | 2-F | 2-Cl | -CH₂-CH₂- | |
| -1323 | 3-F | 2-Cl | -CH₂-CH₂- | |
| -1324 | 4-F | 2-Cl | -CH₂-CH₂- | |
| -1325 | 2-Cl | 2-Cl | -CH₂-CH₂- | |
| -1326 | 3-Cl | 2-Cl | -CH₂-CH₂- | |
| -1327 | 4-Cl | 2-Cl | -CH₂-CH₂- | |
| -1328 | 2-CF₃ | 2-Cl | -CH₂-CH₂- | |
| -1329 | 3-CF₃ | 2-Cl | -CH₂-CH₂- | |
| -1330 | 4-CF₃ | 2-Cl | -CH₂-CH₂- | |
| -1331 | 2,4-F₂ | 2-Cl | -CH₂-CH₂- | |
| -1332 | 2,4-Cl₂ | 2-Cl | -CH₂-CH₂- | |
| -1333 | 2,6-Cl₂ | 2-Cl | -CH₂-CH₂- | |
| -1334 | 2-CN | 2-Cl | -CH₂-CH₂- | |
| -1335 | 3-CN | 2-Cl | -CH₂-CH₂- | |
| -1336 | 4-CN | 2-Cl | -CH₂-CH₂- | |
| -1337 | 2-F-4-CN | 2-Cl | -CH₂-CH₂- | |
| -1338 | 2-Cl-4-CN | 2-Cl | -CH₂-CH₂- | |
| -1339 | 2-Cl-4-CF3 | 2-Cl | -CH₂-CH₂- | |
| -1340 | 2-F-4-CF3 | 2-Cl | -CH₂-CH₂- | |
| -1341 | H | 6-Cl | -CH₂-CH₂- | |
| -1342 | 2-F | 6-Cl | -CH₂-CH₂- | |
| -1343 | 3-F | 6-Cl | -CH₂-CH₂- | |
| -1344 | 4-F | 6-Cl | -CH₂-CH₂- | |
| -1345 | 2-Cl | 6-Cl | -CH₂-CH₂- | |
| -1346 | 3-Cl | 6-Cl | -CH₂-CH₂- | |
| -1347 | 4-Cl | 6-Cl | -CH₂-CH₂- | |
| -1348 | 2-CF₃ | 6-Cl | -CH₂-CH₂- | |
| -1349 | 3-CF₃ | 6-Cl | -CH₂-CH₂- | |
| -1350 | 4-CF₃ | 6-Cl | -CH₂-CH₂- | |
| -1351 | 2,4-F₂ | 6-Cl | -CH₂-CH₂- | |
| -1352 | 2,4-Cl₂ | 6-Cl | -CH₂-CH₂- | |
| -1353 | 2,6-Cl₂ | 6-Cl | -CH₂-CH₂- | |
| -1354 | 2-CN | 6-Cl | -CH₂-CH₂- | |
| -1355 | 3-CN | 6-Cl | -CH₂-CH₂- | |
| -1356 | 4-CN | 6-Cl | -CH₂-CH₂- | |
| -1357 | 2-F-4-CN | 6-Cl | -CH₂-CH₂- | |
| -1358 | 2-Cl-4-CN | 6-Cl | -CH₂-CH₂- | |
| -1359 | 2-Cl-4-CF3 | 6-Cl | -CH₂-CH₂- | |
| -1360 | 2-F-4-CF3 | 6-Cl | -CH₂-CH₂- | |
| -1361 | H | 2-Br | -CH₂-CH₂- | |
| -1362 | 2-F | 2-Br | -CH₂-CH₂- | |
| -1363 | 3-F | 2-Br | -CH₂-CH₂- | |
| -1364 | 4-F | 2-Br | -CH₂-CH₂- | |
| -1365 | 2-Cl | 2-Br | -CH₂-CH₂- | |
| -1366 | 3-Cl | 2-Br | -CH₂-CH₂- | |
| -1367 | 4-Cl | 2-Br | -CH₂-CH₂- | |
| -1368 | 2-CF₃ | 2-Br | -CH₂-CH₂- | |
| -1369 | 3-CF₃ | 2-Br | -CH₂-CH₂- | |
| -1370 | 4-CF₃ | 2-Br | -CH₂-CH₂- | |
| -1371 | 2,4-F₂ | 2-Br | -CH₂-CH₂- | |
| -1372 | 2,4-Cl₂ | 2-Br | -CH₂-CH₂- | |
| -1373 | 2,6-Cl₂ | 2-Br | -CH₂-CH₂- | |
| -1374 | 2-CN | 2-Br | -CH₂-CH₂- | |
| -1375 | 3-CN | 2-Br | -CH₂-CH₂- | |
| -1376 | 4-CN | 2-Br | -CH₂-CH₂- | |
| -1377 | 2-F-4-CN | 2-Br | -CH₂-CH₂- | |
| -1378 | 2-Cl-4-CN | 2-Br | -CH₂-CH₂- | |
| -1379 | 2-Cl-4-CF3 | 2-Br | -CH₂-CH₂- | |
| -1380 | 2-F-4-CF3 | 2-Br | -CH₂-CH₂- | |
| -1381 | H | 6-Br | -CH₂-CH₂- | |
| -1382 | 2-F | 6-Br | -CH₂-CH₂- | |
| -1383 | 3-F | 6-Br | -CH₂-CH₂- | |
| -1384 | 4-F | 6-Br | -CH₂-CH₂- | |
| -1385 | 2-Cl | 6-Br | -CH₂-CH₂- | |
| -1386 | 3-Cl | 6-Br | -CH₂-CH₂- | |
| -1387 | 4-Cl | 6-Br | -CH₂-CH₂- | |
| -1388 | 2-CF₃ | 6-Br | -CH₂-CH₂- | |
| -1389 | 3-CF₃ | 6-Br | -CH₂-CH₂- | |
| -1390 | 4-CF₃ | 6-Br | -CH₂-CH₂- | |
| -1391 | 2,4-F₂ | 6-Br | -CH₂-CH₂- | |
| -1392 | 2,4-Cl₂ | 6-Br | -CH₂-CH₂- | |
| -1393 | 2,6-Cl₂ | 6-Br | -CH₂-CH₂- | |
| -1394 | 2-CN | 6-Br | -CH₂-CH₂- | |
| -1395 | 3-CN | 6-Br | -CH₂-CH₂- | |
| -1396 | 4-CN | 6-Br | -CH₂-CH₂- | |
| -1397 | 2-F-4-CN | 6-Br | -CH₂-CH₂- | |
| -1398 | 2-Cl-4-CN | 6-Br | -CH₂-CH₂- | |
| -1399 | 2-Cl-4-CF3 | 6-Br | -CH₂-CH₂- | |
| -1400 | 2-F-4-CF3 | 6-Br | -CH₂-CH₂- | |
| -1401 | H | 2-F | -CH₂-CH₂- | |
| -1402 | 2-F | 2-F | -CH₂-CH₂- | |
| -1403 | 3-F | 2-F | -CH₂-CH₂- | |
| -1404 | 4-F | 2-F | -CH₂-CH₂- | |
| -1405 | 2-Cl | 2-F | -CH₂-CH₂- | |
| -1406 | 3-Cl | 2-F | -CH₂-CH₂- | |
| -1407 | 4-Cl | 2-F | -CH₂-CH₂- | |
| -1408 | 2-CF₃ | 2-F | -CH₂-CH₂- | |
| -1409 | 3-CF₃ | 2-F | -CH₂-CH₂- | |
| -1410 | 4-CF₃ | 2-F | -CH₂-CH₂- | |
| -1411 | 2,4-F₂ | 2-F | -CH₂-CH₂- | |
| -1412 | 2,4-Cl₂ | 2-F | -CH₂-CH₂- | |
| -1413 | 2,6-Cl₂ | 2-F | -CH₂-CH₂- | |
| -1414 | 2-CN | 2-F | -CH₂-CH₂- | |
| -1415 | 3-CN | 2-F | -CH₂-CH₂- | |
| -1416 | 4-CN | 2-F | -CH₂-CH₂- | |
| -1417 | 2-F-4-CN | 2-F | -CH₂-CH₂- | |
| -1418 | 2-Cl-4-CN | 2-F | -CH₂-CH₂- | |
| -1419 | 2-Cl-4-CF3 | 2-F | -CH₂-CH₂- | |
| -1420 | 2-F-4-CF3 | 2-F | -CH₂-CH₂- | |
| -1421 | H | 6-F | -CH₂-CH₂- | |
| -1422 | 2-F | 6-F | -CH₂-CH₂- | |
| -1423 | 3-F | 6-F | -CH₂-CH₂- | |
| -1424 | 4-F | 6-F | -CH₂-CH₂- | |
| -1425 | 2-Cl | 6-F | -CH₂-CH₂- | |
| -1426 | 3-Cl | 6-F | -CH₂-CH₂- | |
| -1427 | 4-Cl | 6-F | -CH₂-CH₂- | |
| -1428 | 2-CF₃ | 6-F | -CH₂-CH₂- | |
| -1429 | 3-CF₃ | 6-F | -CH₂-CH₂- | |
| -1430 | 4-CF₃ | 6-F | -CH₂-CH₂- | |
| -1431 | 2,4-F₂ | 6-F | -CH₂-CH₂- | |
| -1432 | 2,4-Cl₂ | 6-F | -CH₂-CH₂- | |
| -1433 | 2,6-Cl₂ | 6-F | -CH₂-CH₂- | |
| -1434 | 2-CN | 6-F | -CH₂-CH₂- | |
| -1435 | 3-CN | 6-F | -CH₂-CH₂- | |
| -1436 | 4-CN | 6-F | -CH₂-CH₂- | |
| -1437 | 2-F-4-CN | 6-F | -CH₂-CH₂- | |
| -1438 | 2-Cl-4-CN | 6-F | -CH₂-CH₂- | |
| -1439 | 2-Cl-4-CF3 | 6-F | -CH₂-CH₂- | |
| -1440 | 2-F-4-CF3 | 6-F | -CH₂-CH₂- | |
| -1441 | H | 2-CN | -CH₂-CH₂- | |
| -1442 | 2-F | 2-CN | -CH₂-CH₂- | |
| -1443 | 3-F | 2-CN | -CH₂-CH₂- | |
| -1444 | 4-F | 2-CN | -CH₂-CH₂- | |
| -1445 | 2-Cl | 2-CN | -CH₂-CH₂- | |
| -1446 | 3-Cl | 2-CN | -CH₂-CH₂- | |
| -1447 | 4-Cl | 2-CN | -CH₂-CH₂- | |
| -1448 | 2-CF₃ | 2-CN | -CH₂-CH₂- | |
| -1449 | 3-CF₃ | 2-CN | -CH₂-CH₂- | |
| -1450 | 4-CF₃ | 2-CN | -CH₂-CH₂- | |
| -1451 | 2,4-F₂ | 2-CN | -CH₂-CH₂- | |
| -1452 | 2,4-Cl₂ | 2-CN | -CH₂-CH₂- | |
| -1453 | 2,6-Cl₂ | 2-CN | -CH₂-CH₂- | |
| -1454 | 2-CN | 2-CN | -CH₂-CH₂- | |
| -1455 | 3-CN | 2-CN | -CH₂-CH₂- | |
| -1456 | 4-CN | 2-CN | -CH₂-CH₂- | |
| -1457 | 2-F-4-CN | 2-CN | -CH₂-CH₂- | |
| -1458 | 2-Cl-4-CN | 2-CN | -CH₂-CH₂- | |
| -1459 | 2-Cl-4-CF3 | 2-CN | -CH₂-CH₂- | |
| -1460 | 2-F-4-CF3 | 2-CN | -CH₂-CH₂- | |
| -1461 | H | 6-CN | -CH₂-CH₂- | |
| -1462 | 2-F | 6-CN | -CH₂-CH₂- | |
| -1463 | 3-F | 6-CN | -CH₂-CH₂- | |
| -1464 | 4-F | 6-CN | -CH₂-CH₂- | |
| -1465 | 2-Cl | 6-CN | -CH₂-CH₂- | |
| -1466 | 3-Cl | 6-CN | -CH₂-CH₂- | |
| -1467 | 4-Cl | 6-CN | -CH₂-CH₂- | |
| -1468 | 2-CF₃ | 6-CN | -CH₂-CH₂- | |
| -1469 | 3-CF₃ | 6-CN | -CH₂-CH₂- | |
| -1470 | 4-CF₃ | 6-CN | -CH₂-CH₂- | |
| -1471 | 2,4-F₂ | 6-CN | -CH₂-CH₂- | |
| -1472 | 2,4-Cl₂ | 6-CN | -CH₂-CH₂- | |
| -1473 | 2,6-Cl₂ | 6-CN | -CH₂-CH₂- | |
| -1474 | 2-CN | 6-CN | -CH₂-CH₂- | |
| -1475 | 3-CN | 6-CN | -CH₂-CH₂- | |
| -1476 | 4-CN | 6-CN | -CH₂-CH₂- | |
| -1477 | 2-F-4-CN | 6-CN | -CH₂-CH₂- | |
| -1478 | 2-Cl-4-CN | 6-CN | -CH₂-CH₂- | |
| -1479 | 2-Cl-4-CF3 | 6-CN | -CH₂-CH₂- | |
| -1480 | 2-F-4-CF3 | 6-CN | -CH₂-CH₂- | |
| -1481 | H | 2-CF₃ | -CH₂-CH₂- | |
| -1482 | 2-F | 2-CF₃ | -CH₂-CH₂- | |
| -1483 | 3-F | 2-CF₃ | -CH₂-CH₂- | |
| -1484 | 4-F | 2-CF₃ | -CH₂-CH₂- | |
| -1485 | 2-Cl | 2-CF₃ | -CH₂-CH₂- | |
| -1486 | 3-Cl | 2-CF₃ | -CH₂-CH₂- | |
| -1487 | 4-Cl | 2-CF₃ | -CH₂-CH₂- | |
| -1488 | 2-CF₃ | 2-CF₃ | -CH₂-CH₂- | |
| -1489 | 3-CF₃ | 2-CF₃ | -CH₂-CH₂- | |
| -1490 | 4-CF₃ | 2-CF₃ | -CH₂-CH₂- | |
| -1491 | 2,4-F₂ | 2-CF₃ | -CH₂-CH₂- | |
| -1492 | 2,4-Cl₂ | 2-CF₃ | -CH₂-CH₂- | |
| -1493 | 2,6-Cl₂ | 2-CF₃ | -CH₂-CH₂- | |
| -1494 | 2-CN | 2-CF₃ | -CH₂-CH₂- | |
| -1495 | 3-CN | 2-CF₃ | -CH₂-CH₂- | |
| -1496 | 4-CN | 2-CF₃ | -CH₂-CH₂- | |
| -1497 | 2-F-4-CN | 2-CF₃ | -CH₂-CH₂- | |
| -1498 | 2-Cl-4-CN | 2-CF₃ | -CH₂-CH₂- | |
| -1499 | 2-Cl-4-CF3 | 2-CF₃ | -CH₂-CH₂- | |
| -1500 | 2-F-4-CF3 | 2-CF₃ | -CH₂-CH₂- | |
| -1501 | H | 6-CF₃ | -CH₂-CH₂- | |
| -1502 | 2-F | 6-CF₃ | -CH₂-CH₂- | |
| -1503 | 3-F | 6-CF₃ | -CH₂-CH₂- | |
| -1504 | 4-F | 6-CF₃ | -CH₂-CH₂- | |
| -1505 | 2-Cl | 6-CF₃ | -CH₂-CH₂- | |
| -1506 | 3-Cl | 6-CF₃ | -CH₂-CH₂- | |
| -1507 | 4-Cl | 6-CF₃ | -CH₂-CH₂- | |
| -1508 | 2-CF₃ | 6-CF₃ | -CH₂-CH₂- | |
| -1509 | 3-CF₃ | 6-CF₃ | -CH₂-CH₂- | |
| -1510 | 4-CF₃ | 6-CF₃ | -CH₂-CH₂- | |
| -1511 | 2,4-F₂ | 6-CF₃ | -CH₂-CH₂- | |
| -1512 | 2,4-Cl₂ | 6-CF₃ | -CH₂-CH₂- | |
| -1513 | 2,6-Cl₂ | 6-CF₃ | -CH₂-CH₂- | |
| -1514 | 2-CN | 6-CF₃ | -CH₂-CH₂- | |
| -1515 | 3-CN | 6-CF₃ | -CH₂-CH₂- | |
| -1516 | 4-CN | 6-CF₃ | -CH₂-CH₂- | |
| -1517 | 2-F-4-CN | 6-CF₃ | -CH₂-CH₂- | |
| -1518 | 2-Cl-4-CN | 6-CF₃ | -CH₂-CH₂- | |
| -1519 | 2-Cl-4-CF3 | 6-CF₃ | -CH₂-CH₂- | |
| -1520 | 2-F-4-CF3 | 6-CF₃ | -CH₂-CH₂- | |
| -1521 | H | 2-OCH₃ | -CH₂-CH₂- | |
| -1522 | 2-F | 2-OCH₃ | -CH₂-CH₂- | |
| -1523 | 3-F | 2-OCH₃ | -CH₂-CH₂- | |
| -1524 | 4-F | 2-OCH₃ | -CH₂-CH₂- | |
| -1525 | 2-Cl | 2-OCH₃ | -CH₂-CH₂- | |
| -1526 | 3-Cl | 2-OCH₃ | -CH₂-CH₂- | |
| -1527 | 4-Cl | 2-OCH₃ | -CH₂-CH₂- | |
| -1528 | 2-CF₃ | 2-OCH₃ | -CH₂-CH₂- | |
| -1529 | 3-CF₃ | 2-OCH₃ | -CH₂-CH₂- | |
| -1530 | 4-CF₃ | 2-OCH₃ | -CH₂-CH₂- | |
| -1531 | 2,4-F₂ | 2-OCH₃ | -CH₂-CH₂- | |
| -1532 | 2,4-Cl₂ | 2-OCH₃ | -CH₂-CH₂- | |
| -1533 | 2,6-Cl₂ | 2-OCH₃ | -CH₂-CH₂- | |
| -1534 | 2-CN | 2-OCH₃ | -CH₂-CH₂- | |
| -1535 | 3-CN | 2-OCH₃ | -CH₂-CH₂- | |
| -1536 | 4-CN | 2-OCH₃ | -CH₂-CH₂- | |
| -1537 | 2-F-4-CN | 2-OCH₃ | -CH₂-CH₂- | |
| -1538 | 2-Cl-4-CN | 2-OCH₃ | -CH₂-CH₂- | |
| -1539 | 2-Cl-4-CF3 | 2-OCH₃ | -CH₂-CH₂- | |
| -1540 | 2-F-4-CF3 | 2-OCH₃ | -CH₂-CH₂- | |
| -1541 | H | 6-OCH₃ | -CH₂-CH₂- | |
| -1542 | 2-F | 6-OCH₃ | -CH₂-CH₂- | |
| -1543 | 3-F | 6-OCH₃ | -CH₂-CH₂- | |
| -1544 | 4-F | 6-OCH₃ | -CH₂-CH₂- | |
| -1545 | 2-Cl | 6-OCH₃ | -CH₂-CH₂- | |
| -1546 | 3-Cl | 6-OCH₃ | -CH₂-CH₂- | |
| -1547 | 4-Cl | 6-OCH₃ | -CH₂-CH₂- | |
| -1548 | 2-CF₃ | 6-OCH₃ | -CH₂-CH₂- | |
| -1549 | 3-CF₃ | 6-OCH₃ | -CH₂-CH₂- | |
| -1550 | 4-CF₃ | 6-OCH₃ | -CH₂-CH₂- | |
| -1551 | 2,4-F₂ | 6-OCH₃ | -CH₂-CH₂- | |
| -1552 | 2,4-Cl₂ | 6-OCH₃ | -CH₂-CH₂- | |
| -1553 | 2,6-Cl₂ | 6-OCH₃ | -CH₂-CH₂- | |
| -1554 | 2-CN | 6-OCH₃ | -CH₂-CH₂- | |
| -1555 | 3-CN | 6-OCH₃ | -CH₂-CH₂- | |
| -1556 | 4-CN | 6-OCH₃ | -CH₂-CH₂- | |
| -1557 | 2-F-4-CN | 6-OCH₃ | -CH₂-CH₂- | |
| -1558 | 2-Cl-4-CN | 6-OCH₃ | -CH₂-CH₂- | |
| -1559 | 2-Cl-4-CF3 | 6-OCH₃ | -CH₂-CH₂- | |
| -1560 | 2-F-4-CF3 | 6-OCH₃ | -CH₂-CH₂- | |
| -1561 | H | H | -CH₂-CH₂-CH₂- | |
| -1562 | 2-F | H | -CH₂-CH₂-CH₂- | |
| -1563 | 3-F | H | -CH₂-CH₂-CH₂- | |
| -1564 | 4-F | H | -CH₂-CH₂-CH₂- | |
| -1565 | 2-Cl | H | -CH₂-CH₂-CH₂- | |
| -1566 | 3-Cl | H | -CH₂-CH₂-CH₂- | |
| -1567 | 4-Cl | H | -CH₂-CH₂-CH₂- | |
| -1568 | 2-CF₃ | H | -CH₂-CH₂-CH₂- | |
| -1569 | 3-CF₃ | H | -CH₂-CH₂-CH₂- | |
| -1570 | 4-CF₃ | H | -CH₂-CH₂-CH₂- | |
| -1571 | 2,4-F₂ | H | -CH₂-CH₂-CH₂- | |
| -1572 | 2,4-Cl₂ | H | -CH₂-CH₂-CH₂- | |
| -1573 | 2,6-Cl₂ | H | -CH₂-CH₂-CH₂- | |
| -1574 | 2-CN | H | -CH₂-CH₂-CH₂- | |
| -1575 | 3-CN | H | -CH₂-CH₂-CH₂- | |
| -1576 | 4-CN | H | -CH₂-CH₂-CH₂- | |
| -1577 | 2-F-4-CN | H | -CH₂-CH₂-CH₂- | |
| -1578 | 2-Cl-4-CN | H | -CH₂-CH₂-CH₂- | |
| -1579 | 2-Cl-4-CF3 | H | -CH₂-CH₂-CH₂- | |
| -1580 | 2-F-4-CF3 | H | -CH₂-CH₂-CH₂- | |
| -1581 | H | 2-Cl | -CH₂-CH₂-CH₂- | |
| -1582 | 2-F | 2-Cl | -CH₂-CH₂-CH₂- | |
| -1583 | 3-F | 2-Cl | -CH₂-CH₂-CH₂- | |
| -1584 | 4-F | 2-Cl | -CH₂-CH₂-CH₂- | |
| -1585 | 2-Cl | 2-Cl | -CH₂-CH₂-CH₂- | |
| -1586 | 3-Cl | 2-Cl | -CH₂-CH₂-CH₂- | |
| -1587 | 4-Cl | 2-Cl | -CH₂-CH₂-CH₂- | |
| -1588 | 2-CF₃ | 2-Cl | -CH₂-CH₂-CH₂- | |
| -1589 | 3-CF₃ | 2-Cl | -CH₂-CH₂-CH₂- | |
| -1590 | 4-CF₃ | 2-Cl | -CH₂-CH₂-CH₂- | |
| -1591 | 2,4-F₂ | 2-Cl | -CH₂-CH₂-CH₂- | |
| -1592 | 2,4-Cl₂ | 2-Cl | -CH₂-CH₂-CH₂- | |
| -1593 | 2,6-Cl₂ | 2-Cl | -CH₂-CH₂-CH₂- | |
| -1594 | 2-CN | 2-Cl | -CH₂-CH₂-CH₂- | |
| -1595 | 3-CN | 2-Cl | -CH₂-CH₂-CH₂- | |
| -1596 | 4-CN | 2-Cl | -CH₂-CH₂-CH₂- | |
| -1597 | 2-F-4-CN | 2-Cl | -CH₂-CH₂-CH₂- | |
| -1598 | 2-Cl-4-CN | 2-Cl | -CH₂-CH₂-CH₂- | |
| -1599 | 2-Cl-4-CF3 | 2-Cl | -CH₂-CH₂-CH₂- | |
| -1600 | 2-F-4-CF3 | 2-Cl | -CH₂-CH₂-CH₂- | |
| -1601 | H | 6-Cl | -CH₂-CH₂-CH₂- | |
| -1602 | 2-F | 6-Cl | -CH₂-CH₂-CH₂- | |
| -1603 | 3-F | 6-Cl | -CH₂-CH₂-CH₂- | |
| -1604 | 4-F | 6-Cl | -CH₂-CH₂-CH₂- | |
| -1605 | 2-Cl | 6-Cl | -CH₂-CH₂-CH₂- | |
| -1606 | 3-Cl | 6-Cl | -CH₂-CH₂-CH₂- | |
| -1607 | 4-Cl | 6-Cl | -CH₂-CH₂-CH₂- | |
| -1608 | 2-CF₃ | 6-Cl | -CH₂-CH₂-CH₂- | |
| -1609 | 3-CF₃ | 6-Cl | -CH₂-CH₂-CH₂- | |
| -1610 | 4-CF₃ | 6-Cl | -CH₂-CH₂-CH₂- | |
| -1611 | 2,4-F₂ | 6-Cl | -CH₂-CH₂-CH₂- | |
| -1612 | 2,4-Cl₂ | 6-Cl | -CH₂-CH₂-CH₂- | |
| -1613 | 2,6-Cl₂ | 6-Cl | -CH₂-CH₂-CH₂- | |
| -1614 | 2-CN | 6-Cl | -CH₂-CH₂-CH₂- | |
| -1615 | 3-CN | 6-Cl | -CH₂-CH₂-CH₂- | |
| -1616 | 4-CN | 6-Cl | -CH₂-CH₂-CH₂- | |
| -1617 | 2-F-4-CN | 6-Cl | -CH₂-CH₂-CH₂- | |
| -1618 | 2-Cl-4-CN | 6-Cl | -CH₂-CH₂-CH₂- | |
| -1619 | 2-Cl-4-CF3 | 6-Cl | -CH₂-CH₂-CH₂- | |
| -1620 | 2-F-4-CF3 | 6-Cl | -CH₂-CH₂-CH₂- | |
| -1621 | H | 2-Br | -CH₂-CH₂-CH₂- | |
| -1622 | 2-F | 2-Br | -CH₂-CH₂-CH₂- | |
| -1623 | 3-F | 2-Br | -CH₂-CH₂-CH₂- | |
| -1624 | 4-F | 2-Br | -CH₂-CH₂-CH₂- | |
| -1625 | 2-Cl | 2-Br | -CH₂-CH₂-CH₂- | |
| -1626 | 3-Cl | 2-Br | -CH₂-CH₂-CH₂- | |
| -1627 | 4-Cl | 2-Br | -CH₂-CH₂-CH₂- | |
| -1628 | 2-CF₃ | 2-Br | -CH₂-CH₂-CH₂- | |
| -1629 | 3-CF₃ | 2-Br | -CH₂-CH₂-CH₂- | |
| -1630 | 4-CF₃ | 2-Br | -CH₂-CH₂-CH₂- | |
| -1631 | 2,4-F₂ | 2-Br | -CH₂-CH₂-CH₂- | |
| -1632 | 2,4-Cl₂ | 2-Br | -CH₂-CH₂-CH₂- | |
| -1633 | 2,6-Cl₂ | 2-Br | -CH₂-CH₂-CH₂- | |
| -1634 | 2-CN | 2-Br | -CH₂-CH₂-CH₂- | |
| -1635 | 3-CN | 2-Br | -CH₂-CH₂-CH₂- | |
| -1636 | 4-CN | 2-Br | -CH₂-CH₂-CH₂- | |
| -1637 | 2-F-4-CN | 2-Br | -CH₂-CH₂-CH₂- | |
| -1638 | 2-Cl-4-CN | 2-Br | -CH₂-CH₂-CH₂- | |
| -1639 | 2-Cl-4-CF3 | 2-Br | -CH₂-CH₂-CH₂- | |
| -1640 | 2-F-4-CF3 | 2-Br | -CH₂-CH₂-CH₂- | |
| -1641 | H | 6-Br | -CH₂-CH₂-CH₂- | |
| -1642 | 2-F | 6-Br | -CH₂-CH₂-CH₂- | |
| -1643 | 3-F | 6-Br | -CH₂-CH₂-CH₂- | |
| -1644 | 4-F | 6-Br | -CH₂-CH₂-CH₂- | |
| -1645 | 2-Cl | 6-Br | -CH₂-CH₂-CH₂- | |
| -1646 | 3-Cl | 6-Br | -CH₂-CH₂-CH₂- | |
| -1647 | 4-Cl | 6-Br | -CH₂-CH₂-CH₂- | |
| -1648 | 2-CF₃ | 6-Br | -CH₂-CH₂-CH₂- | |
| -1649 | 3-CF₃ | 6-Br | -CH₂-CH₂-CH₂- | |
| -1650 | 4-CF₃ | 6-Br | -CH₂-CH₂-CH₂- | |
| -1651 | 2,4-F₂ | 6-Br | -CH₂-CH₂-CH₂- | |
| -1652 | 2,4-Cl₂ | 6-Br | -CH₂-CH₂-CH₂- | |
| -1653 | 2,6-Cl₂ | 6-Br | -CH₂-CH₂-CH₂- | |
| -1654 | 2-CN | 6-Br | -CH₂-CH₂-CH₂- | |
| -1655 | 3-CN | 6-Br | -CH₂-CH₂-CH₂- | |
| -1656 | 4-CN | 6-Br | -CH₂-CH₂-CH₂- | |
| -1657 | 2-F-4-CN | 6-Br | -CH₂-CH₂-CH₂- | |
| -1658 | 2-Cl-4-CN | 6-Br | -CH₂-CH₂-CH₂- | |
| -1659 | 2-Cl-4-CF3 | 6-Br | -CH₂-CH₂-CH₂- | |
| -1660 | 2-F-4-CF3 | 6-Br | -CH₂-CH₂-CH₂- | |
| -1661 | H | 2-F | -CH₂-CH₂-CH₂- | |
| -1662 | 2-F | 2-F | -CH₂-CH₂-CH₂- | |
| -1663 | 3-F | 2-F | -CH₂-CH₂-CH₂- | |
| -1664 | 4-F | 2-F | -CH₂-CH₂-CH₂- | |
| -1665 | 2-Cl | 2-F | -CH₂-CH₂-CH₂- | |
| -1666 | 3-Cl | 2-F | -CH₂-CH₂-CH₂- | |
| -1667 | 4-Cl | 2-F | -CH₂-CH₂-CH₂- | |
| -1668 | 2-CF₃ | 2-F | -CH₂-CH₂-CH₂- | |
| -1669 | 3-CF₃ | 2-F | -CH₂-CH₂-CH₂- | |
| -1670 | 4-CF₃ | 2-F | -CH₂-CH₂-CH₂- | |
| -1671 | 2,4-F₂ | 2-F | -CH₂-CH₂-CH₂- | |
| -1672 | 2,4-Cl₂ | 2-F | -CH₂-CH₂-CH₂- | |
| -1673 | 2,6-Cl₂ | 2-F | -CH₂-CH₂-CH₂- | |
| -1674 | 2-CN | 2-F | -CH₂-CH₂-CH₂- | |
| -1675 | 3-CN | 2-F | -CH₂-CH₂-CH₂- | |
| -1676 | 4-CN | 2-F | -CH₂-CH₂-CH₂- | |
| -1677 | 2-F-4-CN | 2-F | -CH₂-CH₂-CH₂- | |
| -1678 | 2-Cl-4-CN | 2-F | -CH₂-CH₂-CH₂- | |
| -1679 | 2-Cl-4-CF3 | 2-F | -CH₂-CH₂-CH₂- | |
| -1680 | 2-F-4-CF3 | 2-F | -CH₂-CH₂-CH₂- | |
| -1681 | H | 6-F | -CH₂-CH₂-CH₂- | |
| -1682 | 2-F | 6-F | -CH₂-CH₂-CH₂- | |
| -1683 | 3-F | 6-F | -CH₂-CH₂-CH₂- | |
| -1684 | 4-F | 6-F | -CH₂-CH₂-CH₂- | |
| -1685 | 2-Cl | 6-F | -CH₂-CH₂-CH₂- | |
| -1686 | 3-Cl | 6-F | -CH₂-CH₂-CH₂- | |
| -1687 | 4-Cl | 6-F | -CH₂-CH₂-CH₂- | |
| -1688 | 2-CF₃ | 6-F | -CH₂-CH₂-CH₂- | |
| -1689 | 3-CF₃ | 6-F | -CH₂-CH₂-CH₂- | |
| -1690 | 4-CF₃ | 6-F | -CH₂-CH₂-CH₂- | |
| -1691 | 2,4-F₂ | 6-F | -CH₂-CH₂-CH₂- | |
| -1692 | 2,4-Cl₂ | 6-F | -CH₂-CH₂-CH₂- | |
| -1693 | 2,6-Cl₂ | 6-F | -CH₂-CH₂-CH₂- | |
| -1694 | 2-CN | 6-F | -CH₂-CH₂-CH₂- | |
| -1695 | 3-CN | 6-F | -CH₂-CH₂-CH₂- | |
| -1696 | 4-CN | 6-F | -CH₂-CH₂-CH₂- | |
| -1697 | 2-F-4-CN | 6-F | -CH₂-CH₂-CH₂- | |
| -1698 | 2-Cl-4-CN | 6-F | -CH₂-CH₂-CH₂- | |
| -1699 | 2-Cl-4-CF3 | 6-F | -CH₂-CH₂-CH₂- | |
| -1700 | 2-F-4-CF3 | 6-F | -CH₂-CH₂-CH₂- | |
| -1701 | H | 2-CN | -CH₂-CH₂-CH₂- | |
| -1702 | 2-F | 2-CN | -CH₂-CH₂-CH₂- | |
| -1703 | 3-F | 2-CN | -CH₂-CH₂-CH₂- | |
| -1704 | 4-F | 2-CN | -CH₂-CH₂-CH₂- | |
| -1705 | 2-Cl | 2-CN | -CH₂-CH₂-CH₂- | |
| -1706 | 3-Cl | 2-CN | -CH₂-CH₂-CH₂- | |
| -1707 | 4-Cl | 2-CN | -CH₂-CH₂-CH₂- | |
| -1708 | 2-CF₃ | 2-CN | -CH₂-CH₂-CH₂- | |
| -1709 | 3-CF₃ | 2-CN | -CH₂-CH₂-CH₂- | |
| -1710 | 4-CF₃ | 2-CN | -CH₂-CH₂-CH₂- | |
| -1711 | 2,4-F₂ | 2-CN | -CH₂-CH₂-CH₂- | |
| -1712 | 2,4-Cl₂ | 2-CN | -CH₂-CH₂-CH₂- | |
| -1713 | 2,6-Cl₂ | 2-CN | -CH₂-CH₂-CH₂- | |
| -1714 | 2-CN | 2-CN | -CH₂-CH₂-CH₂- | |
| -1715 | 3-CN | 2-CN | -CH₂-CH₂-CH₂- | |
| -1716 | 4-CN | 2-CN | -CH₂-CH₂-CH₂- | |
| -1717 | 2-F-4-CN | 2-CN | -CH₂-CH₂-CH₂- | |
| -1718 | 2-Cl-4-CN | 2-CN | -CH₂-CH₂-CH₂- | |
| -1719 | 2-Cl-4-CF3 | 2-CN | -CH₂-CH₂-CH₂- | |
| -1720 | 2-F-4-CF3 | 2-CN | -CH₂-CH₂-CH₂- | |
| -1721 | H | 6-CN | -CH₂-CH₂-CH₂- | |
| -1722 | 2-F | 6-CN | -CH₂-CH₂-CH₂- | |
| -1723 | 3-F | 6-CN | -CH₂-CH₂-CH₂- | |
| -1724 | 4-F | 6-CN | -CH₂-CH₂-CH₂- | |
| -1725 | 2-Cl | 6-CN | -CH₂-CH₂-CH₂- | |
| -1726 | 3-Cl | 6-CN | -CH₂-CH₂-CH₂- | |
| -1727 | 4-Cl | 6-CN | -CH₂-CH₂-CH₂- | |
| -1728 | 2-CF₃ | 6-CN | -CH₂-CH₂-CH₂- | |
| -1729 | 3-CF₃ | 6-CN | -CH₂-CH₂-CH₂- | |
| -1730 | 4-CF₃ | 6-CN | -CH₂-CH₂-CH₂- | |
| -1731 | 2,4-F₂ | 6-CN | -CH₂-CH₂-CH₂- | |
| -1732 | 2,4-Cl₂ | 6-CN | -CH₂-CH₂-CH₂- | |
| -1733 | 2,6-Cl₂ | 6-CN | -CH₂-CH₂-CH₂- | |
| -1734 | 2-CN | 6-CN | -CH₂-CH₂-CH₂- | |
| -1735 | 3-CN | 6-CN | -CH₂-CH₂-CH₂- | |
| -1736 | 4-CN | 6-CN | -CH₂-CH₂-CH₂- | |
| -1737 | 2-F-4-CN | 6-CN | -CH₂-CH₂-CH₂- | |
| -1738 | 2-Cl-4-CN | 6-CN | -CH₂-CH₂-CH₂- | |
| -1739 | 2-Cl-4-CF3 | 6-CN | -CH₂-CH₂-CH₂- | |
| -1740 | 2-F-4-CF3 | 6-CN | -CH₂-CH₂-CH₂- | |
| -1741 | H | 2-CF₃ | -CH₂-CH₂-CH₂- | |
| -1742 | 2-F | 2-CF₃ | -CH₂-CH₂-CH₂- | |
| -1743 | 3-F | 2-CF₃ | -CH₂-CH₂-CH₂- | |
| -1744 | 4-F | 2-CF₃ | -CH₂-CH₂-CH₂- | |
| -1745 | 2-Cl | 2-CF₃ | -CH₂-CH₂-CH₂- | |
| -1746 | 3-Cl | 2-CF₃ | -CH₂-CH₂-CH₂- | |
| -1747 | 4-Cl | 2-CF₃ | -CH₂-CH₂-CH₂- | |
| -1748 | 2-CF₃ | 2-CF₃ | -CH₂-CH₂-CH₂- | |
| -1749 | 3-CF₃ | 2-CF₃ | -CH₂-CH₂-CH₂- | |
| -1750 | 4-CF₃ | 2-CF₃ | -CH₂-CH₂-CH₂- | |
| -1751 | 2,4-F₂ | 2-CF₃ | -CH₂-CH₂-CH₂- | |
| -1752 | 2,4-Cl₂ | 2-CF₃ | -CH₂-CH₂-CH₂- | |
| -1753 | 2,6-Cl₂ | 2-CF₃ | -CH₂-CH₂-CH₂- | |
| -1754 | 2-CN | 2-CF₃ | -CH₂-CH₂-CH₂- | |
| -1755 | 3-CN | 2-CF₃ | -CH₂-CH₂-CH₂- | |
| -1756 | 4-CN | 2-CF₃ | -CH₂-CH₂-CH₂- | |
| -1757 | 2-F-4-CN | 2-CF₃ | -CH₂-CH₂-CH₂- | |
| -1758 | 2-Cl-4-CN | 2-CF₃ | -CH₂-CH₂-CH₂- | |
| -1759 | 2-Cl-4-CF3 | 2-CF₃ | -CH₂-CH₂-CH₂- | |
| -1760 | 2-F-4-CF3 | 2-CF₃ | -CH₂-CH₂-CH₂- | |
| -1761 | H | 6-CF₃ | -CH₂-CH₂-CH₂- | |
| -1762 | 2-F | 6-CF₃ | -CH₂-CH₂-CH₂- | |
| -1763 | 3-F | 6-CF₃ | -CH₂-CH₂-CH₂- | |
| -1764 | 4-F | 6-CF₃ | -CH₂-CH₂-CH₂- | |
| -1765 | 2-Cl | 6-CF₃ | -CH₂-CH₂-CH₂- | |
| -1766 | 3-Cl | 6-CF₃ | -CH₂-CH₂-CH₂- | |
| -1767 | 4-Cl | 6-CF₃ | -CH₂-CH₂-CH₂- | |
| -1768 | 2-CF₃ | 6-CF₃ | -CH₂-CH₂-CH₂- | |
| -1769 | 3-CF₃ | 6-CF₃ | -CH₂-CH₂-CH₂- | |
| -1770 | 4-CF₃ | 6-CF₃ | -CH₂-CH₂-CH₂- | |
| -1771 | 2,4-F₂ | 6-CF₃ | -CH₂-CH₂-CH₂- | |
| -1772 | 2,4-Cl₂ | 6-CF₃ | -CH₂-CH₂-CH₂- | |
| -1773 | 2,6-Cl₂ | 6-CF₃ | -CH₂-CH₂-CH₂- | |
| -1774 | 2-CN | 6-CF₃ | -CH₂-CH₂-CH₂- | |
| -1775 | 3-CN | 6-CF₃ | -CH₂-CH₂-CH₂- | |
| -1776 | 4-CN | 6-CF₃ | -CH₂-CH₂-CH₂- | |
| -1777 | 2-F-4-CN | 6-CF₃ | -CH₂-CH₂-CH₂- | |
| -1778 | 2-Cl-4-CN | 6-CF₃ | -CH₂-CH₂-CH₂- | |
| -1779 | 2-Cl-4-CF3 | 6-CF₃ | -CH₂-CH₂-CH₂- | |
| -1780 | 2-F-4-CF3 | 6-CF₃ | -CH₂-CH₂-CH₂- | |
| -1781 | H | 2-OCH₃ | -CH₂-CH₂-CH₂- | |
| -1782 | 2-F | 2-OCH₃ | -CH₂-CH₂-CH₂- | |
| -1783 | 3-F | 2-OCH₃ | -CH₂-CH₂-CH₂- | |
| -1784 | 4-F | 2-OCH₃ | -CH₂-CH₂-CH₂- | |
| -1785 | 2-Cl | 2-OCH₃ | -CH₂-CH₂-CH₂- | |
| -1786 | 3-Cl | 2-OCH₃ | -CH₂-CH₂-CH₂- | |
| -1787 | 4-Cl | 2-OCH₃ | -CH₂-CH₂-CH₂- | |
| -1788 | 2-CF₃ | 2-OCH₃ | -CH₂-CH₂-CH₂- | |
| -1789 | 3-CF₃ | 2-OCH₃ | -CH₂-CH₂-CH₂- | |
| -1790 | 4-CF₃ | 2-OCH₃ | -CH₂-CH₂-CH₂- | |
| -1791 | 2,4-F₂ | 2-OCH₃ | -CH₂-CH₂-CH₂- | |
| -1792 | 2,4-Cl₂ | 2-OCH₃ | -CH₂-CH₂-CH₂- | |
| -1793 | 2,6-Cl₂ | 2-OCH₃ | -CH₂-CH₂-CH₂- | |
| -1794 | 2-CN | 2-OCH₃ | -CH₂-CH₂-CH₂- | |
| -1795 | 3-CN | 2-OCH₃ | -CH₂-CH₂-CH₂- | |
| -1796 | 4-CN | 2-OCH₃ | -CH₂-CH₂-CH₂- | |
| -1797 | 2-F-4-CN | 2-OCH₃ | -CH₂-CH₂-CH₂- | |
| -1798 | 2-Cl-4-CN | 2-OCH₃ | -CH₂-CH₂-CH₂- | |
| -1799 | 2-Cl-4-CF3 | 2-OCH₃ | -CH₂-CH₂-CH₂- | |
| -1800 | 2-F-4-CF3 | 2-OCH₃ | -CH₂-CH₂-CH₂- | |
| -1801 | H | 6-OCH₃ | -CH₂-CH₂-CH₂- | |
| -1802 | 2-F | 6-OCH₃ | -CH₂-CH₂-CH₂- | |
| -1803 | 3-F | 6-OCH₃ | -CH₂-CH₂-CH₂- | |
| -1804 | 4-F | 6-OCH₃ | -CH₂-CH₂-CH₂- | |
| -1805 | 2-Cl | 6-OCH₃ | -CH₂-CH₂-CH₂- | |
| -1806 | 3-Cl | 6-OCH₃ | -CH₂-CH₂-CH₂- | |
| -1807 | 4-Cl | 6-OCH₃ | -CH₂-CH₂-CH₂- | |
| -1808 | 2-CF₃ | 6-OCH₃ | -CH₂-CH₂-CH₂- | |
| -1809 | 3-CF₃ | 6-OCH₃ | -CH₂-CH₂-CH₂- | |
| -1810 | 4-CF₃ | 6-OCH₃ | -CH₂-CH₂-CH₂- | |
| -1811 | 2,4-F₂ | 6-OCH₃ | -CH₂-CH₂-CH₂- | |
| -1812 | 2,4-Cl₂ | 6-OCH₃ | -CH₂-CH₂-CH₂- | |
| -1813 | 2,6-Cl₂ | 6-OCH₃ | -CH₂-CH₂-CH₂- | |
| -1814 | 2-CN | 6-OCH₃ | -CH₂-CH₂-CH₂- | |
| -1815 | 3-CN | 6-OCH₃ | -CH₂-CH₂-CH₂- | |
| -1816 | 4-CN | 6-OCH₃ | -CH₂-CH₂-CH₂- | |
| -1817 | 2-F-4-CN | 6-OCH₃ | -CH₂-CH₂-CH₂- | |
| -1818 | 2-Cl-4-CN | 6-OCH₃ | -CH₂-CH₂-CH₂- | |
| -1819 | 2-Cl-4-CF3 | 6-OCH₃ | -CH₂-CH₂-CH₂- | |
| -1820 | 2-F-4-CF3 | 6-OCH₃ | -CH₂-CH₂-CH₂- | |
| -1821 | H | H | -CH₂-CH₂-CH₂-CH₂- | |
| -1822 | 2-F | H | -CH₂-CH₂-CH₂-CH₂- | |
| -1823 | 3-F | H | -CH₂-CH₂-CH₂-CH₂- | |
| -1824 | 4-F | H | -CH₂-CH₂-CH₂-CH₂- | |
| -1825 | 2-Cl | H | -CH₂-CH₂-CH₂-CH₂- | |
| -1826 | 3-Cl | H | -CH₂-CH₂-CH₂-CH₂- | |
| -1827 | 4-Cl | H | -CH₂-CH₂-CH₂-CH₂- | |
| -1828 | 2-CF₃ | H | -CH₂-CH₂-CH₂-CH₂- | |
| -1829 | 3-CF₃ | H | -CH₂-CH₂-CH₂-CH₂- | |
| -1830 | 4-CF₃ | H | -CH₂-CH₂-CH₂-CH₂- | |
| -1831 | 2,4-F₂ | H | -CH₂-CH₂-CH₂-CH₂- | |
| -1832 | 2,4-Cl₂ | H | -CH₂-CH₂-CH₂-CH₂- | |
| -1833 | 2,6-Cl₂ | H | -CH₂-CH₂-CH₂-CH₂- | |
| -1834 | 2-CN | H | -CH₂-CH₂-CH₂-CH₂- | |
| -1835 | 3-CN | H | -CH₂-CH₂-CH₂-CH₂- | |
| -1836 | 4-CN | H | -CH₂-CH₂-CH₂-CH₂- | |
| -1837 | 2-F-4-CN | H | -CH₂-CH₂-CH₂-CH₂- | |
| -1838 | 2-Cl-4-CN | H | -CH₂-CH₂-CH₂-CH₂- | |
| -1839 | 2-Cl-4-CF3 | H | -CH₂-CH₂-CH₂-CH₂- | |
| -1840 | 2-F-4-CF3 | H | -CH₂-CH₂-CH₂-CH₂- | |
| -1841 | H | 2-Cl | -CH₂-CH₂-CH₂-CH₂- | |
| -1842 | 2-F | 2-Cl | -CH₂-CH₂-CH₂-CH₂- | |
| -1843 | 3-F | 2-Cl | -CH₂-CH₂-CH₂-CH₂- | |
| -1844 | 4-F | 2-Cl | -CH₂-CH₂-CH₂-CH₂- | |
| -1845 | 2-Cl | 2-Cl | -CH₂-CH₂-CH₂-CH₂- | |
| -1846 | 3-Cl | 2-Cl | -CH₂-CH₂-CH₂-CH₂- | |
| -1847 | 4-Cl | 2-Cl | -CH₂-CH₂-CH₂-CH₂- | |
| -1848 | 2-CF₃ | 2-Cl | -CH₂-CH₂-CH₂-CH₂- | |
| -1849 | 3-CF₃ | 2-Cl | -CH₂-CH₂-CH₂-CH₂- | |
| -1850 | 4-CF₃ | 2-Cl | -CH₂-CH₂-CH₂-CH₂- | |
| -1851 | 2,4-F₂ | 2-Cl | -CH₂-CH₂-CH₂-CH₂- | |
| -1852 | 2,4-Cl₂ | 2-Cl | -CH₂-CH₂-CH₂-CH₂- | |
| -1853 | 2,6-Cl₂ | 2-Cl | -CH₂-CH₂-CH₂-CH₂- | |
| -1854 | 2-CN | 2-Cl | -CH₂-CH₂-CH₂-CH₂- | |
| -1855 | 3-CN | 2-Cl | -CH₂-CH₂-CH₂-CH₂- | |
| -1856 | 4-CN | 2-Cl | -CH₂-CH₂-CH₂-CH₂- | |
| -1857 | 2-F-4-CN | 2-Cl | -CH₂-CH₂-CH₂-CH₂- | |
| -1858 | 2-Cl-4-CN | 2-Cl | -CH₂-CH₂-CH₂-CH₂- | |
| -1859 | 2-Cl-4-CF3 | 2-Cl | -CH₂-CH₂-CH₂-CH₂- | |
| -1860 | 2-F-4-CF3 | 2-Cl | -CH₂-CH₂-CH₂-CH₂- | |
| -1861 | H | 6-Cl | -CH₂-CH₂-CH₂-CH₂- | |
| -1862 | 2-F | 6-Cl | -CH₂-CH₂-CH₂-CH₂- | |
| -1863 | 3-F | 6-Cl | -CH₂-CH₂-CH₂-CH₂- | |
| -1864 | 4-F | 6-Cl | -CH₂-CH₂-CH₂-CH₂- | |
| -1865 | 2-Cl | 6-Cl | -CH₂-CH₂-CH₂-CH₂- | |
| -1866 | 3-Cl | 6-Cl | -CH₂-CH₂-CH₂-CH₂- | |
| -1867 | 4-Cl | 6-Cl | -CH₂-CH₂-CH₂-CH₂- | |
| -1868 | 2-CF₃ | 6-Cl | -CH₂-CH₂-CH₂-CH₂- | |
| -1869 | 3-CF₃ | 6-Cl | -CH₂-CH₂-CH₂-CH₂- | |
| -1870 | 4-CF₃ | 6-Cl | -CH₂-CH₂-CH₂-CH₂- | |
| -1871 | 2,4-F₂ | 6-Cl | -CH₂-CH₂-CH₂-CH₂- | |
| -1872 | 2,4-Cl₂ | 6-Cl | -CH₂-CH₂-CH₂-CH₂- | |
| -1873 | 2,6-Cl₂ | 6-Cl | -CH₂-CH₂-CH₂-CH₂- | |
| -1874 | 2-CN | 6-Cl | -CH₂-CH₂-CH₂-CH₂- | |
| -1875 | 3-CN | 6-Cl | -CH₂-CH₂-CH₂-CH₂- | |
| -1876 | 4-CN | 6-Cl | -CH₂-CH₂-CH₂-CH₂- | |
| -1877 | 2-F-4-CN | 6-Cl | -CH₂-CH₂-CH₂-CH₂- | |
| -1878 | 2-Cl-4-CN | 6-Cl | -CH₂-CH₂-CH₂-CH₂- | |
| -1879 | 2-Cl-4-CF3 | 6-Cl | -CH₂-CH₂-CH₂-CH₂- | |
| -1880 | 2-F-4-CF3 | 6-Cl | -CH₂-CH₂-CH₂-CH₂- | |
| -1881 | H | 2-Br | -CH₂-CH₂-CH₂-CH₂- | |
| -1882 | 2-F | 2-Br | -CH₂-CH₂-CH₂-CH₂- | |
| -1883 | 3-F | 2-Br | -CH₂-CH₂-CH₂-CH₂- | |
| -1884 | 4-F | 2-Br | -CH₂-CH₂-CH₂-CH₂- | |
| -1885 | 2-Cl | 2-Br | -CH₂-CH₂-CH₂-CH₂- | |
| -1886 | 3-Cl | 2-Br | -CH₂-CH₂-CH₂-CH₂- | |
| -1887 | 4-Cl | 2-Br | -CH₂-CH₂-CH₂-CH₂- | |
| -1888 | 2-CF₃ | 2-Br | -CH₂-CH₂-CH₂-CH₂- | |
| -1889 | 3-CF₃ | 2-Br | -CH₂-CH₂-CH₂-CH₂- | |
| -1890 | 4-CF₃ | 2-Br | -CH₂-CH₂-CH₂-CH₂- | |
| -1891 | 2,4-F₂ | 2-Br | -CH₂-CH₂-CH₂-CH₂- | |
| -1892 | 2,4-Cl₂ | 2-Br | -CH₂-CH₂-CH₂-CH₂- | |
| -1893 | 2,6-Cl₂ | 2-Br | -CH₂-CH₂-CH₂-CH₂- | |
| -1894 | 2-CN | 2-Br | -CH₂-CH₂-CH₂-CH₂- | |
| -1895 | 3-CN | 2-Br | -CH₂-CH₂-CH₂-CH₂- | |
| -1896 | 4-CN | 2-Br | -CH₂-CH₂-CH₂-CH₂- | |
| -1897 | 2-F-4-CN | 2-Br | -CH₂-CH₂-CH₂-CH₂- | |
| -1898 | 2-Cl-4-CN | 2-Br | -CH₂-CH₂-CH₂-CH₂- | |
| -1899 | 2-Cl-4-CF3 | 2-Br | -CH₂-CH₂-CH₂-CH₂- | |
| -1900 | 2-F-4-CF3 | 2-Br | -CH₂-CH₂-CH₂-CH₂- | |
| -1901 | H | 6-Br | -CH₂-CH₂-CH₂-CH₂- | |
| -1902 | 2-F | 6-Br | -CH₂-CH₂-CH₂-CH₂- | |
| -1903 | 3-F | 6-Br | -CH₂-CH₂-CH₂-CH₂- | |
| -1904 | 4-F | 6-Br | -CH₂-CH₂-CH₂-CH₂- | |
| -1905 | 2-Cl | 6-Br | -CH₂-CH₂-CH₂-CH₂- | |
| -1906 | 3-Cl | 6-Br | -CH₂-CH₂-CH₂-CH₂- | |
| -1907 | 4-Cl | 6-Br | -CH₂-CH₂-CH₂-CH₂- | |
| -1908 | 2-CF₃ | 6-Br | -CH₂-CH₂-CH₂-CH₂- | |
| -1909 | 3-CF₃ | 6-Br | -CH₂-CH₂-CH₂-CH₂- | |
| -1910 | 4-CF₃ | 6-Br | -CH₂-CH₂-CH₂-CH₂- | |
| -1911 | 2,4-F₂ | 6-Br | -CH₂-CH₂-CH₂-CH₂- | |
| -1912 | 2,4-Cl₂ | 6-Br | -CH₂-CH₂-CH₂-CH₂- | |
| -1913 | 2,6-Cl₂ | 6-Br | -CH₂-CH₂-CH₂-CH₂- | |
| -1914 | 2-CN | 6-Br | -CH₂-CH₂-CH₂-CH₂- | |
| -1915 | 3-CN | 6-Br | -CH₂-CH₂-CH₂-CH₂- | |
| -1916 | 4-CN | 6-Br | -CH₂-CH₂-CH₂-CH₂- | |
| -1917 | 2-F-4-CN | 6-Br | -CH₂-CH₂-CH₂-CH₂- | |
| -1918 | 2-Cl-4-CN | 6-Br | -CH₂-CH₂-CH₂-CH₂- | |
| -1919 | 2-Cl-4-CF3 | 6-Br | -CH₂-CH₂-CH₂-CH₂- | |
| -1920 | 2-F-4-CF3 | 6-Br | -CH₂-CH₂-CH₂-CH₂- | |
| -1921 | H | 2-F | -CH₂-CH₂-CH₂-CH₂- | |
| -1922 | 2-F | 2-F | -CH₂-CH₂-CH₂-CH₂- | |
| -1923 | 3-F | 2-F | -CH₂-CH₂-CH₂-CH₂- | |
| -1924 | 4-F | 2-F | -CH₂-CH₂-CH₂-CH₂- | |
| -1925 | 2-Cl | 2-F | -CH₂-CH₂-CH₂-CH₂- | |
| -1926 | 3-Cl | 2-F | -CH₂-CH₂-CH₂-CH₂- | |
| -1927 | 4-Cl | 2-F | -CH₂-CH₂-CH₂-CH₂- | |
| -1928 | 2-CF₃ | 2-F | -CH₂-CH₂-CH₂-CH₂- | |
| -1929 | 3-CF₃ | 2-F | -CH₂-CH₂-CH₂-CH₂- | |
| -1930 | 4-CF₃ | 2-F | -CH₂-CH₂-CH₂-CH₂- | |
| -1931 | 2,4-F₂ | 2-F | -CH₂-CH₂-CH₂-CH₂- | |
| -1932 | 2,4-Cl₂ | 2-F | -CH₂-CH₂-CH₂-CH₂- | |
| -1933 | 2,6-Cl₂ | 2-F | -CH₂-CH₂-CH₂-CH₂- | |
| -1934 | 2-CN | 2-F | -CH₂-CH₂-CH₂-CH₂- | |
| -1935 | 3-CN | 2-F | -CH₂-CH₂-CH₂-CH₂- | |
| -1936 | 4-CN | 2-F | -CH₂-CH₂-CH₂-CH₂- | |
| -1937 | 2-F-4-CN | 2-F | -CH₂-CH₂-CH₂-CH₂- | |
| -1938 | 2-Cl-4-CN | 2-F | -CH₂-CH₂-CH₂-CH₂- | |
| -1939 | 2-Cl-4-CF3 | 2-F | -CH₂-CH₂-CH₂-CH₂- | |
| -1940 | 2-F-4-CF3 | 2-F | -CH₂-CH₂-CH₂-CH₂- | |
| -1941 | H | 6-F | -CH₂-CH₂-CH₂-CH₂- | |
| -1942 | 2-F | 6-F | -CH₂-CH₂-CH₂-CH₂- | |
| -1943 | 3-F | 6-F | -CH₂-CH₂-CH₂-CH₂- | |
| -1944 | 4-F | 6-F | -CH₂-CH₂-CH₂-CH₂- | |
| -1945 | 2-Cl | 6-F | -CH₂-CH₂-CH₂-CH₂- | |
| -1946 | 3-Cl | 6-F | -CH₂-CH₂-CH₂-CH₂- | |
| -1947 | 4-Cl | 6-F | -CH₂-CH₂-CH₂-CH₂- | |
| -1948 | 2-CF₃ | 6-F | -CH₂-CH₂-CH₂-CH₂- | |
| -1949 | 3-CF₃ | 6-F | -CH₂-CH₂-CH₂-CH₂- | |
| -1950 | 4-CF₃ | 6-F | -CH₂-CH₂-CH₂-CH₂- | |
| -1951 | 2,4-F₂ | 6-F | -CH₂-CH₂-CH₂-CH₂- | |
| -1952 | 2,4-Cl₂ | 6-F | -CH₂-CH₂-CH₂-CH₂- | |
| -1953 | 2,6-Cl₂ | 6-F | -CH₂-CH₂-CH₂-CH₂- | |
| -1954 | 2-CN | 6-F | -CH₂-CH₂-CH₂-CH₂- | |
| -1955 | 3-CN | 6-F | -CH₂-CH₂-CH₂-CH₂- | |
| -1956 | 4-CN | 6-F | -CH₂-CH₂-CH₂-CH₂- | |
| -1957 | 2-F-4-CN | 6-F | -CH₂-CH₂-CH₂-CH₂- | |
| -1958 | 2-Cl-4-CN | 6-F | -CH₂-CH₂-CH₂-CH₂- | |
| -1959 | 2-Cl-4-CF3 | 6-F | -CH₂-CH₂-CH₂-CH₂- | |
| -1960 | 2-F-4-CF3 | 6-F | -CH₂-CH₂-CH₂-CH₂- | |
| -1961 | H | 2-CN | -CH₂-CH₂-CH₂-CH₂- | |
| -1962 | 2-F | 2-CN | -CH₂-CH₂-CH₂-CH₂- | |
| -1963 | 3-F | 2-CN | -CH₂-CH₂-CH₂-CH₂- | |
| -1964 | 4-F | 2-CN | -CH₂-CH₂-CH₂-CH₂- | |
| -1965 | 2-Cl | 2-CN | -CH₂-CH₂-CH₂-CH₂- | |
| -1966 | 3-Cl | 2-CN | -CH₂-CH₂-CH₂-CH₂- | |
| -1967 | 4-Cl | 2-CN | -CH₂-CH₂-CH₂-CH₂- | |
| -1968 | 2-CF₃ | 2-CN | -CH₂-CH₂-CH₂-CH₂- | |
| -1969 | 3-CF₃ | 2-CN | -CH₂-CH₂-CH₂-CH₂- | |
| -1970 | 4-CF₃ | 2-CN | -CH₂-CH₂-CH₂-CH₂- | |
| -1971 | 2,4-F₂ | 2-CN | -CH₂-CH₂-CH₂-CH₂- | |
| -1972 | 2,4-Cl₂ | 2-CN | -CH₂-CH₂-CH₂-CH₂- | |
| -1973 | 2,6-Cl₂ | 2-CN | -CH₂-CH₂-CH₂-CH₂- | |
| -1974 | 2-CN | 2-CN | -CH₂-CH₂-CH₂-CH₂- | |
| -1975 | 3-CN | 2-CN | -CH₂-CH₂-CH₂-CH₂- | |
| -1976 | 4-CN | 2-CN | -CH₂-CH₂-CH₂-CH₂- | |
| -1977 | 2-F-4-CN | 2-CN | -CH₂-CH₂-CH₂-CH₂- | |
| -1978 | 2-Cl-4-CN | 2-CN | -CH₂-CH₂-CH₂-CH₂- | |
| -1979 | 2-Cl-4-CF3 | 2-CN | -CH₂-CH₂-CH₂-CH₂- | |
| -1980 | 2-F-4-CF3 | 2-CN | -CH₂-CH₂-CH₂-CH₂- | |
| -1981 | H | 6-CN | -CH₂-CH₂-CH₂-CH₂- | |
| -1982 | 2-F | 6-CN | -CH₂-CH₂-CH₂-CH₂- | |
| -1983 | 3-F | 6-CN | -CH₂-CH₂-CH₂-CH₂- | |
| -1984 | 4-F | 6-CN | -CH₂-CH₂-CH₂-CH₂- | |
| -1985 | 2-Cl | 6-CN | -CH₂-CH₂-CH₂-CH₂- | |
| -1986 | 3-Cl | 6-CN | -CH₂-CH₂-CH₂-CH₂- | |
| -1987 | 4-Cl | 6-CN | -CH₂-CH₂-CH₂-CH₂- | |
| -1988 | 2-CF₃ | 6-CN | -CH₂-CH₂-CH₂-CH₂- | |
| -1989 | 3-CF₃ | 6-CN | -CH₂-CH₂-CH₂-CH₂- | |
| -1990 | 4-CF₃ | 6-CN | -CH₂-CH₂-CH₂-CH₂- | |
| -1991 | 2,4-F₂ | 6-CN | -CH₂-CH₂-CH₂-CH₂- | |
| -1992 | 2,4-Cl₂ | 6-CN | -CH₂-CH₂-CH₂-CH₂- | |
| -1993 | 2,6-Cl₂ | 6-CN | -CH₂-CH₂-CH₂-CH₂- | |
| -1994 | 2-CN | 6-CN | -CH₂-CH₂-CH₂-CH₂- | |
| -1995 | 3-CN | 6-CN | -CH₂-CH₂-CH₂-CH₂- | |
| -1996 | 4-CN | 6-CN | -CH₂-CH₂-CH₂-CH₂- | |
| -1997 | 2-F-4-CN | 6-CN | -CH₂-CH₂-CH₂-CH₂- | |
| -1998 | 2-Cl-4-CN | 6-CN | -CH₂-CH₂-CH₂-CH₂- | |
| -1999 | 2-Cl-4-CF3 | 6-CN | -CH₂-CH₂-CH₂-CH₂- | |
| -2000 | 2-F-4-CF3 | 6-CN | -CH₂-CH₂-CH₂-CH₂- | |
| -2001 | H | 2-CF₃ | -CH₂-CH₂-CH₂-CH₂- | |
| -2002 | 2-F | 2-CF₃ | -CH₂-CH₂-CH₂-CH₂- | |
| -2003 | 3-F | 2-CF₃ | -CH₂-CH₂-CH₂-CH₂- | |
| -2004 | 4-F | 2-CF₃ | -CH₂-CH₂-CH₂-CH₂- | |
| -2005 | 2-Cl | 2-CF₃ | -CH₂-CH₂-CH₂-CH₂- | |
| -2006 | 3-Cl | 2-CF₃ | -CH₂-CH₂-CH₂-CH₂- | |
| -2007 | 4-Cl | 2-CF₃ | -CH₂-CH₂-CH₂-CH₂- | |
| -2008 | 2-CF₃ | 2-CF₃ | -CH₂-CH₂-CH₂-CH₂- | |
| -2009 | 3-CF₃ | 2-CF₃ | -CH₂-CH₂-CH₂-CH₂- | |
| -2010 | 4-CF₃ | 2-CF₃ | -CH₂-CH₂-CH₂-CH₂- | |
| -2011 | 2,4-F₂ | 2-CF₃ | -CH₂-CH₂-CH₂-CH₂- | |
| -2012 | 2,4-Cl₂ | 2-CF₃ | -CH₂-CH₂-CH₂-CH₂- | |
| -2013 | 2,6-Cl₂ | 2-CF₃ | -CH₂-CH₂-CH₂-CH₂- | |
| -2014 | 2-CN | 2-CF₃ | -CH₂-CH₂-CH₂-CH₂- | |
| -2015 | 3-CN | 2-CF₃ | -CH₂-CH₂-CH₂-CH₂- | |
| -2016 | 4-CN | 2-CF₃ | -CH₂-CH₂-CH₂-CH₂- | |
| -2017 | 2-F-4-CN | 2-CF₃ | -CH₂-CH₂-CH₂-CH₂- | |
| -2018 | 2-Cl-4-CN | 2-CF₃ | -CH₂-CH₂-CH₂-CH₂- | |
| -2019 | 2-Cl-4-CF3 | 2-CF₃ | -CH₂-CH₂-CH₂-CH₂- | |
| -2020 | 2-F-4-CF3 | 2-CF₃ | -CH₂-CH₂-CH₂-CH₂- | |
| -2021 | H | 6-CF₃ | -CH₂-CH₂-CH₂-CH₂- | |
| -2022 | 2-F | 6-CF₃ | -CH₂-CH₂-CH₂-CH₂- | |
| -2023 | 3-F | 6-CF₃ | -CH₂-CH₂-CH₂-CH₂- | |
| -2024 | 4-F | 6-CF₃ | -CH₂-CH₂-CH₂-CH₂- | |
| -2025 | 2-Cl | 6-CF₃ | -CH₂-CH₂-CH₂-CH₂- | |
| -2026 | 3-Cl | 6-CF₃ | -CH₂-CH₂-CH₂-CH₂- | |
| -2027 | 4-Cl | 6-CF₃ | -CH₂-CH₂-CH₂-CH₂- | |
| -2028 | 2-CF₃ | 6-CF₃ | -CH₂-CH₂-CH₂-CH₂- | |
| -2029 | 3-CF₃ | 6-CF₃ | -CH₂-CH₂-CH₂-CH₂- | |
| -2030 | 4-CF₃ | 6-CF₃ | -CH₂-CH₂-CH₂-CH₂- | |
| -2031 | 2,4-F₂ | 6-CF₃ | -CH₂-CH₂-CH₂-CH₂- | |
| -2032 | 2,4-Cl₂ | 6-CF₃ | -CH₂-CH₂-CH₂-CH₂- | |
| -2033 | 2,6-Cl₂ | 6-CF₃ | -CH₂-CH₂-CH₂-CH₂- | |
| -2034 | 2-CN | 6-CF₃ | -CH₂-CH₂-CH₂-CH₂- | |
| -2035 | 3-CN | 6-CF₃ | -CH₂-CH₂-CH₂-CH₂- | |
| -2036 | 4-CN | 6-CF₃ | -CH₂-CH₂-CH₂-CH₂- | |
| -2037 | 2-F-4-CN | 6-CF₃ | -CH₂-CH₂-CH₂-CH₂- | |
| -2038 | 2-Cl-4-CN | 6-CF₃ | -CH₂-CH₂-CH₂-CH₂- | |
| -2039 | 2-Cl-4-CF3 | 6-CF₃ | -CH₂-CH₂-CH₂-CH₂- | |
| -2040 | 2-F-4-CF3 | 6-CF₃ | -CH₂-CH₂-CH₂-CH₂- | |
| -2041 | H | 2-OCH₃ | -CH₂-CH₂-CH₂-CH₂- | |
| -2042 | 2-F | 2-OCH₃ | -CH₂-CH₂-CH₂-CH₂- | |
| -2043 | 3-F | 2-OCH₃ | -CH₂-CH₂-CH₂-CH₂- | |
| -2044 | 4-F | 2-OCH₃ | -CH₂-CH₂-CH₂-CH₂- | |
| -2045 | 2-Cl | 2-OCH₃ | -CH₂-CH₂-CH₂-CH₂- | |
| -2046 | 3-Cl | 2-OCH₃ | -CH₂-CH₂-CH₂-CH₂- | |
| -2047 | 4-Cl | 2-OCH₃ | -CH₂-CH₂-CH₂-CH₂- | |
| -2048 | 2-CF₃ | 2-OCH₃ | -CH₂-CH₂-CH₂-CH₂- | |
| -2049 | 3-CF₃ | 2-OCH₃ | -CH₂-CH₂-CH₂-CH₂- | |
| -2050 | 4-CF₃ | 2-OCH₃ | -CH₂-CH₂-CH₂-CH₂- | |
| -2051 | 2,4-F₂ | 2-OCH₃ | -CH₂-CH₂-CH₂-CH₂- | |
| -2052 | 2,4-Cl₂ | 2-OCH₃ | -CH₂-CH₂-CH₂-CH₂- | |
| -2053 | 2,6-Cl₂ | 2-OCH₃ | -CH₂-CH₂-CH₂-CH₂- | |
| -2054 | 2-CN | 2-OCH₃ | -CH₂-CH₂-CH₂-CH₂- | |
| -2055 | 3-CN | 2-OCH₃ | -CH₂-CH₂-CH₂-CH₂- | |
| -2056 | 4-CN | 2-OCH₃ | -CH₂-CH₂-CH₂-CH₂- | |
| -2057 | 2-F-4-CN | 2-OCH₃ | -CH₂-CH₂-CH₂-CH₂- | |
| -2058 | 2-Cl-4-CN | 2-OCH₃ | -CH₂-CH₂-CH₂-CH₂- | |
| -2059 | 2-Cl-4-CF3 | 2-OCH₃ | -CH₂-CH₂-CH₂-CH₂- | |
| -2060 | 2-F-4-CF3 | 2-OCH₃ | -CH₂-CH₂-CH₂-CH₂- | |
| -2061 | H | 6-OCH₃ | -CH₂-CH₂-CH₂-CH₂- | |
| -2062 | 2-F | 6-OCH₃ | -CH₂-CH₂-CH₂-CH₂- | |
| -2063 | 3-F | 6-OCH₃ | -CH₂-CH₂-CH₂-CH₂- | |
| -2064 | 4-F | 6-OCH₃ | -CH₂-CH₂-CH₂-CH₂- | |
| -2065 | 2-Cl | 6-OCH₃ | -CH₂-CH₂-CH₂-CH₂- | |
| -2066 | 3-Cl | 6-OCH₃ | -CH₂-CH₂-CH₂-CH₂- | |
| -2067 | 4-Cl | 6-OCH₃ | -CH₂-CH₂-CH₂-CH₂- | |
| -2068 | 2-CF₃ | 6-OCH₃ | -CH₂-CH₂-CH₂-CH₂- | |
| -2069 | 3-CF₃ | 6-OCH₃ | -CH₂-CH₂-CH₂-CH₂- | |
| -2070 | 4-CF₃ | 6-OCH₃ | -CH₂-CH₂-CH₂-CH₂- | |
| -2071 | 2,4-F₂ | 6-OCH₃ | -CH₂-CH₂-CH₂-CH₂- | |
| -2072 | 2,4-Cl₂ | 6-OCH₃ | -CH₂-CH₂-CH₂-CH₂- | |
| -2073 | 2,6-Cl₂ | 6-OCH₃ | -CH₂-CH₂-CH₂-CH₂- | |
| -2074 | 2-CN | 6-OCH₃ | -CH₂-CH₂-CH₂-CH₂- | |
| -2075 | 3-CN | 6-OCH₃ | -CH₂-CH₂-CH₂-CH₂- | |
| -2076 | 4-CN | 6-OCH₃ | -CH₂-CH₂-CH₂-CH₂- | |
| -2077 | 2-F-4-CN | 6-OCH₃ | -CH₂-CH₂-CH₂-CH₂- | |
| -2078 | 2-Cl-4-CN | 6-OCH₃ | -CH₂-CH₂-CH₂-CH₂- | |
| -2079 | 2-Cl-4-CF3 | 6-OCH₃ | -CH₂-CH₂-CH₂-CH₂- | |
| -2080 | 2-F-4-CF3 | 6-OCH₃ | -CH₂-CH₂-CH₂-CH₂- | |
| -2081 | H | H | -CH₂-CH₂-CH₂-CH₂-CH₂- | |
| -2082 | 2-F | H | -CH₂-CH₂-CH₂-CH₂-CH₂- | |
| -2083 | 3-F | H | -CH₂-CH₂-CH₂-CH₂-CH₂- | |
| -2084 | 4-F | H | -CH₂-CH₂-CH₂-CH₂-CH₂- | |
| -2085 | 2-Cl | H | -CH₂-CH₂-CH₂-CH₂-CH₂- | |
| -2086 | 3-Cl | H | -CH₂-CH₂-CH₂-CH₂-CH₂- | |
| -2087 | 4-Cl | H | -CH₂-CH₂-CH₂-CH₂-CH₂- | |
| -2088 | 2-CF₃ | H | -CH₂-CH₂-CH₂-CH₂-CH₂- | |
| -2089 | 3-CF₃ | H | -CH₂-CH₂-CH₂-CH₂-CH₂- | |
| -2090 | 4-CF₃ | H | -CH₂-CH₂-CH₂-CH₂-CH₂- | |
| -2091 | 2,4-F₂ | H | -CH₂-CH₂-CH₂-CH₂-CH₂- | |
| -2092 | 2,4-Cl₂ | H | -CH₂-CH₂-CH₂-CH₂-CH₂- | |
| -2093 | 2,6-Cl₂ | H | -CH₂-CH₂-CH₂-CH₂-CH₂- | |
| -2094 | 2-CN | H | -CH₂-CH₂-CH₂-CH₂-CH₂- | |
| -2095 | 3-CN | H | -CH₂-CH₂-CH₂-CH₂-CH₂- | |
| -2096 | 4-CN | H | -CH₂-CH₂-CH₂-CH₂-CH₂- | |
| -2097 | 2-F-4-CN | H | -CH₂-CH₂-CH₂-CH₂-CH₂- | |
| -2098 | 2-Cl-4-CN | H | -CH₂-CH₂-CH₂-CH₂-CH₂- | |
| -2099 | 2-Cl-4-CF3 | H | -CH₂-CH₂-CH₂-CH₂-CH₂- | |
| -2100 | 2-F-4-CF3 | H | -CH₂-CH₂-CH₂-CH₂-CH₂- | |
| -2101 | H | 2-Cl | -CH₂-CH₂-CH₂-CH₂-CH₂- | |
| -2102 | 2-F | 2-Cl | -CH₂-CH₂-CH₂-CH₂-CH₂- | |
| -2103 | 3-F | 2-Cl | -CH₂-CH₂-CH₂-CH₂-CH₂- | |
| -2104 | 4-F | 2-Cl | -CH₂-CH₂-CH₂-CH₂-CH₂- | |
| -2105 | 2-Cl | 2-Cl | -CH₂-CH₂-CH₂-CH₂-CH₂- | |
| -2106 | 3-Cl | 2-Cl | -CH₂-CH₂-CH₂-CH₂-CH₂- | |
| -2107 | 4-Cl | 2-Cl | -CH₂-CH₂-CH₂-CH₂-CH₂- | |
| -2108 | 2-CF₃ | 2-Cl | -CH₂-CH₂-CH₂-CH₂-CH₂- | |
| -2109 | 3-CF₃ | 2-Cl | -CH₂-CH₂-CH₂-CH₂-CH₂- | |
| -2110 | 4-CF₃ | 2-Cl | -CH₂-CH₂-CH₂-CH₂-CH₂- | |
| -2111 | 2,4-F₂ | 2-Cl | -CH₂-CH₂-CH₂-CH₂-CH₂- | |
| -2112 | 2,4-Cl₂ | 2-Cl | -CH₂-CH₂-CH₂-CH₂-CH₂- | |
| -2113 | 2,6-Cl₂ | 2-Cl | -CH₂-CH₂-CH₂-CH₂-CH₂- | |
| -2114 | 2-CN | 2-Cl | -CH₂-CH₂-CH₂-CH₂-CH₂- | |
| -2115 | 3-CN | 2-Cl | -CH₂-CH₂-CH₂-CH₂-CH₂- | |
| -2116 | 4-CN | 2-Cl | -CH₂-CH₂-CH₂-CH₂-CH₂- | |
| -2117 | 2-F-4-CN | 2-Cl | -CH₂-CH₂-CH₂-CH₂-CH₂- | |
| -2118 | 2-Cl-4-CN | 2-Cl | -CH₂-CH₂-CH₂-CH₂-CH₂- | |
| -2119 | 2-Cl-4-CF3 | 2-Cl | -CH₂-CH₂-CH₂-CH₂-CH₂- | |
| -2120 | 2-F-4-CF3 | 2-Cl | -CH₂-CH₂-CH₂-CH₂-CH₂- | |
| -2121 | H | 6-Cl | -CH₂-CH₂-CH₂-CH₂-CH₂- | |
| -2122 | 2-F | 6-Cl | -CH₂-CH₂-CH₂-CH₂-CH₂- | |
| -2123 | 3-F | 6-Cl | -CH₂-CH₂-CH₂-CH₂-CH₂- | |
| -2124 | 4-F | 6-Cl | -CH₂-CH₂-CH₂-CH₂-CH₂- | |
| -2125 | 2-Cl | 6-Cl | -CH₂-CH₂-CH₂-CH₂-CH₂- | |
| -2126 | 3-Cl | 6-Cl | -CH₂-CH₂-CH₂-CH₂-CH₂- | |
| -2127 | 4-Cl | 6-Cl | -CH₂-CH₂-CH₂-CH₂-CH₂- | |
| -2128 | 2-CF₃ | 6-Cl | -CH₂-CH₂-CH₂-CH₂-CH₂- | |
| -2129 | 3-CF₃ | 6-Cl | -CH₂-CH₂-CH₂-CH₂-CH₂- | |
| -2130 | 4-CF₃ | 6-Cl | -CH₂-CH₂-CH₂-CH₂-CH₂- | |
| -2131 | 2,4-F₂ | 6-Cl | -CH₂-CH₂-CH₂-CH₂-CH₂- | |
| -2132 | 2,4-Cl₂ | 6-Cl | -CH₂-CH₂-CH₂-CH₂-CH₂- | |
| -2133 | 2,6-Cl₂ | 6-Cl | -CH₂-CH₂-CH₂-CH₂-CH₂- | |
| -2134 | 2-CN | 6-Cl | -CH₂-CH₂-CH₂-CH₂-CH₂- | |
| -2135 | 3-CN | 6-Cl | -CH₂-CH₂-CH₂-CH₂-CH₂- | |
| -2136 | 4-CN | 6-Cl | -CH₂-CH₂-CH₂-CH₂-CH₂- | |
| -2137 | 2-F-4-CN | 6-Cl | -CH₂-CH₂-CH₂-CH₂-CH₂- | |
| -2138 | 2-Cl-4-CN | 6-Cl | -CH₂-CH₂-CH₂-CH₂-CH₂- | |
| -2139 | 2-Cl-4-CF3 | 6-Cl | -CH₂-CH₂-CH₂-CH₂-CH₂- | |
| -2140 | 2-F-4-CF3 | 6-Cl | -CH₂-CH₂-CH₂-CH₂-CH₂- | |
| -2141 | H | 2-Br | -CH₂-CH₂-CH₂-CH₂-CH₂- | |
| -2142 | 2-F | 2-Br | -CH₂-CH₂-CH₂-CH₂-CH₂- | |
| -2143 | 3-F | 2-Br | -CH₂-CH₂-CH₂-CH₂-CH₂- | |
| -2144 | 4-F | 2-Br | -CH₂-CH₂-CH₂-CH₂-CH₂- | |
| -2145 | 2-Cl | 2-Br | -CH₂-CH₂-CH₂-CH₂-CH₂- | |
| -2146 | 3-Cl | 2-Br | -CH₂-CH₂-CH₂-CH₂-CH₂- | |
| -2147 | 4-Cl | 2-Br | -CH₂-CH₂-CH₂-CH₂-CH₂- | |
| -2148 | 2-CF₃ | 2-Br | -CH₂-CH₂-CH₂-CH₂-CH₂- | |
| -2149 | 3-CF₃ | 2-Br | -CH₂-CH₂-CH₂-CH₂-CH₂- | |
| -2150 | 4-CF₃ | 2-Br | -CH₂-CH₂-CH₂-CH₂-CH₂- | |
| -2151 | 2,4-F₂ | 2-Br | -CH₂-CH₂-CH₂-CH₂-CH₂- | |
| -2152 | 2,4-Cl₂ | 2-Br | -CH₂-CH₂-CH₂-CH₂-CH₂- | |
| -2153 | 2,6-Cl₂ | 2-Br | -CH₂-CH₂-CH₂-CH₂-CH₂- | |
| -2154 | 2-CN | 2-Br | -CH₂-CH₂-CH₂-CH₂-CH₂- | |
| -2155 | 3-CN | 2-Br | -CH₂-CH₂-CH₂-CH₂-CH₂- | |
| -2156 | 4-CN | 2-Br | -CH₂-CH₂-CH₂-CH₂-CH₂- | |
| -2157 | 2-F-4-CN | 2-Br | -CH₂-CH₂-CH₂-CH₂-CH₂- | |
| -2158 | 2-Cl-4-CN | 2-Br | -CH₂-CH₂-CH₂-CH₂-CH₂- | |
| -2159 | 2-Cl-4-CF3 | 2-Br | -CH₂-CH₂-CH₂-CH₂-CH₂- | |
| -2160 | 2-F-4-CF3 | 2-Br | -CH₂-CH₂-CH₂-CH₂-CH₂- | |
| -2161 | H | 6-Br | -CH₂-CH₂-CH₂-CH₂-CH₂- | |
| -2162 | 2-F | 6-Br | -CH₂-CH₂-CH₂-CH₂-CH₂- | |
| -2163 | 3-F | 6-Br | -CH₂-CH₂-CH₂-CH₂-CH₂- | |
| -2164 | 4-F | 6-Br | -CH₂-CH₂-CH₂-CH₂-CH₂- | |
| -2165 | 2-Cl | 6-Br | -CH₂-CH₂-CH₂-CH₂-CH₂- | |
| -2166 | 3-Cl | 6-Br | -CH₂-CH₂-CH₂-CH₂-CH₂- | |
| -2167 | 4-Cl | 6-Br | -CH₂-CH₂-CH₂-CH₂-CH₂- | |
| -2168 | 2-CF₃ | 6-Br | -CH₂-CH₂-CH₂-CH₂-CH₂- | |
| -2169 | 3-CF₃ | 6-Br | -CH₂-CH₂-CH₂-CH₂-CH₂- | |
| -2170 | 4-CF₃ | 6-Br | -CH₂-CH₂-CH₂-CH₂-CH₂- | |
| -2171 | 2,4-F₂ | 6-Br | -CH₂-CH₂-CH₂-CH₂-CH₂- | |
| -2172 | 2,4-Cl₂ | 6-Br | -CH₂-CH₂-CH₂-CH₂-CH₂- | |
| -2173 | 2,6-Cl₂ | 6-Br | -CH₂-CH₂-CH₂-CH₂-CH₂- | |
| -2174 | 2-CN | 6-Br | -CH₂-CH₂-CH₂-CH₂-CH₂- | |
| -2175 | 3-CN | 6-Br | -CH₂-CH₂-CH₂-CH₂-CH₂- | |
| -2176 | 4-CN | 6-Br | -CH₂-CH₂-CH₂-CH₂-CH₂- | |
| -2177 | 2-F-4-CN | 6-Br | -CH₂-CH₂-CH₂-CH₂-CH₂- | |
| -2178 | 2-Cl-4-CN | 6-Br | -CH₂-CH₂-CH₂-CH₂-CH₂- | |
| -2179 | 2-Cl-4-CF3 | 6-Br | -CH₂-CH₂-CH₂-CH₂-CH₂- | |
| -2180 | 2-F-4-CF3 | 6-Br | -CH₂-CH₂-CH₂-CH₂-CH₂- | |
| -2181 | H | 2-F | -CH₂-CH₂-CH₂-CH₂-CH₂- | |
| -2182 | 2-F | 2-F | -CH₂-CH₂-CH₂-CH₂-CH₂- | |
| -2183 | 3-F | 2-F | -CH₂-CH₂-CH₂-CH₂-CH₂- | |
| -2184 | 4-F | 2-F | -CH₂-CH₂-CH₂-CH₂-CH₂- | |
| -2185 | 2-Cl | 2-F | -CH₂-CH₂-CH₂-CH₂-CH₂- | |
| -2186 | 3-Cl | 2-F | -CH₂-CH₂-CH₂-CH₂-CH₂- | |
| -2187 | 4-Cl | 2-F | -CH₂-CH₂-CH₂-CH₂-CH₂- | |
| -2188 | 2-CF₃ | 2-F | -CH₂-CH₂-CH₂-CH₂-CH₂- | |
| -2189 | 3-CF₃ | 2-F | -CH₂-CH₂-CH₂-CH₂-CH₂- | |
| -2190 | 4-CF₃ | 2-F | -CH₂-CH₂-CH₂-CH₂-CH₂- | |
| -2191 | 2,4-F₂ | 2-F | -CH₂-CH₂-CH₂-CH₂-CH₂- | |
| -2192 | 2,4-Cl₂ | 2-F | -CH₂-CH₂-CH₂-CH₂-CH₂- | |
| -2193 | 2,6-Cl₂ | 2-F | -CH₂-CH₂-CH₂-CH₂-CH₂- | |
| -2194 | 2-CN | 2-F | -CH₂-CH₂-CH₂-CH₂-CH₂- | |
| -2195 | 3-CN | 2-F | -CH₂-CH₂-CH₂-CH₂-CH₂- | |
| -2196 | 4-CN | 2-F | -CH₂-CH₂-CH₂-CH₂-CH₂- | |
| -2197 | 2-F-4-CN | 2-F | -CH₂-CH₂-CH₂-CH₂-CH₂- | |
| -2198 | 2-Cl-4-CN | 2-F | -CH₂-CH₂-CH₂-CH₂-CH₂- | |
| -2199 | 2-Cl-4-CF3 | 2-F | -CH₂-CH₂-CH₂-CH₂-CH₂- | |
| -2200 | 2-F-4-CF3 | 2-F | -CH₂-CH₂-CH₂-CH₂-CH₂- | |
| -2201 | H | 6-F | -CH₂-CH₂-CH₂-CH₂-CH₂- | |
| -2202 | 2-F | 6-F | -CH₂-CH₂-CH₂-CH₂-CH₂- | |
| -2203 | 3-F | 6-F | -CH₂-CH₂-CH₂-CH₂-CH₂- | |
| -2204 | 4-F | 6-F | -CH₂-CH₂-CH₂-CH₂-CH₂- | |
| -2205 | 2-Cl | 6-F | -CH₂-CH₂-CH₂-CH₂-CH₂- | |
| -2206 | 3-Cl | 6-F | -CH₂-CH₂-CH₂-CH₂-CH₂- | |
| -2207 | 4-Cl | 6-F | -CH₂-CH₂-CH₂-CH₂-CH₂- | |
| -2208 | 2-CF₃ | 6-F | -CH₂-CH₂-CH₂-CH₂-CH₂- | |
| -2209 | 3-CF₃ | 6-F | -CH₂-CH₂-CH₂-CH₂-CH₂- | |
| -2210 | 4-CF₃ | 6-F | -CH₂-CH₂-CH₂-CH₂-CH₂- | |
| -2211 | 2,4-F₂ | 6-F | -CH₂-CH₂-CH₂-CH₂-CH₂- | |
| -2212 | 2,4-Cl₂ | 6-F | -CH₂-CH₂-CH₂-CH₂-CH₂- | |
| -2213 | 2,6-Cl₂ | 6-F | -CH₂-CH₂-CH₂-CH₂-CH₂- | |
| -2214 | 2-CN | 6-F | -CH₂-CH₂-CH₂-CH₂-CH₂- | |
| -2215 | 3-CN | 6-F | -CH₂-CH₂-CH₂-CH₂-CH₂- | |
| -2216 | 4-CN | 6-F | -CH₂-CH₂-CH₂-CH₂-CH₂- | |
| -2217 | 2-F-4-CN | 6-F | -CH₂-CH₂-CH₂-CH₂-CH₂- | |
| -2218 | 2-Cl-4-CN | 6-F | -CH₂-CH₂-CH₂-CH₂-CH₂- | |
| -2219 | 2-Cl-4-CF3 | 6-F | -CH₂-CH₂-CH₂-CH₂-CH₂- | |
| -2220 | 2-F-4-CF3 | 6-F | -CH₂-CH₂-CH₂-CH₂-CH₂- | |
| -2221 | H | 2-CN | -CH₂-CH₂-CH₂-CH₂-CH₂- | |
| -2222 | 2-F | 2-CN | -CH₂-CH₂-CH₂-CH₂-CH₂- | |
| -2223 | 3-F | 2-CN | -CH₂-CH₂-CH₂-CH₂-CH₂- | |
| -2224 | 4-F | 2-CN | -CH₂-CH₂-CH₂-CH₂-CH₂- | |
| -2225 | 2-Cl | 2-CN | -CH₂-CH₂-CH₂-CH₂-CH₂- | |
| -2226 | 3-Cl | 2-CN | -CH₂-CH₂-CH₂-CH₂-CH₂- | |
| -2227 | 4-Cl | 2-CN | -CH₂-CH₂-CH₂-CH₂-CH₂- | |
| -2228 | 2-CF₃ | 2-CN | -CH₂-CH₂-CH₂-CH₂-CH₂- | |
| -2229 | 3-CF₃ | 2-CN | -CH₂-CH₂-CH₂-CH₂-CH₂- | |
| -2230 | 4-CF₃ | 2-CN | -CH₂-CH₂-CH₂-CH₂-CH₂- | |
| -2231 | 2,4-F₂ | 2-CN | -CH₂-CH₂-CH₂-CH₂-CH₂- | |
| -2232 | 2,4-Cl₂ | 2-CN | -CH₂-CH₂-CH₂-CH₂-CH₂- | |
| -2233 | 2,6-Cl₂ | 2-CN | -CH₂-CH₂-CH₂-CH₂-CH₂- | |
| -2234 | 2-CN | 2-CN | -CH₂-CH₂-CH₂-CH₂-CH₂- | |
| -2235 | 3-CN | 2-CN | -CH₂-CH₂-CH₂-CH₂-CH₂- | |
| -2236 | 4-CN | 2-CN | -CH₂-CH₂-CH₂-CH₂-CH₂- | |
| -2237 | 2-F-4-CN | 2-CN | -CH₂-CH₂-CH₂-CH₂-CH₂- | |
| -2238 | 2-Cl-4-CN | 2-CN | -CH₂-CH₂-CH₂-CH₂-CH₂- | |
| -2239 | 2-Cl-4-CF3 | 2-CN | -CH₂-CH₂-CH₂-CH₂-CH₂- | |
| -2240 | 2-F-4-CF3 | 2-CN | -CH₂-CH₂-CH₂-CH₂-CH₂- | |
| -2241 | H | 6-CN | -CH₂-CH₂-CH₂-CH₂-CH₂- | |
| -2242 | 2-F | 6-CN | -CH₂-CH₂-CH₂-CH₂-CH₂- | |
| -2243 | 3-F | 6-CN | -CH₂-CH₂-CH₂-CH₂-CH₂- | |
| -2244 | 4-F | 6-CN | -CH₂-CH₂-CH₂-CH₂-CH₂- | |
| -2245 | 2-Cl | 6-CN | -CH₂-CH₂-CH₂-CH₂-CH₂- | |
| -2246 | 3-Cl | 6-CN | -CH₂-CH₂-CH₂-CH₂-CH₂- | |
| -2247 | 4-Cl | 6-CN | -CH₂-CH₂-CH₂-CH₂-CH₂- | |
| -2248 | 2-CF₃ | 6-CN | -CH₂-CH₂-CH₂-CH₂-CH₂- | |
| -2249 | 3-CF₃ | 6-CN | -CH₂-CH₂-CH₂-CH₂-CH₂- | |
| -2250 | 4-CF₃ | 6-CN | -CH₂-CH₂-CH₂-CH₂-CH₂- | |
| -2251 | 2,4-F₂ | 6-CN | -CH₂-CH₂-CH₂-CH₂-CH₂- | |
| -2252 | 2,4-Cl₂ | 6-CN | -CH₂-CH₂-CH₂-CH₂-CH₂- | |
| -2253 | 2,6-Cl₂ | 6-CN | -CH₂-CH₂-CH₂-CH₂-CH₂- | |
| -2254 | 2-CN | 6-CN | -CH₂-CH₂-CH₂-CH₂-CH₂- | |
| -2255 | 3-CN | 6-CN | -CH₂-CH₂-CH₂-CH₂-CH₂- | |
| -2256 | 4-CN | 6-CN | -CH₂-CH₂-CH₂-CH₂-CH₂- | |
| -2257 | 2-F-4-CN | 6-CN | -CH₂-CH₂-CH₂-CH₂-CH₂- | |
| -2258 | 2-Cl-4-CN | 6-CN | -CH₂-CH₂-CH₂-CH₂-CH₂- | |
| -2259 | 2-Cl-4-CF3 | 6-CN | -CH₂-CH₂-CH₂-CH₂-CH₂- | |
| -2260 | 2-F-4-CF3 | 6-CN | -CH₂-CH₂-CH₂-CH₂-CH₂- | |
| -2261 | H | 2-CF₃ | -CH₂-CH₂-CH₂-CH₂-CH₂- | |
| -2262 | 2-F | 2-CF₃ | -CH₂-CH₂-CH₂-CH₂-CH₂- | |
| -2263 | 3-F | 2-CF₃ | -CH₂-CH₂-CH₂-CH₂-CH₂- | |
| -2264 | 4-F | 2-CF₃ | -CH₂-CH₂-CH₂-CH₂-CH₂- | |
| -2265 | 2-Cl | 2-CF₃ | -CH₂-CH₂-CH₂-CH₂-CH₂- | |
| -2266 | 3-Cl | 2-CF₃ | -CH₂-CH₂-CH₂-CH₂-CH₂- | |
| -2267 | 4-Cl | 2-CF₃ | -CH₂-CH₂-CH₂-CH₂-CH₂- | |
| -2268 | 2-CF₃ | 2-CF₃ | -CH₂-CH₂-CH₂-CH₂-CH₂- | |
| -2269 | 3-CF₃ | 2-CF₃ | -CH₂-CH₂-CH₂-CH₂-CH₂- | |
| -2270 | 4-CF₃ | 2-CF₃ | -CH₂-CH₂-CH₂-CH₂-CH₂- | |
| -2271 | 2,4-F₂ | 2-CF₃ | -CH₂-CH₂-CH₂-CH₂-CH₂- | |
| -2272 | 2,4-Cl₂ | 2-CF₃ | -CH₂-CH₂-CH₂-CH₂-CH₂- | |
| -2273 | 2,6-Cl₂ | 2-CF₃ | -CH₂-CH₂-CH₂-CH₂-CH₂- | |
| -2274 | 2-CN | 2-CF₃ | -CH₂-CH₂-CH₂-CH₂-CH₂- | |
| -2275 | 3-CN | 2-CF₃ | -CH₂-CH₂-CH₂-CH₂-CH₂- | |
| -2276 | 4-CN | 2-CF₃ | -CH₂-CH₂-CH₂-CH₂-CH₂- | |
| -2277 | 2-F-4-CN | 2-CF₃ | -CH₂-CH₂-CH₂-CH₂-CH₂- | |
| -2278 | 2-Cl-4-CN | 2-CF₃ | -CH₂-CH₂-CH₂-CH₂-CH₂- | |
| -2279 | 2-Cl-4-CF3 | 2-CF₃ | -CH₂-CH₂-CH₂-CH₂-CH₂- | |
| -2280 | 2-F-4-CF3 | 2-CF₃ | -CH₂-CH₂-CH₂-CH₂-CH₂- | |
| -2281 | H | 6-CF₃ | -CH₂-CH₂-CH₂-CH₂-CH₂- | |
| -2282 | 2-F | 6-CF₃ | -CH₂-CH₂-CH₂-CH₂-CH₂- | |
| -2283 | 3-F | 6-CF₃ | -CH₂-CH₂-CH₂-CH₂-CH₂- | |
| -2284 | 4-F | 6-CF₃ | -CH₂-CH₂-CH₂-CH₂-CH₂- | |
| -2285 | 2-Cl | 6-CF₃ | -CH₂-CH₂-CH₂-CH₂-CH₂- | |
| -2286 | 3-Cl | 6-CF₃ | -CH₂-CH₂-CH₂-CH₂-CH₂- | |
| -2287 | 4-Cl | 6-CF₃ | -CH₂-CH₂-CH₂-CH₂-CH₂- | |
| -2288 | 2-CF₃ | 6-CF₃ | -CH₂-CH₂-CH₂-CH₂-CH₂- | |
| -2289 | 3-CF₃ | 6-CF₃ | -CH₂-CH₂-CH₂-CH₂-CH₂- | |
| -2290 | 4-CF₃ | 6-CF₃ | -CH₂-CH₂-CH₂-CH₂-CH₂- | |
| -2291 | 2,4-F₂ | 6-CF₃ | -CH₂-CH₂-CH₂-CH₂-CH₂- | |
| -2292 | 2,4-Cl₂ | 6-CF₃ | -CH₂-CH₂-CH₂-CH₂-CH₂- | |
| -2293 | 2,6-Cl₂ | 6-CF₃ | -CH₂-CH₂-CH₂-CH₂-CH₂- | |
| -2294 | 2-CN | 6-CF₃ | -CH₂-CH₂-CH₂-CH₂-CH₂- | |
| -2295 | 3-CN | 6-CF₃ | -CH₂-CH₂-CH₂-CH₂-CH₂- | |
| -2296 | 4-CN | 6-CF₃ | -CH₂-CH₂-CH₂-CH₂-CH₂- | |
| -2297 | 2-F-4-CN | 6-CF₃ | -CH₂-CH₂-CH₂-CH₂-CH₂- | |
| -2298 | 2-Cl-4-CN | 6-CF₃ | -CH₂-CH₂-CH₂-CH₂-CH₂- | |
| -2299 | 2-Cl-4-CF3 | 6-CF₃ | -CH₂-CH₂-CH₂-CH₂-CH₂- | |
| -2300 | 2-F-4-CF3 | 6-CF₃ | -CH₂-CH₂-CH₂-CH₂-CH₂- | |
| -2301 | H | 2-OCH₃ | -CH₂-CH₂-CH₂-CH₂-CH₂- | |
| -2302 | 2-F | 2-OCH₃ | -CH₂-CH₂-CH₂-CH₂-CH₂- | |
| -2303 | 3-F | 2-OCH₃ | -CH₂-CH₂-CH₂-CH₂-CH₂- | |
| -2304 | 4-F | 2-OCH₃ | -CH₂-CH₂-CH₂-CH₂-CH₂- | |
| -2305 | 2-Cl | 2-OCH₃ | -CH₂-CH₂-CH₂-CH₂-CH₂- | |
| -2306 | 3-Cl | 2-OCH₃ | -CH₂-CH₂-CH₂-CH₂-CH₂- | |
| -2307 | 4-Cl | 2-OCH₃ | -CH₂-CH₂-CH₂-CH₂-CH₂- | |
| -2308 | 2-CF₃ | 2-OCH₃ | -CH₂-CH₂-CH₂-CH₂-CH₂- | |
| -2309 | 3-CF₃ | 2-OCH₃ | -CH₂-CH₂-CH₂-CH₂-CH₂- | |
| -2310 | 4-CF₃ | 2-OCH₃ | -CH₂-CH₂-CH₂-CH₂-CH₂- | |
| -2311 | 2,4-F₂ | 2-OCH₃ | -CH₂-CH₂-CH₂-CH₂-CH₂- | |
| -2312 | 2,4-Cl₂ | 2-OCH₃ | -CH₂-CH₂-CH₂-CH₂-CH₂- | |
| -2313 | 2,6-Cl₂ | 2-OCH₃ | -CH₂-CH₂-CH₂-CH₂-CH₂- | |
| -2314 | 2-CN | 2-OCH₃ | -CH₂-CH₂-CH₂-CH₂-CH₂- | |
| -2315 | 3-CN | 2-OCH₃ | -CH₂-CH₂-CH₂-CH₂-CH₂- | |
| -2316 | 4-CN | 2-OCH₃ | -CH₂-CH₂-CH₂-CH₂-CH₂- | |
| -2317 | 2-F-4-CN | 2-OCH₃ | -CH₂-CH₂-CH₂-CH₂-CH₂- | |
| -2318 | 2-Cl-4-CN | 2-OCH₃ | -CH₂-CH₂-CH₂-CH₂-CH₂- | |
| -2319 | 2-Cl-4-CF3 | 2-OCH₃ | -CH₂-CH₂-CH₂-CH₂-CH₂- | |
| -2320 | 2-F-4-CF3 | 2-OCH₃ | -CH₂-CH₂-CH₂-CH₂-CH₂- | |
| -2321 | H | 6-OCH₃ | -CH₂-CH₂-CH₂-CH₂-CH₂- | |
| -2322 | 2-F | 6-OCH₃ | -CH₂-CH₂-CH₂-CH₂-CH₂- | |
| -2323 | 3-F | 6-OCH₃ | -CH₂-CH₂-CH₂-CH₂-CH₂- | |
| -2324 | 4-F | 6-OCH₃ | -CH₂-CH₂-CH₂-CH₂-CH₂- | |
| -2325 | 2-Cl | 6-OCH₃ | -CH₂-CH₂-CH₂-CH₂-CH₂- | |
| -2326 | 3-Cl | 6-OCH₃ | -CH₂-CH₂-CH₂-CH₂-CH₂- | |
| -2327 | 4-Cl | 6-OCH₃ | -CH₂-CH₂-CH₂-CH₂-CH₂- | |
| -2328 | 2-CF₃ | 6-OCH₃ | -CH₂-CH₂-CH₂-CH₂-CH₂- | |
| -2329 | 3-CF₃ | 6-OCH₃ | -CH₂-CH₂-CH₂-CH₂-CH₂- | |
| -2330 | 4-CF₃ | 6-OCH₃ | -CH₂-CH₂-CH₂-CH₂-CH₂- | |
| -2331 | 2,4-F₂ | 6-OCH₃ | -CH₂-CH₂-CH₂-CH₂-CH₂- | |
| -2332 | 2,4-Cl₂ | 6-OCH₃ | -CH₂-CH₂-CH₂-CH₂-CH₂- | |
| -2333 | 2,6-Cl₂ | 6-OCH₃ | -CH₂-CH₂-CH₂-CH₂-CH₂- | |
| -2334 | 2-CN | 6-OCH₃ | -CH₂-CH₂-CH₂-CH₂-CH₂- | |
| -2335 | 3-CN | 6-OCH₃ | -CH₂-CH₂-CH₂-CH₂-CH₂- | |
| -2336 | 4-CN | 6-OCH₃ | -CH₂-CH₂-CH₂-CH₂-CH₂- | |
| -2337 | 2-F-4-CN | 6-OCH₃ | -CH₂-CH₂-CH₂-CH₂-CH₂- | |
| -2338 | 2-Cl-4-CN | 6-OCH₃ | -CH₂-CH₂-CH₂-CH₂-CH₂- | |
| -2339 | 2-Cl-4-CF3 | 6-OCH₃ | -CH₂-CH₂-CH₂-CH₂-CH₂- | |
| -2340 | 2-F-4-CF3 | 6-OCH₃ | -CH₂-CH₂-CH₂-CH₂-CH₂- | |

According to one embodiment, the present invention relates to compounds of the formulae III.A to III.R

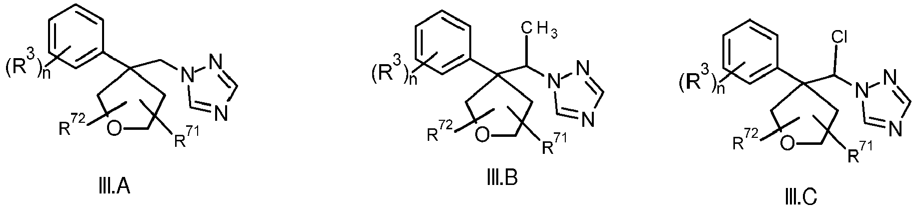

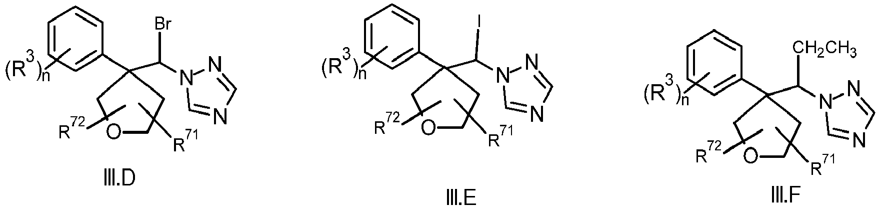

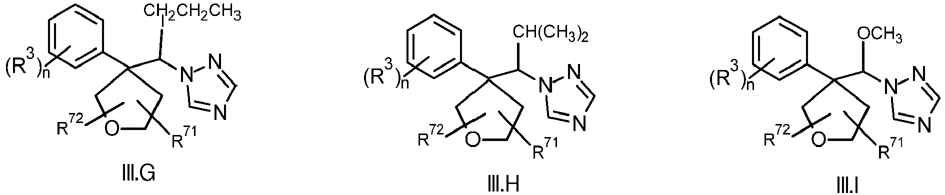

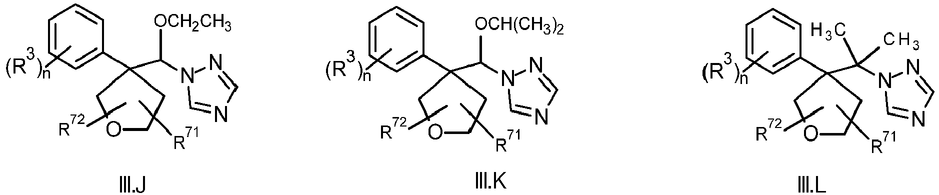

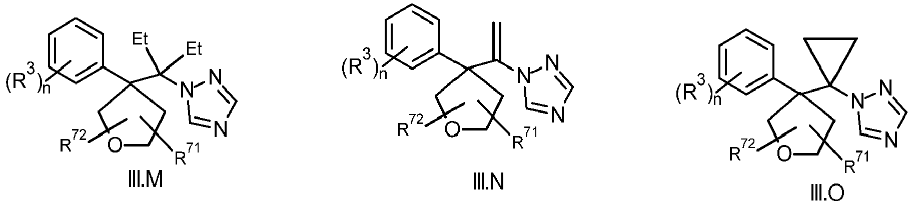

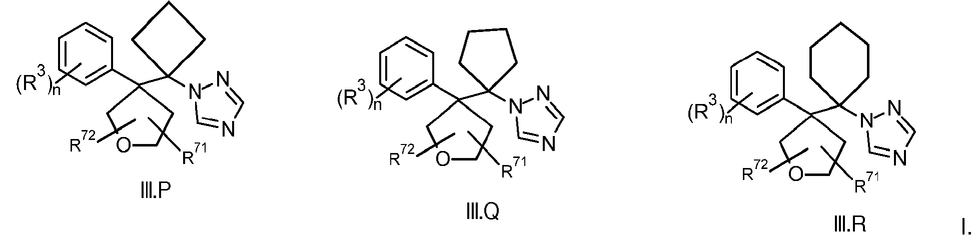

According to one embodiment, the present invention relates to compounds of the formulae IV.A to IV.R

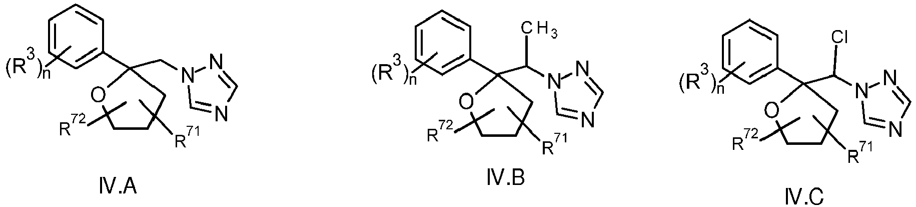

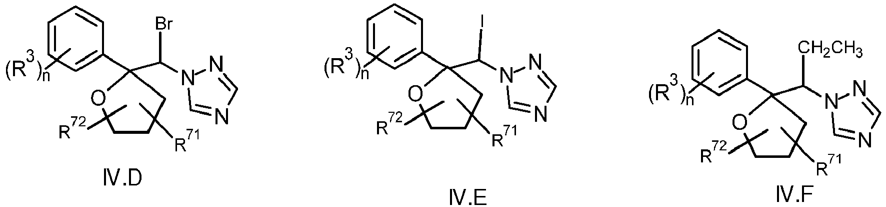

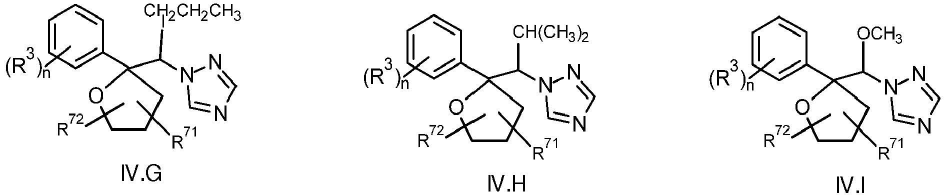

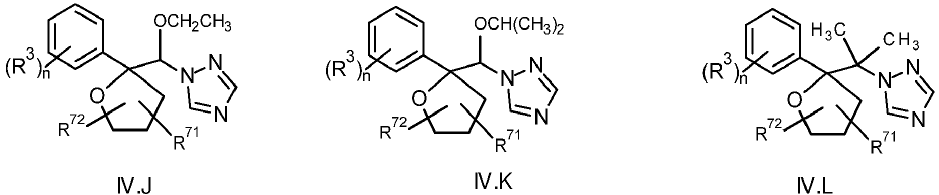

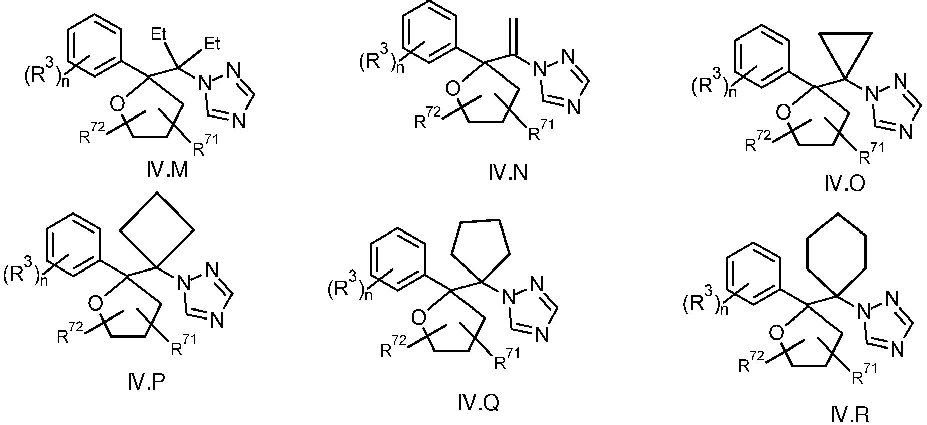

Preference is given to the compounds I according to the invention compiled in Tables 1a-III to 9a-III, 1a-IV to 9a-IV below with the proviso as defined above. The groups mentioned for a substituent in the tables are furthermore per se, independently of the combination in which they are mentioned, a particularly preferred aspect of the substituent in question.

### Table 1a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-1 of Table C and the meaning for (R³)ₙ for each individual compound corresponds in each case to one line of Table P (compounds III.A.C1.P1 to III.A.C1.P155; compounds III.B.C1.P1 to III.B.C1.P155; compounds III.C.C1.P1 to III.C.C1.P155; compounds III.D.C1.P1 to III.D.C1.P155; compounds III.E.C1.P1 to III.E.C1.P155; compounds III.F.C1.P1 to III.F.C1.P155; compounds III.G.C1.P1 to III.G.C1.P155; compounds III.H.C1.P1 to III.H.C1.P155; compounds III.I.C1.P1 to III.I.C1.P155; compounds III.J.C1.P1 to III.J.C1.P155; compounds III.K.C1.P1 to III.K.C1.P155; compounds III.L.C1.P1 to III.L.C1.P155; compounds III.M.C1.P1 to III.M.C1.P155; compounds III.N.C1.P1 to III.N.C1.P155; compounds III.O.C1.P1 to III.O.C1.P155; compounds III.P.C1.P1 to III.P.C1.P155; compounds III.Q.C1.P1 to III.Q.C1.P155; compounds III.R.C1.P1 to III.R.C1.P155;).

### Table 2a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-2 of Table C and the meaning for (R³)ₙ for each individual compound corresponds in each case to one line of Table P (compounds III.A.C2.P1 to III.A.C2.P155; compounds III.B.C2.P1 to III.B.C2.P155; compounds III.C.C2.P1 to III.C.C2.P155; compounds III.D.C2.P1 to III.D.C2.P155; compounds III.E.C2.P1 to III.E.C2.P155; compounds III.F.C2.P1 to III.F.C2.P155; compounds III.G.C2.P1 to III.G.C2.P155; compounds III.H.C2.P1 to III.H.C2.P155; compounds III.I.C2.P1 to III.I.C2.P155; compounds III.J.C2.P1 to III.J.C2.P155; compounds III.K.C2.P1 to III.K.C2.P155; compounds III.L.C1.P1 to III.L.C1.P155; compounds III.M.C2.P1 to III.M.C2.P155; compounds III.N.C2.P1 to III.N.C2.P155; compounds III.O.C2.P1 to III.O.C2.P155; compounds III.P.C2.P1 to III.P.C2.P155; compounds III.Q.C2.P1 to III.Q.C2.P155; compounds III.R.C2.P1 to III.R.C2.P155;).

### Table 3a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-3 of Table C and the meaning for (R³)ₙ for each individual compound corresponds in each case to one line of Table P (compounds III.A.C3.P1 to III.A.C3.P155; compounds III.B.C3.P1 to III.B.C3.P155; compounds III.C.C3.P1 to III.C.C3.P155; compounds III.D.C3.P1 to III.D.C3.P155; compounds III.E.C3.P1 to III.E.C3.P155; compounds III.F.C3.P1 to III.F.C3.P155; compounds III.G.C3.P1 to III.G.C3.P155; compounds III.H.C3.P1 to III.H.C3.P155; compounds III.I.C3.P1 to III.I.C3.P155; compounds III.J.C3.P1 to III.J.C3.P155; compounds III.K.C3.P1 to III.K.C3.P155; compounds III.L.C1.P1 to III.L.C1.P155; compounds III.M.C3.P1 to III.M.C3.P155; compounds III.N.C3.P1 to III.N.C3.P155; compounds III.O.C3.P1 to III.O.C3.P155; compounds III.P.C3.P1 to III.P.C3.P155; compounds III.Q.C3.P1 to III.Q.C3.P155; compounds III.R.C3.P1 to III.R.C3.P155;).

### Table 4a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-4 of Table C and the meaning for (R³)ₙ for each individual compound corresponds in each case to one line of Table P (compounds III.A.C4.P1 to III.A.C4.P155; compounds III.B.C4.P1 to III.B.C4.P155; compounds III.C.C4.P1 to III.C.C4.P155; compounds III.D.C4.P1 to III.D.C4.P155; compounds III.E.C4.P1 to III.E.C4.P155; compounds III.F.C4.P1 to III.F.C4.P155; compounds III.G.C4.P1 to III.G.C4.P155; compounds III.H.C4.P1 to III.H.C4.P155; compounds III.I.C4.P1 to III.I.C4.P155; compounds III.J.C4.P1 to III.J.C4.P155; compounds III.K.C4.P1 to III.K.C4.P155; compounds III.L.C1.P1 to III.L.C1.P155; compounds III.M.C4.P1 to III.M.C4.P155; compounds III.N.C4.P1 to III.N.C4.P155; compounds III.O.C4.P1 to III.O.C4.P155; compounds III.P.C4.P1 to III.P.C4.P155; compounds III.Q.C4.P1 to III.Q.C4.P155; compounds III.R.C4.P1 to III.R.C4.P155;).

### Table 5a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-5 of Table C and the meaning for (R³)ₙ for each individual compound corresponds in each case to one line of Table P (compounds III.A.C5.P1 to III.A.C5.P155; compounds III.B.C5.P1 to III.B.C5.P155; compounds III.C.C5.P1 to III.C.C5.P155; compounds III.D.C5.P1 to III.D.C5.P155; compounds III.E.C5.P1 to III.E.C5.P155; compounds III.F.C5.P1 to III.F.C5.P155; compounds III.G.C5.P1 to III.G.C5.P155; compounds III.H.C5.P1 to III.H.C5.P155; compounds III.I.C5.P1 to III.I.C5.P155; compounds III.J.C5.P1 to III.J.C5.P155; compounds III.K.C5.P1 to III.K.C5.P155; compounds III.L.C1.P1 to III.L.C1.P155; compounds III.M.C5.P1 to III.M.C5.P155; compounds III.N.C5.P1 to III.N.C5.P155; compounds III.O.C5.P1 to III.O.C5.P155; compounds III.P.C5.P1 to III.P.C5.P155; compounds III.Q.C5.P1 to III.Q.C5.P155; compounds III.R.C5.P1 to III.R.C5.P155;).

### Table 6a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-6 of Table C and the meaning for (R³)ₙ for each individual compound corresponds in each case to one line of Table P (compounds III.A.C6.P1 to III.A.C6.P155; compounds III.B.C6.P1 to III.B.C6.P155; compounds III.C.C6.P1 to III.C.C6.P155; compounds III.D.C6.P1 to III.D.C6.P155; compounds III.E.C6.P1 to III.E.C6.P155; compounds III.F.C6.P1 to III.F.C6.P155; compounds III.G.C6.P1 to III.G.C6.P155; compounds III.H.C6.P1 to III.H.C6.P155; compounds III.I.C6.P1 to III.I.C6.P155; compounds III.J.C6.P1 to III.J.C6.P155; compounds III.K.C6.P1 to III.K.C6.P155; compounds III.L.C1.P1 to III.L.C1.P155; compounds III.M.C6.P1 to III.M.C6.P155; compounds III.N.C6.P1 to III.N.C6.P155; compounds III.O.C6.P1 to III.O.C6.P155; compounds III.P.C6.P1 to III.P.C6.P155; compounds III.Q.C6.P1 to III.Q.C6.P155; compounds III.R.C6.P1 to III.R.C6.P155;).

### Table 7a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-7 of Table C and the meaning for (R³)ₙ for each individual compound corresponds in each case to one line of Table P (compounds III.A.C7.P1 to III.A.C7.P155; compounds III.B.C7.P1 to III.B.C7.P155; compounds III.C.C7.P1 to III.C.C7.P155; compounds III.D.C7.P1 to III.D.C7.P155; compounds III.E.C7.P1 to III.E.C7.P155; compounds III.F.C7.P1 to III.F.C7.P155; compounds III.G.C7.P1 to III.G.C7.P155; compounds III.H.C7.P1 to III.H.C7.P155; compounds III.I.C7.P1 to III.I.C7.P155; compounds III.J.C7.P1 to III.J.C7.P155; compounds III.K.C7.P1 to III.K.C7.P155; compounds III.L.C1.P1 to III.L.C1.P155; compounds III.M.C7.P1 to III.M.C7.P155; compounds III.N.C7.P1 to III.N.C7.P155; compounds III.O.C7.P1 to III.O.C7.P155; compounds III.P.C7.P1 to III.P.C7.P155; compounds III.Q.C7.P1 to III.Q.C7.P155; compounds III.R.C7.P1 to III.R.C7.P155;).

### Table 8a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-8 of Table C and the meaning for (R³)ₙ for each individual compound corresponds in each case to one line of Table P (compounds III.A.C8.P1 to III.A.C8.P155; compounds III.B.C8.P1 to III.B.C8.P155; compounds III.C.C8.P1 to III.C.C8.P155; compounds III.D.C8.P1 to III.D.C8.P155; compounds III.E.C8.P1 to III.E.C8.P155; compounds III.F.C8.P1 to III.F.C8.P155; compounds III.G.C8.P1 to III.G.C8.P155; compounds III.H.C8.P1 to III.H.C8.P155; compounds III.I.C8.P1 to III.I.C8.P155; compounds III.J.C8.P1 to III.J.C8.P155; compounds III.K.C8.P1 to III.K.C8.P155; compounds III.L.C1.P1 to III.L.C1.P155; compounds III.M.C8.P1 to III.M.C8.P155; compounds III.N.C8.P1 to III.N.C8.P155; compounds III.O.C8.P1 to III.O.C8.P155; compounds III.P.C8.P1 to III.P.C8.P155; compounds III.Q.C8.P1 to III.Q.C8.P155; compounds III.R.C8.P1 to III.R.C8.P155;).

### Table 9a-III

Compounds of the formula III.A, III.B, III.C, III.D, III.E, III.F, III.G, III.H, III.I, III.J, III.K, III.L, III.M, III.N, III.O, III.P, III.Q, III.R in which the combination of R⁷¹ and R⁷² corresponds to line C-9 of Table C and the meaning for (R³)ₙ for each individual compound corresponds in each case to one line of Table P (compounds III.A.C9.P1 to III.A.C9.P155; compounds III.B.C9.P1 to III.B.C9.P155; compounds III.C.C9.P1 to III.C.C9.P155; compounds III.D.C9.P1 to III.D.C9.P155; compounds III.E.C9.P1 to III.E.C9.P155; compounds III.F.C9.P1 to III.F.C9.P155; compounds III.G.C9.P1 to III.G.C9.P155; compounds III.H.C9.P1 to III.H.C9.P155; compounds III.I.C9.P1 to III.I.C9.P155; compounds III.J.C9.P1 to III.J.C9.P155; compounds III.K.C9.P1 to III.K.C9.P155; compounds III.L.C1.P1 to III.L.C1.P155; compounds III.M.C9.P1 to III.M.C9.P155; compounds III.N.C9.P1 to III.N.C9.P155; compounds III.O.C9.P1 to III.O.C9.P155; compounds III.P.C9.P1 to III.P.C9.P155; compounds III.Q.C9.P1 to III.Q.C9.P155; compounds III.R.C9.P1 to III.R.C9.P155;).

### Table 1a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-1 of Table C and the meaning for (R³)ₙ for each individual compound corresponds in each case to one line of Table P (compounds IV.A.C1.P1 to IV.A.C1.P155; compounds IV.B.C1.P1 to IV.B.C1.P155; compounds IV.C.C1.P1 to IV.C.C1.P155; compounds IV.D.C1.P1 to IV.D.C1.P155; compounds IV.E.C1.P1 to IV.E.C1.P155; compounds IV.F.C1.P1 to IV.F.C1.P155; compounds IV.G.C1.P1 to IV.G.C1.P155; compounds IV.H.C1.P1 to IV.H.C1.P155; compounds IV.I.C1.P1 to IV.I.C1.P155; compounds IV.J.C1.P1 to IV.J.C1.P155; compounds IV.K.C1.P1 to IV.K.C1.P155; compounds IV.L.C1.P1 to IV.L.C1.P155; compounds IV.M.C1.P1 to IV.M.C1.P155; compounds IV.N.C1.P1 to IV.N.C1.P155; compounds IV.O.C1.P1 to IV.O.C1.P155; compounds IV.P.C1.P1 to IV.P.C1.P155; compounds IV.Q.C1.P1 to IV.Q.C1.P155; compounds IV.R.C1.P1 to IV.R.C1.P155;).

### Table 2a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-2 of Table C and the meaning for (R³)ₙ for each individual compound corresponds in each case to one line of Table P (compounds IV.A.C2.P1 to IV.A.C2.P155; compounds IV.B.C2.P1 to IV.B.C2.P155; compounds IV.C.C2.P1 to IV.C.C2.P155; compounds IV.D.C2.P1 to IV.D.C2.P155; compounds IV.E.C2.P1 to IV.E.C2.P155; compounds IV.F.C2.P1 to IV.F.C2.P155; compounds IV.G.C2.P1 to IV.G.C2.P155; compounds IV.H.C2.P1 to IV.H.C2.P155; compounds IV.I.C2.P1 to IV.I.C2.P155; compounds IV.J.C2.P1 to IV.J.C2.P155; compounds IV.K.C2.P1 to IV.K.C2.P155; compounds IV.L.C1.P1 to IV.L.C1.P155; compounds IV.M.C2.P1 to IV.M.C2.P155; compounds IV.N.C2.P1 to IV.N.C2.P155; compounds IV.O.C2.P1 to IV.O.C2.P155; compounds IV.P.C2.P1 to IV.P.C2.P155; compounds IV.Q.C2.P1 to IV.Q.C2.P155; compounds IV.R.C2.P1 to IV.R.C2.P155;).

### Table 3a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-3 of Table C and the meaning for (R³)ₙ for each individual compound corresponds in each case to one line of Table P (compounds IV.A.C3.P1 to IV.A.C3.P155; compounds IV.B.C3.P1 to IV.B.C3.P155; compounds IV.C.C3.P1 to IV.C.C3.P155; compounds IV.D.C3.P1 to IV.D.C3.P155; compounds IV.E.C3.P1 to IV.E.C3.P155; compounds IV.F.C3.P1 to IV.F.C3.P155; compounds IV.G.C3.P1 to IV.G.C3.P155; compounds IV.H.C3.P1 to IV.H.C3.P155; compounds IV.I.C3.P1 to IV.I.C3.P155; compounds IV.J.C3.P1 to IV.J.C3.P155; compounds IV.K.C3.P1 to IV.K.C3.P155; compounds IV.L.C1.P1 to IV.L.C1.P155; compounds IV.M.C3.P1 to IV.M.C3.P155; compounds IV.N.C3.P1 to IV.N.C3.P155; compounds IV.O.C3.P1 to IV.O.C3.P155; compounds IV.P.C3.P1 to IV.P.C3.P155; compounds IV.Q.C3.P1 to IV.Q.C3.P155; compounds IV.R.C3.P1 to IV.R.C3.P155;).

### Table 4a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-4 of Table C and the meaning for (R³)ₙ for each individual compound corresponds in each case to one line of Table P (compounds IV.A.C4.P1 to IV.A.C4.P155; compounds IV.B.C4.P1 to IV.B.C4.P155; compounds IV.C.C4.P1 to IV.C.C4.P155; compounds IV.D.C4.P1 to IV.D.C4.P155; compounds IV.E.C4.P1 to IV.E.C4.P155; compounds IV.F.C4.P1 to IV.F.C4.P155; compounds IV.G.C4.P1 to IV.G.C4.P155; compounds IV.H.C4.P1 to IV.H.C4.P155; compounds IV.I.C4.P1 to IV.I.C4.P155; compounds IV.J.C4.P1 to IV.J.C4.P155; compounds IV.K.C4.P1 to IV.K.C4.P155; compounds IV.L.C1.P1 to IV.L.C1.P155; compounds IV.M.C4.P1 to IV.M.C4.P155; compounds IV.N.C4.P1 to IV.N.C4.P155; compounds IV.O.C4.P1 to IV.O.C4.P155; compounds IV.P.C4.P1 to IV.P.C4.P155; compounds IV.Q.C4.P1 to IV.Q.C4.P155; compounds IV.R.C4.P1 to IV.R.C4.P155;).

### Table 5a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-5 of Table C and the meaning for (R³)ₙ for each individual compound corresponds in each case to one line of Table P (compounds IV.A.C5.P1 to IV.A.C5.P155; compounds IV.B.C5.P1 to IV.B.C5.P155; compounds IV.C.C5.P1 to IV.C.C5.P155; compounds IV.D.C5.P1 to IV.D.C5.P155; compounds IV.E.C5.P1 to IV.E.C5.P155; compounds IV.F.C5.P1 to IV.F.C5.P155; compounds IV.G.C5.P1 to IV.G.C5.P155; compounds IV.H.C5.P1 to IV.H.C5.P155; compounds IV.I.C5.P1 to IV.I.C5.P155; compounds IV.J.C5.P1 to IV.J.C5.P155; compounds IV.K.C5.P1 to IV.K.C5.P155; compounds IV.L.C1.P1 to IV.L.C1.P155; compounds IV.M.C5.P1 to IV.M.C5.P155; compounds IV.N.C5.P1 to IV.N.C5.P155; compounds IV.O.C5.P1 to IV.O.C5.P155; compounds IV.P.C5.P1 to IV.P.C5.P155; compounds IV.Q.C5.P1 to IV.Q.C5.P155; compounds IV.R.C5.P1 to IV.R.C5.P155;).

### Table 6a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-6 of Table C and the meaning for (R³)ₙ for each individual compound corresponds in each case to one line of Table P (compounds IV.A.C6.P1 to IV.A.C6.P155; compounds IV.B.C6.P1 to IV.B.C6.P155; compounds IV.C.C6.P1 to IV.C.C6.P155; compounds IV.D.C6.P1 to IV.D.C6.P155; compounds IV.E.C6.P1 to IV.E.C6.P155; compounds IV.F.C6.P1 to IV.F.C6.P155; compounds IV.G.C6.P1 to IV.G.C6.P155; compounds IV.H.C6.P1 to IV.H.C6.P155; compounds IV.I.C6.P1 to IV.I.C6.P155; compounds IV.J.C6.P1 to IV.J.C6.P155; compounds IV.K.C6.P1 to IV.K.C6.P155; compounds IV.L.C1.P1 to IV.L.C1.P155; compounds IV.M.C6.P1 to IV.M.C6.P155; compounds IV.N.C6.P1 to IV.N.C6.P155; compounds IV.O.C6.P1 to IV.O.C6.P155; compounds IV.P.C6.P1 to IV.P.C6.P155; compounds IV.Q.C6.P1 to IV.Q.C6.P155; compounds IV.R.C6.P1 to IV.R.C6.P155;).

### Table 7a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-7 of Table C and the meaning for (R³)ₙ for each individual compound corresponds in each case to one line of Table P (compounds IV.A.C7.P1 to IV.A.C7.P155; compounds IV.B.C7.P1 to IV.B.C7.P155; compounds IV.C.C7.P1 to IV.C.C7.P155; compounds IV.D.C7.P1 to IV.D.C7.P155; compounds IV.E.C7.P1 to IV.E.C7.P155; compounds IV.F.C7.P1 to IV.F.C7.P155; compounds IV.G.C7.P1 to IV.G.C7.P155; compounds IV.H.C7.P1 to IV.H.C7.P155; compounds IV.I.C7.P1 to IV.I.C7.P155; compounds IV.J.C7.P1 to IV.J.C7.P155; compounds IV.K.C7.P1 to IV.K.C7.P155; compounds IV.L.C1.P1 to IV.L.C1.P155; compounds IV.M.C7.P1 to IV.M.C7.P155; compounds IV.N.C7.P1 to IV.N.C7.P155; compounds IV.O.C7.P1 to IV.O.C7.P155; compounds IV.P.C7.P1 to IV.P.C7.P155; compounds IV.Q.C7.P1 to IV.Q.C7.P155; compounds IV.R.C7.P1 to IV.R.C7.P155;).

### Table 8a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-8 of Table C and the meaning for (R³)ₙ for each individual compound corresponds in each case to one line of Table P (compounds IV.A.C8.P1 to IV.A.C8.P155; compounds IV.B.C8.P1 to IV.B.C8.P155; compounds IV.C.C8.P1 to IV.C.C8.P155; compounds IV.D.C8.P1 to IV.D.C8.P155; compounds IV.E.C8.P1 to IV.E.C8.P155; compounds IV.F.C8.P1 to IV.F.C8.P155; compounds IV.G.C8.P1 to IV.G.C8.P155; compounds IV.H.C8.P1 to IV.H.C8.P155; compounds IV.I.C8.P1 to IV.I.C8.P155; compounds IV.J.C8.P1 to IV.J.C8.P155; compounds IV.K.C8.P1 to IV.K.C8.P155; compounds IV.L.C1.P1 to IV.L.C1.P155; compounds IV.M.C8.P1 to IV.M.C8.P155; compounds IV.N.C8.P1 to IV.N.C8.P155; compounds IV.O.C8.P1 to IV.O.C8.P155; compounds IV.P.C8.P1 to IV.P.C8.P155; compounds IV.Q.C8.P1 to IV.Q.C8.P155; compounds IV.R.C8.P1 to IV.R.C8.P155;).

### Table 9a-IV

Compounds of the formula IV.A, IV.B, IV.C, IV.D, IV.E, IV.F, IV.G, IV.H, IV.I, IV.J, IV.K, IV.L, IV.M, IV.N, IV.O, IV.P, IV.Q, IV.R in which the combination of R⁷¹ and R⁷² corresponds to line C-9 of Table C and the meaning for (R³)ₙ for each individual compound corresponds in each case to one line of Table P (compounds IV.A.C9.P1 to IV.A.C9.P155; compounds IV.B.C9.P1 to IV.B.C9.P155; compounds IV.C.C9.P1 to IV.C.C9.P155; compounds IV.D.C9.P1 to IV.D.C9.P155; compounds IV.E.C9.P1 to IV.E.C9.P155; compounds IV.F.C9.P1 to IV.F.C9.P155; compounds IV.G.C9.P1 to IV.G.C9.P155; compounds IV.H.C9.P1 to IV.H.C9.P155; compounds IV.I.C9.P1 to IV.I.C9.P155; compounds IV.J.C9.P1 to IV.J.C9.P155; compounds IV.K.C9.P1 to IV.K.C9.P155; compounds IV.L.C1.P1 to IV.L.C1.P155; compounds IV.M.C9.P1 to IV.M.C9.P155; compounds IV.N.C9.P1 to IV.N.C9.P155; compounds IV.O.C9.P1 to IV.O.C9.P155; compounds IV.P.C9.P1 to IV.P.C9.P155; compounds IV.Q.C9.P1 to IV.Q.C9.P155; compounds IV.R.C9.P1 to IV.R.C9.P155;)

**Table C**

| No. | R⁷¹ | R⁷² |
|---|---|---|
| C-1 | CH₃ | H |
| C-2 | CH₂CH₃ | H |
| C-3 | CH₂CH₂CH₃ | H |
| C-4 | CH₃ | CH₃ |
| C-5 | CH₂CH₃ | CH₂CH₃ |
| C-6 | -CH₂-CH2- | |
| C-7 | -CH₂-CH2-CH2- | |
| C-7 | -CH₂-CH2-CH2-CH2- | |
| C-9 | -CH₂-CH2-CH2-CH2-CH2- | |

The compounds I and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants. Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties. Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CrylA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum)* or T4-lysozym (e. g. potato cultivars capable of synthesizing these proteins with increased resistance against bacteria such as *Erwinia amylvora).* The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugospp.* (white rust) on ornamentals, vegetables (e. g. *A. candida)* and sunflowers (e. g. *A. tragopogonis); Alternaria* spp. (Alternaria leaf spot) on vegetables, rape *(A. brassicola* or *brassicae),* sugar beets *(A. tenuis),* fruits, rice, soybeans, potatoes (e. g. *A. solani or A. alternata),* tomatoes (e. g. *A. solani or A. alternata)* and wheat; *Aphanomyces* spp. on sugar beets and vegetables; *Ascochyta spp.* on cereals and vegetables, e. g. *A. tritici* (anthracnose) on wheat and *A. hordei on* barley; *Bipolaris* and *Drechslera spp.* (teleomorph: *Cochliobolus spp.),* e. g. Southern leaf blight *(D. maydis)* or Northern leaf blight *(B. zeicola)* on corn, e. g. spot blotch *(B. sorokiniana)* on cereals and e.g. *B. oryzae* on rice and turfs; *Blumeria* (formerly *Erysiphe) graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma)* spp. (rot or wilt) on broad-leaved trees and evergreens, e. g. C. *ulmi*(Dutch elm disease) on elms; *Cercosporaspp.* (Cercospora leaf spots) on corn (e.g. Gray leaf spot: *C. zeae-maydis),* rice, sugar beets (e. g. *C. beticola),* sugar cane, vegetables, coffee, soybeans (e. g. *C. sojina* or *C. kikuchii)* and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris)* spp. (leaf spots) on corn *(C. carbonum),* cereals (e. g. *C. sativus,* anamorph: *B. sorokiniana)* and rice (e. g. *C. miyabeanus,* anamorph: *H. oryzae); Colletotrichum* (teleomorph: *Glomerella)* spp. (anthracnose) on cotton (e. g. *C. gossypii),* corn (e. g. *C. graminicola: An*thracnose stalk rot), soft fruits, potatoes (e. g. *C. coccodes:* black dot), beans (e. g. *C. lindemuthianum)* and soybeans (e. g. *C. truncatum* or *C. gloeosporioides); Corticium* spp., e. g. *C. sasakii (sheath* blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri:* Black Foot Disease) and ornamentals; *Dematophora* (teleomorph: *Rosellinia)* necatrix (root and stem rot) on soybeans; *Diaporthe spp.,* e. g. *D. phaseolorum* (damping off) on soybeans; *Drechslera* (syn. *Helminthosporium,* teleomorph: *Pyrenophora)* spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia* (syn. *Phellinus) punctata, F. mediterranea, Phaeomoniella chlamydospora* (earlier *Phaeoacremonium chlamydosporum), Phaeoacremonium aleophilum* and/or *Botryosphaeria obtusa; Elsinoe* spp. on pome fruits *(E pyri),* soft fruits *(E veneta:* anthracnose) and vines *(E ampelina:* anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* on soybeans and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii on* cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M. fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakopsora pachyrhizi and P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp*.* on sunflowers, vines (e. g. *P. viticola*: can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans*: late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp*.* (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P. teres (net* blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*); *Septoria* spp. on various plants, e. g. *S. glycines* (brown spot) on soybeans, *S. tritici* (*Septoria* blotch) on wheat and *S.* (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum,* syn. *Helminthosporium turcicum*) and turf; *Sphacelotheca* spp. (smut) on corn, (e. g. *S. reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonosporaspp.* on cereals, e. g. *S. nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis*: corn smut) and sugar cane; *Venturia* spp. (scab) on apples (e. *g. V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The compounds I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting. The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and compositions thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005. Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders. Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and compositions thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and compositions thereof.

Suitable surfactants are surface-active compounds, such as anionic, cationic, nonionic and amphoteric surfactants, block polymers, polyelectrolytes, and compositions thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and compositions thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates. Suitable nonionic surfactants are alkoxylates, N-subsituted fatty acid amides, amine oxides, esters, sugar-based surfactants, polymeric surfactants, and compositions thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This composition is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This composition is stirred for 1 h to produce spontaneously a thermodynamically stable microemulsion.
x) Microcapsules (CS)
   An oil phase comprising 5-50 wt% of a compound I, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e.g. finely divided kaolin) ad 100 wt%.
xii) Granules (GR, FG)
   0.5-30 wt% of a compound I is ground finely and associated with solid carrier (e.g. silicate) ad 100 wt%. Granulation is achieved by extrusion, spray-drying or fluidized bed.
xiii) Ultra-low volume liquids (UL)
   1-50 wt% of a compound I are dissolved in organic solvent (e.g. aromatic hydrocarbon) ad 100 wt%.

The compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary composition may be mixed by the user himself in a spray tank and further auxiliaries may be added, if appropriate.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the compounds I can be used, is intended to illustrate the possible combinations but does not limit them:
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxystrobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)-phenyl)-2-methoxyimino-N-methyl-acetamide, pyribencarb, triclopyricarb/chlorodincarb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl]2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl]2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl]2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl2-methylpropanoate
   - inhibitors of complex II (e. g. carboxamides): benodanil, benzovindiflupyr, bixafen, boscalid, carboxin, fenfuram, fluopyram, flutolanil, fluxapyroxad, furametpyr, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide;
   - other respiration inhibitors (e.g. complex I, uncouplers): diflumetorim, (5,8-difluoroquinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 1-[*rel*-(2*S*,3*R*)-3-(2-chlorophenyl)-2-(2,4-difluorophenyl)-oxiranylmethyl]-5-thiocyanato-1H-[1,2,4]triazole, 2-[*rel*-(2*S*;3*R*)-3-(2-chlorophenyl)-2-(2,4-difluorophenyl)-oxiranylmethyl]-2H-[1,2,4]triazole-3-thiol; imidazoles: imazalil, pefurazoate, prochloraz, triflumizol; pyrimidines, pyridines and piperazines: fenarimol, nuarimol, pyrifenox, triforine;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methyl-piperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)-ethanesulfonyl)-but-2-yl) carbamic acid-(4-fluorophenyl) ester;
   - compounds affecting cell membrane permeability and fatty acides: propamocarb, propamocarb-hydrochlorid
   - fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone H) Inhibitors with Multi Site Action
   - inorganic active substances: Bordeaux composition, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlorphenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1H,5H-[1,4]dithiino[2,3-c:5,6-c']dipyrrole-1,3,5,7(2H,6H)-tetraone;
I) Cell wall synthesis inhibitors
   - inhibitors of glucan synthesis: validamycin, polyoxin B; melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxy-acetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole), N-(6-methoxy-pyridin-3-yl) cyclopropanecarboxylic acid amide, 5-chloro-1-(4,6-dimethoxy-pyrimidin-2-yl)-2-methyl-1 H-benzoimidazole, 2-(4-chloro-phenyl)-N-[4-(3,4-dimethoxy-phenyl)-isoxazol-5-yl]-2-prop-2-ynyloxy-acetamide;
L) Antifungal biocontrol agents, plant bioactivators: *Ampelomyces quisqualis* (e.g. AQ 10^{®} from Intrachem Bio GmbH & Co. KG, Germany), *Aspergillus flavus* (e.g. AFLAGUARD^{®} from Syngenta, CH), *Aureobasidium pullulans* (e.g. BOTECTOR^{®} from bio-ferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSO-DY^{®}, SERENADE^{®} MAX and SERENADE^{®} ASO from AgraQuest Inc., USA), *Bacillus subtilis* var. *amyloliquefaciens* FZB24 (e.g. TAEGRO^{®} from Novozyme Biologicals, Inc., USA), *Candida oleophila* I-82 (e.g. ASPIRE^{®} from Ecogen Inc., USA), *Candida saitoana* (e.g. BIO-CURE^{®} (in composition with lysozyme) and BIOCOAT^{®} from Micro Flo Company, USA (BASF SE) and Arysta), Chitosan (e.g. ARMOUR-ZEN from BotriZen Ltd., NZ), *Clonostachys rosea* f*. catenulata,* also named *Gliocladium catenulatum* (e.g. isolate J 1446: PRES-TOP^{®} from Verdera, Finland), *Coniothyrium minitans* (e.g. CONTANS^{®} from Prophyta, Germany), *Cryphonectria parasitica* (e.g. *Endothia parasiticafrom* CNICM, France), *Cryptococcus albidus* (e.g. YIELD PLUS^{®} from Anchor Bio-Technologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Bio-preparaty, Czech Rep.), *Reynoutria sachlinensis* (e.g. REGALIA^{®} from Marrone BioUInnovations, USA), *Talaromyces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma BioWorks Inc., USA), *T. harzianum* TH 35 (e.g. ROOT PRO^{®} from Mycontrol Ltd., Israel), *T. harzianum* T-39 (e.g. TRICHODEX^{®} and TRICHO-DERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isagro Ricerca, Italy), *T. polysporum* and *T. harzianum* (e.g. BINAB^{®} from BINAB Bio-Innovation AB, Sweden), *T. stromaticum* (e.g. TRICO-VAB^{®} from C.E.P.L.A.C., Brazil), *T. virens* GL-21 (e.g. SOILGARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN^{®} from Botry-Zen Ltd, NZ);
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron, tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - others: benclothiaz, bifenazate, cartap, flonicamid, pyridalyl, pymetrozine, sulfur, thiocyclam, flubendiamide, chlorantraniliprole, cyazypyr (HGW86), cyenopyrafen, flupyrazofos, cyflumetofen, amidoflumet, imicyafos, bistrifluron, and pyrifluquinazon.

The present invention furthermore relates to agrochemical compositions comprising a composition of at least one compound I (component 1) and at least one further active substance useful for plant protection, e. g. selected from the groups A) to O) (component 2), in particular one further fungicide, e. g. one or more fungicide from the groups A) to L), as described above, and if desired one suitable solvent or solid carrier. Those compositions are of particular interest, since many of them at the same application rate show higher efficiencies against harmful fungi. Furthermore, combating harmful fungi with a composition of compounds I and at least one fungicide from groups A) to L), as described above, is more efficient than combating those fungi with individual compounds I or individual fungicides from groups A) to L). By applying compounds I together with at least one active substance from groups A) to O) a synergistic effect can be obtained, i.e. more then simple addition of the individual effects is obtained (synergistic compositions).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

In binary compositions, i.e. compositions according to the invention comprising one compound I (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1. In ternary compositions, i.e. compositions according to the invention comprising one compound I (component 1) and a first further active substance (component 2) and a second further active substance (component 3), e. g. two active substances from groups A) to O), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to compositions comprising a compound I (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; benzovindiflupyr, bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to compositions comprising a compound of formula I (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to compositions comprising a compound of formula I (component 1) and at least one active substance selected from group C) (component 2) and particularly selected from metalaxyl, (metalaxyl-M) mefenoxam, ofurace.

Preference is given to compositions comprising a compound of formula I (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to compositions comprising a compound I (component 1) and at least one active substance selected from group E) (component 2) and particularly selected from cyprodinil, mepanipyrim, pyrimethanil.

Preference is also given to compositions comprising a compound I (component 1) and at least one active substance selected from group F) (component 2) and particularly selected from iprodione, fludioxonil, vinclozolin, quinoxyfen.

Preference is also given to compositions comprising a compound I (component 1) and at least one active substance selected from group G) (component 2) and particularly selected from dimethomorph, flumorph, iprovalicarb, benthiavalicarb, mandipropamid, propamocarb. Preference is also given to compositions comprising a compound I (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to compositions comprising a compound I (component 1) and at least one active substance selected from group I) (component 2) and particularly selected from carpropamid and fenoxanil.

Preference is also given to compositions comprising a compound I (component 1) and at least one active substance selected from group J) (component 2) and particularly selected from acibenzolar-S-methyl, probenazole, tiadinil, fosetyl, fosetyl-aluminium, H₃PO₃ and salts thereof. Preference is also given to compositions comprising a compound I (component 1) and at least one active substance selected from group K) (component 2) and particularly selected from cymoxanil, proquinazid and *N-*methyl-2-{1-[(5-methyl-3-trifluoromethyl-1H-pyrazol-1-yl)-acetyl]-piperidin-4-yl}-*N-*[(1R)-1,2,3,4-tetrahydronaphthalen-1-yl]-4-thiazolecarboxamide.

Preference is also given to compositions comprising a compound I (component 1) and at least one active substance selected from group L) (component 2) and particularly selected from *Bacillus subtilis* strain NRRL No. B-21661, *Bacillus pumilus* strain NRRL No. B-30087 and *Ulocladium oudemansii.*

Accordingly, the present invention furthermore relates to compositions comprising one compound I (component 1) and one further active substance (component 2), which further active substance is selected from the column "Component 2" of the lines B-1 to B-369 of Table B.

A further embodiment relates to the compositions B-1 to B-369 listed in Table B, where a row of Table B corresponds in each case to a fungicidal composition comprising one of the in the present specification individualized compounds of formula I (component 1) and the respective further active substance from groups A) to O) (component 2) stated in the row in question. Preferably, the compositions described comprise the active substances in synergistically effective amounts.

**Table B: Composition comprising one indiviualized compound I and one further active substance from groups A) to O)**

| Composition | Component 1 | Component 2 |
|---|---|---|
| B-1 | one individualized compound I | Azoxystrobin |
| B-2 | one individualized compound I | Coumethoxystrobin |
| B-3 | one individualized compound I | Coumoxystrobin |
| B-4 | one individualized compound I | Dimoxystrobin |
| B-5 | one individualized compound I | Enestroburin |
| B-6 | one individualized compound I | Fenaminstrobin |
| B-7 | one individualized compound I | Fenoxystrobin/Flufenoxystrobin |
| B-8 | one individualized compound I | Fluoxastrobin |
| B-9 | one individualized compound I | Kresoxim-methyl |
| B-10 | one individualized compound I | Metominostrobin |
| B-11 | one individualized compound I | Orysastrobin |
| B-12 | one individualized compound I | Picoxystrobin |
| B-13 | one individualized compound I | Pyraclostrobin |
| B-14 | one individualized compound I | Pyrametostrobin |
| B-15 | one individualized compound I | Pyraoxystrobin |
| B-16 | one individualized compound I | Pyribencarb |
| B-17 | one individualized compound I | Trifloxystrobin |
| B-18 | one individualized compound I | Triclopyricarb/Chlorodincarb |
| B-19 | one individualized compound I | 2-[2-(2,5-dimethyl-phenoxymethyl)-phenyl]-3-methoxy-acrylic acid methyl ester |
| B-20 | one individualized compound I | 2-(2-(3-(2,6-dichlorophenyl)-1-methyl-allylideneaminooxymethyl)-phenyl)-2-methoxyimino-N-methyl-acetamide |
| B-21 | one individualized compound I | Benalaxyl |
| B-22 | one individualized compound I | Benalaxyl-M |
| B-23 | one individualized compound I | Benodanil |
| B-24 | one individualized compound I | Benzovindiflupyr |
| B-25 | one individualized compound I | Bixafen |
| B-26 | one individualized compound I | Boscalid |
| B-27 | one individualized compound I | Carboxin |
| B-28 | one individualized compound I | Fenfuram |
| B-29 | one individualized compound I | Fenhexamid |
| B-30 | one individualized compound I | Flutolanil |
| B-31 | one individualized compound I | Fluxapyroxad |
| B-32 | one individualized compound I | Furametpyr |
| B-33 | one individualized compound I | Isopyrazam |
| B-34 | one individualized compound I | Isotianil |
| B-35 | one individualized compound I | Kiralaxyl |
| B-36 | one individualized compound I | Mepronil |
| B-37 | one individualized compound I | Metalaxyl |
| B-38 | one individualized compound I | Metalaxyl-M |
| B-39 | one individualized compound I | Ofurace |
| B-40 | one individualized compound I | Oxadixyl |
| B-41 | one individualized compound I | Oxycarboxin |
| B-42 | one individualized compound I | Penflufen |
| B-43 | one individualized compound I | Penthiopyrad |
| B-44 | one individualized compound I | Sedaxane |
| B-45 | one individualized compound I | Tecloftalam |
| B-46 | one individualized compound I | Thifluzamide |
| B-47 | one individualized compound I | Tiadinil |
| B-48 | one individualized compound I | 2-Amino-4-methyl-thiazole-5-carboxylic acid anilide |
| B-49 | one individualized compound I | N-(4'-trifluoromethylthiobiphenyl-2-yl)-3-difluoromethyl-1-methyl-1H-pyrazole-4-carboxamide |
| B-50 | one individualized compound I | N-(2-(1,3,3-trimethyl-butyl)-phenyl)-1,3-dimethyl-5-fluoro-1H-pyrazole-4-carboxamide |
| B-51 | one individualized compound I | 3-(difluoromethyl)-1-methyl-N-(1,1,3-tri-methylindan-4-yl)pyrazole-4-carbox-amide |
| B-52 | one individualized compound I | 3-(trifluoromethyl)-1-methyl-N-(1,1,3-tri-methylindan-4-yl)pyrazole-4-carbox-amide |
| B-53 | one individualized compound I | 1,3-dimethyl-N-(1,1,3-trimethylindan-4-yl)pyrazole-4-carboxamide |
| B-54 | one individualized compound I | 3-(trifluoromethyl)-1,5-dimethyl-N-(1,1,3-trimethylindan-4-yl)pyrazole-4-carboxamide |
| B-55 | one individualized compound I | 3-(difluoromethyl)-1,5-dimethyl-N-(1,1,3-trimethylindan-4-yl)pyrazole-4-carboxamide |
| B-56 | one individualized compound I | 1,3,5-trimethyl-N-(1,1,3-trimethylindan-4-yl)pyrazole-4-carboxamide |
| B-57 | one individualized compound I | Dimethomorph |
| B-58 | one individualized compound I | Flumorph |
| B-59 | one individualized compound I | Pyrimorph |
| B-60 | one individualized compound I | Flumetover |
| B-61 | one individualized compound I | Fluopicolide |
| B-62 | one individualized compound I | Fluopyram |
| B-63 | one individualized compound I | Zoxamide |
| B-64 | one individualized compound I | Carpropamid |
| B-65 | one individualized compound I | Diclocymet |
| B-66 | one individualized compound I | Mandipropamid |
| B-67 | one individualized compound I | Oxytetracyclin |
| B-68 | one individualized compound I | Silthiofam |
| B-69 | one individualized compound I | N-(6-methoxy-pyridin-3-yl) cyclopro-panecarboxylic acid amide |
| B-70 | one individualized compound I | Azaconazole |
| B-71 | one individualized compound I | Bitertanol |
| B-72 | one individualized compound I | Bromuconazole |
| B-73 | one individualized compound I | Cyproconazole |
| B-74 | one individualized compound I | Difenoconazole |
| B-75 | one individualized compound I | Diniconazole |
| B-76 | one individualized compound I | Diniconazole-M |
| B-77 | one individualized compound I | Epoxiconazole |
| B-78 | one individualized compound I | Fenbuconazole |
| B-79 | one individualized compound I | Fluquinconazole |
| B-80 | one individualized compound I | Flusilazole |
| B-81 | one individualized compound I | Flutriafol |
| B-82 | one individualized compound I | Hexaconazol |
| B-83 | one individualized compound I | Imibenconazole |
| B-84 | one individualized compound I | Ipconazole |
| B-85 | one individualized compound I | Metconazole |
| B-86 | one individualized compound I | Myclobutanil |
| B-87 | one individualized compound I | Oxpoconazol |
| B-88 | one individualized compound I | Paclobutrazol |
| B-89 | one individualized compound I | Penconazole |
| B-90 | one individualized compound I | Propiconazole |
| B-91 | one individualized compound I | Prothioconazole |
| B-92 | one individualized compound I | Simeconazole |
| B-93 | one individualized compound I | Tebuconazole |
| B-94 | one individualized compound I | Tetraconazole |
| B-95 | one individualized compound I | Triadimefon |
| B-96 | one individualized compound I | Triadimenol |
| B-97 | one individualized compound I | Triticonazole |
| B-98 | one individualized compound I | Uniconazole |
| B-99 | one individualized compound I | 1-[*re*/-(2*S*,3*R*)-3-(2-chlorophenyl)-2-(2,4-difluorophenyl)-oxiranylmethyl]-5-thiocyanato-1H-[1,2,4]triazole, |
| B-100 | one individualized compound I | 2-[*rel*(2*S;*3*R*)-3-(2-chlorophenyl)-2-(2,4-difluorophenyl)-oxiranylmethyl]-2H-[1,2,4]triazole-3-thiol |
| B-101 | one individualized compound I | Cyazofamid |
| B-102 | one individualized compound I | Amisulbrom |
| B-103 | one individualized compound I | Imazalil |
| B-104 | one individualized compound I | Imazalil-sulfate |
| B-105 | one individualized compound I | Pefurazoate |
| B-106 | one individualized compound I | Prochloraz |
| B-107 | one individualized compound I | Triflumizole |
| B-108 | one individualized compound I | Benomyl |
| B-109 | one individualized compound I | Carbendazim |
| B-110 | one individualized compound I | Fuberidazole |
| B-111 | one individualized compound I | Thiabendazole |
| B-112 | one individualized compound I | Ethaboxam |
| B-113 | one individualized compound I | Etridiazole |
| B-114 | one individualized compound I | Hymexazole |
| B-115 | one individualized compound I | 2-(4-Chloro-phenyl)-N-[4-(3,4-dimeth-oxy-phenyl)-isoxazol-5-yl]-2-prop-2-yn-yloxy-acetamide |
| B-116 | one individualized compound I | Fluazinam |
| B-117 | one individualized compound I | Pyrifenox |
| B-118 | one individualized compound I | 3-[5-(4-Chloro-phenyl)-2,3-dimethyl-is-oxazolidin-3-yl]-pyridine (Pyrisoxazole) |
| B-119 | one individualized compound I | 3-[5-(4-Methyl-phenyl)-2,3-dimethyl-isoxazolidin-3-yl]-pyridine |
| B-120 | one individualized compound I | Bupirimate |
| B-121 | one individualized compound I | Cyprodinil |
| B-122 | one individualized compound I | 5-Fluorocytosine |
| B-123 | one individualized compound I | 5-Fluoro-2-(p-tolylmethoxy)pyrimidin-4-amine |
| B-124 | one individualized compound I | 5-Fluoro-2-(4-fluorophenylmethoxy)-pyrimidin-4-amine |
| B-125 | one individualized compound I | Diflumetorim |
| B-126 | one individualized compound I | (5,8-Difluoroquinazolin-4-yl)-{2-[2-fluo-ro-4-(4-trifluoromethylpyridin-2-yloxy)-phenyl]-ethyl}-amine |
| B-127 | one individualized compound I | Fenarimol |
| B-128 | one individualized compound I | Ferimzone |
| B-129 | one individualized compound I | Mepanipyrim |
| B-130 | one individualized compound I | Nitrapyrin |
| B-131 | one individualized compound I | Nuarimol |
| B-132 | one individualized compound I | Pyrimethanil |
| B-133 | one individualized compound I | Triforine |
| B-134 | one individualized compound I | Fenpiclonil |
| B-135 | one individualized compound I | Fludioxonil |
| B-136 | one individualized compound I | Aldimorph |
| B-137 | one individualized compound I | Dodemorph |
| B-138 | one individualized compound I | Dodemorph-acetate |
| B-139 | one individualized compound I | Fenpropimorph |
| B-140 | one individualized compound I | Tridemorph |
| B-141 | one individualized compound I | Fenpropidin |
| B-142 | one individualized compound I | Fluoroimid |
| B-143 | one individualized compound I | Iprodione |
| B-144 | one individualized compound I | Procymidone |
| B-145 | one individualized compound I | Vinclozolin |
| B-146 | one individualized compound I | Famoxadone |
| B-147 | one individualized compound I | Fenamidone |
| B-148 | one individualized compound I | Flutianil |
| B-149 | one individualized compound I | Octhilinone |
| B-150 | one individualized compound I | Probenazole |
| B-151 | one individualized compound I | Fenpyrazamine |
| B-152 | one individualized compound I | Acibenzolar-S-methyl |
| B-153 | one individualized compound I | Ametoctradin |
| B-154 | one individualized compound I | Amisulbrom |
| B-155 | one individualized compound I | [(3S,6S,7R,8R)-8-benzyl-3-[(3-isobuty-ryloxymethoxy-4-methoxypyridine-2-carbonyl)amino]-6-methyl-4,9-dioxo-[1,5]dioxonan-7-yl]2-methylpropanoate |
| B-156 | one individualized compound I | [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate |
| B-157 | one individualized compound I | [(3S,6S,7R,8R)-8-benzyl-3-[[3-(acet-oxymethoxy)-4-methoxy-pyridine-2-carbonyl]amino]-6-methyl-4,9-dioxo-1,5-dioxonan-7-yl]2-methylpropanoate |
| B-158 | one individualized compound I | [(3S,6S,7R,8R)-8-benzyl-3-[(3-isobut-oxycarbonyloxy-4-meth oxy-pyrid i ne-2-carbonyl)amino]-6-methyl-4,9-dioxo-1,5-dioxonan-7-yl]2-methylpropanoate |
| B-159 | one individualized compound I | [(3S,6S,7R,8R)-8-benzyl-3-[[3-(1,3-ben-zodioxol-5-ylmethoxy)-4-methoxy-pyri-dine-2-carbonyl]amino]-6-methyl-4,9-di-oxo-1,5-dioxonan-7-yl]2-methyl-propanoate |
| B-160 | one individualized compound I | (3S,6S, 7 R,8 R)-3-[[(3-hydroxy-4-meth-oxy-2-pyridinyl)carbonyl]amino]-6-methyl-4,9-dioxo-8-(phenylmethyl)-1,5-dioxonan-7-yl 2-methylpropanoate |
| B-161 | one individualized compound I | Anilazin |
| B-162 | one individualized compound I | Blasticidin-S |
| B-163 | one individualized compound I | Captafol |
| B-164 | one individualized compound I | Captan |
| B-165 | one individualized compound I | Chinomethionat |
| B-166 | one individualized compound I | Dazomet |
| B-167 | one individualized compound I | Debacarb |
| B-168 | one individualized compound I | Diclomezine |
| B-169 | one individualized compound I | Difenzoquat, |
| B-170 | one individualized compound I | Difenzoquat-methylsulfate |
| B-171 | one individualized compound I | Fenoxanil |
| B-172 | one individualized compound I | Folpet |
| B-173 | one individualized compound I | Oxolinsäure |
| B-174 | one individualized compound I | Piperalin |
| B-175 | one individualized compound I | Proquinazid |
| B-176 | one individualized compound I | Pyroquilon |
| B-177 | one individualized compound I | Quinoxyfen |
| B-178 | one individualized compound I | Triazoxid |
| B-179 | one individualized compound I | Tricyclazole |
| B-180 | one individualized compound I | 2-Butoxy-6-iodo-3-propyl-chromen-4-one |
| B-181 | one individualized compound I | 5-Chloro-1-(4,6-dimethoxy-pyrimidin-2-yl)-2-methyl-1H-benzoimidazole |
| B-182 | one individualized compound I | 5-Chloro-7-(4-methyl-piperidin-1-yl)-6-(2,4,6-trifluoro-phenyl)-[1,2,4]tri-azolo[1,5-a]pyrimidine |
| B-183 | one individualized compound I | Ferbam |
| B-184 | one individualized compound I | Mancozeb |
| B-185 | one individualized compound I | Maneb |
| B-186 | one individualized compound I | Metam |
| B-187 | one individualized compound I | Methasulphocarb |
| B-188 | one individualized compound I | Metiram |
| B-189 | one individualized compound I | Propineb |
| B-190 | one individualized compound I | Thiram |
| B-191 | one individualized compound I | Zineb |
| B-192 | one individualized compound I | Ziram |
| B-193 | one individualized compound I | Diethofencarb |
| B-194 | one individualized compound I | Benthiavalicarb |
| B-195 | one individualized compound I | Iprovalicarb |
| B-196 | one individualized compound I | Propamocarb |
| B-197 | one individualized compound I | Propamocarb hydrochlorid |
| B-198 | one individualized compound I | Valifenalate |
| B-199 | one individualized compound I | N-(1-(1-(4-cyanophenyl)ethanesulfon-yl)-but-2-yl) carbamic acid-(4-fluoro-phenyl) ester |
| B-200 | one individualized compound I | Dodine |
| B-201 | one individualized compound I | Dodine free base |
| B-202 | one individualized compound I | Guazatine |
| B-203 | one individualized compound I | Guazatine-acetate |
| B-204 | one individualized compound I | Iminoctadine |
| B-205 | one individualized compound I | Iminoctadine-triacetate |
| B-206 | one individualized compound I | Iminoctadine-tris(albesilate) |
| B-207 | one individualized compound I | Kasugamycin |
| B-208 | one individualized compound I | Kasugamycin-hydrochloride-hydrate |
| B-209 | one individualized compound I | Polyoxine |
| B-210 | one individualized compound I | Streptomycin |
| B-211 | one individualized compound I | Validamycin A |
| B-212 | one individualized compound I | Binapacryl |
| B-213 | one individualized compound I | Dicloran |
| B-214 | one individualized compound I | Dinobuton |
| B-215 | one individualized compound I | Dinocap |
| B-216 | one individualized compound I | Nitrothal-isopropyl |
| B-217 | one individualized compound I | Tecnazen |
| B-218 | one individualized compound I | Fentin salts |
| B-219 | one individualized compound I | Dithianon |
| B-220 | one individualized compound I | 2,6-dimethyl-1H,5H-[1,4]dithiino [2,3-c:5,6-c']dipyrrole-1,3,5,7(2H,6H)-tetraone |
| B-221 | one individualized compound I | Isoprothiolane |
| B-222 | one individualized compound I | Edifenphos |
| B-223 | one individualized compound I | Fosetyl, Fosetyl-aluminium |
| B-224 | one individualized compound I | Iprobenfos |
| B-225 | one individualized compound I | Phosphorous acid (H₃PO₃) and deriva-tives |
| B-226 | one individualized compound I | Pyrazophos |
| B-227 | one individualized compound I | Tolclofos-methyl |
| B-228 | one individualized compound I | Chlorothalonil |
| B-229 | one individualized compound I | Dichlofluanid |
| B-230 | one individualized compound I | Dichlorophen |
| B-231 | one individualized compound I | Flusulfamide |
| B-232 | one individualized compound I | Hexachlorbenzene |
| B-233 | one individualized compound I | Pencycuron |
| B-234 | one individualized compound I | Pentachlorophenol and salts |
| B-235 | one individualized compound I | Phthalide |
| B-236 | one individualized compound I | Quintozene |
| B-237 | one individualized compound I | Thiophanate Methyl |
| B-238 | one individualized compound I | Tolylfluanid |
| B-239 | one individualized compound I | N-(4-chloro-2-nitro-phenyl)-N-ethyl-4-methyl-benzenesulfonamide |
| B-240 | one individualized compound I | Bordeaux composition |
| B-241 | one individualized compound I | Copper acetate |
| B-242 | one individualized compound I | Copper hydroxide |
| B-243 | one individualized compound I | Copper oxychloride |
| B-244 | one individualized compound I | basic Copper sulfate |
| B-245 | one individualized compound I | Sulfur |
| B-246 | one individualized compound I | Biphenyl |
| B-247 | one individualized compound I | Bronopol |
| B-248 | one individualized compound I | Cyflufenamid |
| B-249 | one individualized compound I | Cymoxanil |
| B-250 | one individualized compound I | Diphenylamin |
| B-251 | one individualized compound I | Metrafenone |
| B-252 | one individualized compound I | Pyriofenone |
| B-253 | one individualized compound I | Mildiomycin |
| B-254 | one individualized compound I | Oxin-copper |
| B-255 | one individualized compound I | Oxathiapiprolin |
| B-256 | one individualized compound I | Prohexadione calcium |
| B-257 | one individualized compound I | Spiroxamine |
| B-258 | one individualized compound I | Tebufloquin |
| B-259 | one individualized compound I | Tolylfluanid |
| B-260 | one individualized compound I | N-(Cyclopropylmethoxyimino-(6-difluoromethoxy-2,3-difluoro-phenyl)-methyl)-2-phenyl acetamide |
| B-261 | one individualized compound I | N'-(4-(4-chloro-3-trifluoromethyl-phenoxy)-2,5-dimethyl-phenyl)-N-ethyl-N-methyl formamidine |
| B-262 | one individualized compound I | N'-(4-(4-fluoro-3-trifluoromethyl-phenoxy)-2,5-dimethyl-phenyl)-N-ethyl-N-methyl formamidine |
| B-263 | one individualized compound I | N'-(2-methyl-5-trifluoromethyl-4-(3-tri-methylsilanyl-propoxy)-phenyl)-N-ethyl-N-methyl formamidine |
| B-264 | one individualized compound I | N'-(5-difluoromethyl-2-methyl-4-(3-tri-methylsilanyl-propoxy)-phenyl)-N-ethyl-N-methyl formamidine |
| B-265 | one individualized compound I | Methoxy-acetic acid 6-tert-butyl-8-fluoro-2,3-dimethyl-quinolin-4-yl ester |
| B-266 | one individualized compound I | *Bacillus subtilis* NRRL No. B-21661 |
| B-267 | one individualized compound I | *Bacillus pumilus* NRRL No. B-30087 |
| B-268 | one individualized compound I | *Ulocladium oudemansii* |
| B-269 | one individualized compound I | Carbaryl |
| B-270 | one individualized compound I | Carbofuran |
| B-271 | one individualized compound I | Carbosulfan |
| B-272 | one individualized compound I | Methomylthiodicarb |
| B-273 | one individualized compound I | Bifenthrin |
| B-274 | one individualized compound I | Cyfluthrin |
| B-275 | one individualized compound I | Cypermethrin |
| B-276 | one individualized compound I | alpha-Cypermethrin |
| B-277 | one individualized compound I | zeta-Cypermethrin |
| B-278 | one individualized compound I | Deltamethrin |
| B-279 | one individualized compound I | Esfenvalerate |
| B-280 | one individualized compound I | Lambda-cyhalothrin |
| B-281 | one individualized compound I | Permethrin |
| B-282 | one individualized compound I | Tefluthrin |
| B-283 | one individualized compound I | Diflubenzuron |
| B-284 | one individualized compound I | Flufenoxuron |
| B-285 | one individualized compound I | Lufenuron |
| B-286 | one individualized compound I | Teflubenzuron |
| B-287 | one individualized compound I | Spirotetramate |
| B-288 | one individualized compound I | Clothianidin |
| B-289 | one individualized compound I | Dinotefuran |
| B-290 | one individualized compound I | Imidacloprid |
| B-291 | one individualized compound I | Thiamethoxam |
| B-292 | one individualized compound I | Flupyradifurone |
| B-293 | one individualized compound I | Acetamiprid |
| B-294 | one individualized compound I | Thiacloprid |
| B-295 | one individualized compound I | Endosulfan |
| B-296 | one individualized compound I | Fipronil |
| B-297 | one individualized compound I | Abamectin |
| B-298 | one individualized compound I | Emamectin |
| B-299 | one individualized compound I | Spinosad |
| B-300 | one individualized compound I | Spinetoram |
| B-301 | one individualized compound I | Hydramethylnon |
| B-302 | one individualized compound I | Chlorfenapyr |
| B-303 | one individualized compound I | Fenbutatin oxide |
| B-304 | one individualized compound I | Indoxacarb |
| B-305 | one individualized compound I | Metaflumizone |
| B-306 | one individualized compound I | Flonicamid |
| B-307 | one individualized compound I | Lubendiamide |
| B-308 | one individualized compound I | Chlorantraniliprole |
| B-309 | one individualized compound I | Cyazypyr (HGW86) |
| B-310 | one individualized compound I | Cyflumetofen |
| B-311 | one individualized compound I | Acetochlor |
| B-312 | one individualized compound I | Dimethenamid |
| B-313 | one individualized compound I | metolachlor |
| B-314 | one individualized compound I | Metazachlor |
| B-315 | one individualized compound I | Glyphosate |
| B-316 | one individualized compound I | Glufosinate |
| B-317 | one individualized compound I | Sulfosate |
| B-318 | one individualized compound I | Clodinafop |
| B-319 | one individualized compound I | Fenoxaprop |
| B-320 | one individualized compound I | Fluazifop |
| B-321 | one individualized compound I | Haloxyfop |
| B-322 | one individualized compound I | Paraquat |
| B-323 | one individualized compound I | Phenmedipham |
| B-324 | one individualized compound I | Clethodim |
| B-325 | one individualized compound I | Cycloxydim |
| B-326 | one individualized compound I | Profoxydim |
| B-327 | one individualized compound I | Sethoxydim |
| B-328 | one individualized compound I | Tepraloxydim |
| B-329 | one individualized compound I | Pendimethalin |
| B-330 | one individualized compound I | Prodiamine |
| B-331 | one individualized compound I | Trifluralin |
| B-332 | one individualized compound I | Acifluorfen |
| B-333 | one individualized compound I | Bromoxynil |
| B-334 | one individualized compound I | Imazamethabenz |
| B-335 | one individualized compound I | Imazamox |
| B-336 | one individualized compound I | Imazapic |
| B-337 | one individualized compound I | Imazapyr |
| B-338 | one individualized compound I | Imazaquin |
| B-339 | one individualized compound I | Imazethapyr |
| B-340 | one individualized compound I | 2,4-Dichlorophenoxyacetic acid (2,4-D) |
| B-341 | one individualized compound I | Chloridazon |
| B-342 | one individualized compound I | Clopyralid |
| B-343 | one individualized compound I | Fluroxypyr |
| B-344 | one individualized compound I | Picloram |
| B-345 | one individualized compound I | Picolinafen |
| B-346 | one individualized compound I | Bensulfuron |
| B-347 | one individualized compound I | Chlorimuron-ethyl |
| B-348 | one individualized compound I | Cyclosulfamuron |
| B-349 | one individualized compound I | lodosulfuron |
| B-350 | one individualized compound I | Mesosulfuron |
| B-351 | one individualized compound I | Metsulfuron-methyl |
| B-352 | one individualized compound I | Nicosulfuron |
| B-353 | one individualized compound I | Rimsulfuron |
| B-354 | one individualized compound I | Triflusulfuron |
| B-355 | one individualized compound I | Atrazine |
| B-356 | one individualized compound I | Hexazinone |
| B-357 | one individualized compound I | Diuron |
| B-358 | one individualized compound I | Florasulam |
| B-359 | one individualized compound I | Pyroxasulfone |
| B-360 | one individualized compound I | Bentazone |
| B-361 | one individualized compound I | Cinidon-ethyl |
| B-362 | one individualized compound I | Cinmethylin |
| B-363 | one individualized compound I | Dicamba |
| B-364 | one individualized compound I | Diflufenzopyr |
| B-365 | one individualized compound I | Quinclorac |
| B-366 | one individualized compound I | Quinmerac |
| B-367 | one individualized compound I | Mesotrione |
| B-368 | one individualized compound I | Saflufenacil |
| B-369 | one individualized compound I | Topramezone |

The active substances referred to as component 2, their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657).

The compositions of active substances can be prepared as compositions comprising besides the active ingridients at least one inert ingredient by usual means, e. g. by the means given for the compositions of compounds I.

Concerning usual ingredients of such compositions reference is made to the explanations given for the compositions containing compounds I.

The compositions of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

### Synthesis examples

With due modification of the starting compounds, the procedures shown in the synthesis examples below were used to obtain further compounds I. The resulting compounds, together with physical data, are listed in Table I below.

## Claims

1. Compounds of formula I 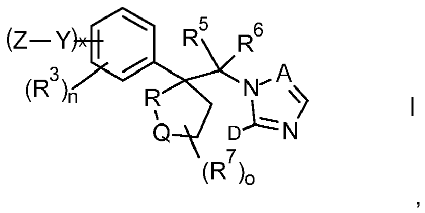 wherein:
R is O or CH₂;
Q is O or CH₂;
wherein either R or Q is O;
A is CH or N;
D is H, halogen or SR^{D}, wherein
R^{D} is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl or CN;
R³ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R³ is unsubstituted or further substituted by one, two, three or four R^{3a}; wherein
R^{3a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
n is 0, 1, 2, 3 or 4;
Y is a direct bond or a divalent group selected from the group consisting of -O-, -S-, SO-, - SO₂-; -NH-, -N(C₁-C₄-alkyl)-, CR¹⁵R⁸-, -CR⁹R¹⁰-CR¹¹R¹²-, -CR¹³=CR¹⁴ and -C≡C-;
wherein
R⁸,R⁹,R¹⁰,R¹¹,R¹²,R¹³,R¹⁴,R¹⁵ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl, wherein the heteroaryl contains 1, 2, 3 or 4 heteroatoms selected from the group consisting of O, N and S, or phenyl, wherein the heteroaryl or phenyl is unsubstituted (m=0) or substituted by (R⁴)ₘ, wherein
m is 0, 1, 2, 3, 4 or 5;
R⁴ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a}; wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-haloalkoxy;
p is 0, 1 or 2;
x is 0, 1, 2, 3 or 4;
R⁵ is H, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, phenyl, heteroaryl, phenyl-C₁-C₄-alkyl and heteroaryl-C₁-C₄-alkyl, wherein the heteroaryl radical in the last-mentioned groups is 5- or 6-membered and wherein the aliphatic, alicyclic and aromatic moieties of R⁵ are unsubstituted or substituted by one, two, three or four or up to the maximum possible number of R^{5a}; wherein
R^{5a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R⁶ is H or is selected from the substituents defined for R⁵, wherein the aliphatic, alicyclic and aromatic moieties of R⁶ are unsubstituted or substituted by one, two, three or four or up to the maximum possible number of R^{6a}, wherein R^{6a} is defined as R^{5a};
or
R⁵ and R⁶ together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S);
or R⁵ and R⁶ together with the carbon atom to which they are bound (denominated C*) form a group (C*=CR⁵⁵R⁶⁶), wherein
R⁵⁵ R⁶⁶ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R⁷ is independently selected from H, C₁-C₆-alkyl, C₂-C₆-alkenyl, C₂-C₆-alkynyl, C₃-C₈-cycloalkyl, wherein each of R⁷ is unsubstituted or further substituted by one, two, three or four R^{7a}; wherein
R^{7a} is independently selected from halogen, OH and C₁-C₆-alkoxy;
or
two substituents R⁷: R⁷¹ and R⁷² together with the carbon atom(s) to which they are bound form a saturated three-, four-, five-, six- or seven-membered carbocycle or heterocycle,
wherein the heterocycle contains one, two, three or four O atoms; o is 0, 1, 2, 3 or 4;
wherein
and the N-oxides and the agricucturally acceptable salts thereof with the proviso that
if R is O, x is 1, R⁵ and R⁶ are H R³ is not halogen.

2. The compounds according to claim 1, wherein
A is N.

3. The compounds according to any of the claims 1 to 2, wherein
DisH.

4. The compounds according to any of the claims 1 to 3, wherein
RisO.

5. The compounds according to any of the claims 1 to 3, wherein
Q is O.

6. The compounds according to any of the claims 1 to 5, wherein
R³ is halogen, CN, NO₂, C₁-C₆-alkoxy, C₁-C₆-haloalkyl or S(O)₂(C₁-C₄-alkyl).

7. The compounds according to any of the claims 1 to 6, wherein
R⁵ and R⁶ are independently H, halogen, C₁-C₄-alkyl, C₁-C₄-alkoxy; or
R⁵ and R⁶ together with the carbon atom to which they are bound form a saturated three-, four-, five-, six- or seven-membered carbocycle; or
R⁵ and R⁶ together with the carbon atom to which they are bound (denominated C*) form a group C*=CH₂.

8. A process for preparing compounds of formula I as defined in any of claims 1 to 7, which comprises reacting a compound of formula comprises reacting a compound of formula which comprises following steps:
cross coupling a compound of formula II 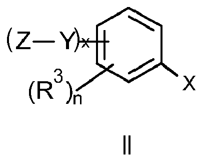
wherein Z, Y, R³, n are as defined in any of claims 1 to 7 and X is a halogen to compound of formula III 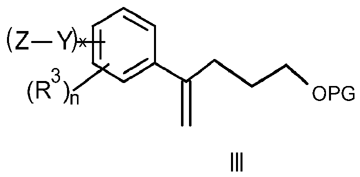
wherein Z, Y, R³, n are as defined in any of claims 1 to 7 and PG is preferably THP, MOM, TMS, TES, TBDMS, TIPS, TBDPS;
epoxidation of the compound of formula III using an reductions agent to obtain an intermediate IV 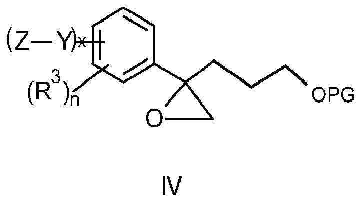
wherein Z, Y, R³, n are as defined in any of claims 1 to 7 and PG is preferably THP, MOM, TMS, TES, TBDMS, TIPS, TBDPS;
cyclisation of the intermediate IV to provide a compound of the formula V 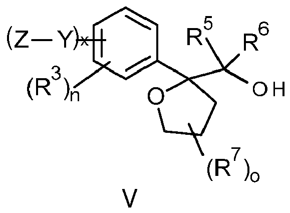
wherein Z, Y, R³, R⁵, R⁶, R⁷, o, n, x are as defined in any of claims 1 to 7;
activation by sulfonation or halogenation leads to obtain compounds of formula I.

9. A process for preparing compounds of formula I as defined in any of claims 1 to7, which comprises following steps:
transforming a compound of formula VI 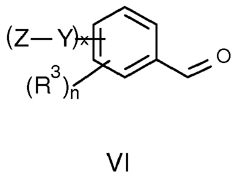
wherein Z, Y, R³, n are as defined in any of claims 1 to 7 to compound of formula VII 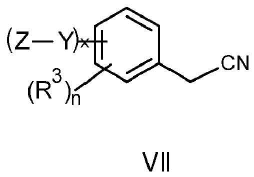 wherein Z, Y, R³, n are as defined in any of claims 1 to 7;
cyclisation to obtain an intermediate VIII 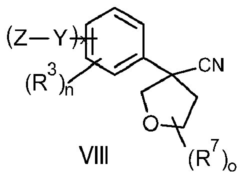
wherein Z, Y, R³, R⁷, n are as defined in any of claims 1 to 7;
reduction of the intermediate VIII to provide a compound of the formula IX 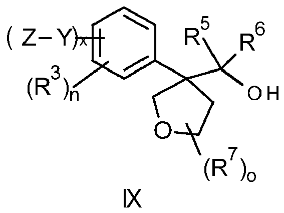
wherein Z, Y, R³, R⁵, R⁶, R⁷, o, n, x are as defined in any of claims 1 to 7;
activation by sulfonation or halogenation leads to obtain compounds of formula I.

10. Compounds of formulae III, IV, V, VIII, IX
wherein Z, Y, R³, R⁵, R⁶, R⁷, o, n, x, if applicable, are as defined in any of claims 1 to 7.

11. Agrochemical compositions, wherein said composition comprise an auxiliary and at least one compound of formula I, as defined in any of the claims 1 to 7, an N-oxide or an agriculturally acceptable salt thereof.

12. The compositions according to claim 11, comprising additionally a further active substance.

13. Use of a compound of the formula I, as defined in any of the claims 1 to 7, and/or of an agriculturally acceptable salt thereof or of the compositions, as defined in any of the claims 11 to 12, for combating phytopathogenic fungi.

14. A method for combating harmful fungi, comprising treating the fungi or the materials, plants, the soil or seeds to be protected against fungal attack with an effective amount of at least one compound of formula I, as defined in any of the claims 1 to 7, or with a composition, as defined in any of the claims 11 to 12.

15. Seed, coated with at least one compound of the formula I, as defined in any of the claims 1 to 7, and/or an agriculturally acceptable salt thereof or with a composition, as defined in any of the claims 11 to 12, in an amount of from 0.1 to 10 kg per 100 kg of seed.
